# EUROPEAN PATENT SPECIFICATION

(11) **EP 3 956 334 B1**
(45) Date of publication and mention of the grant of the patent: **24.09.2025**
(21) Application number: 20718675.0
(22) Date of filing: 17.04.2020
(51) Int. Cl.: B29D 11/02, C08F 220/18, C08F 220/30, C08F 220/38, C07F 7/18, C07D 309/10, C07C 69/54, C07C 69/653, C07C 255/54, C07C 309/67, C07C 317/18, C07C 323/12, C07C 323/65, C07C 255/47, C07C 309/65, C07C 323/18, C07C 327/28, B29D 11/00, G02B 1/04

(54) **COMPOUNDS FOR OPTICALLY ACTIVE DEVICES**
VERBINDUNGEN FÜR OPTISCH AKTIVE VORRICHTUNGEN
COMPOSÉS POUR DISPOSITIFS OPTIQUEMENT ACTIFS

(30) Priority: 18.04.2019 EP 19170336
(43) Date of publication of application: 23.02.2022
(73) Proprietor: AMO Ireland, Dublin (IE)
(72) Inventor: GERSTENECKER, Stefan, 72469 MESSSTETTEN (DE)
(74) Representative: Carpmaels & Ransford LLP
(86) International application number: PCT/EP2020/060798
(87) International publication number: WO 2020/212543

(56) References cited:
- EP-A1- 3 133 065
- JUNYOU ZHANG ET AL: "Enantioselective Phosphine-Catalyzed Allylic Alkylations of mix -Indene with MBH Carbonates", ORGANIC LETTERS, vol. 19, no. 22, 27 October 2017 (2017-10-27), US, pages 6080 - 6083, XP055704918, ISSN: 1523-7060, DOI: 10.1021/acs.orglett.7b02895

## Description

### Field of the Invention

The present invention relates to novel ophthalmic devices comprising polymerized compounds comprising a photoactive unit, said polymerized compounds, and special monomer compounds being particularly suitable for compositions and ophthalmic devices.

### Background of the Invention

Cataract is a general term for an affection of the eye that leads to a loss of vision and in the extreme to blindness by clouding of the normally clear lens of the eye. It is the major cause of blindness in the world, affecting more than 100 million people. Due to the fact that its major cause is age and the population's average age is increasing, it is expected that the number of cataracts will continue to increase substantially in the future.

Effective treatment of cataract is only possible by surgical intervention, whereby the natural lens of the eye is removed through an incision in the cornea and replaced with an artificial lens, often also referred to as "intraocular lens". In preparation of surgery current state-of-the-art surgical methods employ eye mapping so as to approximate the refractive power best suited to the respective patient.

Even though cataract surgery is one of the most widely used and safest surgical procedures it is not without specific post-surgery problems. It frequently happens that the refractive power of the implanted intraocular lens (IOL) is insufficient for restoring good vision. Such problems may, for example, be caused by changes in eye geometry as consequence of the surgery as well as irregular wound healing and positioning errors that result in the artificial lens not having the optimal optical properties. As a result the patient will still require corrective vision aids, e.g. glasses, to be able to see correctly. In some cases the resulting refractive power of the implanted artificial lens is so far removed from the required refractive power that further surgery will be required. Particularly for aged persons this is not desirable because the body's capability for healing is reduced with increasing age. Furthermore, there is the risk of attracting endophthalmitis, an inflammation of the eye, which can even lead to a complete loss of vision or worse, loss of the eye.

There is therefore a need in the health sector for optically active ophthalmic devices, and particularly for artificial intraocular lenses, that would allow for non-invasive adjustment of refractive power after implantation of the lens, thereby preferably further reducing the need for post-surgery vision aids.

Some developments in this sense have already been made, as for example evidenced by WO 2007/033831, WO 2009/074520, US 2010/0324165, WO 2017/032442, WO 2017/032443, WO 2017/032444, WO 2018/149850, WO 2018/149852, WO 2018/149853, WO 2018/149855, WO 2018/149856 or WO 2018/149857.

M. Schraub et al, European Polymer Journal 51 (2014) 21-27 describes the photochemistry of 3-phenyl-coumarin containing polymethacrylates. EP 3 133 065 A1 discloses compounds used in ophthalmic devices.

The photochemistry of 3-phenyl-1,2-dihydronaphthalenes is subject of the articles of F. Keijzer et al, Journal of Photochemistry and Photobiology, A: Chemistry, 1990, 50, 401-406, J.J.M Lamberts et al, Recueil, Journal of the Royal Netherlands Chemical Society, 1984, 103/4,131-135 and H. Behm et al, Journal of Crystallographic and Spectroscopic Research 1988, 18(4), 471-475.

JP3417969 describes 5,6-difluoro-1H-indene derivatives and liquid crystal compositions comprising the same.

WO200059861 describes selective retinoic acid analogs useful in various dermatological diseases, in the treatment of malignant tumors and as agents for the minimization or prevention of post-surgical adhesion formation and pharmaceutical compositions comprising said compounds.

JP2003238459 describes indene compounds useful as a component of a liquid crystal composition and liquid crystal electrooptic elements comprising these.

EP 1342770 describes polymerizable liquid crystal compounds bearing two polymerizable end groups, their polymers, liquid crystal compositions, an optical retardation film, an optically anisotropic element, a nonlinear optical device and an electro-optical device comprising the liquid crystal polymers.

US20070087289 describes dipole-like polymer brushes which may consist of poly[styrene-co-2-(4-vinylphenyl)indene]/P2VP polymer systems to be used within rewritable and erasable printing forms.

WO2008043567 describes phenyl-substituted 3H-indenes for use in the treatment or prophylaxis of a condition associated with a disease or disorder associated with estrogen receptor activity and pharmaceutical compositions comprising said compounds.

WO2009047343 describes benzocycloheptene derivatives as estrogens having selective activity and pharmaceutical compositions comprising said compounds.

M. Silverman et al, Journal of Organic Chemistry, 1946, 11,34-49 describe the synthesis of 1-ethyl-3,4-dihydro-6-(2-propen-1-yloxy)-2-[4-(2-propen-1-yloxy)phenyl]naphthalene.

T. Kametani et al, J. Chem. Soc. (C), 1971, (6), 1032-43 describe the synthesis of 3-(2-ethenyl-4,5-dimethoxyphenyl)-4,5,6-trimethoxy-1H-indene.

S. Kano et al, Chem. Pharm. Bull. 1975, 23(5), 1171-1172 describe the synthesis of 2-(2-ethenyl-4,5-dimethoxyphenyl)-5,6-dimethoxy-1H-indene.

A. Padwa et al, J. Am. Chem. Soc. 1983, 105, 4446-4456 describe the synthesis of 1,2-diphenyl-3-methyl-4-vinylindene, 1,2-diphenyl-1-vinyl-3-methylindene and 2-methyl-1-phenyl-3-(o-vinyl-phenyl)indene.

S.C. Diehl et al, Israel Journal of Chemistry, 2003, Volume Date 2002, 42(4), 393-401 describe polystyrene-supported 2-arylindenyl zirconocene catalysts for propylene polymerization. 2-[4-(2-propen-1-yl)phenyl]-1H-indene is described therein.

R. Schinner, T. Wolff, Colloid Polym. Sci. 2001, 279, 1225-1230 describe cross-linking in solution of polystyrene containing phenylindene units. Styrene and 2-(4'-styryl)indene are used for copolymerization.

J.N. D'Amour et al, Proceedings of SPIE - The international Society for Optical Engineering, 2003, 5039 (Pt. 2, Advances in resist Technology and Processing XX) describe photocrosslinkable polystyrene films show a linear decrease in glass transition temperature with decreasing film thickness. 2-(4'-Styryl)-indene is used to synthesize said photocrosslinkable polystyrene.

Frank Hoffmann et al, Journal of Colloid and Interface Science, 2008, 322, 434-447 describe polymer brushes grafted on silica wafers. Said polymer brushes are made up from poly-4-vinylpyridine, polymethacrylic acid and polystyrene copolymerized with 2-(4'-styryl)-indene. They are reported to be switchable polymer brushes that can be fixed in one state via photo-cross-linking via photodimerization of two phenylindene moieties. 2-Trimethylsilyloxy-2-(4-vinylphenyl)-indane is used within the monomer solutions.

Frank Hoffmann et al, Polymeric Materials: Science & Engineering 2004, 90, 374 describe photochemical structuring of binary polymer brush layers via photodimerization by using a photosensitive styrene/2-(4-styryl)-indene copolymer. 2-Trimethylsilyloxy-2-(4-vinylphenyl)-indane is used within the monomer solutions.

Xinfei Ji et al, ACS Omega, 2018, 3, 10099-10106 describe the facile synthesis of 3-arylindenes by HMPA-promoted direct arylation of indenes with aryl fluorides.

Moon Gon Jeong et al, Macromolecules, 2017, 50(1), 223-234 report the cross-linking of hydrophobic compartments of complex self-assembled structures of amphiphilic block copolymers by the [2π+2π]-cycloaddition of an indene moiety such as 2-(4-vinylphenyl)indene present in a hydrophobic block based on polystryrene. They further demonstrate that the micellar structures and complex inverse bicontinuous bilayers could be covalently cross-linked in aqueous solutions upon irradiation with long-wavelength UV light (λ = 365 nm).

However, there is still a need to provide alternative or improved ophthalmic devices to be implanted by state of the art cataract surgical methods and there is still a need to provide special compounds for the manufacture of intraocular lenses to be implanted by state of the art cataract surgical methods, particularly by state of the art micro-incision cataract surgical methods.

Consequently, it is an objective of the present application to provide for alternative or improved ophthalmic devices and suitable compounds for the manufacture of such ophthalmic devices.

It is also an objective of the present application to provide for compounds, the optical properties of which may be changed, preferably by non-invasive techniques.

It is a further objective of the present application to provide alternative compounds or compounds having advantages over currently known compounds, preferably in combination with being suitable for ophthalmic devices.

An advantage for the monomers of formula (I) to be used for the preparation of the ophthalmic device according to the invention is the better handling through low melting points by using them in compositions and or polymers/copolymers. A further advantage is that the liquid to low melting monomers of formula (I) to be used for the preparation of the ophthalmic device according to the invention enable a higher flexibility in the choice of initiators for a thermally activated polymerization.

Advantages for polymers or copolymers comprising polymerized monomers of formula (I) according to the invention are better flexibilities and low glass transition temperatures. The polymers or copolymers according to the invention preferably show a shift of 20 to 50 nm of the absorption maximum compared to coumarin containing polymers or copolymers and 5 to 25 nm of the absorption maximum compared to benzofuran containing polymers or copolymers. This results in a wider non-absorption range of the polymer and enables a higher flexibility in adjusting the absorption edge of an ophthalmic material by e.g. choice of UV absorber.

### Summary of the Invention

The present inventors have now found that the above objects may be attained either individually or in any combination by ophthalmic devices and the compounds of the present application.

The invention relates to an ophthalmic device or a precursor article for an ophthalmic device comprising at least one polymerized compound of formula (I), 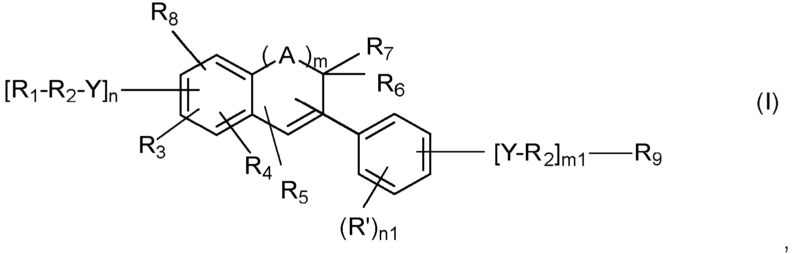 wherein
- A: is the same or different at each instance and is CH₂, CHR₀ or C(R₀)₂;
- m: is 0, 1, 2 or 3;
- Y: is independently of each other O, S, SO₂, or a bond;
- n: is 0 or 1 ;
- m1: is 0 or 1 ;
- n+m1: is 1;
- n1: is 0, 1, 2, 3 or 4;
- R₀: is at each occurrence independently selected from the group consisting of F, a linear or branched alkyl group having 1 to 4 C atoms and a linear or branched partially or fully fluorinated alkyl group having 1 to 4 C atoms;
- R': is at each occurrence independently selected from the group consisting of F, a linear or branched, non-halogenated, partially or completely halogenated alkyl group having 1 to 20 C atoms, a non-halogenated, partially or completely halogenated cycloalkyl group having 3 to 6 C atoms, a linear or branched, non-halogenated, partially or completely halogenated alkoxy group having 1 to 20 C atoms and a linear or branched, non-halogenated, partially or completely halogenated thioalkyl group having 1 to 20 C atoms;
- R₁: is a trialkoxysilyl group or a dialkoxyalkylsilyl group where the alkyl and/or alkoxy groups are each independently linear or branched having 1 to 6 C atoms, or a silyl group of formula (1), (2) or (3) or a polymerizable group of formula (4), 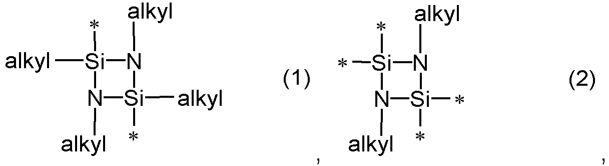 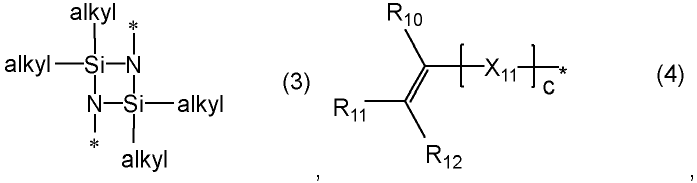
where alkyl means at each occurrence independently of each other a linear or branched alkyl group having 1 to 6 C atoms and the asterisk "*" denotes at each occurrence independently of each other a linkage to the linker [-R₂-Y]ₙ or [Y-R₂-]ₘ₁;
and wherein
X₁₁ is selected from the group consisting of O, S, O-SO₂, SO₂-O, C(=O), OC(=O), C(=O)O, S(C=O) and (C=O)S ,
R₁₀, R₁₁, R₁₂ are at each occurrence independently of each other selected from the group consisting of H, F, a linear or branched, non-fluorinated, partially or completely fluorinated alkyl group having 1 to 20 C atoms and aryl with 6 to 14 C atoms and
c is 0 or 1 ;
- -R₂-: is -(C(R)₂)ₒ-, or -(C(R)₂)ₚ-X₈-(C(R)₂)_{q}-(X₉)ₛ-(C(R)₂)ᵣ(X₁₀)ₜ-(C(R)₂)ᵤ-;
R is at each occurrence independently selected from the group consisting of H, F, a linear or branched alkyl group having 1 to 4 C atoms or a linear or branched partially or fully fluorinated alkyl group having 1 to 4 C atoms;
o is selected from the group consisting of 0 to 20,
X₈, X₉, X₁₀ are at each occurrence independently O, S, SO₂, or NR₀,
s, t is 0 or 1,
p, q are at each occurrence independently selected from the group consisting of 1 to 10,
r, u are at each occurrence independently selected from the group consisting of 0 to 10, wherein the overall number of atoms for -(C(R)₂)ₚ-X₈-(C(R)₂)_{q}-(X₉)ₛ-(C(R)₂)ᵣ(X₁₀)ₜ-(C(R)₂)ᵤ- is up to 20 atoms,
- R₃, R₄, R₅, R₆, R₇, R₈: are at each occurrence independently selected from the group consisting of H, F, Cl, Br, CN, SO₂CF₃, a linear or branched, non-halogenated, partially or completely halogenated alkyl group having 1 to 20 C atoms, a non-halogenated, partially or completely halogenated cycloalkyl group having 3 to 6 C atoms, a linear or branched, non-halogenated, partially or completely halogenated alkoxy group having 1 to 20 C atoms and a linear or branched, non-halogenated and partially or completely halogenated thioalkyl group having 1 to 20 C atoms;
- R₉: is H or R' in case m1 is 0 and
- R₉: is R₁ in case m1 is 1.

The invention relates further to a process of forming an ophthalmic device or a precursor article for an ophthalmic device as described before or preferably described below, said process comprising the steps of - providing a composition comprising at least one compound of formulae (I) as described before or preferably described below and/or an oligomer or polymer derived from a compound of formula (I) as described below or preferably described below but having at least one reactive group left for polymerization and optionally further monomers different from compounds of formula (I) and/or crosslinking agents and/or UV absorbers and/or radical initiators;
- subsequently forming the ophthalmic device or precursor article of said composition.

The invention relates further to a process of changing the optical properties of an ophthalmic device or a precursor article for an ophthalmic device as described before or preferably described below said process comprising the steps of
- providing an ophthalmic device or a precursor article with the process as described before or preferably described below, and
- subsequently exposing said ophthalmic device or precursor article to irradiation having a wavelength of at least 200 nm and at most 1500 nm.

The invention relates further to oligomers, polymers or copolymer comprising at least one polymerized compound of formula (I) as described before where oligomers, polymers and copolymers of 2-(4-vinylphenyl)indene and 2-[4-(2-propen-1-yl)phenyl-1H-indene are excluded.

The invention relates further to compositions for polymerization comprising at least one compound of formulae (I) as described before or preferably described below and/or an oligomer or polymer derived from compounds of formula (I) as described before or preferably described below but having at least one reactive group left for polymerization excluding 2-(vinylphenyl)indene, 2-[4-(2-propen-1-yl)phenyl-1H-indene and any oligomer, polymer or copolymer derived from 2-(vinylphenyl)indene and 2-[4-(2-propen-1-yl)phenyl-1H-indene and/or a crosslinking agent and/or a UV absorber and/or a radical initiator and optionally further monomers different from compounds of formula (I).

The invention relates further to compounds of formula (I), 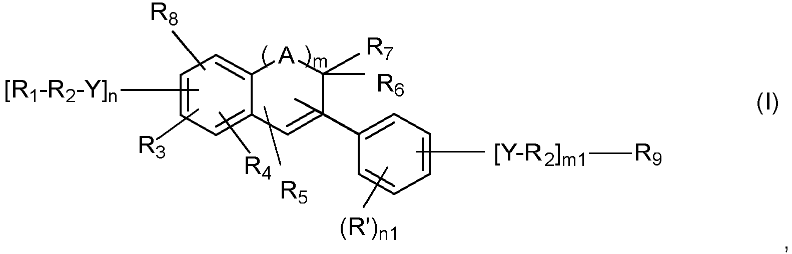 wherein
- A: is the same or different at each instance and is CH₂, CHR₀ or C(R₀)₂;
- m: is 0, 1, 2 or 3;
- Y: is independently of each other O, S, SO₂, or a bond;
- n: is 0 or 1 ;
- m1: is 0 or 1 ;
- n+m1: is 1;
- n1: is 0, 1, 2, 3 or 4;
- R₀: is at each occurrence independently selected from the group consisting of F, a linear or branched alkyl group having 1 to 4 C atoms and a linear or branched partially or fully fluorinated alkyl group having 1 to 4 C atoms;
- R': is at each occurrence independently selected from the group consisting of F, a linear or branched, non-halogenated, partially or completely halogenated alkyl group having 1 to 20 C atoms, a non-halogenated, partially or completely halogenated cycloalkyl group having 3 to 6 C atoms, a linear or branched, non-halogenated, partially or completely halogenated alkoxy group having 1 to 20 C atoms and a linear or branched, non-halogenated, partially or completely halogenated thioalkyl group having 1 to 20 C atoms;
- R₁: is a trialkoxysilyl group or a dialkoxyalkylsilyl group where the alkyl and/or alkoxy groups are each independently linear or branched having 1 to 6 C atoms, or a silyl group of formula (1), (2) or (3) or a polymerizable group of formula (4), 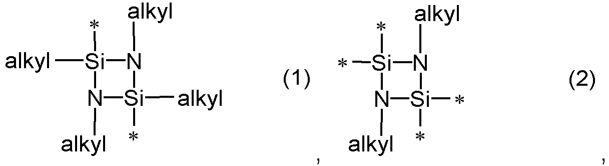 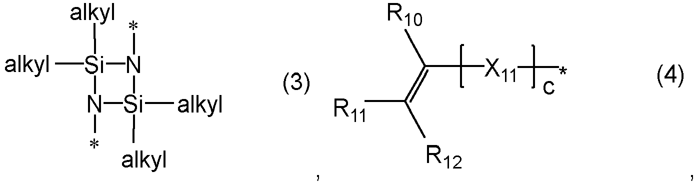
where alkyl means at each occurrence independently of each other a linear or branched alkyl group having 1 to 6 C atoms and the asterisk "*" denotes at each occurrence independently of each other a linkage to the linker [-R₂-Y]ₙ or [Y-R₂-]ₘ₁;
and wherein
X₁₁ is selected from the group consisting of O, S, O-SO₂, SO₂-O, C(=O), OC(=O), C(=O)O, S(C=O) and (C=O)S ,
R₁₀, R₁₁, R₁₂ are at each occurrence independently of each other selected from the group consisting of H, F, a linear or branched, non-fluorinated, partially or completely fluorinated alkyl group having 1 to 20 C atoms and aryl with 6 to 14 C atoms and
c is 0 or 1 ;
- -R₂-: is -(C(R)₂)ₒ-, or -(C(R)₂)ₚ-X₈-(C(R)₂)_{q}-(X₉)ₛ-(C(R)₂)ᵣ-(X₁₀)ₜ-(C(R)₂)ᵤ-;
R is at each occurrence independently selected from the group consisting of H, F, a linear or branched alkyl group having 1 to 4 C atoms or a linear or branched partially or fully fluorinated alkyl group having 1 to 4 C atoms;
o is selected from the group consisting of 0 to 20,
X₈, X₉, X₁₀ are at each occurrence independently O, S, SO₂, or NR₀,
s, t is 0 or 1,
p, q are at each occurrence independently selected from the group consisting of 1 to 10,
r, u are at each occurrence independently selected from the group consisting of 0 to 10, wherein the overall number of atoms for -(C(R)₂)ₚ-X₈-(C(R)₂)_{q}-(X₉)ₛ-(C(R)₂)ᵣ(X₁₀)ₜ-(C(R)₂)ᵤ- is up to 20 atoms,
- R₃, R₄, R₅, R₆, R₇, R₈: are at each occurrence independently selected from the group consisting of H, F, Cl, Br, CN, SO₂CF₃, a linear or branched, non-halogenated, partially or completely halogenated alkyl group having 1 to 20 C atoms, a non-halogenated, partially or completely halogenated cycloalkyl group having 3 to 6 C atoms, a linear or branched, non-halogenated, partially or completely halogenated alkoxy group having 1 to 20 C atoms and a linear or branched, non-halogenated and partially or completely halogenated thioalkyl group having 1 to 20 C atoms;
- R₉: is H or R' in case m1 is 0 and
- R₉: is R₁ in case m1 is 1,

provided that in case of m is 0, m1 is 1 and Y is a bond, o is selected from the group consisting of 3 to 20; and
provided that in case of m is 0 and m1 is 1, two substituents from the group of R₃, R₄ and R₈ are not simultaneously F; and
provided that in case of m is 0, m1 is 1 and Y is a bond or O, R' is on each occurrence not F; and
provided that in case of m is 0, R₆ and R₇ are on each occurrence not a non-halogenated, partially or completely halogenated cycloalkyl group having 3 to 6 C atoms; and
provided that in case of m is 1, n is 1 and Y is a bond, o is selected from 1 to 20; and
provided that in case of m is 2, m1 is 1 and Y is a bond, o is selected from the group consisting of 3 to 20; and
provided that in case of m is 2, m1 is 1 and Y is O, o is selected from the group consisting of 1 to 20; and
provided that in case of m is 2, n is 1 and Y is a bond, o is selected from the group consisting of 3 to 20; and
provided that in case of m is 2, n is 1 and Y is O, o is selected from the group consisting of 1 to 20.

### Detailed Description of the Invention

Compounds of formula (I) as described before or preferably described below can be preferably used as monomers for the preparation of a precursor article such as a blank which may be transformed to an ophthalmic device such as an eye-implant or specifically an intraocular lens or can be preferably used for the preparation of the ophthalmic device as such as described before or preferably described below.

The compounds of formula (I) and all preferred embodiments of compounds of formula (I) including any monomeric units according to the present invention include all stereoisomers or racemic mixtures.

The compounds of formula (I) provide several advantages over prior art materials for the preparation of ophthalmic devices or precursor articles for an ophthalmic device
- the number of C atoms of the linker -R₂- is reduced compared to the 1-benzopyran-2-one, 1-benzopyran-2-thione, thiochromene-2-one, thiochromene-2-thione, quinolin-2-one or quinolin-2-thione ring systems of the state of the art but still their melting points or glass transition temperatures allow for foldable or bendable ophthalmic devices or precursor articles for an ophthalmic device comprising polymerized compounds of formula (I),
- due to the low melting points and/or liquid monomers of formula (I) at room temperature, the handling of the materials is much easier and their use enable a higher flexibility in the choice of initiators for a thermally activated polymerization within the process of manufacturing the ophthalmic device or precursor article for an ophthalmic device according to the invention.

Polymers that are foldable at room temperature generally exhibit glass transition temperatures (T_{g}) lower than room temperature (ca. 21 °C). They are easily deformable at this temperature without causing physical damage to the polymer, for example by inducing creep, stress or fissures. For polymers in intraocular lenses, T_{g}s of less than or equal to 15 °C are preferred.

Polymers used in ophthalmic device manufacturing, preferably in intraocular lens manufacturing, have preferably relatively high refractive indices, which enable the fabrication of thinner ophthalmic devices such as intraocular lenses. Preferably, the polymer used in an ophthalmic device, preferably an intraocular lens, will have a refractive index greater than about 1.5 and presently most preferably greater than about 1.55.

In case an asterisk ("*") is used within the description of the present invention, it denotes a linkage to an adjacent unit or group or, in case of a polymer, to an adjacent repeating unit or any other group whenever it is not specifically defined.

A linear or branched alkyl group having 1 to 10 C atoms denotes an alkyl group having 1, 2, 3, 4, 5, 6, 7, 8, 9 or 10 C atoms, for example methyl, ethyl, *iso*-propyl, *n*-propyl, *iso*-butyl, *n*-butyl, *tert*-butyl, *n*-pentyl, 1-, 2- or 3-methylbutyl, 1,1-, 1,2- or 2,2-dimethylpropyl, 1-ethylpropyl, *n*-hexyl, *n-*heptyl, *n*-octyl, ethylhexyl, *n*-nonyl or *n*-decyl. A linear or branched alkyl group having 1 to 20 C atoms include all examples for a linear or branched alkyl group having 1 to 10 C atoms including any alkyl group having 11, 12, 13, 14, 15, 16, 17, 18, 19 and 20 C atoms such as *n*-undecyl, *n*-dodecyl, *n-*tridecyl, *n*-tetradecyl, *n*-pentadecyl, *n*-hexadecyl, *n*-heptadecyl, *n*-octadecyl, *n*-nonadecyl and *n*-eicosyl.

The term partially halogenated alkyl group denotes that at least one H atom of the alkyl group is replaced by F, Cl, Br or I. Preferably, the alkyl group is partially fluorinated meaning that at least one H atom of the alkyl group is replaced by F. A preferred partially halogenated alkyl group is CH₂CF₃.

The term completely halogenated alkyl group denotes that all H atoms of the alkyl group are replaced by F, Cl, Br and/or I. Preferably, the alkyl group is completely fluorinated meaning that all H atoms of the alkyl group are replaced by F. A preferred completely fluorinated alkyl group is trifluoromethyl or pentafluoroethyl.

The term halogenated or preferably fluorinated corresponds additionally to other groups such as a halogenated cycloalkyl group, a halogenated alkoxy group or a halogenated thioalkyl group.

A cycloalkyl group having 3 to 6 C atoms includes cyclopropyl, cyclobutyl, cyclopentyl and cyclohexyl which may be partially or completely halogenated or fluorinated as explained before. Preferably, the cycloalkyl group is cyclopropyl.

A linear or branched alkoxy group having 1 to 20 C atoms denotes an O-alkyl group having 1, 2, 3, 4, 5, 6, 7, 8, 9, 10, 11, 12, 13, 14, 15, 16, 17, 18, 19 or 20 C atoms, for example methoxy, ethoxy, iso-propoxy, n-propoxy, *iso*-butoxy, *n*-butoxy, *tert*-butoxy, *n*-pentyloxy, 1-, 2- or 3-methylbutyloxy, 1,1-, 1,2- or 2,2-dimethylpropoxy, 1-ethylpropoxy, *n-*hexyloxy, *n*-heptyloxy, *n*-octyloxy, ethylhexyloxy, *n*-nonyloxy, *n*-decyloxy, *n-*undecyloxy, *n*-dodecyloxy, *n*-tridecyloxy, *n*-tetradecyloxy, *n*-pentadecyloxy, *n*-hexadecyloxy, *n*-heptadecyloxy, *n*-octadecyloxy, *n*-nonadecyloxy and *n-*eicosyloxy which may be partially or completely halogenated or preferably may be partially or completely fluorinated. A preferred completely fluorinated alkoxy group is trifluoromethoxy.

A linear or branched thioalkyl group having 1 to 20 C atoms denotes a S-alkyl group having 1, 2, 3, 4, 5, 6, 7, 8, 9, 10, 11, 12, 13, 14, 15, 16, 17, 18, 19 or 20 C atoms, for example thiomethyl, 1-thioethyl, 1-thio-iso-propyl, 1-thio-*n*-propoyl, 1-thio-*iso*-butyl, 1-thio-*n*-butyl, 1-thio-*tert*-butyl, 1-thio-*n-*pentyl, 1-thio-1-, -2- or -3-methylbutyl, 1-thio-1,1-, -1,2- or -2,2-dimethylpropyl, 1-thio-1-ethylpropyl, 1-thio-*n*-hexyl, 1-thio-*n*-heptyl, 1-thio-*n-*octyl, 1-thio-ethylhexyl, 1-thio-*n*-nonyl, 1-thio-*n*-decyl, 1-thio-*n*-undecyl, 1-thio-*n*-dodecyl, 1-thio-*n*-tridecyl, 1-thio-*n*-tetradecyl, 1-thio-*n*-pentadecyl, 1-thio-*n*-hexadecyl, 1-thio-*n*-heptadecyl, 1-thio-*n*-octadecyl, 1-thio-*n-*nonadecyl and 1-thio-*n*-eicosyl which may be partially or completely halogenated or preferably may be partially or completely fluorinated. A preferred completely fluorinated thioether group is trifluoromethyl thioether.

Preferred alkyl and alkoxy radicals have 1, 2, 3, 4, 5, 6, 7, 8, 9 or 10 C atoms.

An aryl group in the context of this invention contains 6 to 40 ring atoms and a heteroaryl group in the context of this invention contains 5 to 40 ring atoms comprising at least one heteroatom. The heteroatoms are preferably selected from N, O and/or S. An aryl group or heteroaryl group is understood here to mean either a simple aromatic cycle, i.e. phenyl, or a simple heteroaromatic cycle, for example pyridinyl, pyrimidinyl, thiophenyl, etc., or a fused (annelated) aryl or heteroaryl group, for example naphthyl, anthracenyl, phenanthrenyl, quinolinyl or isoquinolinyl.

An aryl group or heteroaryl group is preferably derived from benzene, naphthalene, anthracene, phenanthrene, pyrene, benzanthracene, chrysene, perylene, fluoranthene, naphthacene, pentacene, benzopyrene, biphenyl, biphenylene, terphenyl, triphenylene, fluorene, spirobifluorene, dihydrophenanthrene, dihydropyrene, tetrahydropyrene, cis- or trans-indenofluorene, cis- or trans-indenocarbazole, cis- or trans-indolocarbazole, truxene, isotruxene, spirotruxene, spiroisotruxene, furan, benzofuran, isobenzofuran, dibenzofuran, thiophene, benzothiophene, isobenzothiophene, dibenzothiophene, pyrrole, indole, isoindole, carbazole, pyridine, quinoline, isoquinoline, acridine, phenanthridine, benzo-5,6-quinoline, benzo-6,7-quinoline, benzo-7,8-quinoline, phenothiazine, phenoxazine, pyrazole, indazole, imidazole, benzimidazole, naphthimidazole, phenanthrimidazole, pyridimidazole, pyrazinimidazole, quinoxalinimidazole, oxazole, benzoxazole, naphthoxazole, anthroxazole, phenanthroxazole, isoxazole, 1,2-thiazole, 1,3-thiazole, benzothiazole, pyridazine, hexaazatriphenylene, benzopyridazine, pyrimidine, benzopyrimidine, quinoxaline, 1,5-diazaanthracene, 2,7-diazapyrene, 2,3-diazapyrene, 1,6-diazapyrene, 1,8-diazapyrene, 4,5-diazapyrene, 4,5,9,10-tetraazaperylene, pyrazine, phenazine, phenoxazine, phenothiazine, fluorubine, naphthyridine, azacarbazole, benzocarboline, phenanthroline, 1,2,3-triazole, 1,2,4-triazole, benzotriazole, 1,2,3-oxadiazole, 1,2,4-oxadiazole, 1,2,5-oxadiazole, 1,3,4-oxadiazole, 1,2,3-thiadiazole, 1,2,4-thiadiazole, 1,2,5-thiadiazole, 1,3,4-thiadiazole, 1,3,5-triazine, 1,2,4-triazine, 1,2,3-triazine, tetrazole, 1,2,4,5-tetrazine, 1,2,3,4-tetrazine, 1,2,3,5-tetrazine, purine, pteridine, indolizine and benzothiadiazole.

A polymerizable group is a group which can be subject to or can undergo polymerization thus forming an oligomer or a polymer.

Polymerization is the process of taking individual monomers and chaining them together to make longer units. These longer units are called polymers. The compounds of formula (I) as described before and preferably described below are suitable monomers for the preparation of an ophthalmic device or a precursor article for an ophthalmic device.

Within the gist of the invention, the polymerizable group R₁ once oligomerized or polymerized thus forms or is part of the backbone of the oligomer, polymer or copolymer comprising polymerized compounds of formula (I). Suitable polymerizable groups are defined to be a trialkoxysilyl group or a dialkoxyalkylsilyl group where the alkyl and/or alkoxy groups are each independently linear or branched having 1 to 6 C atoms, or a silyl group of formula (1), (2) or (3) or a polymerizable group of formula (4), 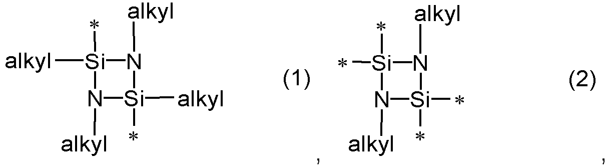 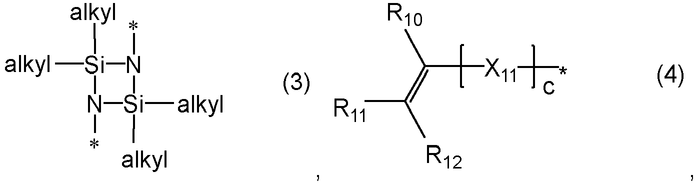
where alkyl means at each occurrence independently of each other a linear or branched alkyl group having 1 to 6 C atoms and the asterisk "*" denotes at each occurrence independently of each other a linkage to the linker [-R₂-Y]ₙ or [Y-R₂-]ₘ₁;
and wherein
X₁₁ is selected from the group consisting of O, S, O-SO₂, SO₂-O, C(=O), OC(=O), C(=O)O, S(C=O) and (C=O)S,
R₁₀, R₁₁, R₁₂ are at each occurrence independently of each other selected from the group consisting of H, F, a linear or branched, non-fluorinated, partially or completely fluorinated alkyl group having 1 to 20 C atoms and aryl with 6 to 14 C atoms and
c is 0 or 1.

Particularly preferred polymerizable groups are described below.

Particularly preferred polymerized groups are described below.

Aryl with 6 to 14 C atoms is an aryl group preferably selected from the group consisting of phenyl, naphthyl or anthryl, particularly preferably phenyl.

In one preferred embodiment, the compounds of formula (I) acting as monomers for the preparation of the ophthalmic device or precursor article of the ophthalmic device as described before or for the preparation of an oligomer, polymer or copolymer according to the invention or as compound according to the invention contain the polymerizable group R₁ attached via - R₂- and Y to the photoactive ring system. This is the case for compounds of formula (I) in which n is 1 and m1 is 0 which can be described accordingly in formula (I').

The invention is therefore additionally directed to an ophthalmic device or a precursor article for an ophthalmic device comprising at least one polymerized compound of formula (I'), 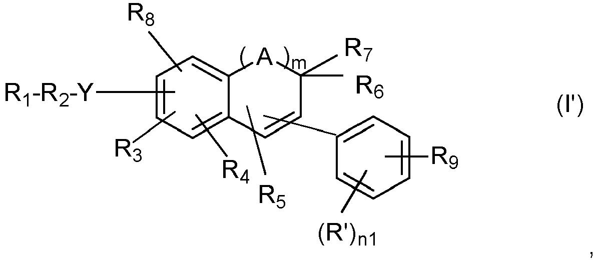 wherein R₁, -R₂-, Y, R₃, R₄, R₅, R₆, R₇, R₈, A, m, R', and n1 have a meaning as described before or preferably described before or below and R⁹ is H or R' as described before or preferably described before.

The invention is therefore additionally directed to compounds of formula (I) wherein n is 1 and m1 is 0 which can preferably be described according to formula (I'), 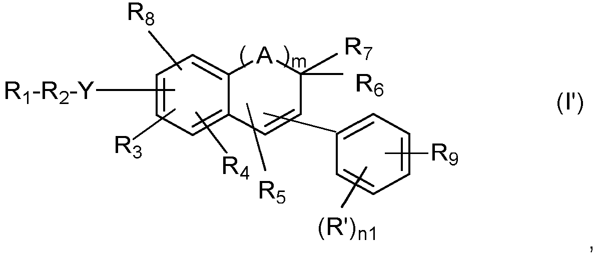
wherein R₁, -R₂-, Y, R₃, R₄, R₅, R₆, R₇, R₈, A, m, R', and n1 have a meaning as described before or preferably described before or below and R⁹ is H or R' as described before or preferably described before,
provided that in case of m is 0, R₆ and R₇ are on each occurrence not a non-halogenated, partially or completely halogenated cycloalkyl group having 3 to 6 C atoms; and
provided that in case of m is 1, n is 1 and Y is a bond, o is selected from 1 to 20; and
provided that in case of m is 2, n is 1 and Y is a bond, o is selected from the group consisting of 3 to 20; and
provided that in case of m is 2, n is 1 and Y is O, o is selected from the group consisting of 1 to 20.

The position of substituent R₁-R₂-Y in formulae (I) or (I') is not limited. In compounds of formula (I'-a), the substituent R₁-R₂-Y is in position 8 of the annellated aromatic ring.

In compounds of formula (I'-b), the substituent R₁-R₂-Y is in position 7 of the annellated aromatic ring.

In compounds of formula (I'-c), the substituent R₁-R₂-Y is in position 6 of the annellated aromatic ring.

In compounds of formula (I'-d), the substituent R₁-R₂-Y is in position 5 of the annellated aromatic ring.

The invention is therefore additionally directed to an ophthalmic device or a precursor article for an ophthalmic device comprising at least one polymerized compound of formula (I) wherein n is 1 and m1 is 0 which can preferably be described according to formulae (I'-a), (I'-b), (I'-c) and (I'-d), 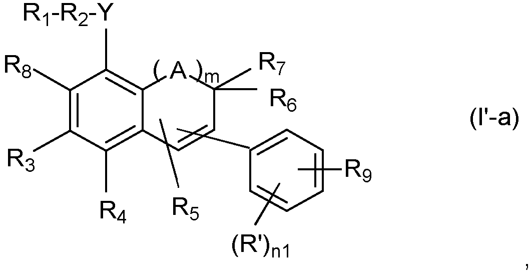 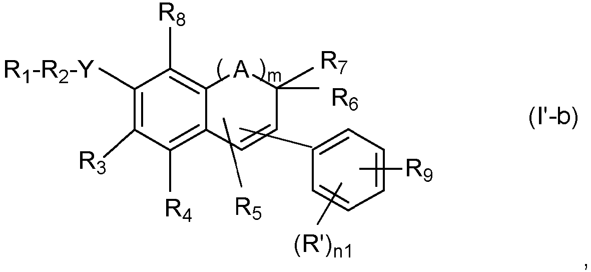 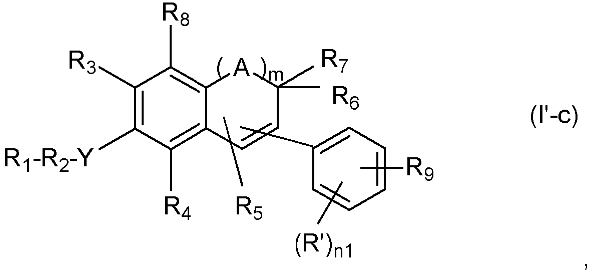 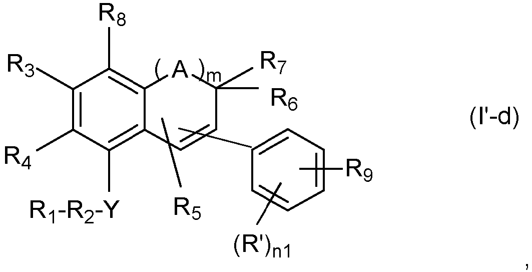 wherein R₁, -R₂-, Y, R₃, R₄, R₅, R₆, R₇, R₈, R₉, A, m, R', and n1 have a meaning as described before or preferably described before or below.

The invention is therefore additionally directed to compounds of formula (I) wherein n is 1 and m1 is 0 which can preferably be described according to formulae (I'-a), (I'-b), (I'-c) and (I'-d), 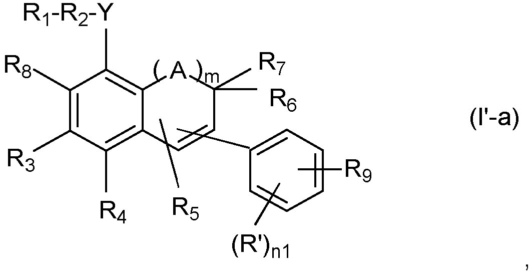 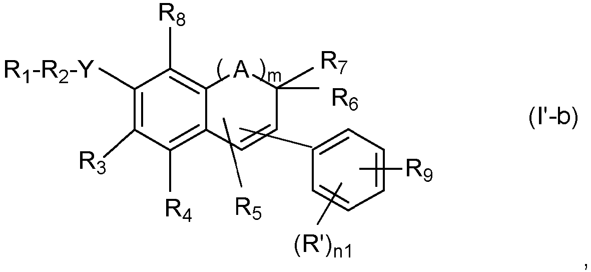 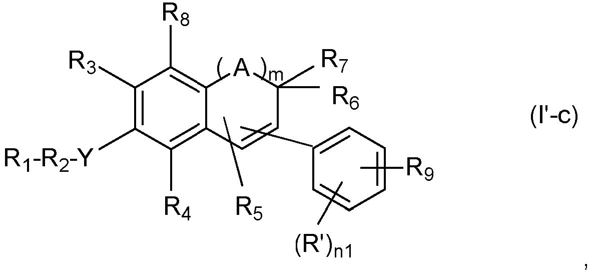 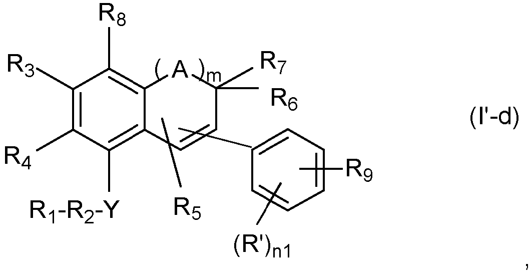
wherein R₁, -R₂-, Y, R₃, R₄, R₅, R₆, R₇, R₈, R₉, A, m, R', and n1 have a meaning as described before or preferably described before or below, provided that in case of m is 0, R₆ and R₇ are on each occurrence not a non-halogenated, partially or completely halogenated cycloalkyl group having 3 to 6 C atoms; and
provided that in case of m is 1, n is 1 and Y is a bond, o is selected from 1 to 20; and
provided that in case of m is 2, n is 1 and Y is a bond, o is selected from the group consisting of 3 to 20; and
provided that in case of m is 2, n is 1 and Y is O, o is selected from the group consisting of 1 to 20.

Preferred positions of R₁-R₂-X are position 8 and/or 7 of the annellated aromatic ring as e.g. visualized in formulae (I'-a) and (I'-b).

In another preferred embodiment of the invention, the compounds of formula (I) acting as monomers for the preparation of the ophthalmic device or precursor article of the ophthalmic device as described before or for the preparation of an oligomer, polymer or copolymer according to the invention or as compound according to the invention as described before contain the polymerizable group R₁ attached via -R₂- and Y to the phenyl substituent of the photoactive ring system. This is the case for compounds of formula (I) in which n is 0 and m1 is 1 which can be described accordingly in formula (I").

The invention is therefore additionally directed to an ophthalmic device or a precursor article for an ophthalmic device comprising at least one polymerized compound of formula (I"), 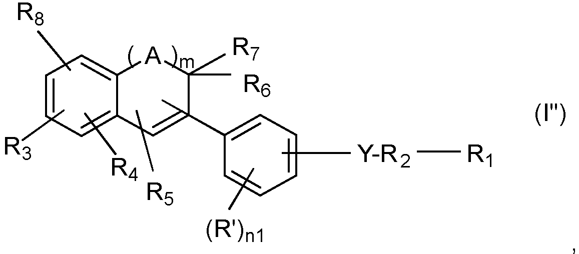 wherein R₁, -R₂-, Y, R₃, R₄, R₅, R₆, R₇, R₈, A, m, R', and n1 have a meaning as described before or preferably described before or below.

The invention is therefore additionally directed to compounds of formula (I) wherein n is 0 and m1 is 1 which can preferably be described according to formula (I"), 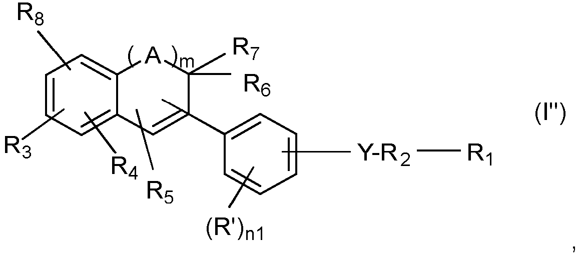
wherein R₁, -R₂-, Y, R₃, R₄, R₅, R₆, R₇, R₈, R₉, A, m, R', and n1 have a meaning as described before or preferably described before or below, provided that in case of m is 0, m1 is 1 and Y is a bond, o is selected from the group consisting of 3 to 20; and
provided that in case of m is 0 and m1 is 1, two substituents from the group of R₃, R₄ and R₈ are not simultaneously F; and
provided that in case of m is 0, m1 is 1 and Y is a bond or O, R' is on each occurrence not F; and
provided that in case of m is 0, R₆ and R₇ are on each occurrence not a non-halogenated, partially or completely halogenated cycloalkyl group having 3 to 6 C atoms; and
provided that in case of m is 2, m1 is 1 and Y is a bond, o is selected from the group consisting of 3 to 20; and
provided that in case of m is 2, m1 is 1 and Y is O, o is selected from the group consisting of 1 to 20.

As described before within the ophthalmic device, precursor article, compounds of formula (I), (I'), (I") and any oligomers, polymers or copolymers derived therefrom according to the invention, the substituents R₃, R₄, R₅, R₆, R₇ and R₈ are at each occurrence independently selected from the group consisting of H, F, Cl, Br, CN, SO₂CF₃, a linear or branched, non-halogenated, partially or completely halogenated alkyl group having 1 to 20 C atoms, a non-halogenated, partially or completely halogenated cycloalkyl group having 3 to 6 C atoms, a linear or branched, non-halogenated, partially or completely halogenated alkoxy group having 1 to 20 C atoms and a linear or branched, non-halogenated and partially or completely halogenated thioalkyl group having 1 to 20 C atoms.

R₃, R₄ and R₈ are preferably H, F, a straight-chain alkyl group with 1 to 4 C atoms or a straight-chain alkoxy group with 1 to 4 C atoms. R₃, R₄ and R₈ are particularly preferably H.

R₅ is preferably H, F, CN, SO₂CF₃, CF₃, CF₂CF₃, or CH₂CF₃. R₅ is particularly preferably H, F, CF₃. R₅ is very particularly preferably H or F. R₅ is very particularly preferably H.

R₆ and R₇ are preferably independently of each other H, F, a linear or branched alkyl group having 1 to 4 C atoms or a linear or branched partially or fully fluorinated alkyl group having 1 to 4 C atoms. R₆ is preferably H and R₇ has a meaning as described or preferably described before or below. R₆ and R₇ are independently of each other particularly preferably H or F. R₆ and R₇ are preferably identical. R₆ and R₇ are very particularly preferably H.

With regards to the compounds according to the invention, the described disclaimers have to be considered for the definition and preferred embodiments of R₃, R₄, R₅, R₆, R₇ and R₈.

Compounds of formula (I), (I') or (I") wherein m is 0 are based on an indene ring system and can be described as compounds of formulae (1-1), (I'-1) or (I"-1) 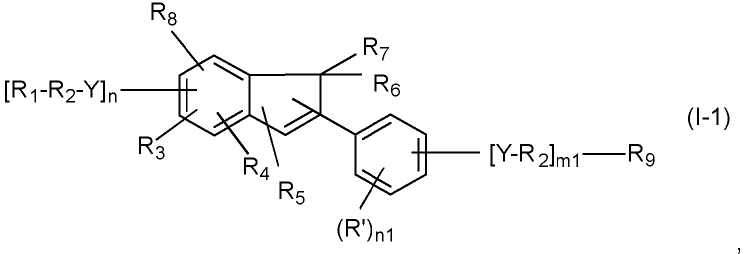 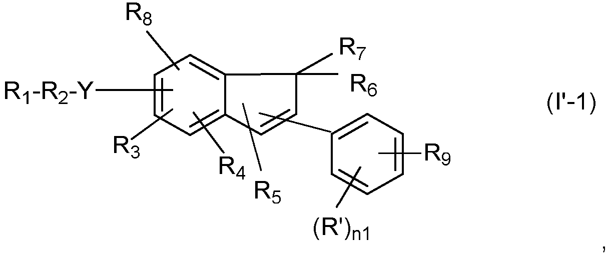 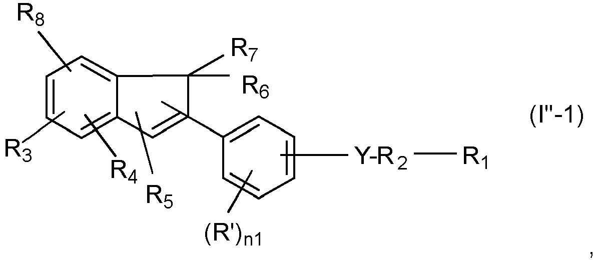 wherein R₁, -R₂-, Y, n, R₃, R₄, R₅, R₆, R₇, R₈, R₉, m1, n1 and R' have a meaning as described before or preferably described before or below and act preferably as monomers for the preparation of the ophthalmic device or precursor article of the ophthalmic device as described before or for the preparation of an oligomer, polymer or copolymer according to the invention.

Compounds of formula (I), (I') or (I") wherein m is 1 are based on a 1,2-dihydronaphthalene ring system and can be described as compounds of formula (I-2), (I'-2) or (I"-2) 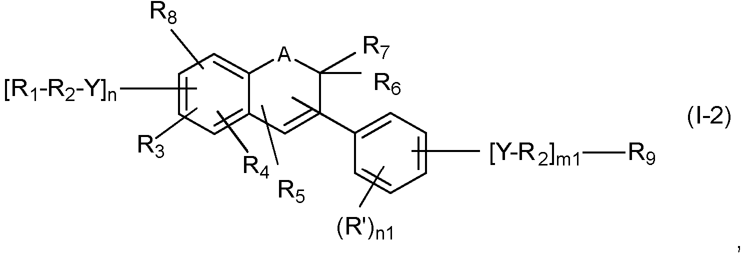 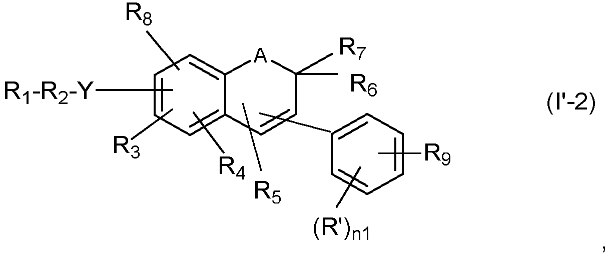 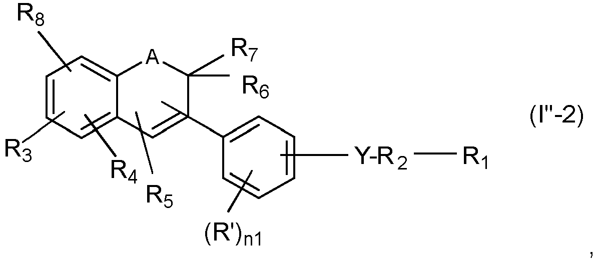 wherein A, R₁, -R₂-, Y, n, R₃, R₄, R₅, R₆, R₇, R₈, R₉, m1, n1 and R' have a meaning as described before or preferably described before or below and act preferably as monomers for the preparation of the ophthalmic device or precursor article of the ophthalmic device as described before or for the preparation of an oligomer, polymer or copolymer according to the invention.

A is the same or different at each instance and is CH₂, CHR₀ or C(R₀)₂ and R₀ has a meaning as described before or preferably described below. Preferably, A is the same or different at each instance and is CH₂ or CHR₀ and R₀ has a meaning as described before or preferably described below. Particularly preferably, A is at each instance CH₂.

Compounds of formula (I-2), (I'-2) and (I"-2) wherein A is CH₂ correspond to formulae (I-2-H), (I'-2-H) and (I"-2-H), 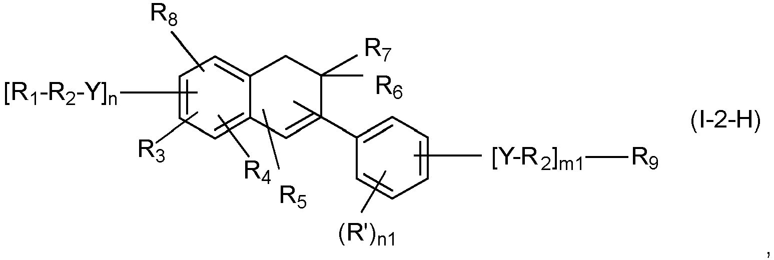 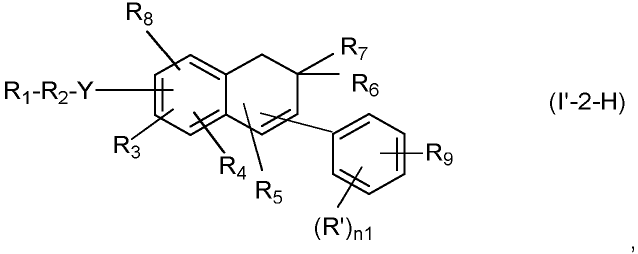 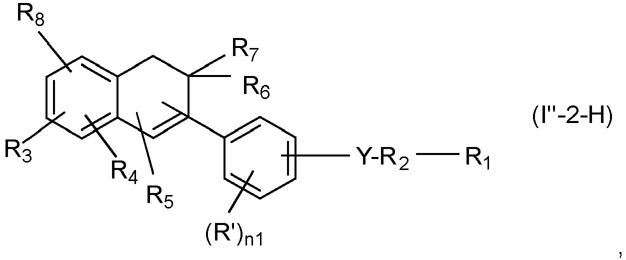 wherein R₁, -R₂-, Y, n, R₃, R₄, R₅, R₆, R₇, R₈, R₉, m1, n1 and R' have a meaning as described before or preferably described before or below and act preferably as monomers of formulae (I), (I') and (I") for the preparation of the ophthalmic device or precursor article of the ophthalmic device as described before or for the preparation of an oligomer, polymer or copolymer according to the invention.

Compounds of formula (I), (I') or (I") wherein m is 2 are based on a 6,7-dihydro-5H-benzo[7]annulene ring system and can be described as compounds of formula (I-3), (I'-3) or (I"-3) 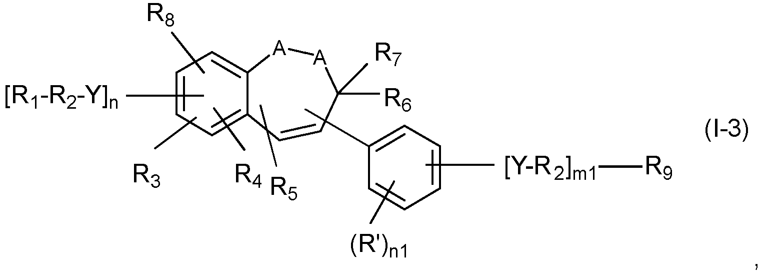 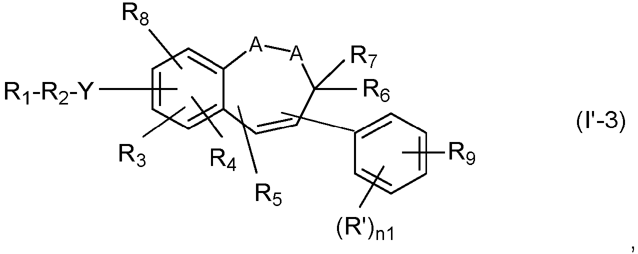 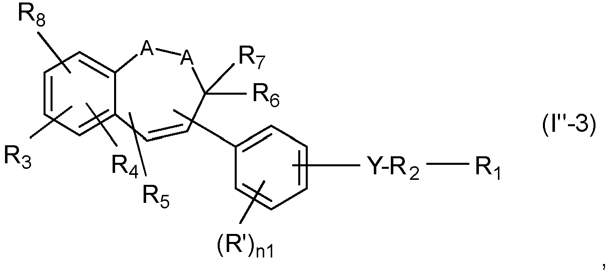 wherein A, R₁, -R₂-, Y, n, R₃, R₄, R₅, R₆, R₇, R₈, R₉, m1, n1 and R' have a meaning as described before or preferably described before or below and act preferably as monomers for the preparation of the ophthalmic device or precursor article of the ophthalmic device as described before or for the preparation of an oligomer, polymer or copolymer according to the invention.

A is the same or different at each instance and is CH₂, CHR₀ or C(R₀)₂ and R₀ has a meaning as described before or preferably described below. Preferably, A is the same or different at each instance and is CH₂ or CHR₀ and R₀ has a meaning as described before or preferably described below Particularly preferably, A is at each instance CH₂.

Compounds of formula (I-3), (I'-3) and (I"-3) wherein each A is CH₂ correspond to formulae (I-3-H), (I'-3-H) and (I"-3-H), 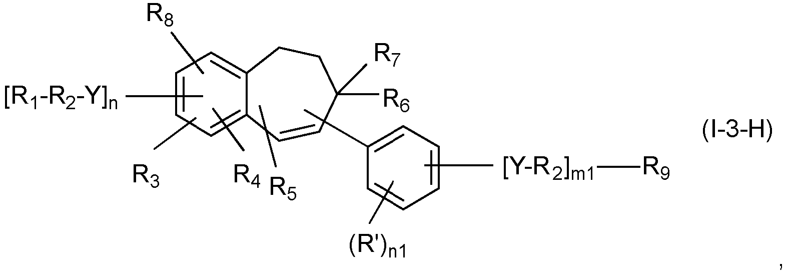 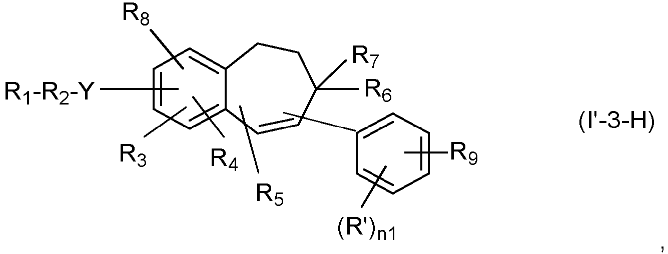 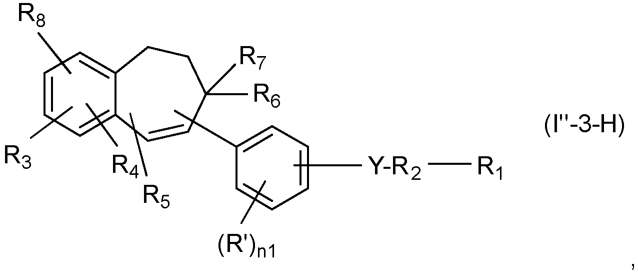 wherein R₁, -R₂-, Y, n, R₃, R₄, R₅, R₆, R₇, R₈, R₉, m1, n1 and R' have a meaning as described before or preferably described before or below and act preferably as monomers of formulae (I), (I') and (I") for the preparation of the ophthalmic device or precursor article of the ophthalmic device as described before or for the preparation of an oligomer, polymer or copolymer according to the invention.

Compounds of formula (I), (I') or (I") wherein m is 2 are based on a (Z)-5,6,7,8-tetrahydrobenzo[8]annulene ring system and can be described as compounds of formula (I-4), (I'-4) or (I"-4) 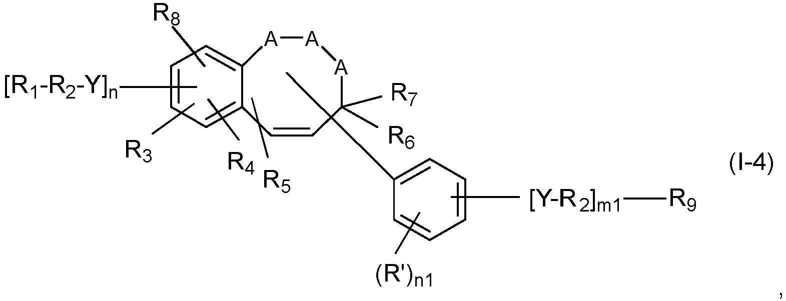 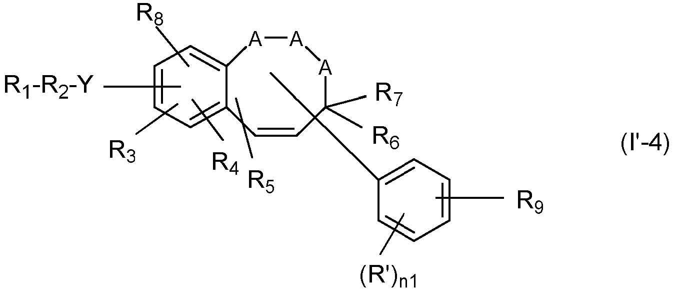 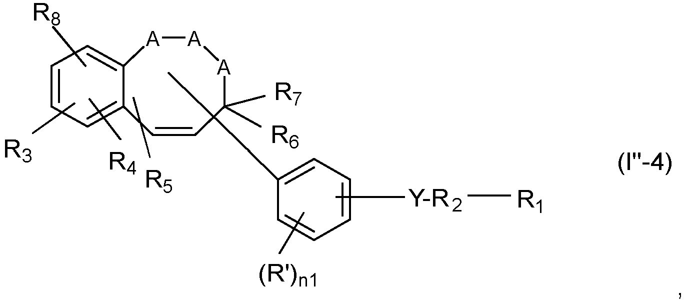 wherein A, R₁, -R₂-, Y, n, R₃, R₄, R₅, R₆, R₇, R₈, R₉, m1, n1 and R' have a meaning as described before or preferably described before or below as monomers for the preparation of the ophthalmic device or precursor article of the ophthalmic device as described before or for the preparation of an oligomer, polymer or copolymer according to the invention.

A is the same or different at each instance and is CH₂, CHR₀ or C(R₀)₂ and R₀ has a meaning as described before or preferably described below. Preferably, A is the same or different at each instance and is CH₂ or CHR₀ and R₀ has a meaning as described before or preferably described below Particularly preferably, A is at each instance CH₂.

Compounds of formula (I-4), (I'-4) and (I"-4) wherein each A is CH₂ correspond to formulae (I-4-H), (I'-4-H) and (I"-4-H), 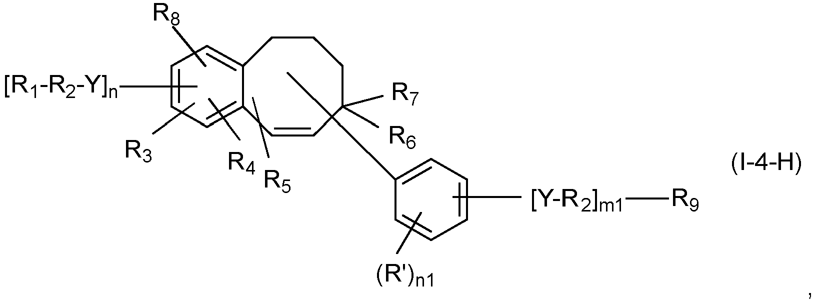 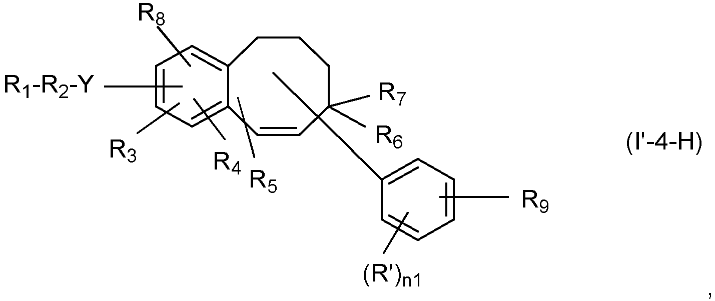 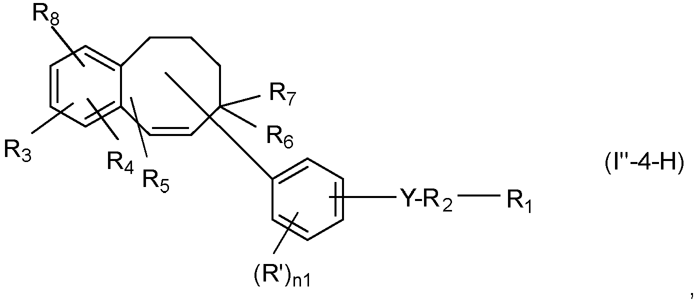 wherein R₁, -R₂-, Y, n, R₃, R₄, R₅, R₆, R₇, R₈, R₉, m1, n1 and R' have a meaning as described before or preferably described before or below and act preferably as monomers of formulae (I), (I') and (I") for the preparation of the ophthalmic device or precursor article of the ophthalmic device as described before or for the preparation of an oligomer, polymer or copolymer according to the invention.

As described before within the ophthalmic device, precursor article, compounds of formulae (I), (1-1), (I-2), (I-2H), (I-3), (I-3-H), (I-4), (I-4-H), (I'), (I'-a), (I'-b), (I'-c), (I'-d), (I'-1), (I'-2), (I'-2-H), (I'-3), (I'-3-H), (I'-4) and (I'-4-H) and any oligomers, polymers or copolymers derived therefrom according to the invention, R₀ is at each occurrence independently selected from the group consisting of F, a linear or branched alkyl group having 1 to 4 C atoms and a linear or branched partially or fully fluorinated alkyl group having 1 to 4 C atoms. R₀ is independently at each occurrence and preferably F, Methyl, Ethyl or Trifluoromethyl. R₀ is independently at each occurrence particularly preferably Methyl. R₀ is independently at each occurrence particularly preferably F.

The invention furthermore relates to an ophthalmic device or a precursor article for an ophthalmic device comprising at least one polymerized compound of formulae (I), (I'), (I'-a), (I'-b), (I'-c), (I'-d) or (I") as described before or preferably described before wherein A is independently at each occurrence CH₂ and m is 1, 2 or 3.

The invention therefore relates to an ophthalmic device or a precursor article for an ophthalmic device comprising at least one polymerized compound of formulae (I), (I'), (I'-a), (I'-b), (I'-c), (I'-d) or (I") as described before or preferably described before wherein A is independently at each occurrence CH₂ and m is 1.

The invention therefore relates to an ophthalmic device or a precursor article for an ophthalmic device comprising at least one polymerized compound of formulae (I), (I'), (I'-a), (I'-b), (I'-c), (I'-d) or (I") as described before or preferably described before wherein A is independently at each occurrence CH₂ and m is 2.

The invention therefore relates to an ophthalmic device or a precursor article for an ophthalmic device comprising at least one polymerized compound of formulae (I), (I'), (I'-a), (I'-b), (I'-c), (I'-d) or (I") as described before or preferably described before wherein A is independently at each occurrence CH₂ and m is 3.

The invention furthermore relates to compounds of formula (I), (I'), (I'-a), (I'-b), (I'-c), (I'-d) or (I") as described before or preferably described before wherein A is independently at each occurrence CH₂ and m is 1, 2 or 3.

The invention therefore relates to compounds of formula (I), (I'), (I'-a), (I'-b), (I'-c), (I'-d) or (I") as described before or preferably described before wherein A is independently at each occurrence CH₂ and m is 1.

The invention therefore relates to compounds of formula (I), (I'), (I'-a), (I'-b), (I'-c), (I'-d) or (I") as described before or preferably described before wherein A is independently at each occurrence CH₂ and m is 2.

The invention therefore relates to compounds of formula (I), (I'), (I'-a), (I'-b), (I'-c), (I'-d) or (I") as described before or preferably described before wherein A is independently at each occurrence CH₂ and m is 3.

With regards to the compounds according to the invention, the described disclaimers have to be considered when m is 0, 1 and/or 2.

As described before, the substituent R₉ corresponds to H or R' in case m1 is 0 wherein R' has a meaning as described before or a preferred or particularly preferred meaning as described below.

In one embodiment of the invention, R₉ is preferably H in case m1 is 0. Therefore, the invention is furthermore directed to an ophthalmic device or a precursor article for an ophthalmic device comprising at least one polymerized compound of formulae (I), (1-1), (I-2), (I-2H), (I-3), (I-3-H), (I-4), (I-4-H), (I'), (I'-a), (I'-b), (I'-c), (I'-d), (I'-1), (I'-2), (I'-2-H), (I'-3), (I'-3-H), (I'-4) and (I'-4-H) where R₉ is H in case m1 is 0 and n1 and R' have a meaning as described before or preferably described below.

Therefore, the invention is furthermore directed to compounds of formulae (I), (1-1), (I-2), (I-2H), (I-3), (I-3-H), (I-4), (I-4-H), (I'), (I'-a), (I'-b), (I'-c), (I'-d), (I'-1), (I'-2), (I'-2-H), (I'-3), (I'-3-H), (I'-4) and (I'-4-H) where R₉ is H in case m1 is 0 and n1 and R' have a meaning as described before or preferably described below.

In this embodiment, it is preferred that n1 is 0.

Therefore, the invention is furthermore directed to an ophthalmic device or a precursor article for an ophthalmic device comprising at least one polymerized compound of formulae (I), (1-1), (I-2), (I-2H), (I-3), (I-3-H), (I-4), (I-4-H), (I'), (I'-a), (I'-b), (I'-c), (I'-d), (I'-1), (I'-2), (I'-2-H), (I'-3), (I'-3-H), (I'-4) and (I'-4-H) wherein R₉ is H in case m1 is 0 and n1 is 0.

Therefore, the invention is furthermore directed to compounds of formulae (I), (I-1), (I-2), (I-2H), (I-3), (I-3-H), (I-4), (I-4-H), (I'), (I'-a), (I'-b), (I'-c), (I'-d), (I'-1), (I'-2), (I'-2-H), (I'-3), (I'-3-H), (I'-4) and (I'-4-H) wherein R₉ is H in case m1 is 0 and n1 is 0.

In another embodiment of the invention, R₉ is preferably R' in case m1 is 0. Therefore, the invention is furthermore directed to an ophthalmic device or a precursor article for an ophthalmic device comprising at least one polymerized compound of formulae (I), (1-1), (I-2), (I-2H), (I-3), (I-3-H), (I-4), (I-4-H), (I'), (I'-a), (I'-b), (I'-c), (I'-d), (I'-1), (I'-2), (I'-2-H), (I'-3), (I'-3-H), (I'-4) and (I'-4-H) wherein R₉ is R' in case m1 is 0 and n1 and R' have a meaning as described before or preferably described below.

Therefore, the invention is furthermore directed to compounds of formulae (I), (1-1), (I-2), (I-2H), (I-3), (I-3-H), (I-4), (I-4-H), (I'), (I'-a), (I'-b), (I'-c), (I'-d), (I'-1), (I'-2), (I'-2-H), (I'-3), (I'-3-H), (I'-4) and (I'-4-H) wherein R₉ is R' in case m1 is 0 and n1 and R' have a meaning as described before or preferably described below.

In this embodiment, it is preferred that n1 is 0, 1 or 2, preferably 0 or 1. Therefore, the invention is furthermore directed to an ophthalmic device or a precursor article for an ophthalmic device comprising at least one polymerized compound of formulae (I), (1-1), (I-2), (I-2H), (I-3), (I-3-H), (I-4), (I-4-H), (I'), (I'-a), (I'-b), (I'-c), (I'-d), (I'-1), (I'-2), (I'-2-H), (I'-3), (I'-3-H), (I'-4) and (I'-4-H) wherein R₉ is R' in case m1 is 0 and n1 is 0, 1 or 2 and R' has a meaning as described before or preferably described below.

Therefore, the invention is furthermore directed to an ophthalmic device or a precursor article for an ophthalmic device comprising at least one polymerized compound of formulae (I), (1-1), (I-2), (I-2H), (I-3), (I-3-H), (I-4), (I-4-H), (I'), (I'-a), (I'-b), (I'-c), (I'-d), (I'-1), (I'-2), (I'-2-H), (I'-3), (I'-3-H), (I'-4) and (I'-4-H) wherein R₉ is R' in case m1 is 0 and n1 is 0 or 1 and R' has a meaning as described before or preferably described below.

Therefore, the invention is furthermore directed to compounds of formulae (I), (1-1), (I-2), (I-2H), (I-3), (I-3-H), (I-4), (I-4-H), (I'), (I'-a), (I'-b), (I'-c), (I'-d), (I'-1), (I'-2), (I'-2-H), (I'-3), (I'-3-H), (I'-4) and (I'-4-H) wherein R₉ is R' in case m1 is 0 and n1 is 0, 1 or 2 and R' has a meaning as described before or preferably described below.

Therefore, the invention is furthermore directed to compounds of formulae (I), (1-1), (I-2), (I-2H), (I-3), (I-3-H), (I-4), (I-4-H), (I'), (I'-a), (I'-b), (I'-c), (I'-d), (I'-1), (I'-2), (I'-2-H), (I'-3), (I'-3-H), (I'-4) and (I'-4-H) wherein R₉ is R' in case m1 is 0 and n1 is 0 or 1 and R' has a meaning as described before or preferably described below.

According to the invention, R' is independently of each other preferably selected from the group consisting of F, a linear or branched partially or fully fluorinated alkyl group having 1 to 10 C atoms, a linear or branched alkyl group having 1 to 10 C atoms, a linear or branched alkoxy group having 1 to 10 C atoms, a linear or branched partially or fully fluorinated alkoxy group having 1 to 10 C atoms and a linear or branched thioalkyl group having 1 to 10 C atoms.

R' is independently of each other particularly preferably selected from the group consisting of F, methyl, ethyl, *n*-propyl, *n*-butyl, *n*-pentyl, *n*-hexyl, *n-*heptyl, *n*-octyl, trifluoromethyl, pentafluoroethyl, heptafluoropropyl, methoxy, ethoxy, propoxy, trifluoromethoxy, pentafluoroethoxy, thiomethyl and thioethyl.

R' is independently of each other particularly preferably selected from the group consisting of F, ethyl, *n*-pentyl, trifluoromethyl, methoxy and trifluoromethoxy.

With regards to the compounds according to the invention, the described disclaimers have to be considered for the definition of R'.

In these embodiments of the invention when m1 is 0, R₉ is H or R' and n1 is 0 or 1, the substitution pattern of the substituents R' is preferably selected from (S-1) to (S-12) 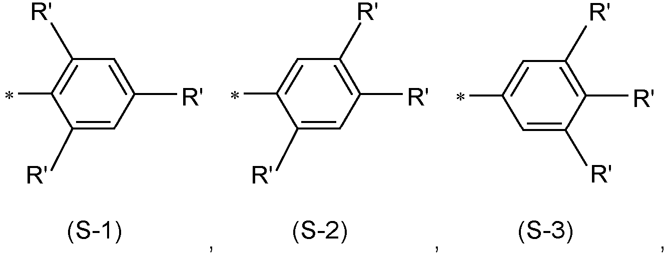 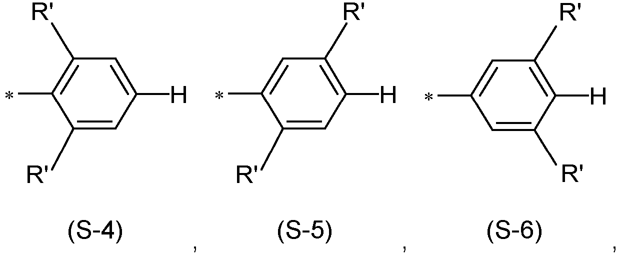 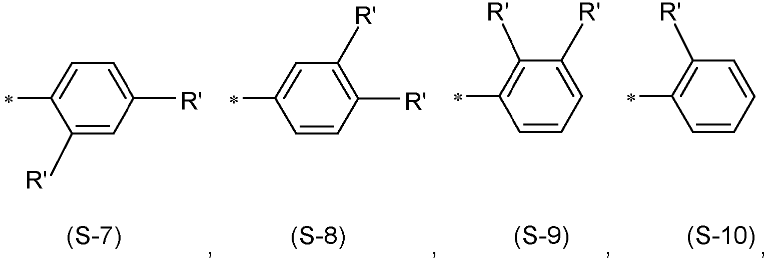 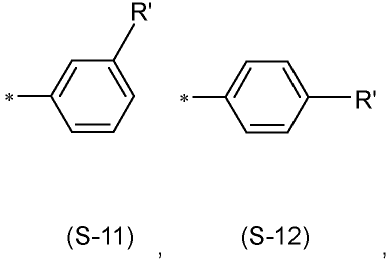 wherein R' has independently of each other a meaning as described before or preferably described below. Preferred substitution patterns are (S-1), (S-7), (S-10), (S-11) and (S-12). Particularly preferred substitution patterns are (S-7) and/or (S-10) and/or (S-12). A very particularly preferred substitution pattern is (S-7). A very particularly preferred substitution pattern is (S-10). A very particularly preferred substitution pattern is (S-12).

The invention therefore relates additionally to an ophthalmic device or precursor article for an ophthalmic device comprising polymerized compounds of formulae (I), (1-1), (I-2), (I-2H), (I-3), (I-3-H), (I-4), (I-4-H), (I'), (I'-a), (I'-b), (I'-c), (I'-d), (I'-1), (I'-2), (I'-2-H), (I'-3), (I'-3-H), (I'-4) and (I'-4-H) as described or preferably described before wherein the substitution pattern of the R' substituents is (S-7) or (S-10) or (S-12).

The invention therefore relates additionally to compounds of formulae (I), (I-1), (I-2), (I-2H), (I-3), (I-3-H), (I-4), (I-4-H), (I'), (I'-a), (I'-b), (I'-c), (I'-d), (I'-1), (I'-2), (I'-2-H), (I'-3), (I'-3-H), (I'-4) and (I'-4-H) as described or preferably described before wherein the substitution pattern of the R' substituents is (S-7) or (S-10) or (S-12).

As described before, the substituent R₉ corresponds to R₁ in case m1 is 1 wherein R' has a meaning as described before or a preferred or particularly preferred meaning as described below.

Therefore, the invention is furthermore directed to an ophthalmic device or precursor article for an ophthalmic device comprising polymerized compounds of formulae (I), (1-1), (I-2), (I-2-H), (I-3), (I-3-H), (I-4), (I-4-H), (I"), (I"-1), (I"-2), (I"-2-H), (I"-3), (I"-3-H), (I"-4) and (I"-4-H) wherein R₉ is R₁ in case m1 is 1 and n1 and R' have a meaning as described before or preferably described below.

Therefore, the invention is furthermore directed to compounds of formulae (I), (1-1), (I-2), (I-2-H), (I-3), (I-3-H), (I-4), (I-4-H), (I"), (I"-1), (I"-2), (I"-2-H), (I"-3), (I"-3-H), (I"-4) and (I"-4-H) wherein R₉ is R₁ in case m1 is 1 and n1 and R' have a meaning as described before or preferably described below. In this embodiment of the invention, n1 is preferably 0 or 1 and R' has a meaning as described or preferably described before. In this embodiment where R₉ is R₁ and R₁ is linked via -R₂-Y- to the phenyl group, such R₁-R₂-Y-group is preferably in ortho, meta or para position to the bond of said phenyl group linked to the rest of formulae (I), (1-1), (I-2), (I-2-H), (I-3), (I-3-H), (I-4), (I-4-H), (I"), (I"-1), (I"-2), (I"-2-H), (I"-3), (I"-3-H), (I"-4) or (I"-4-H). In this embodiment where R₉ is R₁ and R₁ is linked via -R₂-Y- to the phenyl group, such R₁-R₂-Y-group is particularly preferably in ortho or para position to the bond of said phenyl group linked to the rest of formulae (I), (1-1), (I-2), (I-2-H), (I-3), (I-3-H), (I-4), (I-4-H), (I"), (I"-1), (I"-2), (I"-2-H), (I"-3), (I"-3-H), (I"-4) or (I"-4-H). In this embodiment where R₉ is R₁ and R₁ is linked via -R₂-Y- to the phenyl group, such R₁-R₂-Y-group is very particularly preferably in para position to the bond of said phenyl group linked to the rest of formulae (I), (1-1), (I-2), (I-2-H), (I-3), (I-3-H), (I-4), (I-4-H), (I"), (I"-1), (I"-2), (I"-2-H), (I"-3), (I"-3-H), (I"-4) or (I"-4-H).

Therefore, the invention is furthermore directed to an ophthalmic device or precursor article for an ophthalmic device comprising polymerized compounds of formulae (I), (1-1), (I-2), (I-2-H), (I-3), (I-3-H), (I-4), (I-4-H), (I"), (I"-1), (I"-2), (I"-2-H), (I"-3), (I"-3-H), (I"-4) and (I"-4-H) wherein R₉ is R₁, n1 is 0 or 1 and R' has a meaning as described before or preferably described below and R₁ is linked via -R₂-Y- to the phenyl group and such R₁-R₂-Y-group is in ortho, meta or para position, preferably in meta or para position, particularly preferably in para position to the bond of said phenyl group linked to the rest of said formulae.

Therefore, the invention is furthermore directed to compounds of formulae (I), (1-1), (I-2), (I-2-H), (I-3), (I-3-H), (I-4), (I-4-H), (I"), (I"-1), (I"-2), (I"-2-H), (I"-3), (I"-3-H), (I"-4) and (I"-4-H) wherein R₉ is R₁, n1 is 0 or 1 and R' has a meaning as described before or preferably described below and R₁ is linked via -R₂-Y- to the phenyl group and such R₁-R₂-Y-group is in ortho, meta or para position, preferably in meta or para position, particularly preferably in para position to the bond of said phenyl group linked to the rest of said formulae.

In this embodiment, it is preferred that n1 is 0.

Therefore, the invention is furthermore directed to an ophthalmic device or precursor article for an ophthalmic device comprising polymerized compounds of formulae (I), (1-1), (I-2), (I-2-H), (I-3), (I-3-H), (I-4), (I-4-H), (I"), (I"-1), (I"-2), (I"-2-H), (I"-3), (I"-3-H), (I"-4) and (I"-4-H) wherein R₉ is R₁ in case m1 is 1 and n1 is 0.

Therefore, the invention is furthermore directed to compounds of formulae (I), (1-1), (I-2), (I-2-H), (I-3), (I-3-H), (I-4), (I-4-H), (I"), (I"-1), (I"-2), (I"-2-H), (I"-3), (I"-3-H), (I"-4) and (I"-4-H) wherein R₉ is R₁ in case m1 is 1 and n1 is 0.

Therefore, the invention is furthermore directed to an ophthalmic device or precursor article for an ophthalmic device comprising polymerized compounds of formulae (I), (1-1), (I-2), (I-2-H), (I-3), (I-3-H), (I-4), (I-4-H), (I"), (I"-1), (I"-2), (I"-2-H), (I"-3), (I"-3-H), (I"-4) and (I"-4-H) wherein R₉ is R₁ in case m1 is 1 and n1 is 0 and R₁ is linked via -R₂-Y- to the phenyl group and such R₁-R₂-Y-group is in ortho, meta or para position, preferably in meta or para position, particularly preferably in para position to the bond of said phenyl group linked to the rest of said formulae.

Therefore, the invention is furthermore directed to an ophthalmic device or precursor article for an ophthalmic device comprising polymerized compounds of formulae (I), (1-1), (I-2), (I-2-H), (I-3), (I-3-H), (I-4), (I-4-H), (I"), (I"-1), (I"-2), (I"-2-H), (I"-3), (I"-3-H), (I"-4) and (I"-4-H) wherein R₉ is R₁ in case m1 is 1 and n1 is 0 and R₁ is linked via -R₂-Y- to the phenyl group and such R₁-R₂-Y-group is in para position to the bond of said phenyl group linked to the rest of said formulae.

Therefore, the invention is furthermore directed to compounds of formulae (I), (1-1), (I-2), (I-2-H), (I-3), (I-3-H), (I-4), (I-4-H), (I"), (I"-1), (I"-2), (I"-2-H), (I"-3), (I"-3-H), (I"-4) and (I"-4-H) wherein R₉ is R₁ in case m1 is 1 and n1 is 0 and R₁ is linked via -R₂-Y- to the phenyl group and such R₁-R₂-Y-group is in ortho, meta or para position, preferably in meta or para position, particularly preferably in para position to the bond of said phenyl group linked to the rest of said formulae.

Therefore, the invention is furthermore directed to compounds of formulae (I), (1-1), (I-2), (I-2-H), (I-3), (I-3-H), (I-4), (I-4-H), (I"), (I"-1), (I"-2), (I"-2-H), (I"-3), (I"-3-H), (I"-4) and (I"-4-H) wherein R₉ is R₁ in case m1 is 1 and n1 is 0 and R₁ is linked via -R₂-Y- to the phenyl group and such R₁-R₂-Y-group is in para position to the bond of said phenyl group linked to the rest of said formulae.

An ophthalmic device or precursor article for an ophthalmic device comprising polymerized compounds of formula (I) with linkers and substituents as described before or preferably described before or below are preferred when m is 1 or 2.

Compounds of formula (I) with linkers and substituents as described before or preferably described before or below are preferred when m is 1 or 2.

An ophthalmic device or precursor article for an ophthalmic device comprising polymerized compounds of formula (I) with linkers and substituents as described before or preferably described before or below are preferred when m is 2.

Compounds of formula (I) with linkers and substituents as described before or preferably described before or below are particularly preferred when m is 2.

The preferred position of the phenyl group in all compounds of formulae (I), (I-1), (I-2), (I-2H), (I-3), (I-3-H), (I-4), (I-4-H), (I'), (I'-a), (I'-b), (I'-c), (I'-d), (I'-1), (I'-2), (I'-2-H), (I'-3), (I'-3-H), (I'-4), (I'-4-H) (I"), (I"-1), (I"-2), (I"-2-H), (I"-3), (I"-3-H), (I"-4) and (I"-4-H) is at the C atom of the isolated double bond which is adjacent to C(R₆)(R₇). Correspondingly, the preferred position of R₅ in all compounds of formulae (I), (1-1), (I-2), (I-2H), (I-3), (I-3-H), (I-4), (I-4-H), (I'), (I'-a), (I'-b), (I'-c), (I'-d), (I'-1), (I'-2), (I'-2-H), (I'-3), (I'-3-H), (I'-4), (I'-4-H) (I"), (I"-1), (I"-2), (I"-2-H), (I"-3), (I"-3-H), (I"-4) and (I"-4-H) is at the C atom adjacent to the fused aromatic ring.

The following formula summarizes the preferred positions of R₅ and phenyl group for compounds of formula (I) reading formula (I#), 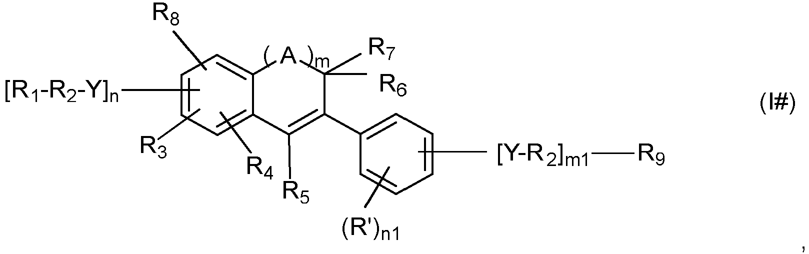 wherein R₁, -R₂-, Y, R₃, R₄, R₅, R₆, R₇, R₈, R₉, A, m, R', and n1 have a meaning as described before or preferably described before or below. Such compounds according to formula (I#) act preferably as monomers of formulae (I) for the preparation of the ophthalmic device or precursor article of the ophthalmic device as described before or for the preparation of an oligomer, polymer or copolymer according to the invention.

According to the invention, compounds of formulae (I), (I#), (1-1), (I-2), (I-2H), (I-3), (I-3-H), (I-4), (I-4-H), (I'), (I'-a), (I'-b), (I'-c), (I'-d), (I'-1), (I'-2), (I'-2-H), (I'-3), (I'-3-H), (I'-4), (I'-4-H) (I"), (I"-1), (I"-2), (I"-2-H), (I"-3), (I"-3-H), (I"-4) and (I"-4-H) with substituents as described before or preferably described before have a polymerizable group as described before or preferably described before or below and have at least one linking element Y-R₂.

According to the invention, Y is independently at each occurrence O, S, O=S=O or a bond.

According to the invention, the linking element -R₂- is selected from the group consisting of -(C(R)₂)ₒ-, or -(C(R)₂)ₚ-X₈-(C(R)₂)_{q}-(X₉)ₛ-(C(R)₂)ᵣ-(X₁₀)ₜ-(C(R)₂)ᵤ-, R is at each occurrence independently selected from the group consisting of H, F, a linear or branched alkyl group having 1 to 4 C atoms or a linear or branched partially or fully fluorinated alkyl group having 1 to 4 C atoms and o is selected from the group consisting of 1 to 20, X₈, X₉ and X₁₀ are at each occurrence O, S, SO₂, or NR₀, s and t are at each occurrence independently 0 or 1, p and q are at each occurrence independently selected from the group consisting of 1 to 10, r and u are at each occurrence independently selected from the group consisting of 0 to 10, wherein the overall number of atoms for -(C(R)₂)ₚ-X₈-(C(R)₂)_{q}-(X₉)ₛ-(C(R)₂)ᵣ(X₁₀)ₜ-(C(R)₂)ᵤ-, is up to 20 C atoms. R₀ in NR₀ has a meaning as described before excluding F.

According to the invention, R is at each occurrence independently selected from the group consisting of H, F, a linear or branched alkyl group having 1 to 8 C atoms or a linear or branched partially or fully fluorinated alkyl group having 1 to 4 C atoms.

R is particularly preferably at each occurrence independently H, F, methyl or ethyl. R is very particularly preferably H.

In another preferred embodiment of the invention, o is preferably selected from the group consisting of 0, 1, 2, 3, 4, 5, 6, 7, 8, 9, 10, 11 and 12 within the compounds of formulae (I), (I#), (1-1), (I-2), (I-2H), (I-3), (I-3-H), (I-4), (I-4-H), (I'), (I'-a), (I'-b), (I'-c), (I'-d), (I'-1), (I'-2), (I'-2-H), (I'-3), (I'-3-H), (I'-4), (I'-4-H) (I"), (I"-1), (I"-2), (I"-2-H), (I"-3), (I"-3-H), (I"-4) and (I"-4-H) acting as monomers for the preparation of the ophthalmic device or precursor article of the ophthalmic device as described before or for the preparation of an oligomer, polymer or copolymer according to the invention or within the compounds according to the invention. Preferably, o is selected from the group consisting of 0, 1, 2, 3, 4, 5, 6, 7, 8, 9, 10, 11 and 12. Particularly preferably, o is selected from the group consisting of 1, 2, 3, 4, 5, 6 and 7 where 1, 3, 5 and 7 or 2, 3, 4, 5 and 6 is especially preferred. Very particularly preferably, o is 5. With regards to the compounds according to the invention, the described disclaimers have to be considered for the definition of o.

In another preferred embodiment of the invention, s, t, X₈, X₉, X₁₀, p, q, r and u within the compounds of formulae (I), (I#), (1-1), (I-2), (I-2H), (I-3), (I-3-H), (I-4), (I-4-H), (I'), (I'-a), (I'-b), (I'-c), (I'-d), (I'-1), (I'-2), (I'-2-H), (I'-3), (I'-3-H), (I'-4), (I'-4-H) (I"), (I"-1), (I"-2), (I"-2-H), (I"-3), (I"-3-H), (I"-4) and (I"-4-H) acting as monomers for the preparation of the ophthalmic device or precursor article of the ophthalmic device as described before or for the preparation of an oligomer, polymer or copolymer according to the invention or within the compounds according to the invention have the following preferred meaning:
Preferably, s is 1. Preferably, s is 0.

Preferably t is 0 or 1.

Preferably, s and t are 0.

Preferably, X₈, X₉ and X₁₀ are O, S or SO₂. Particularly preferably, X₈, X₉ and X₁₀ are O. Particularly preferably, X₈, X₉ and X₁₀ are S. Particularly preferably, X₈, X₉ and X₁₀ are SO₂.

Preferably, p and q are each independently 1, 3, 3, 4, 5 or 6, particularly preferably 1 or 2, very particularly preferably 2

Preferably, r and u are each independently 0, 1, 2 or 3, particularly preferably 0, 1 or 2, very particularly preferably 0.

In case o is 0, -R₂- is a bond.

According to the invention, suitable examples for -R₂- are -(CH₂)-, -(CH₂)₂-, -(CH₂)₃-, -(CH₂)₄-, -(CH₂)₅-, -(CH₂)₆-, -(CH₂)₇-, -(CH₂)₈-, -(CH₂)₉-, -(CH₂)₁₀-, - (CH₂)₁₁-, -(CH₂)₁₂-, -(CH₂)₁₃-, -(CH₂)₁₄-, -(CH₂)₁₅-, -(CH₂)₁₆-, -(CH₂)₁₇-, - (CH₂)₁₈-, -(CH₂)₁₉-, -(CH₂)₂₀-, -(CHCH₃)-, -(CHCH₃)₂-, -(CHCH₃)₃-, - (CHCH₃)₄-, -(CHCH₃)₅-, -(CHCH₃)₆-, -(CHCH₃)₇-, -(CHCH₃)₈-, -(CHCH₃)₉-, - (CHCH₃)₁₀-, -(CHCH₃)₁₁-, -(CHCH₃)₁₂-, -(CHCH₃)₁₃-, -(CHCH₃)₁₄-, - (CHCH₃)₁₅-, -(CHCH₃)₁₆-, -(CHCH₃)₁₇-, -(CHCH₃)₁₈-, -(CHCH₃)₁₉-, - (CHCH₃)₂₀-, -(C(CH₃)₂)-, -(C(CH₃)₂)₂-, -(C(CH₃)₂)₃-, -(C(CH₃)₂)₄-, - (C(CH₃)₂)₅-, -(C(CH₃)₂)₆-, -(C(CH₃)₂)₇-, -(C(CH₃)₂)₈-, -(C(CH₃)₂)₉-, - (C(CH₃)₂)₁₀-, -(C(CH₃)₂)₁₁-, -(C(CH₃)₂)₁₂-, -(C(CH₃)₂)₁₃-, -(C(CH₃)₂)₁₄-, - (C(CH₃)₂)₁₅-, -(C(CH₃)₂)₁₆-, -(C(CH₃)₂)₁₇-, -(C(CH₃)₂)₁₈-, -(C(CH₃)₂)₁₉-, - (C(CH₃)₂)₂₀-, -(CHC₂H₅)-, -(CHC₂H₅)₂-, -(CHC₂H₅)₃-, -(CHC₂H₅)₄-, - (CHC₂H₅)₅-, -(CHC₂H₅)₆-, -(CHC₂H₅)₇-, -(CHC₂H₅)₈-, -(CHC₂H₅)₉-, - (CHC₂H₅)₁₀-, -(CHC₂H₅)₁₁-, -(CHC₂H₅)₁₂-, -(CHC₂H₅)₁₃-, -(CHC₂H₅)₁₄-, - (CHC₂H₅)₁₅-, -(CHC₂H₅)₁₆-, -(CHC₂H₅)₁₇-, -(CHC₂H₅)₁₈-, -(CHC₂H₅)₁₉-, - (CHC₂H₅)₂₀-, -(CH₂)-(CHCH₃)-(CH₂)-, -(CH₂)-(CHCH₃)-(CH₂)₂-, -(CH₂)-(CHCH₃)-(CH₂)₃-, -(CH₂)-(CHCH₃)-(CH₂)₁₁-, -(CH₂)₂-(CHCH₃)-(CH₂)-, - (CH₂)₃-(CHCH₃)-(CH₂)-, -(CH₂)₁₁-(CHCH₃)-(CH₂)-, -(CH₂)₂-O-(CH₂)₂-, - (CH₂)₃-O-(CH₂)₃-, -(CH₂)₂-O-(CH₂)₂-O-(CH₂)₂-, -(CH₂)₃-O-(CH₂)₃-O-(CH₂)₃-, -(CH₂)₂-O-(CH₂)₂-O-(CH₂)₆-, -(CH₂)₆-O-(CH₂)₂-O-(CH₂)₂-, -(CH₂)₂-O-(CH₂)₂-O-(CH₂)₈-, -(CH₂)₈-O-(CH₂)₂-O-(CH₂)₂-, -(CH₂)₂-S-(CH₂)₂-, -(CH₂)₃-S-(CH₂)₃-, -(CH₂)₂-S-(CH₂)₂-S-(CH₂)₂-, -(CH₂)₃-S-(CH₂)₃-S-(CH₂)₃-, -(CH₂)₂-S-(CH₂)₂-S-(CH₂)₆-, -(CH₂)₆-S-(CH₂)₂-S-(CH₂)₂-, -(CH₂)₂-S-(CH₂)₂-S-(CH₂)₈-, -(CH₂)₈-S-(CH₂)₂-S-(CH₂)₂-, -(CH₂)₂-SO₂-(CH₂)₂-, -(CH₂)₃-SO₂-(CH₂)₃-, -(CH₂)₂-SO₂-(CH₂)₂-SO₂-(CH₂)₂-, -(CH₂)₃-SO₂-(CH₂)₃-SO₂-(CH₂)₃-, -(CH₂)₂-SO₂-(CH₂)₂-SO₂-(CH₂)₆-, -(CH₂)₆-SO₂-(CH₂)₂-SO₂-(CH₂)₂-, -(CH₂)₂-SO₂-(CH₂)₂-SO₂-(CH₂)₈-, -(CH₂)₈-SO₂-(CH₂)₂-SO₂-(CH₂)₂-, -(CH₂)-S-(CH₂)₂-O-(CH₂)-, -(CH₂)-SO₂-(CH₂)₂-O-(CH₂)-, -(CH₂)-SO₂-(CH₂)₂-S-(CH₂)-, -(CH₂)-O-(CH₂)₂-S-(CH₂)₂-O-(CH₂)-, -(CH₂)-S-(CH₂)₂-O-(CH₂)₂-S-(CH₂)-, -(CH₂)-SO₂-(CH₂)₂-O-(CH₂)₂-SO₂-(CH₂)-, -(CH₂)-S-(CH₂)₂-S-(CH₂)₂-S-(CH₂)-, -(CH₂)-SO₂-(CH₂)₂-SO₂-(CH₂)₂-SO₂-(CH₂)-, -(CH₂)-O-(CH₂)₂-SO₂-(CH₂)₂-O-(CH₂)-, -(CH₂)₂-(NCH₃)-(CH₂)₂-, -(CH₂)₃-(NCH₃)-(CH₂)₃-, -(CH₂)₂-(NCH₃)-(CH₂)₂-(NCH₃)-(CH₂)₂-, -(CH₂)₃-(NCH₃)-(CH₂)₃-(NCH₃)-(CH₂)₃-, -(CH₂)₂-(NCH₃)-(CH₂)₂-(NCH₃)-(CH₂)₆-, -(CH₂)₆-(NCH₃)-(CH₂)₂-(NCH₃)-(CH₂)₂-, -(CH₂)₂-(NCH₃)-(CH₂)₂-(NCH₃)-(CH₂)₈- and-(CH₂)₈-(NCH₃)-(CH₂)₂-(NCH₃)-(CH₂)₂-; -(CF₂)-(CH₂)-, -(CH₂)-(CF₂)-, -(CH₂)-(CF₂)-(CH₂)-, -(CH₂)-(CF₂)-(CH₂)₂-, - (CH₂)-(CF₂)-(CH₂)₃-, -(CH₂)-(CF₂)-(CH₂)₄-, -(CH₂)-(CF₂)-(CH₂)₅-, -(CH₂)-(CF₂)-(CH₂)₆-, -(CH₂)-(CF₂)-(CH₂)₇-, -(CH₂)-(CF₂)-(CH₂)₈-, -(CH₂)-(CF₂)-(CH₂)₉-, -(CH₂)-(CF₂)-(CH₂)₁₀-, -(CH₂)₂-(CF₂)-(CH₂)-, -(CH₂)₃-(CF₂)-(CH₂)-, - (CH₂)₄-(CF₂)-(CH₂)-, -(CH₂)₅-(CF₂)-(CH₂)-, -(CH₂)₆-(CF₂)-(CH₂)-, -(CH₂)₇-(CF₂)-(CH₂)-, -(CH₂)₈-(CF₂)-(CH₂)-, -(CH₂)₉-(CF₂)-(CH₂)-, -(CH₂)₁₀-(CF₂)-(CH₂)-, -(CH₂)₂-(CF₂)-(CH₂)₂-, -(CH₂)₃-(CF₂)-(CH₂)₃-, -(CH₂)₄-(CF₂)-(CH₂)₄-, -(CH₂)₅-(CF₂)-(CH₂)₅-, -(CH₂)₂-(CF₂)-(CH₂)-, -(CH₂)₂-(CF₂)-(CH₂)₃-, - (CH₂)₂-(CF₂)-(CH₂)₄-, -(CH₂)₂-(CF₂)-(CH₂)₅-, -(CH₂)₂-(CF₂)-(CH₂)₆-, -(CH₂)₂-(CF₂)-(CH₂)₇-, -(CH₂)₂-(CF₂)-(CH₂)₈-, -(CH₂)₂-(CF₂)-(CH₂)₉-, -(CH₂)₃-(CF₂)-(CH₂)-, -(CH₂)₃-(CF₂)-(CH₂)₂-, -(CH₂)₃-(CF₂)-(CH₂)₄-, -(CH₂)₃-(CF₂)-(CH₂)₅-, -(CH₂)₃-(CF₂)-(CH₂)₆-, -(CH₂)₃-(CF₂)-(CH₂)₇-, -(CH₂)₃-(CF₂)-(CH₂)₆-, -(CH₂)₄-(CF₂)-(CH₂)-, -(CH₂)₄-(CF₂)-(CH₂)₂-, -(CH₂)₄-(CF₂)-(CH₂)₃-, -(CH₂)₄-(CF₂)-(CH₂)₅-, -(CH₂)₄-(CF₂)-(CH₂)₆-, -(CH₂)₄-(CF₂)-(CH₂)₇-, -(CH₂)₅-(CF₂)-(CH₂)-, -(CH₂)₅-(CF₂)-(CH₂)₂-, -(CH₂)₅-(CF₂)-(CH₂)₃-, -(CH₂)₅-(CF₂)-(CH₂)₄-, -(CH₂)₅-(CF₂)-(CH₂)₆-, -(CH₂)₆-(CF₂)-(CH₂)-, -(CH₂)₆-(CF₂)-(CH₂)₂-, -(CH₂)₆-(CF₂)-(CH₂)₃-, -(CH₂)₆-(CF₂)-(CH₂)₄-, -(CH₂)₆-(CF₂)-(CH₂)₅-, -(CFH)-(CH₂)-, -(CH₂)-(CFH)-, -(CH₂)-(CFH)-(CH₂)-, -(CH₂)-(CFH)-(CH₂)₂-, - (CH₂)-(CFH)-(CH₂)₃-, -(CH₂)-(CFH)-(CH₂)₄-, -(CH₂)-(CFH)-(CH₂)₅-, -(CH₂)-(CFH)-(CH₂)₆-, -(CH₂)-(CFH)-(CH₂)₇-, -(CH₂)-(CFH)-(CH₂)₈-, -(CH₂)-(CFH)-(CH₂)₉-, -(CH₂)-(CFH)-(CH₂)₁₀-, -(CH₂)₂-(CFH)-(CH₂)-, -(CH₂)₃-(CFH)-(CH₂)-, -(CH₂)₄-(CFH)-(CH₂)-, -(CH₂)₅-(CFH)-(CH₂)-, -(CH₂)₆-(CFH)-(CH₂)-, - (CH₂)₇-(CFH)-(CH₂)-, -(CH₂)₈-(CFH)-(CH₂)-, -(CH₂)₉-(CFH)-(CH₂)-, -(CH₂)₁₀-(CFH)-(CH₂)-, -(CH₂)₂-(CFH)-(CH₂)₂-, -(CH₂)₃-(CFH)-(CH₂)₃-, -(CH₂)₄-(CFH)-(CH₂)₄-, -(CH₂)₅-(CFH)-(CH₂)₅-, -(CH₂)₂-(CFH)-(CH₂)-, -(CH₂)₂-(CFH)-(CH₂)₃-, -(CH₂)₂-(CFH)-(CH₂)₄-, -(CH₂)₂-(CFH)-(CH₂)₅-, -(CH₂)₂-(CFH)-(CH₂)₆-, -(CH₂)₂-(CFH)-(CH₂)₇-, -(CH₂)₂-(CFH)-(CH₂)₈-, -(CH₂)₂-(CFH)-(CH₂)₉-, -(CH₂)₃-(CFH)-(CH₂)-, -(CH₂)₃-(CFH)-(CH₂)₂-, -(CH₂)₃-(CFH)-(CH₂)₄-, -(CH₂)₃-(CFH)-(CH₂)₅-, -(CH₂)₃-(CFH)-(CH₂)₆-, -(CH₂)₃-(CFH)-(CH₂)₇-, -(CH₂)₃-(CFH)-(CH₂)₈-, -(CH₂)₄-(CFH)-(CH₂)-, -(CH₂)₄-(CFH)-(CH₂)₂-, -(CH₂)₄-(CFH)-(CH₂)₃-, -(CH₂)₄-(CFH)-(CH₂)₅-, -(CH₂)₄-(CFH)-(CH₂)₆-, -(CH₂)₄-(CFH)-(CH₂)₇-, -(CH₂)₅-(CFH)-(CH₂)-, -(CH₂)₅-(CFH)-(CH₂)₂-, -(CH₂)₅-(CFH)-(CH₂)₃-, -(CH₂)₅-(CFH)-(CH₂)₄-, -(CH₂)₅-(CFH)-(CH₂)₆-, -(CH₂)₆-(CFH)-(CH₂)-, -(CH₂)₆-(CFH)-(CH₂)₂-, -(CH₂)₆-(CFH)-(CH₂)₃-, -(CH₂)₆-(CFH)-(CH₂)₄-, -(CH₂)₆-(CFH)-(CH₂)₅-, -(CF₂)₂-(CH₂)-, -(CH₂)-(CF₂)₂-, -(CH₂)-(CF₂)₂-(CH₂)-, -(CH₂)-(CF₂)₂-(CH₂)₂-, - (CH₂)-(CF₂)₂-(CH₂)₃-, -(CH₂)-(CF₂)₂-(CH₂)₄-, -(CH₂)-(CF₂)₂-(CH₂)₅-, -(CH₂)-(CF₂)₂-(CH₂)₆-, -(CH₂)-(CF₂)₂-(CH₂)₇-, -(CH₂)-(CF₂)₂-(CH₂)₈-, -(CH₂)-(CF₂)₂-(CH₂)₉-, -(CH₂)₂-(CF₂)₂-(CH₂)-, -(CH₂)₃-(CF₂)₂-(CH₂)-, -(CH₂)₄-(CF₂)₂-(CH₂)-, -(CH₂)₅-(CF₂)₂-(CH₂)-, -(CH₂)₆-(CF₂)₂-(CH₂)-, -(CH₂)₇-(CF₂)₂-(CH₂)-, -(CH₂)₈-(CF₂)₂-(CH₂)-, -(CH₂)₉-(CF₂)₂-(CH₂)-, -(CH₂)₂-(CF₂)₂-(CH₂)₂-, -(CH₂)₃-(CF₂)₂-(CH₂)₃-, -(CH₂)₄-(CF₂)₂-(CH₂)₄-, -(CH₂)₅-(CF₂)₂-(CH₂)₅-, -(CH₂)₂-(CF₂)₂-(CH₂)-,-(CH₂)₂-(CF₂)₂-(CH₂)₃-, -(CH₂)₂-(CF₂)₂-(CH₂)₄-, -(CH₂)₂-(CF₂)₂-(CH₂)₅-, -(CH₂)₂-(CF₂)₂-(CH₂)₆-, -(CH₂)₂-(CF₂)₂-(CH₂)₇-, -(CH₂)₂-(CF₂)₂-(CH₂)₈-, - (CH₂)₃-(CF₂)₂-(CH₂)-, -(CH₂)₃-(CF₂)₂-(CH₂)₂-, -(CH₂)₃-(CF₂)₂-(CH₂)₄-, - (CH₂)₃-(CF₂)₂-(CH₂)₅-, -(CH₂)₃-(CF₂)₂-(CH₂)₆-, -(CH₂)₃-(CF₂)₂-(CH₂)₇-, - (CH₂)₄-(CF₂)₂-(CH₂)-, -(CH₂)₄-(CF₂)₂-(CH₂)₂-, -(CH₂)₄-(CF₂)₂-(CH₂)₃-, - (CH₂)₄-(CF₂)₂-(CH₂)₅-, -(CH₂)₄-(CF₂)₂-(CH₂)₆-, -(CH₂)₅-(CF₂)₂-(CH₂)-, - (CH₂)₅-(CF₂)₂-(CH₂)₂-, -(CH₂)₅-(CF₂)₂-(CH₂)₃-, -(CH₂)₅-(CF₂)₂-(CH₂)₄-, - (CH₂)₆-(CF₂)₂-(CH₂)-, -(CH₂)₆-(CF₂)₂-(CH₂)₂-, -(CH₂)₆-(CF₂)₂-(CH₂)₃-, - (CH₂)₆-(CF₂)₂-(CH₂)₄-, -(CFH)₂-(CH₂)-, -(CH₂)-(CFH)₂-, -(CH₂)-(CFH)₂-(CH₂)-, -(CH₂)-(CFH)₂-(CH₂)₂-, -(CH₂)-(CFH)₂-(CH₂)₃-, -(CH₂)-(CFH)₂-(CH₂)₄-, -(CH₂)-(CFH)₂-(CH₂)₅-, -(CH₂)-(CFH)₂-(CH₂)₆-, -(CH₂)-(CFH)₂-(CH₂)₇-, -(CH₂)-(CFH)₂-(CH₂)₈-, -(CH₂)-(CFH)₂-(CH₂)₉-, -(CH₂)₂-(CFH)₂-(CH₂)-, -(CH₂)₃-(CFH)₂-(CH₂)-, -(CH₂)₄-(CFH)₂-(CH₂)-, -(CH₂)₅-(CFH)₂-(CH₂)-, -(CH₂)₆-(CFH)₂-(CH₂)-, -(CH₂)₇-(CFH)₂-(CH₂)-, -(CH₂)₈-(CFH)₂-(CH₂)-, -(CH₂)₉-(CFH)₂-(CH₂)-, -(CH₂)₂-(CFH)₂-(CH₂)₂-, -(CH₂)₃-(CFH)₂-(CH₂)₃-, -(CH₂)₄-(CFH)₂-(CH₂)₄-, -(CH₂)₅-(CFH)₂-(CH₂)₅-, -(CH₂)₂-(CFH)₂-(CH₂)-,-(CH₂)₂-(CFH)₂-(CH₂)₃-, -(CH₂)₂-(CFH)₂-(CH₂)₄-, -(CH₂)₂-(CFH)₂-(CH₂)₅-, -(CH₂)₂-(CFH)₂-(CH₂)₆-, -(CH₂)₂-(CFH)₂-(CH₂)₇-, -(CH₂)₂-(CFH)₂-(CH₂)₈-, -(CH₂)₃-(CFH)₂-(CH₂)-, -(CH₂)₃-(CFH)₂-(CH₂)₂-, -(CH₂)₃-(CFH)₂-(CH₂)₄-, -(CH₂)₃-(CFH)₂-(CH₂)₅-, -(CH₂)₃-(CFH)₂-(CH₂)₆-, -(CH₂)₃-(CFH)₂-(CH₂)₇-, -(CH₂)₄-(CFH)₂-(CH₂)-, -(CH₂)₄-(CFH)₂-(CH₂)₂-, -(CH₂)₄-(CFH)₂-(CH₂)₃-, -(CH₂)₄-(CFH)₂-(CH₂)₅-, -(CH₂)₄-(CFH)₂-(CH₂)₆-, -(CH₂)₅-(CFH)₂-(CH₂)-, -(CH₂)₅-(CFH)₂-(CH₂)₂-, -(CH₂)₅-(CFH)₂-(CH₂)₃-, -(CH₂)₅-(CFH)₂-(CH₂)₄-, -(CH₂)₆-(CFH)₂-(CH₂)-, -(CH₂)₆-(CFH)₂-(CH₂)₂-, -(CH₂)₆-(CFH)₂-(CH₂)₃-, -(CH₂)₆-(CFH)₂-(CH₂)₄-, -(CF₂)₃-(CH₂)-, -(CH₂)-(CF₂)₃-, -(CH₂)-(CF₂)₃-(CH₂)-, -(CH₂)-(CF₂)₃-(CH₂)₂-, - (CH₂)-(CF₂)₃-(CH₂)₃-, -(CH₂)-(CF₂)₃-(CH₂)₄-, -(CH₂)-(CF₂)₃-(CH₂)₅-, -(CH₂)-(CF₂)₃-(CH₂)₆-, -(CH₂)-(CF₂)₃-(CH₂)₇-, -(CH₂)-(CF₂)₃-(CH₂)₈-, -(CH₂)₂-(CF₂)₃-(CH₂)-, -(CH₂)₃-(CF₂)₃-(CH₂)-, -(CH₂)₄-(CF₂)₃-(CH₂)-, -(CH₂)₅-(CF₂)₃-(CH₂)-, - (CH₂)₆-(CF₂)₃-(CH₂)-, -(CH₂)₇-(CF₂)₃-(CH₂)-, -(CH₂)₈-(CF₂)₃-(CH₂)-, -(CH₂)₂-(CF₂)₃-(CH₂)₂-, -(CH₂)₃-(CF₂)₃-(CH₂)₃-, -(CH₂)₄-(CF₂)₃-(CH₂)₄-, -(CH₂)₂-(CF₂)₃-(CH₂)-,-(CH₂)₂-(CF₂)₃-(CH₂)₃-, -(CH₂)₂-(CF₂)₃-(CH₂)₄-, -(CH₂)₂-(CF₂)₃-(CH₂)₅-, -(CH₂)₂-(CF₂)₃-(CH₂)₆-, -(CH₂)₂-(CF₂)₃-(CH₂)₇-, -(CH₂)₃-(CF₂)₃-(CH₂)-, -(CH₂)₃-(CF₂)₃-(CH₂)₂-, -(CH₂)₃-(CF₂)₃-(CH₂)₄-, -(CH₂)₃-(CF₂)₃-(CH₂)₅-, -(CH₂)₃-(CF₂)₃-(CH₂)₆-, -(CH₂)₄-(CF₂)₃-(CH₂)-, -(CH₂)₄-(CF₂)₃-(CH₂)₂-, -(CH₂)₄-(CF₂)₃-(CH₂)₃-, -(CH₂)₄-(CF₂)₃-(CH₂)₅-, -(CH₂)₅-(CF₂)₃-(CH₂)-, -(CH₂)₅-(CF₂)₃-(CH₂)₂-, -(CH₂)₅-(CF₂)₃-(CH₂)₃-, -(CH₂)₅-(CF₂)₃-(CH₂)₄-, (CH₂)₆-(CF₂)₃-(CH₂)-, -(CH₂)₆-(CF₂)₃-(CH₂)₂-, -(CH₂)₆-(CF₂)₃-(CH₂)₃-, -(CF₂)₄-(CH₂)-, -(CH₂)-(CF₂)₄-, -(CH₂)-(CF₂)₄-(CH₂)-, -(CH₂)-(CF₂)₄-(CH₂)₂-, - (CH₂)-(CF₂)₄-(CH₂)₃-, -(CH₂)-(CF₂)₄-(CH₂)₄-, -(CH₂)-(CF₂)₄-(CH₂)₅-, -(CH₂)-(CF₂)₄-(CH₂)₆-, -(CH₂)-(CF₂)₄-(CH₂)₇-, -(CH₂)-(CF₂)₄-(CH₂)₈-, -(CH₂)-(CF₂)₄-(CH₂)₉-, -(CH₂)-(CF₂)₄-(CH₂)₁₀-, -(CH₂)₂-(CF₂)₄-(CH₂)-, -(CH₂)₃-(CF₂)₄-(CH₂)-, -(CH₂)₄-(CF₂)₄-(CH₂)-, -(CH₂)₅-(CF₂)₄-(CH₂)-, -(CH₂)₆-(CF₂)₄-(CH₂)-, - (CH₂)₇-(CF₂)₄-(CH₂)-, -(CH₂)₂-(CF₂)₄-(CH₂)₂-, -(CH₂)₃-(CF₂)₄-(CH₂)₃-, - (CH₂)₄-(CF₂)₄-(CH₂)₄-, -(CH₂)₅-(CF₂)₄-(CH₂)₅-, -(CH₂)₂-(CF₂)₄-(CH₂)₃-, - (CH₂)₂-(CF₂)₄-(CH₂)₄-, -(CH₂)₂-(CF₂)₄-(CH₂)₅-, -(CH₂)₂-(CF₂)₄-(CH₂)₆-, - (CH₂)₃-(CF₂)₄-(CH₂)₂-, -(CH₂)₃-(CF₂)₄-(CH₂)₄-, -(CH₂)₄-(CF₂)₄-(CH₂)₂-, - (CH₂)₄-(CF₂)₄-(CH₂)₃-, -(CH₂)₅-(CF₂)₄-(CH₂)₂-, -(CH₂)₅-(CF₂)₄-(CH₂)₃-, - (CH₂)₆-(CF₂)₄-(CH₂)₂-, -(CF₂)₅-(CH₂)-, -(CH₂)-(CF₂)₅-, -(CH₂)-(CF₂)₅-(CH₂)-, -(CH₂)-(CF₂)₅-(CH₂)₂-, - (CH₂)-(CF₂)₅-(CH₂)₃-, -(CH₂)-(CF₂)₅-(CH₂)₄-, -(CH₂)-(CF₂)₅-(CH₂)₅-, -(CH₂)-(CF₂)₅-(CH₂)₆-, -(CH₂)₂-(CF₂)₅-(CH₂)-, -(CH₂)₃-(CF₂)₅-(CH₂)-, -(CH₂)₄-(CF₂)₅-(CH₂)-, -(CH₂)₅-(CF₂)₅-(CH₂)-, -(CH₂)₆-(CF₂)₅-(CH₂)-, -(CH₂)₂-(CF₂)₅-(CH₂)₂-, -(CH₂)₃-(CF₂)₅-(CH₂)₃-, -(CH₂)₄-(CF₂)₅-(CH₂)₄-, -(CH₂)₂-(CF₂)₅-(CH₂)₃-, - (CH₂)₂-(CF₂)₅-(CH₂)₄-, -(CH₂)₂-(CF₂)₅-(CH₂)₅-, -(CH₂)₂-(CF₂)₅-(CH₂)₆-, - (CH₂)₃-(CF₂)₅-(CH₂)₂-, -(CH₂)₃-(CF₂)₅-(CH₂)₄-, -(CH₂)₄-(CF₂)₅-(CH₂)₂-, - (CH₂)₄-(CF₂)₅-(CH₂)₃-, -(CH₂)₅-(CF₂)₅-(CH₂)₂-, -(CHCF₃)-(CH₂)-, -(CH₂)-(CHCF₃)-, -(CH₂)-(CHCF₃)-(CH₂)-, -(CH₂)-(CHCF₃)-(CH₂)₂-, -(CH₂)-(CHCF₃)-(CH₂)₃-, -(CH₂)-(CHCF₃)-(CH₂)₄-, -(CH₂)-(CHCF₃)-(CH₂)₅-, -(CH₂)-(CHCF₃)-(CH₂)₆-, -(CH₂)-(CHCF₃)-(CH₂)₇-, -(CH₂)-(CHCF₃)-(CH₂)₆-, -(CH₂)-(CHCF₃)-(CH₂)₉-, -(CH₂)-(CHCF₃)-(CH₂)₁₀-, - (CH₂)₂-(CHCF₃)-(CH₂)-, -(CH₂)₃-(CHCF₃)-(CH₂)-, -(CH₂)₄-(CHCF₃)-(CH₂)-, - (CH₂)₅-(CHCF₃)-(CH₂)-, -(CH₂)₆-(CHCF₃)-(CH₂)-, -(CH₂)₇-(CHCF₃)-(CH₂)-, - (CH₂)₈-(CHCF₃)-(CH₂)-, -(CH₂)₉-(CHCF₃)-(CH₂)-, -(CH₂)₁₀-(CHCF₃)-(CH₂)-, - (CH₂)₂-(CHCF₃)-(CH₂)₂-, -(CH₂)₃-(CHCF₃)-(CH₂)₃-, -(CH₂)₄-(CHCF₃)-(CH₂)₄- , -(CH₂)₅-(CHCF₃)-(CH₂)₅-, -(CH₂)₂-(CHCF₃)-(CH₂)₃-, -(CH₂)₂-(CHCF₃)-(CH₂)₄-, -(CH₂)₂-(CHCF₃)-(CH₂)₅-, -(CH₂)₂-(CHCF₃)-(CH₂)₆-, -(CH₂)₂-(CHCF₃)-(CH₂)₇-, -(CH₂)₂-(CHCF₃)-(CH₂)₈-, -(CH₂)₂-(CHCF₃)-(CH₂)₉-, - (CH₂)₃-(CHCF₃)-(CH₂)₂-, -(CH₂)₃-(CHCF₃)-(CH₂)₄-, -(CH₂)₃-(CHCF₃)-(CH₂)₅- , -(CH₂)₃-(CHCF₃)-(CH₂)₆-, -(CH₂)₃-(CHCF₃)-(CH₂)₇-, -(CH₂)₃-(CHCF₃)-(CH₂)₈-, -(CH₂)₄-(CHCF₃)-(CH₂)₂-, -(CH₂)₄-(CHCF₃)-(CH₂)₃-, -(CH₂)₄-(CHCF₃)-(CH₂)₅-, -(CH₂)₄-(CHCF₃)-(CH₂)₆-, -(CH₂)₄-(CHCF₃)-(CH₂)₇-, - (CH₂)₅-(CHCF₃)-(CH₂)₂-, -(CH₂)₅-(CHCF₃)-(CH₂)₃-, -(CH₂)₅-(CHCF₃)-(CH₂)₄- , -(CH₂)₅-(CHCF₃)-(CH₂)₆-, -(CH₂)₆-(CHCF₃)-(CH₂)₂-, -(CH₂)₆-(CHCF₃)-(CH₂)₃-, -(CH₂)₆-(CHCF₃)-(CH₂)₄-, -(CH₂)₆-(CHCF₃)-(CH₂)₅-, -(CHCF₃)₂-(CH₂)-, -(CH₂)-(CHCF₃)₂-, -(CH₂)-(CHCF₃)₂-(CH₂)-, -(CH₂)-(CHCF₃)₂-(CH₂)₂-, -(CH₂)-(CHCF₃)₂-(CH₂)₃-, -(CH₂)-(CHCF₃)₂-(CH₂)₄-, - (CH₂)-(CHCF₃)₂-(CH₂)₅-, -(CH₂)-(CHCF₃)₂-(CH₂)₆-, -(CH₂)-(CHCF₃)₂-(CH₂)₇- , -(CH₂)-(CHCF₃)₂-(CH₂)₈-, -(CH₂)-(CHCF₃)₂-(CH₂)₉-, -(CH₂)₂-(CHCF₃)₂-(CH₂)-, -(CH₂)₃-(CHCF₃)₂-(CH₂)-, -(CH₂)₄-(CHCF₃)₂-(CH₂)-, -(CH₂)₅-(CHCF₃)₂-(CH₂)-, -(CH₂)₆-(CHCF₃)₂-(CH₂)-, -(CH₂)₇-(CHCF₃)₂-(CH₂)-, - (CH₂)₈-(CHCF₃)₂-(CH₂)-, -(CH₂)₉-(CHCF₃)₂-(CH₂)-, -(CH₂)₂-(CHCF₃)₂-(CH₂)₂-, -(CH₂)₃-(CHCF₃)₂-(CH₂)₃-, -(CH₂)₄-(CHCF₃)₂-(CH₂)₄-, -(CH₂)₅-(CHCF₃)₂-(CH₂)₅-, -(CH₂)₂-(CHCF₃)₂-(CH₂)₃-, -(CH₂)₂-(CHCF₃)₂-(CH₂)₄-, - (CH₂)₂-(CHCF₃)₂-(CH₂)₅-, -(CH₂)₂-(CHCF₃)₂-(CH₂)₆-, -(CH₂)₂-(CHCF₃)₂-(CH₂)₇-, -(CH₂)₂-(CHCF₃)₂-(CH₂)₆-, -(CH₂)₃-(CHCF₃)₂-(CH₂)₂-, -(CH₂)₃-(CHCF₃)₂-(CH₂)₄-, -(CH₂)₃-(CHCF₃)₂-(CH₂)₅-, -(CH₂)₃-(CHCF₃)₂-(CH₂)₆-, - (CH₂)₃-(CHCF₃)₂-(CH₂)₇-, -(CH₂)₄-(CHCF₃)₂-(CH₂)₂-, -(CH₂)₄-(CHCF₃)₂-(CH₂)₃-, -(CH₂)₄-(CHCF₃)₂-(CH₂)₅-, -(CH₂)₄-(CHCF₃)₂-(CH₂)₆-, -(CH₂)₅-(CHCF₃)₂-(CH₂)₂-, -(CH₂)₅-(CHCF₃)₂-(CH₂)₃-, -(CH₂)₅-(CHCF₃)₂-(CH₂)₄-, - (CH₂)₆-(CHCF₃)₂-(CH₂)₂-, -(CH₂)₆-(CHCF₃)₂-(CH₂)₃-, -(CH₂)₆-(CHCF₃)₂-(CH₂)₄-, -(CHCF₃)₃-(CH₂)-, -(CH₂)-(CHCF₃)₃-, -(CH₂)-(CHCF₃)₃-(CH₂)-, -(CH₂)-(CHCF₃)₃-(CH₂)₂-, -(CH₂)-(CHCF₃)₃-(CH₂)₃-, -(CH₂)-(CHCF₃)₃-(CH₂)₄-, - (CH₂)-(CHCF₃)₃-(CH₂)₅-, -(CH₂)-(CHCF₃)₃-(CH₂)₆-, -(CH₂)-(CHCF₃)₃-(CH₂)₇- , -(CH₂)-(CHCF₃)₃-(CH₂)₆-, -(CH₂)₂-(CHCF₃)₃-(CH₂)-, -(CH₂)₃-(CHCF₃)₃-(CH₂)-, -(CH₂)₄-(CHCF₃)₃-(CH₂)-, -(CH₂)₅-(CHCF₃)₃-(CH₂)-, -(CH₂)₆-(CHCF₃)₃-(CH₂)-, -(CH₂)₇-(CHCF₃)₃-(CH₂)-, -(CH₂)₈-(CHCF₃)₃-(CH₂)-, - (CH₂)₂-(CHCF₃)₃-(CH₂)₂-, -(CH₂)₃-(CHCF₃)₃-(CH₂)₃-, -(CH₂)₄-(CHCF₃)₃-(CH₂)₄-, -(CH₂)₂-(CHCF₃)₃-(CH₂)₃-, -(CH₂)₂-(CHCF₃)₃-(CH₂)₄-, -(CH₂)₂-(CHCF₃)₃-(CH₂)₅-, -(CH₂)₂-(CHCF₃)₃-(CH₂)₆-, -(CH₂)₂-(CHCF₃)₃-(CH₂)₇-, - (CH₂)₃-(CHCF₃)₃-(CH₂)₂-, -(CH₂)₃-(CHCF₃)₃-(CH₂)₄-, -(CH₂)₃-(CHCF₃)₃-(CH₂)₅-, -(CH₂)₃-(CHCF₃)₃-(CH₂)₆-, -(CH₂)₄-(CHCF₃)₃-(CH₂)₂-, -(CH₂)₄-(CHCF₃)₃-(CH₂)₃-, -(CH₂)₄-(CHCF₃)₃-(CH₂)₅-, -(CH₂)₅-(CHCF₃)₃-(CH₂)₂-, - (CH₂)₅-(CHCF₃)₃-(CH₂)₃-, -(CH₂)₅-(CHCF₃)₃-(CH₂)₄-, -(CH₂)₆-(CHCF₃)₃-(CH₂)₂-, -(CH₂)₆-(CHCF₃)₃-(CH₂)₃-, -(CHCF₃)₄-(CH₂)-, -(CH₂)-(CHCF₃)₄-, -(CH₂)-(CHCF₃)₄-(CH₂)-, -(CH₂)-(CHCF₃)₄-(CH₂)₂-, -(CH₂)-(CHCF₃)₄-(CH₂)₃-, -(CH₂)-(CHCF₃)₄-(CH₂)₄-, - (CH₂)-(CHCF₃)₄-(CH₂)₅-, -(CH₂)-(CHCF₃)₄-(CH₂)₆-, -(CH₂)-(CHCF₃)₄-(CH₂)₇- , -(CH₂)-(CHCF₃)₄-(CH₂)₈-, -(CH₂)-(CHCF₃)₄-(CH₂)₉-, -(CH₂)-(CHCF₃)₄-(CH₂)₁₀-, -(CH₂)₂-(CHCF₃)₄-(CH₂)-, -(CH₂)₃-(CHCF₃)₄-(CH₂)-, -(CH₂)₄-(CHCF₃)₄-(CH₂)-, -(CH₂)₅-(CHCF₃)₄-(CH₂)-, -(CH₂)₆-(CHCF₃)₄-(CH₂)-, - (CH₂)₇-(CHCF₃)₄-(CH₂)-, -(CH₂)₂-(CHCF₃)₄-(CH₂)₂-, -(CH₂)₃-(CHCF₃)₄-(CH₂)₃-, -(CH₂)₄-(CHCF₃)₄-(CH₂)₄-, -(CH₂)₅-(CHCF₃)₄-(CH₂)₅-, -(CH₂)₂-(CHCF₃)₄-(CH₂)₃-, -(CH₂)₂-(CHCF₃)₄-(CH₂)₄-, -(CH₂)₂-(CHCF₃)₄-(CH₂)₅-, - (CH₂)₂-(CHCF₃)₄-(CH₂)₆-, -(CH₂)₃-(CHCF₃)₄-(CH₂)₂-, -(CH₂)₃-(CHCF₃)₄-(CH₂)₄-, -(CH₂)₄-(CHCF₃)₄-(CH₂)₂-, -(CH₂)₄-(CHCF₃)₄-(CH₂)₃-, -(CH₂)₅-(CHCF₃)₄-(CH₂)₂-, -(CH₂)₅-(CHCF₃)₄-(CH₂)₃-, -(CH₂)₆-(CHCF₃)₄-(CH₂)₂-, -(CHCF₃)₅-(CH₂)-, -(CH₂)-(CHCF₃)₅-, -(CH₂)-(CHCF₃)₅-(CH₂)-, -(CH₂)-(CHCF₃)₅-(CH₂)₂-, -(CH₂)-(CHCF₃)₅-(CH₂)₃-, -(CH₂)-(CHCF₃)₅-(CH₂)₄-, - (CH₂)-(CHCF₃)₅-(CH₂)₅-, -(CH₂)-(CHCF₃)₅-(CH₂)₆-, -(CH₂)₂-(CHCF₃)₅-(CH₂)-, -(CH₂)₃-(CHCF₃)₅-(CH₂)-, -(CH₂)₄-(CHCF₃)₅-(CH₂)-, -(CH₂)₅-(CHCF₃)₅-(CH₂)-, -(CH₂)₆-(CHCF₃)₅-(CH₂)-, -(CH₂)₂-(CHCF₃)₅-(CH₂)₂-, -(CH₂)₃-(CHCF₃)₅-(CH₂)₃-, -(CH₂)₄-(CHCF₃)₅-(CH₂)₄-, -(CH₂)₂-(CHCF₃)₅-(CH₂)₃-, - (CH₂)₂-(CHCF₃)₅-(CH₂)₄-, -(CH₂)₂-(CHCF₃)₅-(CH₂)₅-, -(CH₂)₂-(CHCF₃)₅-(CH₂)₆-, -(CH₂)₃-(CHCF₃)₅-(CH₂)₂-, -(CH₂)₃-(CHCF₃)₅-(CH₂)₄-, -(CH₂)₄-(CHCF₃)₅-(CH₂)₂-, -(CH₂)₄-(CHCF₃)₅-(CH₂)₃-, -(CH₂)₅-(CHCF₃)₅-(CH₂)₂-, -[C(CH₃)CF₃]-(CH₂)-, -(CH₂)-[C(CH₃)CF₃]-, -(CH₂)-[C(CH₃)CF₃]-(CH₂)-, - (CH₂)-[C(CH₃)CF₃]-(CH₂)₂-, -(CH₂)-[C(CH₃)CF₃]-(CH₂)₃-, -(CH₂)-[C(CH₃)CF₃]-(CH₂)₄-, -(CH₂)-[C(CH₃)CF₃]-(CH₂)₅-, -(CH₂)-[C(CH₃)CF₃]-(CH₂)₆-, -(CH₂)-[C(CH₃)CF₃]-(CH₂)₇-, -(CH₂)-[C(CH₃)CF₃]-(CH₂)₈-, -(CH₂)-[C(CH₃)CF₃]-(CH₂)₉-, -(CH₂)-[C(CH₃)CF₃]-(CH₂)₁₀-, -(CH₂)₂-[C(CH₃)CF₃]-(CH₂)-, -(CH₂)₃-[C(CH₃)CF₃]-(CH₂)-, -(CH₂)₄-[C(CH₃)CF₃]-(CH₂)-, -(CH₂)₅-[C(CH₃)CF₃]-(CH₂)-, -(CH₂)₆-[C(CH₃)CF₃]-(CH₂)-, -(CH₂)₇-[C(CH₃)CF₃]-(CH₂)-, -(CH₂)₈-[C(CH₃)CF₃]-(CH₂)-, -(CH₂)₉-[C(CH₃)CF₃]-(CH₂)-, -(CH₂)₁₀-[C(CH₃)CF₃]-(CH₂)-, -(CH₂)₂-[C(CH₃)CF₃]-(CH₂)₂-, -(CH₂)₃-[C(CH₃)CF₃]-(CH₂)₃-, -(CH₂)₄-[C(CH₃)CF₃]-(CH₂)₄-, -(CH₂)₅-[C(CH₃)CF₃]-(CH₂)₅-, -(CH₂)₂-[C(CH₃)CF₃]-(CH₂)₃-, -(CH₂)₂-[C(CH₃)CF₃]-(CH₂)₄-, -(CH₂)₂-[C(CH₃)CF₃]-(CH₂)₅-, -(CH₂)₂-[C(CH₃)CF₃]-(CH₂)₆-, -(CH₂)₂-[C(CH₃)CF₃]-(CH₂)₇-, -(CH₂)₂-[C(CH₃)CF₃]-(CH₂)₈-, -(CH₂)₂-[C(CH₃)CF₃]-(CH₂)₉-, -(CH₂)₃-[C(CH₃)CF₃]-(CH₂)₂-, -(CH₂)₃-[C(CH₃)CF₃]-(CH₂)₄-, -(CH₂)₃-[C(CH₃)CF₃]-(CH₂)₅-, -(CH₂)₃-[C(CH₃)CF₃]-(CH₂)₆-, -(CH₂)₃-[C(CH₃)CF₃]-(CH₂)₇-, -(CH₂)₃-[C(CH₃)CF₃]-(CH₂)₈-, -(CH₂)₄-[C(CH₃)CF₃]-(CH₂)₂-, -(CH₂)₄-[C(CH₃)CF₃]-(CH₂)₃-, -(CH₂)₄-[C(CH₃)CF₃]-(CH₂)₅-, -(CH₂)₄-[C(CH₃)CF₃]-(CH₂)₈-, -(CH₂)₄-[C(CH₃)CF₃]-(CH₂)₇-, -(CH₂)₅-[C(CH₃)CF₃]-(CH₂)₂-, -(CH₂)₅-[C(CH₃)CF₃]-(CH₂)₃-, -(CH₂)₅-[C(CH₃)CF₃]-(CH₂)₄-, -(CH₂)₅-[C(CH₃)CF₃]-(CH₂)₆-, -(CH₂)₆-[C(CH₃)CF₃]-(CH₂)₂-, -(CH₂)₆-[C(CH₃)CF₃]-(CH₂)₃-, -(CH₂)₆-[C(CH₃)CF₃]-(CH₂)₄-, -(CH₂)₆-[C(CH₃)CF₃]-(CH₂)₅-, -[C(CH₃)CF₃]₂-(CH₂)-, -(CH₂)-[C(CH₃)CF₃]₂-, -(CH₂)-[C(CH₃)CF₃]₂-(CH₂)-, - (CH₂)-[C(CH₃)CF₃]₂-(CH₂)₂-, -(CH₂)-[C(CH₃)CF₃]₂-(CH₂)₃-, -(CH₂)-[C(CH₃)CF₃]₂-(CH₂)₄-, -(CH₂)-[C(CH₃)CF₃]₂-(CH₂)₅-, -(CH₂)-[C(CH₃)CF₃]₂-(CH₂)₆-, -(CH₂)-[C(CH₃)CF₃]₂-(CH₂)₇-, -(CH₂)-[C(CH₃)CF₃]₂-(CH₂)₈-, -(CH₂)-[C(CH₃)CF₃]₂-(CH₂)₉-, -(CH₂)₂-[C(CH₃)CF₃]₂-(CH₂)-, -(CH₂)₃-[C(CH₃)CF₃]₂-(CH₂)-, -(CH₂)₄-[C(CH₃)CF₃]₂-(CH₂)-, -(CH₂)₅-[C(CH₃)CF₃]₂-(CH₂)-, -(CH₂)₆-[C(CH₃)CF₃]₂-(CH₂)-, -(CH₂)₇-[C(CH₃)CF₃]₂-(CH₂)-, -(CH₂)₈-[C(CH₃)CF₃]₂-(CH₂)-, -(CH₂)₉-[C(CH₃)CF₃]₂-(CH₂)-, -(CH₂)₂-[C(CH₃)CF₃]₂-(CH₂)₂-, -(CH₂)₃-[C(CH₃)CF₃]₂-(CH₂)₃-, -(CH₂)₄-[C(CH₃)CF₃]₂-(CH₂)₄-, -(CH₂)₅-[C(CH₃)CF₃]₂-(CH₂)₅-, -(CH₂)₂-[C(CH₃)CF₃]₂-(CH₂)₃-, -(CH₂)₂-[C(CH₃)CF₃]₂-(CH₂)₄-, - (CH₂)₂-[C(CH₃)CF₃]₂-(CH₂)₅-, -(CH₂)₂-[C(CH₃)CF₃]₂-(CH₂)₆-, -(CH₂)₂-[C(CH₃)CF₃]₂-(CH₂)₇-, -(CH₂)₂-[C(CH₃)CF₃]₂-(CH₂)₈-, -(CH₂)₃-[C(CH₃)CF₃]₂-(CH₂)₂-, -(CH₂)₃-[C(CH₃)CF₃]₂-(CH₂)₄-, -(CH₂)₃-[C(CH₃)CF₃]₂-(CH₂)₅-, - (CH₂)₃-[C(CH₃)CF₃]₂-(CH₂)₆-, -(CH₂)₃-[C(CH₃)CF₃]₂-(CH₂)₇-, -(CH₂)₄-[C(CH₃)CF₃]₂-(CH₂)₂-, -(CH₂)₄-[C(CH₃)CF₃]₂-(CH₂)₃-, -(CH₂)₄-[C(CH₃)CF₃]₂-(CH₂)₅-, -(CH₂)₄-[C(CH₃)CF₃]₂-(CH₂)₆-, -(CH₂)₅-[C(CH₃)CF₃]₂-(CH₂)₂-, - (CH₂)₅-[C(CH₃)CF₃]₂-(CH₂)₃-, -(CH₂)₅-[C(CH₃)CF₃]₂-(CH₂)₄-, -(CH₂)₆-[C(CH₃)CF₃]₂-(CH₂)₂-, -(CH₂)₆-[C(CH₃)CF₃]₂-(CH₂)₃-, -(CH₂)₆-[C(CH₃)CF₃]₂-(CH₂)₄-, -[C(CH₃)CF₃]₃-(CH₂)-, -(CH₂)-[C(CH₃)CF₃]₃-, -(CH₂)-[C(CH₃)CF₃]₃-(CH₂)-, - (CH₂)-[C(CH₃)CF₃]₃-(CH₂)₂-, -(CH₂)-[C(CH₃)CF₃]₃-(CH₂)₃-, -(CH₂)-[C(CH₃)CF₃]₃-(CH₂)₄-, -(CH₂)-[C(CH₃)CF₃]₃-(CH₂)₅-, -(CH₂)-[C(CH₃)CF₃]₃-(CH₂)₆-, -(CH₂)-[C(CH₃)CF₃]₃-(CH₂)₇-, -(CH₂)-[C(CH₃)CF₃]₃-(CH₂)₈-, -(CH₂)₂-[C(CH₃)CF₃]₃-(CH₂)-, -(CH₂)₃-[C(CH₃)CF₃]₃-(CH₂)-, -(CH₂)₄-[C(CH₃)CF₃]₃-(CH₂)-, -(CH₂)₅-[C(CH₃)CF₃]₃-(CH₂)-, -(CH₂)₆-[C(CH₃)CF₃]₃-(CH₂)-, -(CH₂)₇-[C(CH₃)CF₃]₃-(CH₂)-, -(CH₂)₈-[C(CH₃)CF₃]₃-(CH₂)-, -(CH₂)₂-[C(CH₃)CF₃]₃-(CH₂)₂-, -(CH₂)₃-[C(CH₃)CF₃]₃-(CH₂)₃-, -(CH₂)₄-[C(CH₃)CF₃]₃-(CH₂)₄-, - (CH₂)₂-[C(CH₃)CF₃]₃-(CH₂)₃-, -(CH₂)₂-[C(CH₃)CF₃]₃-(CH₂)₄-, -(CH₂)₂-[C(CH₃)CF₃]₃-(CH₂)₅-, -(CH₂)₂-[C(CH₃)CF₃]₃-(CH₂)₆-, -(CH₂)₂-[C(CH₃)CF₃]₃-(CH₂)₇-, -(CH₂)₃-[C(CH₃)CF₃]₃-(CH₂)₂-, -(CH₂)₃-[C(CH₃)CF₃]₃-(CH₂)₄-, - (CH₂)₃-[C(CH₃)CF₃]₃-(CH₂)₅-, -(CH₂)₃-[C(CH₃)CF₃]₃-(CH₂)₆-, -(CH₂)₄-[C(CH₃)CF₃]₃-(CH₂)₂-, -(CH₂)₄-[C(CH₃)CF₃]₃-(CH₂)₃-, -(CH₂)₄-[C(CH₃)CF₃]₃-(CH₂)₅-, -(CH₂)₅-[C(CH₃)CF₃]₃-(CH₂)₂-, -(CH₂)₅-[C(CH₃)CF₃]₃-(CH₂)₃-, - (CH₂)₅-[C(CH₃)CF₃]₃-(CH₂)₄-, -(CH₂)₆-[C(CH₃)CF₃]₃-(CH₂)₂-, -(CH₂)₆-[C(CH₃)CF₃]₃-(CH₂)₃-, -[C(CH₃)CF₃]₄-(CH₂)-, -(CH₂)-[C(CH₃)CF₃]₄-, -(CH₂)-[C(CH₃)CF₃]₄-(CH₂)-, - (CH₂)-[C(CH₃)CF₃]₄-(CH₂)₂-, -(CH₂)-[C(CH₃)CF₃]₄-(CH₂)₃-, -(CH₂)-[C(CH₃)CF₃]₄-(CH₂)₄-, -(CH₂)-[C(CH₃)CF₃]₄-(CH₂)₅-, -(CH₂)-[C(CH₃)CF₃]₄-(CH₂)₆-, -(CH₂)-[C(CH₃)CF₃]₄-(CH₂)₇-, -(CH₂)-[C(CH₃)CF₃]₄-(CH₂)₈-, -(CH₂)-[C(CH₃)CF₃]₄-(CH₂)₉-, -(CH₂)-[C(CH₃)CF₃]₄-(CH₂)₁₀-, -(CH₂)₂-[C(CH₃)CF₃]₄-(CH₂)-, -(CH₂)₃-[C(CH₃)CF₃]₄-(CH₂)-, -(CH₂)₄-[C(CH₃)CF₃]₄-(CH₂)-, -(CH₂)₅-[C(CH₃)CF₃]₄-(CH₂)-, -(CH₂)₆-[C(CH₃)CF₃]₄-(CH₂)-, -(CH₂)₇-[C(CH₃)CF₃]₄-(CH₂)-, -(CH₂)₂-[C(CH₃)CF₃]₄-(CH₂)₂-, -(CH₂)₃-[C(CH₃)CF₃]₄-(CH₂)₃-, - (CH₂)₄-[C(CH₃)CF₃]₄-(CH₂)₄-, -(CH₂)₅-[C(CH₃)CF₃]₄-(CH₂)₅-, -(CH₂)₂-[C(CH₃)CF₃]₄-(CH₂)₃-, -(CH₂)₂-[C(CH₃)CF₃]₄-(CH₂)₄-, -(CH₂)₂-[C(CH₃)CF₃]₄-(CH₂)₅-, -(CH₂)₂-[C(CH₃)CF₃]₄-(CH₂)₆-, -(CH₂)₃-[C(CH₃)CF₃]₄-(CH₂)₂-, - (CH₂)₃-[C(CH₃)CF₃]₄-(CH₂)₄-, -(CH₂)₄-[C(CH₃)CF₃]₄-(CH₂)₂-, -(CH₂)₄-[C(CH₃)CF₃]₄-(CH₂)₃-, -(CH₂)₅-[C(CH₃)CF₃]₄-(CH₂)₂-, -(CH₂)₅-[C(CH₃)CF₃]₄-(CH₂)₃-, -(CH₂)₆-[C(CH₃)CF₃]₄-(CH₂)₂-, -[C(CH₃)CF₃]₅-(CH₂)-, -(CH₂)-[C(CH₃)CF₃]₅-, -(CH₂)-[C(CH₃)CF₃]₅-(CH₂)-, - (CH₂)-[C(CH₃)CF₃]₅-(CH₂)₂-, -(CH₂)-[C(CH₃)CF₃]₅-(CH₂)₃-, -(CH₂)-[C(CH₃)CF₃]₅-(CH₂)₄-, -(CH₂)-[C(CH₃)CF₃]₅-(CH₂)₅-, -(CH₂)-[C(CH₃)CF₃]₅-(CH₂)₆-, -(CH₂)₂-[C(CH₃)CF₃]₅-(CH₂)-, -(CH₂)₃-[C(CH₃)CF₃]₅-(CH₂)-, -(CH₂)₄-[C(CH₃)CF₃]₅-(CH₂)-, -(CH₂)₅-[C(CH₃)CF₃]₅-(CH₂)-, -(CH₂)₆-[C(CH₃)CF₃]₅-(CH₂)-, -(CH₂)₂-[C(CH₃)CF₃]₅-(CH₂)₂-, -(CH₂)₃-[C(CH₃)CF₃]₅-(CH₂)₃-, - (CH₂)₄-[C(CH₃)CF₃]₅-(CH₂)₄-, -(CH₂)₂-[C(CH₃)CF₃]₅-(CH₂)₃-, -(CH₂)₂-[C(CH₃)CF₃]₅-(CH₂)₄-, -(CH₂)₂-[C(CH₃)CF₃]₅-(CH₂)₅-, -(CH₂)₂-[C(CH₃)CF₃]₅-(CH₂)₆-, -(CH₂)₃-[C(CH₃)CF₃]₅-(CH₂)₂-, -(CH₂)₃-[C(CH₃)CF₃]₅-(CH₂)₄-, - (CH₂)₄-[C(CH₃)CF₃]₅-(CH₂)₂-, -(CH₂)₄-[C(CH₃)CF₃]₅-(CH₂)₃-, -(CH₂)₅-[C(CH₃)CF₃]₅-(CH₂)₂-, -[CH(CH₂CF₃)]-(CH₂)-, -(CH₂)-[CH(CH₂CF₃)]-, -(CH₂)-[CH(CH₂CF₃)]-(CH₂)-, -(CH₂)-[CH(CH₂CF₃)]-(CH₂)₂-, -(CH₂)-[CH(CH₂CF₃)] -(CH₂)₃-, -(CH₂)-[CH(CH₂CF₃)]-(CH₂)₄-, -(CH₂)-[CH(CH₂CF₃)]-(CH₂)₅-, -(CH₂)-[CH(CH₂CF₃)]-(CH₂)₆-, -(CH₂)-[CH(CH₂CF₃)]-(CH₂)₇-, -(CH₂)-[CH(CH₂CF₃)]-(CH₂)₈-, - (CH₂)-[CH(CH₂CF₃)]-(CH₂)₉-, -(CH₂)-[CH(CH₂CF₃)]-(CH₂)₁₀-, -(CH₂)₂-[CH(CH₂CF₃)]-(CH₂)-, -(CH₂)₃-[CH(CH₂CF₃)]-(CH₂)-, -(CH₂)₄-[CH(CH₂CF₃)]-(CH₂)-, -(CH₂)₅-[CH(CH₂CF₃)]-(CH₂)-, -(CH₂)₆-[CH(CH₂CF₃)]-(CH₂)-, - (CH₂)₇-[CH(CH₂CF₃)]-(CH₂)-, -(CH₂)₈-[CH(CH₂CF₃)]-(CH₂)-, -(CH₂)₉-[CH(CH₂CF₃)]-(CH₂)-, -(CH₂)₁₀-[CH(CH₂CF₃)]-(CH₂)-, -(CH₂)₂-[CH(CH₂CF₃)]-(CH₂)₂-, -(CH₂)₃-[CH(CH₂CF₃)]-(CH₂)₃-, -(CH₂)₄-[CH(CH₂CF₃)]-(CH₂)₄-, -(CH₂)₅-[CH(CH₂CF₃)]-(CH₂)₅-, -(CH₂)₂-[CH(CH₂CF₃)]-(CH₂)₃-, -(CH₂)₂-[CH(CH₂CF₃)]-(CH₂)₄-, -(CH₂)₂-[CH(CH₂CF₃)]-(CH₂)₅-, -(CH₂)₂-[CH(CH₂CF₃)]-(CH₂)₆-, -(CH₂)₂-[CH(CH₂CF₃)]-(CH₂)₇-, -(CH₂)₂-[CH(CH₂CF₃)]-(CH₂)₈-, -(CH₂)₂-[CH(CH₂CF₃)]-(CH₂)₉-, -(CH₂)₃-[CH(CH₂CF₃)]-(CH₂)₂-, -(CH₂)₃-[CH(CH₂CF₃)]-(CH₂)₄-, -(CH₂)₃-[CH(CH₂CF₃)]-(CH₂)₅-, -(CH₂)₃-[CH(CH₂CF₃)]-(CH₂)₆-, -(CH₂)₃-[CH(CH₂CF₃)]-(CH₂)₇-, -(CH₂)₃-[CH(CH₂CF₃)]-(CH₂)₈-, -(CH₂)₄-[CH(CH₂CF₃)]-(CH₂)₂-, -(CH₂)₄-[CH(CH₂CF₃)]-(CH₂)₃-, -(CH₂)₄-[CH(CH₂CF₃)]-(CH₂)₅-, -(CH₂)₄-[CH(CH₂CF₃)]-(CH₂)₆-, -(CH₂)₄-[CH(CH₂CF₃)]-(CH₂)₇-, -(CH₂)₅-[CH(CH₂CF₃)]-(CH₂)₂-, -(CH₂)₅-[CH(CH₂CF₃)]-(CH₂)₃-, -(CH₂)₅-[CH(CH₂CF₃)]-(CH₂)₄-, -(CH₂)₅-[CH(CH₂CF₃)]-(CH₂)₆-, -(CH₂)₆-[CH(CH₂CF₃)]-(CH₂)₂-, -(CH₂)₆-[CH(CH₂CF₃)]-(CH₂)₃-, -(CH₂)₆-[CH(CH₂CF₃)]-(CH₂)₄-, -(CH₂)₆-[CH(CH₂CF₃)]-(CH₂)₅-, -[CH(CH₂CF₃)]₂-(CH₂)-, -(CH₂)-[CH(CH₂CF₃)]₂-, -(CH₂)-[CH(CH₂CF₃)]₂-(CH₂)-, -(CH₂)-[CH(CH₂CF₃)]₂-(CH₂)₂-, -(CH₂)-[CH(CH₂CF₃)]₂-(CH₂)₃-, - (CH₂)-[CH(CH₂CF₃)]₂-(CH₂)₄-, -(CH₂)-[CH(CH₂CF₃)]₂-(CH₂)₅-, -(CH₂)-[CH(CH₂CF₃)]₂-(CH₂)₆-, -(CH₂)-[CH(CH₂CF₃)₂-(CH₂)₇-, -(CH₂)-[CH(CH₂CF₃)]₂-(CH₂)₈-, -(CH₂)-[CH(CH₂CF₃)]₂-(CH₂)₉-, -(CH₂)₂-[CH(CH₂CF₃)]₂-(CH₂)-, -(CH₂)₃-[CH(CH₂CF₃)]₂-(CH₂)-, -(CH₂)₄-[CH(CH₂CF₃)]₂-(CH₂)-, -(CH₂)₅-[CH(CH₂CF₃)]₂-(CH₂)-, -(CH₂)₆-[CH(CH₂CF₃)]₂-(CH₂)-, -(CH₂)_{?}-[CH(CH₂CF₃)]₂-(CH₂)-, -(CH₂)₈-[CH(CH₂CF₃)]₂-(CH₂)-, -(CH₂)₉-[CH(CH₂CF₃)]₂-(CH₂)-, -(CH₂)₂-[CH(CH₂CF₃)]₂-(CH₂)₂-, -(CH₂)₃-[CH(CH₂CF₃)]₂-(CH₂)₃-, -(CH₂)₄-[CH(CH₂CF₃)]₂-(CH₂)₄-, -(CH₂)₅-[CH(CH₂CF₃)]₂-(CH₂)₅-, -(CH₂)₂-[CH(CH₂CF₃)]₂-(CH₂)₃-, -(CH₂)₂-[CH(CH₂CF₃)]₂-(CH₂)₄-, -(CH₂)₂-[CH(CH₂CF₃)]₂-(CH₂)₅-, -(CH₂)₂-[CH(CH₂CF₃)]₂-(CH₂)₆-, -(CH₂)₂-[CH(CH₂CF₃)]₂-(CH₂)₇-, -(CH₂)₂-[CH(CH₂CF₃)]₂-(CH₂)₈-, -(CH₂)₃-[CH(CH₂CF₃)]₂-(CH₂)₂-, -(CH₂)₃-[CH(CH₂CF₃)]₂-(CH₂)₄-, -(CH₂)₃-[CH(CH₂CF₃)]₂-(CH₂)₅-, -(CH₂)₃-[CH(CH₂CF₃)]₂-(CH₂)₆-, -(CH₂)₃-[CH(CH₂CF₃)]₂-(CH₂)₇-, -(CH₂)₄-[CH(CH₂CF₃)]₂-(CH₂)₂-, -(CH₂)₄-[CH(CH₂CF₃)]₂-(CH₂)₃-, -(CH₂)₄-[CH(CH₂CF₃)]₂-(CH₂)₅-, -(CH₂)₄-[CH(CH₂CF₃)]₂-(CH₂)₆-, -(CH₂)_{S}-[CH(CH₂CF₃)]₂-(CH₂)₂-, -(CH₂)₅-[CH(CH₂CF₃)]₂-(CH₂)₃-, -(CH₂)₅-[CH(CH₂CF₃)]₂-(CH₂)₄-, -(CH₂)₆-[CH(CH₂CF₃)]₂-(CH₂)₂-, -(CH₂)₆-[CH(CH₂CF₃)]₂-(CH₂)₃-, -(CH₂)₆-[CH(CH_{2C}F₃)]₂-(CH₂)₄-, -[CH(CH₂CF₃)]₃-(CH₂)-, -(CH₂)-[CH(CH₂CF₃)]₃-, -(CH₂)-[CH(CH₂CF₃)]₃-(CH₂)-, -(CH₂)-[CH(CH₂CF₃)]₃-(CH₂)₂-, -(CH₂)-[CH(CH₂CF₃)]₃-(CH₂)₃-, - (CH₂)-[CH(CH₂CF₃)]₃-(CH₂)₄-, -(CH₂)-[CH(CH₂CF₃)]₃-(CH₂)₅-, -(CH₂)-[CH(CH₂CF₃)]₃-(CH₂)₆-, -(CH₂)-[CH(CH₂CF₃)]₃-(CH₂)₇-, -(CH₂)-[CH(CH₂CF₃)]₃-(CH₂)₈-, -(CH₂)₂-[CH(CH₂CF₃)]₃-(CH₂)-, -(CH₂)₃-[CH(CH₂CF₃)]₃-(CH₂)-, -(CH₂)₄-[CH(CH₂CF₃)]₃-(CH₂)-, -(CH₂)₅-[CH(CH₂CF₃)]₃-(CH₂)-, -(CH₂)₆-[CH(CH₂CF₃)]₃-(CH₂)-, -(CH₂)₇-[CH(CH₂CF₃)]₃-(CH₂)-, -(CH₂)₈-[CH(CH₂CF₃)]₃-(CH₂)-, -(CH₂)₂-[CH(CH₂CF₃)]₃-(CH₂)₂-, -(CH₂)₃-[CH(CH₂CF₃)]₃-(CH₂)₃-, -(CH₂)₄-[CH(CH₂CF₃)]₃-(CH₂)₄-, -(CH₂)₂-[CH(CH₂CF₃)]₃-(CH₂)₃-, -(CH₂)₂-[CH(CH₂CF₃)]₃-(CH₂)₄-, -(CH₂)₂-[CH(CH₂CF₃)]₃-(CH₂)₅-, -(CH₂)₂-[CH(CH₂CF₃)]₃-(CH₂)₆-, -(CH₂)₂-[CH(CH₂CF₃)]₃-(CH₂)₇-, -(CH₂)₃-[CH(CH₂CF₃)]₃-(CH₂)₂-, -(CH₂)₃-[CH(CH₂CF₃)]₃-(CH₂)₄-, -(CH₂)₃-[CH(CH₂CF₃)]₃-(CH₂)₅-, -(CH₂)₃-[CH(CH₂CF₃)]₃-(CH₂)₆-, -(CH₂)₄-[CH(CH₂CF₃)]₃-(CH₂)₂-, -(CH₂)₄-[CH(CH₂CF₃)]₃-(CH₂)₃-, -(CH₂)₄-[CH(CH₂CF₃)]₃-(CH₂)₅-, -(CH₂)₅-[CH(CH₂CF₃)]₃-(CH₂)₂-, -(CH₂)₅-[CH(CH₂CF₃)]₃-(CH₂)₃-, -(CH₂)₅-[CH(CH₂CF₃)]₃-(CH₂)₄-, -(CH₂)₆-[CH(CH₂CF₃)]₃-(CH₂)₂-, -(CH₂)₆-[CH(CH₂CF₃)]₃-(CH₂)₃-, -[CH(CH₂CF₃)]₄-(CH₂)-, -(CH₂)-[CH(CH₂CF₃)]₄-, -(CH₂)-[CH(CH₂CF₃)]₄-(CH₂)-, -(CH₂)-[CH(CH₂CF₃)]₄-(CH₂)₂-, -(CH₂)-[CH(CH₂CF₃)]₄-(CH₂)₃-, - (CH₂)-[CH(CH₂CF₃)]₄-(CH₂)₄-, -(CH₂)-[CH(CH₂CF₃)]₄-(CH₂)₅-, -(CH₂)-[CH(CH₂CF₃)]₄-(CH₂)₆-, -(CH₂)-[CH(CH₂CF₃)]₄-(CH₂)₇-, -(CH₂)-[CH(CH₂CF₃)]₄-(CH₂)₈-, -(CH₂)-[CH(CH₂CF₃)]₄-(CH₂)₉-, -(CH₂)-[CH(CH₂CF₃)]₄-(CH₂)₁₀-, -(CH₂)₂-[CH(CH₂CF₃)]₄-(CH₂)-, -(CH₂)₃-[CH(CH₂CF₃)]₄-(CH₂)-, -(CH₂)₄-[CH(CH₂CF₃)]₄-(CH₂)-, -(CH₂)₅-[CH(CH₂CF₃)]₄-(CH₂)-, -(CH₂)₆-[CH(CH₂CF₃)]₄-(CH₂)-, -(CH₂)₇-[CH(CH₂CF₃)]₄-(CH₂)-, -(CH₂)₂-[CH(CH₂CF₃)]₄-(CH₂)₂-, -(CH₂)₃-[CH(CH₂CF₃)]₄-(CH₂)₃-, -(CH₂)₄-[CH(CH₂CF₃)]₄-(CH₂)₄-, -(CH₂)₅-[CH(CH₂CF₃)]₄-(CH₂)₅-, -(CH₂)₂-[CH(CH₂CF₃)]₄-(CH₂)₃-, -(CH₂)₂-[CH(CH₂CF₃)]₄-(CH₂)₄-, -(CH₂)₂-[CH(CH₂CF₃)]₄-(CH₂)₅-, -(CH₂)₂-[CH(CH₂CF₃)]₄-(CH₂)₆-, -(CH₂)₃-[CH(CH₂CF₃)]₄-(CH₂)₂-, -(CH₂)₃-[CH(CH₂CF₃)]₄-(CH₂)₄-, -(CH₂)₄-[CH(CH₂CF₃)]₄-(CH₂)₂-, -(CH₂)₄-[CH(CH₂CF₃)]₄-(CH₂)₃-, -(CH₂)₅-[CH(CH₂CF₃)]₄-(CH₂)₂-, -(CH₂)₅-[CH(CH₂CF₃)]₄-(CH₂)₃-, -(CH₂)₆-[CH(CH₂CF₃)]₄-(CH₂)₂-, -[CH(CH₂CF₃)]₅-(CH₂)-, -(CH₂)-[CH(CH₂CF₃)₅-, -(CH₂)-[CH(CH₂CF₃)]₅-(CH₂)-, -(CH₂)-[CH(CH₂CF₃)]₅-(CH₂)₂-, -(CH₂)-[CH(CH₂CF₃)]₅-(CH₂)₃-, - (CH₂)-[CH(CH₂CF₃)]₅-(CH₂)₄-, -(CH₂)-[CH(CH₂CF₃)]₅-(CH₂)₅-, -(CH₂)-[CH(CH₂CF₃)]₅-(CH₂)₆-, -(CH₂)₂-[CH(CH₂CF₃)]₅-(CH₂)-, -(CH₂)₃-[CH(CH₂CF₃)]₅-(CH₂)-, -(CH₂)₄-[CH(CH₂CF₃)]₅-(CH₂)-, -(CH₂)₅-[CH(CH₂CF₃)]₅-(CH₂)-, -(CH₂)₆-[CH(CH₂CF₃)]₅-(CH₂)-, -(CH₂)₂-[CH(CH₂CF₃)]₅-(CH₂)₂-, -(CH₂)₃-[CH(CH₂CF₃)]₅-(CH₂)₃-, -(CH₂)₄-[CH(CH₂CF₃)]₅-(CH₂)₄-, -(CH₂)₂-[CH(CH₂CF₃)]₅-(CH₂)₃-, -(CH₂)₂-[CH(CH₂CF₃)]₅-(CH₂)₄-, -(CH₂)₂-[CH(CH₂CF₃)]₅-(CH₂)₅-, -(CH₂)₂-[CH(CH₂CF₃)]₅-(CH₂)₆-, -(CH₂)₃-[CH(CH₂CF₃)]₅-(CH₂)₂-, -(CH₂)₃-[CH(CH₂CF₃)]₅-(CH₂)₄-, -(CH₂)₄-[CH(CH₂CF₃)]₅-(CH₂)₂-, -(CH₂)₄-[CH(CH₂CF₃)]₅-(CH₂)₃-, -(CH₂)₅-[CH(CH₂CF₃)]₅-(CH₂)₂-, -[C(CH₃)(CH₂CF₃)]-(CH₂)-, -(CH₂)-[C(CH₃)(CH₂CF₃)]-, -(CH₂)-[C(CH₃)(CH₂CF₃)]-(CH₂)-, -(CH₂)-[C(CH₃)(CH₂CF₃)]-(CH₂)₂-, -(CH₂)-[C(CH₃)(CH₂CF₃)]-(CH₂)₃-, -(CH₂)-[C(CH₃)(CH₂CF₃)]-(CH₂)₄-, -(CH₂)-[C(CH₃)(CH₂CF₃)]-(CH₂)₅-, -(CH₂)-[C(CH₃)(CH₂CF₃)]-(CH₂)₆-, -(CH₂)-[C(CH₃)(CH₂CF₃)]-(CH₂)₇-, -(CH₂)-[C(CH₃)(CH₂CF₃)]-(CH₂)₈-, -(CH₂)-[C(CH₃)(CH₂CF₃)]-(CH₂)₉-, -(CH₂)-[C(CH₃)(CH₂CF₃)]-(CH₂)₁₀-, -(CH₂)₂-[C(CH₃)(CH₂CF₃)]-(CH₂)-, -(CH₂)₃-[C(CH₃)(CH₂CF₃)]-(CH₂)-, -(CH₂)₄-[C(CH₃)(CH₂CF₃)]-(CH₂)-, -(CH₂)₅-[C(CH₃)(CH₂CF₃)]-(CH₂)-, -(CH₂)₆-[C(CH₃)(CH₂CF₃)]-(CH₂)-, -(CH₂)₇-[C(CH₃)(CH₂CF₃)]-(CH₂)-, -(CH₂)₈-[C(CH₃)(CH₂CF₃)]-(CH₂)-, -(CH₂)₉-[C(CH₃)(CH₂CF₃)]-(CH₂)-, -(CH₂)₁₀-[C(CH₃)(CH₂CF₃)]-(CH₂)-, -(CH₂)₂-[C(CH₃)(CH₂CF₃)]-(CH₂)₂-, -(CH₂)₃-[C(CH₃)(CH₂CF₃)]-(CH₂)₃-, -(CH₂)₄-[C(CH₃)(CH₂CF₃)]-(CH₂)₄-, -(CH₂)₅-[C(CH₃)(CH₂CF₃)]-(CH₂)₅-, -(CH₂)₂-[C(CH₃)(CH₂CF₃)]-(CH₂)₃-, -(CH₂)₂-[C(CH₃)(CH₂CF₃)]-(CH₂)₄-, -(CH₂)₂-[C(CH₃)(CH₂CF₃)]-(CH₂)₅-, -(CH₂)₂-[C(CH₃)(CH₂CF₃)]-(CH₂)₆-, -(CH₂)₂-[C(CH₃)(CH₂CF₃)]-(CH₂)₇-, -(CH₂)₂-[C(CH₃)(CH₂CF₃)]-(CH₂)₈-, -(CH₂)₂-[C(CH₃)(CH₂CF₃)]-(CH₂)₉-, -(CH₂)₃-[C(CH₃)(CH₂CF₃)]-(CH₂)₂-, -(CH₂)₃-[C(CH₃)(CH₂CF₃)]-(CH₂)₄-, -(CH₂)₃-[C(CH₃)(CH₂CF₃)]-(CH₂)₅-, -(CH₂)₃-[C(CH₃)(CH₂CF₃)]-(CH₂)₆-, -(CH₂)₃-[C(CH₃)(CH₂CF₃)]-(CH₂)₇-, -(CH₂)₃-[C(CH₃)(CH₂CF₃)]-(CH₂)₈-, -(CH₂)₄-[C(CH₃)(CH₂CF₃)]-(CH₂)₂-, -(CH₂)₄-[C(CH₃)(CH₂CF₃)]-(CH₂)₃-, -(CH₂)₄-[C(CH₃)(CH₂CF₃)]-(CH₂)₅-, -(CH₂)₄-[C(CH₃)(CH₂CF₃)]-(CH₂)₆-, -(CH₂)₄-[C(CH₃)(CH₂CF₃)]-(CH₂)₇-, -(CH₂)₅-[C(CH₃)(CH₂CF₃)]-(CH₂)₂-, -(CH₂)₅-[C(CH₃)(CH₂CF₃)]-(CH₂)₃-, -(CH₂)₅-[C(CH₃)(CH₂CF₃)]-(CH₂)₄-, -(CH₂)₅-[C(CH₃)(CH₂CF₃)]-(CH₂)₆-, -(CH₂)₆-[C(CH₃)(CH₂CF₃)]-(CH₂)₂-, -(CH₂)₆-[C(CH₃)(CH₂CF₃)]-(CH₂)₃-, -(CH₂)₆-[C(CH₃)(CH₂CF₃)]-(CH₂)₄-, -(CH₂)₆-[C(CH₃)(CH₂CF₃)]-(CH₂)₅-, -[C(CH₃)(CH₂CF₃)]₂-(CH₂)-, -(CH₂)-[C(CH₃)(CH₂CF₃)]₂-, -(CH₂)-[C(CH₃)(CH₂CF₃)]₂-(CH₂)-, -(CH₂)-[C(CH₃)(CH₂CF₃)]₂-(CH₂)₂-, -(CH₂)-[C(CH₃)(CH₂CF₃)]₂-(CH₂)₃-, -(CH₂)-[C(CH₃)(CH₂CF₃)]₂-(CH₂)₄-, -(CH₂)-[C(CH₃)(CH₂CF₃)]₂-(CH₂)₅-, -(CH₂)-[C(CH₃)(CH₂CF₃)]₂-(CH₂)₆-, -(CH₂)-[C(CH₃)(CH₂CF₃)]₂-(CH₂)₇-, -(CH₂)-[C(CH₃)(CH₂CF₃)]₂-(CH₂)₈-, -(CH₂)-[C(CH₃)(CH₂CF₃)]₂-(CH₂)₉-, -(CH₂)₂-[C(CH₃)(CH₂CF₃)]₂-(CH₂)-, -(CH₂)₃-[C(CH₃)(CH₂CF₃)]₂-(CH₂)-, -(CH₂)₄-[C(CH₃)(CH₂CF₃)]₂-(CH₂)-, -(CH₂)₅-[C(CH₃)(CH₂CF₃)]₂-(CH₂)-, -(CH₂)₆-[C(CH₃)(CH₂CF₃)]₂-(CH₂)-, -(CH₂)₇-[C(CH₃)(CH₂CF₃)]₂-(CH₂)-, -(CH₂)₈-[C(CH₃)(CH₂CF₃)]₂-(CH₂)-, -(CH₂)₉-[C(CH₃)(CH₂CF₃)]₂-(CH₂)-, -(CH₂)₂-[C(CH₃)(CH₂CF₃)]₂-(CH₂)₂-, -(CH₂)₃-[C(CH₃)(CH₂CF₃)]₂-(CH₂)₃-, -(CH₂)₄-[C(CH₃)(CH₂CF₃)]₂-(CH₂)₄-, -(CH₂)₅-[C(CH₃)(CH₂CF₃)]₂-(CH₂)₅-, -(CH₂)₂-[C(CH₃)(CH₂CF₃)]₂-(CH₂)₃-, -(CH₂)₂-[C(CH₃)(CH₂CF₃)]₂-(CH₂)₄-, -(CH₂)₂-[C(CH₃)(CH₂CF₃)]₂-(CH₂)₅-, -(CH₂)₂-[C(CH₃)(CH₂CF₃)]₂-(CH₂)₆-, -(CH₂)₂-[C(CH₃)(CH₂CF₃)]₂-(CH₂)₇-, -(CH₂)₂-[C(CH₃)(CH₂CF₃)]₂-(CH₂)₈-, -(CH₂)₃-[C(CH₃)(CH₂CF₃)]₂-(CH₂)₂-, -(CH₂)₃-[C(CH₃)(CH₂CF₃)]₂-(CH₂)₄-, -(CH₂)₃-[C(CH₃)(CH₂CF₃)]₂-(CH₂)₅-, -(CH₂)₃-[C(CH₃)(CH₂CF₃)]₂-(CH₂)₆-, -(CH₂)₃-[C(CH₃)(CH₂CF₃)]₂-(CH₂)₇-, -(CH₂)₄-[C(CH₃)(CH₂CF₃)]₂-(CH₂)₂-, -(CH₂)₄-[C(CH₃)(CH₂CF₃)]₂-(CH₂)₃-, -(CH₂)₄-[C(CH₃)(CH₂CF₃)]₂-(CH₂)₅-, -(CH₂)₄-[C(CH₃)(CH₂CF₃)]₂-(CH₂)₆-, -(CH₂)₅-[C(CH₃)(CH₂CF₃)]₂-(CH₂)₂-, -(CH₂)₅-[C(CH₃)(CH₂CF₃)]₂-(CH₂)₃-, -(CH₂)₅-[C(CH₃)(CH₂CF₃)]₂-(CH₂)₄-, -(CH₂)₆-[C(CH₃)(CH₂CF₃)]₂-(CH₂)₂-, -(CH₂)₆-[C(CH₃)(CH₂CF₃)]₂-(CH₂)₃-, -(CH₂)₆-[C(CH₃)(CH₂CF₃)]₂-(CH₂)₄-, -[C(CH₃)(CH₂CF₃)]₃-(CH₂)-, -(CH₂)-[C(CH₃)(CH₂CF₃)]₃-, -(CH₂)-[C(CH₃)(CH₂CF₃)]₃-(CH₂)-, -(CH₂)-[C(CH₃)(CH₂CF₃)]₃-(CH₂)₂-, -(CH₂)-[C(CH₃)(CH₂CF₃)]₃-(CH₂)₃-, -(CH₂)-[C(CH₃)(CH₂CF₃)]₃-(CH₂)₄-, -(CH₂)-[C(CH₃)(CH₂CF₃)]₃-(CH₂)₅-, -(CH₂)-[C(CH₃)(CH₂CF₃)]₃-(CH₂)₆-, -(CH₂)-[C(CH₃)(CH₂CF₃)]₃-(CH₂)₇-, -(CH₂)-[C(CH₃)(CH₂CF₃)]₃-(CH₂)₈-, -(CH₂)₂-[C(CH₃)(CH₂CF₃)]₃-(CH₂)-, -(CH₂)₃-[C(CH₃)(CH₂CF₃)]₃-(CH₂)-, -(CH₂)₄-[C(CH₃)(CH₂CF₃)]₃-(CH₂)-, -(CH₂)₅-[C(CH₃)(CH₂CF₃)]₃-(CH₂)-, -(CH₂)₆-[C(CH₃)(CH₂CF₃)]₃-(CH₂)-, -(CH₂)₇-[C(CH₃)(CH₂CF₃)]₃-(CH₂)-, -(CH₂)₈-[C(CH₃)(CH₂CF₃)]₃-(CH₂)-, -(CH₂)₂-[C(CH₃)(CH₂CF₃)]₃-(CH₂)₂-, -(CH₂)₃-[C(CH₃)(CH₂CF₃)]₃-(CH₂)₃-, -(CH₂)₄-[C(CH₃)(CH₂CF₃)]₃-(CH₂)₄-, -(CH₂)₂-[C(CH₃)(CH₂CF₃)]₃-(CH₂)₃-, -(CH₂)₂-[C(CH₃)(CH₂CF₃)]₃-(CH₂)₄-, -(CH₂)₂-[C(CH₃)(CH₂CF₃)]₃-(CH₂)₅-, -(CH₂)₂-[C(CH₃)(CH₂CF₃)]₃-(CH₂)₆-, -(CH₂)₂-[C(CH₃)(CH₂CF₃)]₃-(CH₂)₇-, -(CH₂)₃-[C(CH₃)(CH₂CF₃)]₃-(CH₂)₂-, -(CH₂)₃-[C(CH₃)(CH₂CF₃)]₃-(CH₂)₄-, -(CH₂)₃-[C(CH₃)(CH₂CF₃)]₃-(CH₂)₅-, -(CH₂)₃-[C(CH₃)(CH₂CF₃)]₃-(CH₂)₆-, -(CH₂)₄-[C(CH₃)(CH₂CF₃)]₃-(CH₂)₂-, -(CH₂)₄-[C(CH₃)(CH₂CF₃)]₃-(CH₂)₃-, -(CH₂)₄-[C(CH₃)(CH₂CF₃)]₃-(CH₂)₅-, -(CH₂)₅-[C(CH₃)(CH₂CF₃)]₃-(CH₂)₂-, -(CH₂)₅-[C(CH₃)(CH₂CF₃)]₃-(CH₂)₃-, -(CH₂)₅-[C(CH₃)(CH₂CF₃)]₃-(CH₂)₄-, -(CH₂)₆-[C(CH₃)(CH₂CF₃)]₃-(CH₂)₂-, -(CH₂)₆-[C(CH₃)(CH₂CF₃)]₃-(CH₂)₃-, -[C(CH₃)(CH₂CF₃)]₄-(CH₂)-, -(CH₂)-[C(CH₃)(CH₂CF₃)]₄-, -(CH₂)-[C(CH₃)(CH₂CF₃)]₄-(CH₂)-, -(CH₂)-[C(CH₃)(CH₂CF₃)]₄-(CH₂)₂-, -(CH₂)-[C(CH₃)(CH₂CF₃)]₄-(CH₂)₃-, -(CH₂)-[C(CH₃)(CH₂CF₃)]₄-(CH₂)₄-, -(CH₂)-[C(CH₃)(CH₂CF₃)]₄-(CH₂)₅-, -(CH₂)-[C(CH₃)(CH₂CF₃)]₄-(CH₂)₆-, -(CH₂)-[C(CH₃)(CH₂CF₃)]₄-(CH₂)₇-, -(CH₂)-[C(CH₃)(CH₂CF₃)]₄-(CH₂)₈-, -(CH₂)-[C(CH₃)(CH₂CF₃)]₄-(CH₂)₉-, -(CH₂)-[C(CH₃)(CH₂CF₃)]₄-(CH₂)₁₀-, -(CH₂)₂-[C(CH₃)(CH₂CF₃)]₄-(CH₂)-, -(CH₂)₃-[C(CH₃)(CH₂CF₃)]₄-(CH₂)-, -(CH₂)₄-[C(CH₃)(CH₂CF₃)]₄-(CH₂)-, -(CH₂)₅-[C(CH₃)(CH₂CF₃)]₄-(CH₂)-, -(CH₂)₆-[C(CH₃)(CH₂CF₃)]₄-(CH₂)-, -(CH₂)₇-[C(CH₃)(CH₂CF₃)]₄-(CH₂)-, -(CH₂)₂-[C(CH₃)(CH₂CF₃)]₄-(CH₂)₂-, -(CH₂)₃-[C(CH₃)(CH₂CF₃)]₄-(CH₂)₃-, -(CH₂)₄-[C(CH₃)(CH₂CF₃)]₄-(CH₂)₄-, -(CH₂)₅-[C(CH₃)(CH₂CF₃)]₄-(CH₂)₅-, -(CH₂)₂-[C(CH₃)(CH₂CF₃)]₄-(CH₂)₃-, -(CH₂)₂-[C(CH₃)(CH₂CF₃)]₄-(CH₂)₄-, -(CH₂)₂-[C(CH₃)(CH₂CF₃)]₄-(CH₂)₅-, -(CH₂)₂-[C(CH₃)(CH₂CF₃)]₄-(CH₂)₆-, -(CH₂)₃- [C(CH₃)(CH₂CF₃)]₄-(CH₂)₂-, -(CH₂)₃-[C(CH₃)(CH₂CF₃)]₄-(CH₂)₄-, -(CH₂)₄-[C(CH₃)(CH₂CF₃)]₄-(CH₂)₂-, -(CH₂)₄-[C(CH₃)(CH₂CF₃)]₄-(CH₂)₃-, -(CH₂)₅-[C(CH₃)(CH₂CF₃)]₄-(CH₂)₂-, -(CH₂)₅-[C(CH₃)(CH₂CF₃)]₄-(CH₂)₃-, -(CH₂)₆-[C(CH₃)(CH₂CF₃)]₄-(CH₂)₂-, -[C(CH₃)(CH₂CF₃)]₅-(CH₂)-, -(CH₂)-[C(CH₃)(CH₂CF₃)]₅-, -(CH₂)-[C(CH₃)(CH₂CF₃)]₅-(CH₂)-, -(CH₂)-[C(CH₃)(CH₂CF₃)]₅-(CH₂)₂-, -(CH₂)-[C(CH₃)(CH₂CF₃)]₅-(CH₂)₃-, -(CH₂)-[C(CH₃)(CH₂CF₃)]₅-(CH₂)₄-, -(CH₂)-[C(CH₃)(CH₂CF₃)]₅-(CH₂)₅-, -(CH₂)-[C(CH₃)(CH₂CF₃)]₅-(CH₂)₆-, -(CH₂)₂-[C(CH₃)(CH₂CF₃)₅-(CH₂)-, -(CH₂)₃-[C(CH₃)(CH₂CF₃)]₅-(CH₂)-, -(CH₂)₄-[C(CH₃)(CH₂CF₃)₅-(CH₂)-, -(CH₂)₅-[C(CH₃)(CH₂CF₃)]₅-(CH₂)-, -(CH₂)₆-[C(CH₃)(CH₂CF₃)₅-(CH₂)-, -(CH₂)₂-[C(CH₃)(CH₂CF₃)]₅-(CH₂)₂-, -(CH₂)₃-[C(CH₃)(CH₂CF₃)]₅-(CH₂)₃-, -(CH₂)₄-[C(CH₃)(CH₂CF₃)]₅-(CH₂)₄-, -(CH₂)₂-[C(CH₃)(CH₂CF₃)]₅-(CH₂)₃-, -(CH₂)₂-[C(CH₃)(CH₂CF₃)]₅-(CH₂)₄-, -(CH₂)₂-[C(CH₃)(CH₂CF₃)]₅-(CH₂)₅-, -(CH₂)₂-[C(CH₃)(CH₂CF₃)]₅-(CH₂)₆-, -(CH₂)₃-[C(CH₃)(CH₂CF₃)]₅-(CH₂)₂-, -(CH₂)₃-[C(CH₃)(CH₂CF₃)]₅-(CH₂)₄-, -(CH₂)₄-[C(CH₃)(CH₂CF₃)]₅-(CH₂)₂-, -(CH₂)₄-[C(CH₃)(CH₂CF₃)]₅-(CH₂)₃-, -(CH₂)₅-[C(CH₃)(CH₂CF₃)]₅-(CH₂)₂-, -(CH₂)₂-(CF₂)-O-(CF₂)-(CH₂)₂-, -(CH₂)₂-(CF₂)-O-(CH₂)-O-(CF₂)-(CH₂)₂- , -(CH₂)₂-(CF₂)-O-(CH₂)₂-O-(CF₂)-(CH₂)₂, -(CH₂)-(CF₂)-O-(CF₂)-O-(CF₂)-(CH₂)-, -(CH₂)₂-(CF₂)-O-(CF₂)-O-(CF₂)-(CH₂)₂-, -(CH₂)₃-(CF₂)-O-(CF₂)-O-(CF₂)-(CH₂)₃-, -(CH₂)₄-(CF₂)-O-(CF₂)-O-(CF₂)-(CH₂)₄-, -(CH₂)-(CF₂)-O-(CF₂)-O-(CF₂)-(CH₂)₂-, -(CH₂)-(CF₂)-O-(CF₂)-O-(CF₂)-(CH₂)₃-, -(CH₂)-(CF₂)-O-(CF₂)-O-(CF₂)-(CH₂)₄-, -(CH₂)-(CF₂)-O-(CF₂)-O-(CF₂)-(CH₂)₅-, -(CH₂)-(CF₂)-O-(CF₂)-O-(CF₂)-(CH₂)₆-, -(CH₂)-(CF₂)-O-(CF₂)-O-(CF₂)-(CH₂)₇-, -(CH₂)₂-(CF₂)-O-(CF₂)-O-(CF₂)-(CH₂)-, -(CH₂)₃-(CF₂)-O-(CF₂)-O-(CF₂)-(CH₂)-, -(CH₂)₄-(CF₂)-O-(CF₂)-O-(CF₂)-(CH₂)- , -(CH₂)₅-(CF₂)-O-(CF₂)-O-(CF₂)-(CH₂)-, -(CH₂)₆-(CF₂)-O-(CF₂)-O-(CF₂)-(CH₂)-, -(CH₂)₇-(CF₂)-O-(CF₂)-O-(CF₂)-(CH₂)-, -(CH₂)₂-(CF₂)-O-(CF₂)-O-(CF₂)-(CH₂)₃-, -(CH₂)₂-(CF₂)-O-(CF₂)-O-(CF₂)-(CH₂)₄-, -(CH₂)₂-(CF₂)-O-(CF₂)-O-(CF₂)-(CH₂)₅-, -(CH₂)₂-(CF₂)-O-(CF₂)-O-(CF₂)-(CH₂)₆-, -(CH₂)₃-(CF₂)-O-(CF₂)-O-(CF₂)-(CH₂)₂-, -(CH₂)₄-(CF₂)-O-(CF₂)-O-(CF₂)-(CH₂)₂- , -(CH₂)₅-(CF₂)-O-(CF₂)-O-(CF₂)-(CH₂)₂-, -(CH₂)₆-(CF₂)-O-(CF₂)-O-(CF₂)-(CH₂)₂-, -(CH₂)₃-(CF₂)-O-(CF₂)-O-(CF₂)-(CH₂)₄-, -(CH₂)₄-(CF₂)-O-(CF₂)-O-(CF₂)-(CH₂)₃-, -(CH₂)₃-(CF₂)-O-(CF₂)-O-(CF₂)-(CH₂)₅-, -(CH₂)₅-(CF₂)-O-(CF₂)-O-(CF₂)-(CH₂)₃-, -(CH₂)-(CF₂)-O-(CF₂)₂-O-(CF₂)-(CH₂)-, -(CH₂)₂-(CF₂)-O-(CF₂)₂-O-(CF₂)-(CH₂)₂-, -(CH₂)₃-(CF₂)-O-(CF₂)₂-O-(CF₂)-(CH₂)₃-, -(CH₂)₄-(CF₂)-O-(CF₂)₂-O-(CF₂)-(CH₂)₄-, -(CH₂)-(CF₂)-O-(CF₂)₂-O-(CF₂)-(CH₂)₂-, -(CH₂)-(CF₂)-O-(CF₂)₂-O-(CF₂)-(CH₂)₃-, -(CH₂)-(CF₂)-O-(CF₂)₂-O-(CF₂)-(CH₂)₄-, -(CH₂)-(CF₂)-O-(CF₂)₂-O-(CF₂)-(CH₂)₅-, -(CH₂)-(CF₂)-O-(CF₂)₂-O-(CF₂)-(CH₂)₆- , -(CH₂)-(CF₂)-O-(CF₂)₂-O-(CF₂)-(CH₂)₇-, -(CH₂)₂-(CF₂)-O-(CF₂)₂-O-(CF₂)-(CH₂)-, -(CH₂)₃-(CF₂)-O-(CF₂)₂-O-(CF₂)-(CH₂)-, -(CH₂)₄-(CF₂)-O-(CF₂)₂-O-(CF₂)-(CH₂)-, -(CH₂)₅-(CF₂)-O-(CF₂)₂-O-(CF₂)-(CH₂)-, -(CH₂)₆-(CF₂)-O-(CF₂)₂-O-(CF₂)-(CH₂)-, -(CH₂)₇-(CF₂)-O-(CF₂)₂-O-(CF₂)-(CH₂)-, -(CH₂)₂-(CF₂)-O-(CF₂)₂-O-(CF₂)-(CH₂)₃-, -(CH₂)₂-(CF₂)-O-(CF₂)₂-O-(CF₂)-(CH₂)₄- , -(CH₂)₂-(CF₂)-O-(CF₂)₂-O-(CF₂)-(CH₂)₅-, -(CH₂)₂-(CF₂)-O-(CF₂)₂-O-(CF₂)-(CH₂)₆-, -(CH₂)₃-(CF₂)-O-(CF₂)₂-O-(CF₂)-(CH₂)₂-, -(CH₂)₄-(CF₂)-O-(CF₂)₂-O-(CF₂)-(CH₂)₂-, -(CH₂)₅-(CF₂)-O-(CF₂)₂-O-(CF₂)-(CH₂)₂-, -(CH₂)₆-(CF₂)-O-(CF₂)₂-O-(CF₂)-(CH₂)₂-, -(CH₂)₃-(CF₂)-O-(CF₂)₂-O-(CF₂)-(CH₂)₄-, -(CH₂)₄-(CF₂)-O-(CF₂)₂-O-(CF₂)-(CH₂)₃-, -(CH₂)₃-(CF₂)-O-(CF₂)₂-O-(CF₂)-(CH₂)₅- , -(CH₂)₅-(CF₂)-O-(CF₂)₂-O-(CF₂)-(CH₂)₃-, -(CH₂)-(CF₂)₂-O-(CF₂)₂-O-(CF₂)₂-(CH₂)-, -(CH₂)₂-(CF₂)₂-O-(CF₂)₂-O-(CF₂)₂-(CH₂)₂-, -(CH₂)₃-(CF₂)₂-O-(CF₂)₂-O-(CF₂)₂-(CH₂)₃-, -(CH₂)₄-(CF₂)₂-O-(CF₂)₂-O-(CF₂)₂-(CH₂)₄-, -(CH₂)-(CF₂)₂-O-(CF₂)₂-O-(CF₂)₂-(CH₂)₂-, -(CH₂)-(CF₂)₂-O-(CF₂)₂-O-(CF₂)₂-(CH₂)₃-, -(CH₂)-(CF₂)₂-O-(CF₂)₂-O-(CF₂)₂-(CH₂)₃- , -(CH₂)-(CF₂)₂-O-(CF₂)₂-O-(CF₂)₂-(CH₂)₄-, -(CH₂)-(CF₂)₂-O-(CF₂)₂-O-(CF₂)₂-(CH₂)₅-, -(CH₂)-(CF₂)₂-O-(CF₂)₂-O-(CF₂)₂-(CH₂)₆-, -(CH₂)-(CF₂)₂-O-(CF₂)₂-O-(CF₂)₂-(CH₂)₇-, -(CH₂)₂-(CF₂)₂-O-(CF₂)₂-O-(CF₂)₂-(CH₂)-, -(CH₂)₃-(CF₂)₂-O-(CF₂)₂-O-(CF₂)₂-(CH₂)-, -(CH₂)₄-(CF₂)₂-O-(CF₂)₂-O-(CF₂)₂-(CH₂)- , -(CH₂)₅-(CF₂)₂-O-(CF₂)₂-O-(CF₂)₂-(CH₂)-, -(CH₂)₆-(CF₂)₂-O-(CF₂)₂-O-(CF₂)₂-(CH₂)-, -(CH₂)₇-(CF₂)₂-O-(CF₂)₂-O-(CF₂)₂-(CH₂)-, -(CH₂)₂-(CF₂)₂-O-(CF₂)₂-O-(CF₂)₂-(CH₂)₃-, -(CH₂)₂-(CF₂)₂-O-(CF₂)₂-O-(CF₂)₂-(CH₂)₄-, -(CH₂)₂-(CF₂)₂-O-(CF₂)₂-O-(CF₂)₂-(CH₂)₅-, -(CH₂)₂-(CF₂)₂-O-(CF₂)₂-O-(CF₂)₂-(CH₂)₆- , -(CH₂)₃-(CF₂)₂-O-(CF₂)₂-O-(CF₂)₂-(CH₂)₂-, -(CH₂)₄-(CF₂)₂-O-(CF₂)₂-O-(CF₂)₂-(CH₂)₂-, -(CH₂)₅-(CF₂)₂-O-(CF₂)₂-O-(CF₂)₂-(CH₂)₂-, -(CH₂)₆-(CF₂)₂-O-(CF₂)₂-O-(CF₂)₂-(CH₂)₂-, -(CH₂)₃-(CF₂)₂-O-(CF₂)₂-O-(CF₂)₂-(CH₂)₄-, -(CH₂)₄-(CF₂)₂-O-(CF₂)₂-O-(CF₂)₂-(CH₂)₃-, -(CH₂)₃-(CF₂)₂-O-(CF₂)₂-O-(CF₂)₂-(CH₂)₅- , -(CH₂)₅-(CF₂)₂-O-(CF₂)₂-O-(CF₂)₂-(CH₂)₃-, -(CH₂)-(CF₂)-O-(CF₂)-O-(CF₂)-O-(CF₂)-(CH₂)-, -(CH₂)₂-(CF₂)-O-(CF₂)-O-(CF₂)-O-(CF₂)-(CH₂)₂-, -(CH₂)₃-(CF₂)-O-(CF₂)-O-(CF₂)-O-(CF₂)-(CH₂)₃- , -(CH₂)₄-(CF₂)-O-(CF₂)-O-(CF₂)-O-(CF₂)-(CH₂)₄-, -(CH₂)-(CF₂)-O-(CF₂)-O-(CF₂)-O-(CF₂)-(CH₂)₂-, -(CH₂)-(CF₂)-O-(CF₂)-O-(CF₂)-O-(CF₂)-(CH₂)₃- , -(CH₂)-(CF₂)-O-(CF₂)-O-(CF₂)-O-(CF₂)-(CH₂)₄-, -(CH₂)-(CF₂)-O-(CF₂)-O-(CF₂)-O-(CF₂)-(CH₂)₅-, -(CH₂)-(CF₂)-O-(CF₂)-O-(CF₂)-O-(CF₂)-(CH₂)₆- , -(CH₂)-(CF₂)-O-(CF₂)-O-(CF₂)-O-(CF₂)-(CH₂)₇-, -(CH₂)₂-(CF₂)-O-(CF₂)-O-(CF₂)-O-(CF₂)-(CH₂)-, -(CH₂)₃-(CF₂)-O-(CF₂)-O-(CF₂)-O-(CF₂)-(CH₂)- , -(CH₂)₄-(CF₂)-O-(CF₂)-O-(CF₂)-O-(CF₂)-(CH₂)-, -(CH₂)₅-(CF₂)-O-(CF₂)-O-(CF₂)-O-(CF₂)-(CH₂)-, -(CH₂)₆-(CF₂)-O-(CF₂)-O-(CF₂)-O-(CF₂)-(CH₂)- , -(CH₂)₇-(CF₂)-O-(CF₂)-O-(CF₂)-O-(CF₂)-(CH₂)-, -(CH₂)₂-(CF₂)-O-(CF₂)-O-(CF₂)-O-(CF₂)-(CH₂)₃-, -(CH₂)₂-(CF₂)-O-(CF₂)-O-(CF₂)-O-(CF₂)-(CH₂)₄- , -(CH₂)₂-(CF₂)-O-(CF₂)-O-(CF₂)-O-(CF₂)-(CH₂)₅-, -(CH₂)₂-(CF₂)-O-(CF₂)-O-(CF₂)-O-(CF₂)-(CH₂)₆-, -(CH₂)₃-(CF₂)-O-(CF₂)-O-(CF₂)-O-(CF₂)-(CH₂)₂- , -(CH₂)₄-(CF₂)-O-(CF₂)-O-(CF₂)-O-(CF₂)-(CH₂)₂-, -(CH₂)₅-(CF₂)-O-(CF₂)-O-(CF₂)-O-(CF₂)-(CH₂)₂-, -(CH₂)₆-(CF₂)-O-(CF₂)-O-(CF₂)-O-(CF₂)-(CH₂)₂- , -(CH₂)₃-(CF₂)-O-(CF₂)-O-(CF₂)-O-(CF₂)-(CH₂)₄-, -(CH₂)₄-(CF₂)-O-(CF₂)-O-(CF₂)-O-(CF₂)-(CH₂)₃-, -(CH₂)₃-(CF₂)-O-(CF₂)-O-(CF₂)-O-(CF₂)-(CH₂)₅- , -(CH₂)₅-(CF₂)-O-(CF₂)-O-(CF₂)-O-(CF₂)-(CH₂)₃-, -(CH₂)-(CF₂)₂-O-(CF₂)₂-O-(CF₂)₂-O-(CF₂)₂-(CH₂)-, -(CH₂)₂-(CF₂)₂-O-(CF₂)₂-O-(CF₂)₂-O-(CF₂)₂-(CH₂)₂-, -(CH₂)₃-(CF₂)₂-O-(CF₂)₂-O-(CF₂)₂-O-(CF₂)₂-(CH₂)₃-, -(CH₂)₄-(CF₂)₂-O-(CF₂)₂-O-(CF₂)₂-O-(CF₂)₂-(CH₂)₄-, -(CH₂)-(CF₂)₂-O-(CF₂)₂-O-(CF₂)₂-O-(CF₂)₂-(CH₂)₂-, -(CH₂)-(CF₂)₂-O-(CF₂)₂-O-(CF₂)₂-O-(CF₂)₂-(CH₂)₃-, -(CH₂)-(CF₂)₂-O-(CF₂)₂-O-(CF₂)₂-O-(CF₂)₂-(CH₂)₄-, -(CH₂)-(CF₂)₂-O-(CF₂)₂-O-(CF₂)₂-O-(CF₂)₂-(CH₂)₅-, -(CH₂)-(CF₂)₂-O-(CF₂)₂-O-(CF₂)₂-O-(CF₂)₂-(CH₂)₆-, -(CH₂)-(CF₂)₂-O-(CF₂)₂-O-(CF₂)₂-O-(CF₂)₂-(CH₂)₇- , -(CH₂)₂-(CF₂)₂-O-(CF₂)₂-O-(CF₂)₂-O-(CF₂)₂-(CH₂)-, -(CH₂)₃-(CF₂)₂-O-(CF₂)₂-O-(CF₂)₂-O-(CF₂)₂-(CH₂)-, -(CH₂)₄-(CF₂)₂-O-(CF₂)₂-O-(CF₂)₂-O-(CF₂)₂-(CH₂)-, -(CH₂)₅-(CF₂)₂-O-(CF₂)₂-O-(CF₂)₂-O-(CF₂)₂-(CH₂)-, -(CH₂)₆-(CF₂)₂-O-(CF₂)₂-O-(CF₂)₂-O-(CF₂)₂-(CH₂)-, -(CH₂)₇-(CF₂)₂-O-(CF₂)₂-O-(CF₂)₂-O-(CF₂)₂-(CH₂)-, -(CH₂)₂-(CF₂)₂-O-(CF₂)₂-O-(CF₂)₂-O-(CF₂)₂-(CH₂)₃- , -(CH₂)₂-(CF₂)₂-O-(CF₂)₂-O-(CF₂)₂-O-(CF₂)₂-(CH₂)₄-, -(CH₂)₂-(CF₂)₂-O-(CF₂)₂-O-(CF₂)₂-O-(CF₂)₂-(CH₂)₅-, -(CH₂)₂-(CF₂)₂-O-(CF₂)₂-O-(CF₂)₂-O-(CF₂)₂-(CH₂)₆-, -(CH₂)₃-(CF₂)₂-O-(CF₂)₂-O-(CF₂)₂-O-(CF₂)₂-(CH₂)₂-, -(CH₂)₄-(CF₂)₂-O-(CF₂)₂-O-(CF₂)₂-O-(CF₂)₂-(CH₂)₂-, -(CH₂)₅-(CF₂)₂-O-(CF₂)₂-O-(CF₂)₂-O-(CF₂)₂-(CH₂)₂-, -(CH₂)₆-(CF₂)₂-O-(CF₂)₂-O-(CF₂)₂-O-(CF₂)₂-(CH₂)₂- , -(CH₂)₃-(CF₂)₂-O-(CF₂)₂-O-(CF₂)₂-O-(CF₂)₂-(CH₂)₄-, -(CH₂)₄-(CF₂)₂-O-(CF₂)₂-O-(CF₂)₂-O-(CF₂)₂-(CH₂)₃-, -(CH₂)₃-(CF₂)₂-O-(CF₂)₂-O-(CF₂)₂-O-(CF₂)₂-(CH₂)₅-, -(CH₂)₅-(CF₂)₂-O-(CF₂)₂-O-(CF₂)₂-O-(CF₂)₂-(CH₂)₃-.

Preferred examples for -R₂- are -(CH₂)₃-, -(CH₂)₄-, -(CH₂)₅-, -(CH₂)₆-, - (CH₂)₇-, -(CH₂)₈-, -(CH₂)₉-, -(CH₂)₁₀-, -(CH₂)₁₁-, -(CH₂)₂-S-(CH₂)₂-, -(CH₂)₂-SO₂-(CH₂)₂-, -(CH₂)-S-(CH₂)₂-O-(CH₂)-, -(CH₂)-SO₂-(CH₂)₂-O-(CH₂)-, - (CH₂)-SO₂-(CH₂)₂-S-(CH₂)-, -(CH₂)-O-(CH₂)₂-S-(CH₂)₂-O-(CH₂)-, -(CH₂)-S-(CH₂)₂-O-(CH₂)₂-S-(CH₂)-, -(CH₂)-SO₂-(CH₂)₂-O-(CH₂)₂-SO₂-(CH₂)-, -(CH₂)-S-(CH₂)₂-S-(CH₂)₂-S-(CH₂)-, -(CH₂)-SO₂-(CH₂)₂-SO₂-(CH₂)₂-SO₂-(CH₂)-, - (CH₂)-O-(CH₂)₂-SO₂-(CH₂)₂-O-(CH₂)-, -(CH₂)₁₂, -(CH₂)₃-(CF₂)-(CH₂)₃-, - (CH₂)-(CF₂)₃-(CH₂)-, -(CH₂)₂-(CF₂)₄-(CH₂)₂-, -(CH₂)-[CH(CF₃)]-(CH₂)-, - (CH₂)-[C(CH₃)CF_{3]}-(CH₂)-, -(CH₂)-[CH(CH₂CF₃)]-(CH₂)-, -(CH₂)-[C(CH₃)(CH₂CF₃)]-(CH₂)-, -(CH₂)₂-(CF₂)-O-(CF₂)-O-(CF₂)-(CH₂)₂- and -(CH₂)-(CF₂)-O-(CF₂)-O-(CF₂)-O-(CF₂)-(CH₂)- according to the invention.

Compounds of formulae (I), (I#), (1-1), (I-2), (I-2H), (I-3), (I-3-H), (I-4), (I-4-H), (I'), (I'-a), (I'-b), (I'-c), (I'-d), (I'-1), (I'-2), (I'-2-H), (I'-3), (I'-3-H), (I'-4), (I'-4-H) (I"), (I"-1), (I"-2), (I"-2-H), (I"-3), (I"-3-H), (I"-4) and (I"-4-H) with substituents as described before or preferably described before having a polymerizable group as described before or preferably described before or below are preferred in case the substituent -R₂- within the at least one linking element Y-R₂- corresponds to -(C(R)₂)ₒ-, wherein R and o has a meaning as described or preferably described before.

Accordingly monomers of formulae (I), (I#), (1-1), (I-2), (I-2H), (I-3), (I-3-H), (I-4), (I-4-H), (I'), (I'-a), (I'-b), (I'-c), (I'-d), (I'-1), (I'-2), (I'-2-H), (I'-3), (I'-3-H), (I'-4), (I'-4-H) (I"), (I"-1), (I"-2), (I"-2-H), (I"-3), (I"-3-H), (I"-4) and (I"-4-H) for the preparation of an ophthalmic device or precursor article for an ophthalmic device as described before with substituents as described before or preferably described before having a polymerizable group as described before or preferably described before or below are preferred in case the substituent -R₂- within the at least one linking element Y-R₂-corresponds to -(C(R)₂)ₒ-, wherein R and o has a meaning as described or preferably described before. Such ophthalmic devices and precursor articles prepared by using these monomers are especially preferred.

Particularly preferred examples for -R₂- are -(CH₂)₃-, -(CH₂)₄-, -(CH₂)₅-, - (CH₂)₆-, -(CH₂)₇- according to the invention. Very particularly preferably, - R₂- is -(CH₂)₅- according to the invention.

Therefore, the invention is furthermore directed to an ophthalmic device or precursor article for an ophthalmic device comprising polymerized compounds of formulae (I), (1-1), (I-2), (I-2-H), (I-3), (I-3-H), (I-4), (I-4-H), (I"), (I"-1), (I"-2), (I"-2-H), (I"-3), (I"-3-H), (I"-4) and (I"-4-H) as described before or preferably described before wherein -R₂- is at each occurrence independently -(C(R)₂)ₒ-, wherein R and o have a meaning as described or preferably described before.

The invention therefore relates to compounds of formulae (I), (I#), (1-1), (I-2), (I-2H), (I-3), (I-3-H), (I-4), (I-4-H), (I'), (I'-a), (I'-b), (I'-c), (I'-d), (I'-1), (I'-2), (I'-2-H), (I'-3), (I'-3-H), (I'-4), (I'-4-H) (I"), (I"-1), (I"-2), (I"-2-H), (I"-3), (I"-3-H), (I"-4) and (I"-4-H) as described before or preferably described before wherein -R₂- is at each occurrence independently -(C(R)₂)ₒ-, wherein R and o have a meaning as described or preferably described before.

The substituent Y-R₂- within formulae (I), (I#), (1-1), (I-2), (I-2H), (I-3), (I-3-H), (I-4), (I-4-H), (I'), (I'-a), (I'-b), (I'-c), (I'-d), (I'-1), (I'-2), (I'-2-H), (I'-3), (I'-3-H), (I'-4), (I'-4-H) (I"), (I"-1), (I"-2), (I"-2-H), (I"-3), (I"-3-H), (I"-4) and (I"-4-H) is selected from the group consisting of O-R₂-, -R₂- where Y is a bond, SO₂-R₂- and S-R₂-, wherein -R₂- has a meaning as described before or preferably or particularly preferably described before.

The substituent Y-R₂- is preferably selected from the group consisting of OR₂- and -R₂- where Y is a bond wherein -R₂- has a meaning as described before or preferably or particularly preferably described before.

The substituent Y-R₂-R₁ within formulae (I), (I#), (1-1), (I-2), (I-2H), (I-3), (I-3-H), (I-4), (I-4-H), (I'), (I'-a), (I'-b), (I'-c), (I'-d), (I'-1), (I'-2), (I'-2-H), (I'-3), (I'-3-H), (I'-4), (I'-4-H) (I"), (I"-1), (I"-2), (I"-2-H), (I"-3), (I"-3-H), (I"-4) and (I"-4-H) is selected from the group consisting of O-R₂-R₁, -R₂-R₁, SO₂-R₂-R₁ and S-R₂-R₁, or preferably selected from the group consisting of O-R₂-R₁ and - R₂-R₁, wherein -R₂- has a meaning as described before or preferably or particularly preferably described before and wherein R₁ is trimethoxysilyl, dimethoxymethylsilyl or a polymerizable group according to formula (4), 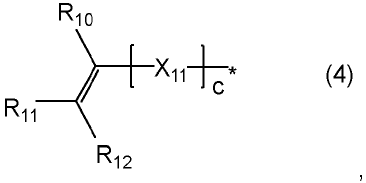 wherein
- X₁₁: is selected from the group consisting of O, S, O-SO₂, SO₂-O, C(=O), OC(=O), C(=O)O, S(C=O) and (C=O)S,
- R₁₀, R₁₁, R₁₂: are at each occurrence independently of each other selected from the group consisting of H, F, a linear or branched, non-fluorinated, partially or completely fluorinated alkyl group having 1 to 20 C atoms or aryl with 6 to 14 C atoms and
- c: is 0 or 1 .

In another preferred embodiment of the invention, c, 11, R₁₀, R₁₁ and R₁₂ within the compounds of formulae (I), (I#), (1-1), (I-2), (I-2H), (I-3), (I-3-H), (I-4), (I-4-H), (I'), (I'-a), (I'-b), (I'-c), (I'-d), (I'-1), (I'-2), (I'-2-H), (I'-3), (I'-3-H), (I'-4), (I'-4-H) (I"), (I"-1), (I"-2), (I"-2-H), (I"-3), (I"-3-H), (I"-4) and (I"-4-H) acting as monomers for the preparation of the ophthalmic device or precursor article of the ophthalmic device as described before or for the preparation of an oligomer, polymer or copolymer according to the invention or within the compounds according to the invention have the following preferred meaning:
Preferably, R₁₁ and R₁₂ are H. Preferably, c is 1.

Preferably, R₁₀ is H, methyl, ethyl or phenyl. Particularly preferably, R₁₀ is H or methyl.

Preferably, X₁₁ is C(=O), OC(=O) or C(=O)O. Particularly preferably, X₁₁ is C(=O)O.

Preferred alkenyl groups of formula (4) as polymerizable groups R₁ according to the invention are therefore represented by any one selected from the group consisting of formulae (4-1), (4-2), (4-3), (4-4), (4-5), (4-6), (4-7), (4-8), (4-9), (4-10), (4-11) and (4-12):

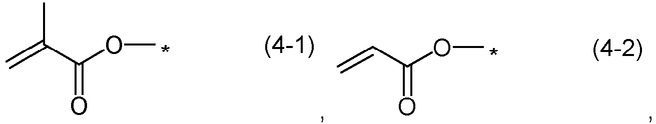

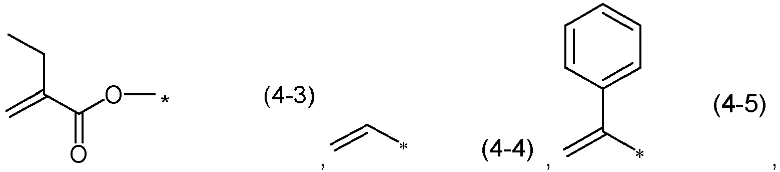

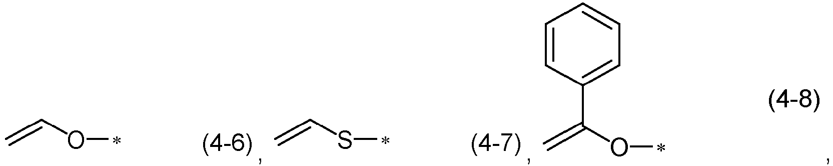

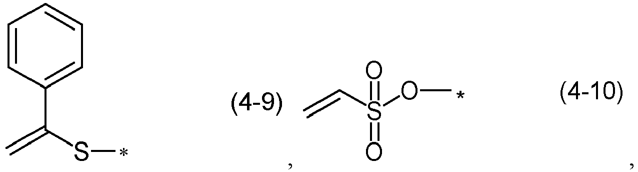

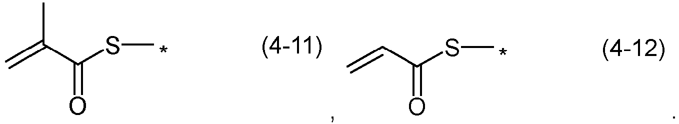

Particularly preferred alkenyl groups of formula (4) as polymerizable groups R₁ according to the invention are represented by any one selected from the group consisting of formulae (4-1), (4-2), (4-3), (4-5), (4-6), (4-11) and (4-12) as described before.

The alkenyl group represented by formula (4-1) is called methacrylate. The alkenyl group represented by formula (4-2) is called acrylate.

The preferred groups R₁ are preferably combined with preferred groups of the linking element -R₂- and/or the linking element Y-R₂-. Combinations are excluded where two O atoms or one O atom and one S atom are directly bonded to each other as known for a skilled artisan in the field of organic chemistry.

The substituent Y-R₂-R₁ within formulae (I), (I#), (I-1), (I-2), (I-2H), (I-3), (I-3-H), (I-4), (I-4-H), (I'), (I'-a), (I'-b), (I'-c), (I'-d), (I'-1), (I'-2), (I'-2-H), (I'-3), (I'-3-H), (I'-4), (I'-4-H) (I"), (I"-1), (I"-2), (I"-2-H), (I"-3), (I"-3-H), (I"-4) and (I"-4-H) is therefore particularly preferably selected from the group consisting of O-R₁, O-(CH₂)₃-R₁, O-(CH₂)₄-R₁, O-(CH₂)₅-R₁, O-(CH₂)₆-R₁, O-(CH₂)₇-R₁, - O-(CH₂)₈-R₁, -O-(CH₂)₉-R₁, -O-(CH₂)₁₀-R₁, -O-(CH₂)₁₁-R₁, -O-(CH₂)₁₂-R₁, -O-(CH₂)₂-S-(CH₂)₂-R₁, -O-(CH₂)₂-SO₂-(CH₂)₂-R₁, O-(CH₂)-S-(CH₂)₂-O-(CH₂)-R₁, O-(CH₂)-SO₂-(CH₂)₂-O-(CH₂)-R₁, O-(CH₂)-SO₂-(CH₂)₂-S-(CH₂)-R₁, O-(CH₂)-O-(CH₂)₂-S-(CH₂)₂-O-(CH₂)-R₁, O-(CH₂)-S-(CH₂)₂-O-(CH₂)₂-S-(CH₂)-R₁, O-(CH₂)-SO₂-(CH₂)₂-O-(CH₂)₂-SO₂-(CH₂)-R₁, O-(CH₂)-S-(CH₂)₂-S-(CH₂)₂-S-(CH₂)-R₁, O-(CH₂)-SO₂-(CH₂)₂-SO₂-(CH₂)₂-SO₂-(CH₂)-R₁, O-(CH₂)-O-(CH₂)₂-SO₂-(CH₂)₂-O-(CH₂)-R₁, -O-(CH₂)₃-(CF₂)-(CH₂)₃-R₁, -O-(CH₂)-(CF₂)₃-(CH₂)-R₁, -O-(CH₂)₂-(CF₂)₄-(CH₂)₂-R₁, -O-(CH₂)-[CH(CF₃)]-(CH₂)-R₁, -O-(CH₂)-[C(CH₃)CF₃]-(CH₂)-R₁, -O-(CH₂)-[CH(CH₂CF₃)]-(CH₂)-R₁, -O-(CH₂)-[C(CH₃)(CH₂CF₃)]-(CH₂)-R₁, -O-(CH₂)₂-(CF₂)-O-(CF₂)-O-(CF₂)-(CH₂)₂-R₁ and -O-(CH₂)-(CF₂)-O-(CF₂)-O-(CF₂)-O-(CF₂)-(CH₂)-R₁, wherein R₁ is selected from the group consisting of an alkenyl of formula (4-1), (4-2), (4-3), (4-4), (4-5), (4-6), (4-7), (4-8), (4-9), (4-10), (4-11), or (4-12);
-(CH₂)₃-R₁, -(CH₂)₄-R₁, -(CH₂)₅-R₁, -(CH₂)₆-R₁, -(CH₂)₇-R₁, -(CH₂)₈-R₁, - (CH₂)₉-R₁, -(CH₂)₁₀-R₁, -(CH₂)₁₁-R₁, -(CH₂)₁₂-R₁,-(CH₂)₂-S-(CH₂)₂-R₁, - (CH₂)₂-SO₂-(CH₂)₂-R₁, -(CH₂)-S-(CH₂)₂-O-(CH₂)-R₁, -(CH₂)-SO₂-(CH₂)₂-O-(CH₂)-R₁, -(CH₂)-SO₂-(CH₂)₂-S-(CH₂)-R₁, -(CH₂)-O-(CH₂)₂-S-(CH₂)₂-O-(CH₂)-R₁, -(CH₂)-S-(CH₂)₂-O-(CH₂)₂-S-(CH₂)-R₁, -(CH₂)-SO₂-(CH₂)₂-O-(CH₂)₂-SO₂-(CH₂)-R₁, -(CH₂)-S-(CH₂)₂-S-(CH₂)₂-S-(CH₂)-R₁, -(CH₂)-SO₂-(CH₂)₂-SO₂-(CH₂)₂-SO₂-(CH₂)-R₁, -(CH₂)-O-(CH₂)₂-SO₂-(CH₂)₂-O-(CH₂)-R₁, {(CH₂)₃-(CF₂)-(CH₂)₃-R₁, -(CH₂)-(CF₂)₃-(CH₂)-R₁, -(CH₂)₂-(CF₂)₄-(CH₂)₂-R₁, - (CH₂)-[CH(CF₃)]-(CH₂)-R₁, -(CH₂)-[C(CH₃)CF₃]-(CH₂)-R₁, -(CH₂)-[CH(CH₂CF₃)]-(CH₂)-R₁, -(CH₂)-[C(CH₃)(CH₂CF₃)]-(CH₂)-R₁, -(CH₂)₂-(CF₂)-O-(CF₂)-O-(CF₂)-(CH₂)₂-R₁ and -(CH₂)-(CF₂)-O-(CF₂)-O-(CF₂)-O-(CF₂)-(CH₂)-R₁, -, where Y is a bond and wherein R₁ is selected from the group consisting of an alkenyl of formula (4-1), (4-2), (4-3), (4-4), (4-5), (4-6), (4-7), (4-8), (4-9), (4-10), (4-11), or (4-12);
S-R₁, S-(CH₂)₃-R₁, S-(CH₂)₄-R₁, S-(CH₂)₅-R₁, S-(CH₂)₆-R₁, S-(CH₂)₇-R₁, S-(CH₂)₈-R₁, S-(CH₂)₉-R₁, S-(CH₂)₁₀-R₁, S-(CH₂)₁₁-R₁, S-(CH₂)₁₂-R₁, -S-(CH₂)₂-S-(CH₂)₂-R₁, -S-(CH₂)₂-SO₂-(CH₂)₂-R₁, S-(CH₂)-S-(CH₂)₂-O-(CH₂)-R₁, S-(CH₂)-SO₂-(CH₂)₂-O-(CH₂)-R₁, S-(CH₂)-SO₂-(CH₂)₂-S-(CH₂)-R₁, S-(CH₂)-O-(CH₂)₂-S-(CH₂)₂-O-(CH₂)-R₁, S-(CH₂)-S-(CH₂)₂-O-(CH₂)₂-S-(CH₂)-R₁, S-(CH₂)-SO₂-(CH₂)₂-O-(CH₂)₂-SO₂-(CH₂)-R₁, S-(CH₂)-S-(CH₂)₂-S-(CH₂)₂-S-(CH₂)-R₁, S-(CH₂)-SO₂-(CH₂)₂-SO₂-(CH₂)₂-SO₂-(CH₂)-R₁, S-(CH₂)-O-(CH₂)₂-SO₂-(CH₂)₂-O-(CH₂)-R₁, S-(CH₂)₃-(CF₂)-(CH₂)₃-R₁, S-(CH₂)-(CF₂)₃-(CH₂)-R₁, S-(CH₂)₂-(CF₂)₄-(CH₂)₂-R₁, S-(CH₂)-[CH(CF₃)]-(CH₂)-R₁, S-(CH₂)-[C(CH₃)CF_{3]}-(CH₂)-R₁, S-(CH₂)-[CH(CH₂CF₃)]-(CH₂)-R₁, S-(CH₂)-[C(CH₃)(CH₂CF₃)]-(CH₂)-R₁, S-(CH₂)₂-(CF₂)-O-(CF₂)-O-(CF₂)-(CH₂)₂-R₁ and S-(CH₂)-(CF₂)-O-(CF₂)-O-(CF₂)-O-(CF₂)-(CH₂)-R₁, wherein R₁ is selected from the group consisting of an alkenyl of formula (4-1), (4-2), (4-3), (4-4), (4-5), (4-6), (4-7), (4-8), (4-9), (4-10), (4-11), or (4-12);
SO₂-(CH₂)₃-R₁, SO₂-(CH₂)₄-R₁, SO₂-(CH₂)₅-R₁, SO₂-(CH₂)₆-R₁, SO₂-(CH₂)₇-R₁, SO₂-(CH₂)₈-R₁, SO₂-(CH₂)₉-R₁, SO₂-(CH₂)₁₀-R₁, SO₂-(CH₂)₁₁-R₁, SO₂-(CH₂)₁₂-R₁, SO₂-(CH₂)₂-S-(CH₂)₂-R₁, SO₂-(CH₂)₂-SO₂-(CH₂)₂-R₁, SO₂-(CH₂)-S-(CH₂)₂-O-(CH₂)-R₁, SO₂-(CH₂)-SO₂-(CH₂)₂-O-(CH₂)-R₁, SO₂-(CH₂)-SO₂-(CH₂)₂-S-(CH₂)-R₁, SO₂-(CH₂)-O-(CH₂)₂-S-(CH₂)₂-O-(CH₂)-R₁, SO₂-(CH₂)-S-(CH₂)₂-O-(CH₂)₂-S-(CH₂)-R₁, SO₂-(CH₂)-SO₂-(CH₂)₂-O-(CH₂)₂-SO₂-(CH₂)-R₁, SO₂-(CH₂)-S-(CH₂)₂-S-(CH₂)₂-S-(CH₂)-R₁, SO₂-(CH₂)-SO₂-(CH₂)₂-SO₂-(CH₂)₂-SO₂-(CH₂)-R₁, SO₂-(CH₂)-O-(CH₂)₂-SO₂-(CH₂)₂-O-(CH₂)-R₁, SO₂-(CH₂)₃-(CF₂)-(CH₂)₃-R₁, SO₂-(CH₂)-(CF₂)₃-(CH₂)-R₁, SO₂-(CH₂)₂-(CF₂)₄-(CH₂)₂-R₁, SO₂-(CH₂)-[CH(CF₃)]-(CH₂)-R₁, SO₂-(CH₂)-[C(CH₃)CF₃]-(CH₂)-R₁, SO₂-(CH₂)-[CH(CH₂CF₃)]-(CH₂)-R₁, SO₂-(CH₂)-[C(CH₃)(CH₂CF₃)]-(CH₂)-R₁, SO₂-(CH₂)₂-(CF₂)-O-(CF₂)-O-(CF₂)-(CH₂)₂-R₁ and SO₂-(CH₂)-(CF₂)-O-(CF₂)-O-(CF₂)-O-(CF₂)-(CH₂)-R₁, wherein R₁ is selected from the group consisting of an alkenyl of formula (4-1), (4-2), (4-3), (4-4), (4-5), (4-6), (4-7), (4-8), (4-9), (4-10), (4-11), or (4-12).

Particularly preferably, the compounds of formulae (I), (I#), (1-1), (I-2), (I-2H), (1-3), (I-3-H), (I-4), (I-4-H), (I'), (I'-1), (I'-2), (I'-2-H), (I'-3), (I'-3-H), (I'-4), (I'-4-H) (I"), (I"-1), (I"-2), (I"-2-H), (I"-3), (I"-3-H), (I"-4) and (I"-4-H) comprise a polymerizable group R₁ which is represented by formulae (4-1), (4-2), (4-5), (4-6), (4-11) and (4-12).

Very particularly preferably, the compounds of formulae (I), (I#), (1-1), (I-2), (I-2H), (1-3), (I-3-H), (I-4), (I-4-H), (I'), (I'-1), (I'-2), (I'-2-H), (I'-3), (I'-3-H), (I'-4), (I'-4-H) (I"), (I"-1), (I"-2), (I"-2-H), (I"-3), (I"-3-H), (I"-4) and (I"-4-H) comprise a polymerizable group R₁ which is a methacryl or an acryl group represented by formula (4-1) and (4-2).

The invention therefore relates further to an ophthalmic device or a precursor article for an ophthalmic device comprising polymerized compounds of formulae (I), (I#), (1-1), (I-2), (I-2H), (I-3), (I-3-H), (I-4), (I-4-H), (I'), (I'-1), (I'-2), (1'-2-H), (I'-3), (I'-3-H), (I'-4), (I'-4-H) (I"), (I"-1), (I"-2), (I"-2-H), (I"-3), (I"-3-H), (I"-4) and/or (I"-4-H) as described before or preferably described before wherein R₁ is at each occurrence independently an acryl or methacryl group.

The invention therefore relates further to compounds of formulae (I), (I#), (I-1), (I-2), (I-2H), (I-3), (I-3-H), (I-4), (I-4-H), (I'), (I'-1), (I'-2), (I'-2-H), (I'-3), (I'-3-H), (I'-4), (I'-4-H) (I"), (I"-1), (I"-2), (I"-2-H), (I"-3), (I"-3-H), (I"-4) and/or (I"-4-H) as described before or preferably described before wherein R₁ is at each occurrence independently an acryl or methacryl group.

Examples for compounds/monomers of formulae (I), (I#), (1-1), (I-2), (I-2H), (I-3), (I-3-H), (I-4), (I-4-H), (I'), (I'-a), (I'-b), (I'-c), (I'-d), (I'-1), (I'-2), (I'-2-H), (I'-3), (I'-3-H), (I'-4), (I'-4-H) (I"), (I"-1), (I"-2), (I"-2-H), (I"-3), (I"-3-H), (I"-4) and/or (I"-4-H) are the following compounds (A-001) to (A-180) as shown in tables 1 and 1-1.

**Table 1:**

| | |
|---|---|
| 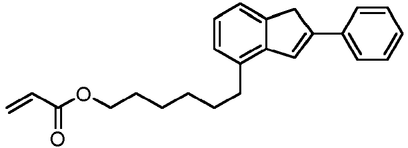 | A-001 |
| 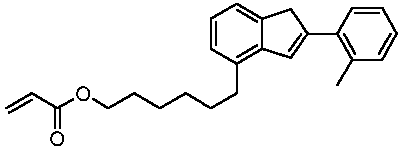 | A-002 |
| 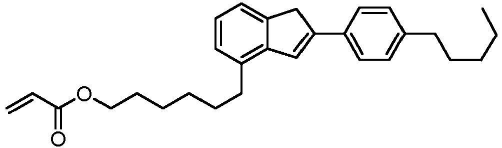 | A-003 |
| 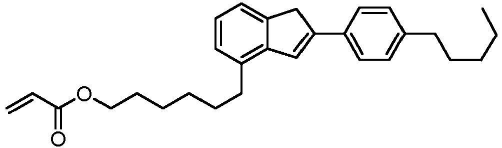 | A-004 |
| 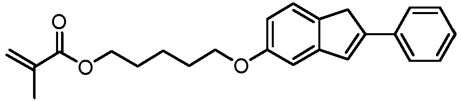 | A-005 |
| 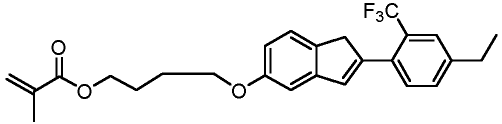 | A-006 |
| 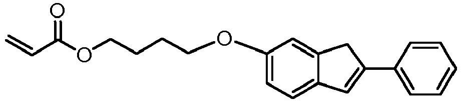 | A-007 |
| 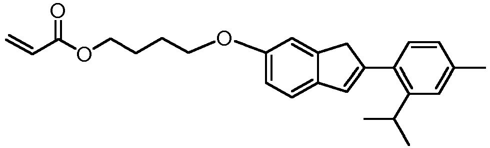 | A-008 |
| 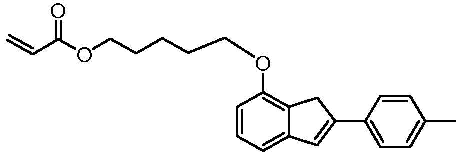 | A-009 |
| 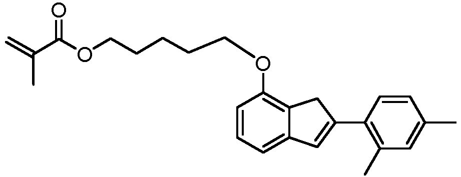 | A-010 |
| 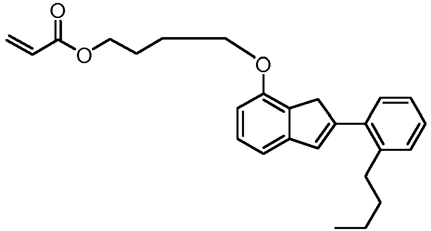 | A-011 |
| 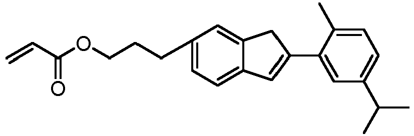 | A-012 |
| 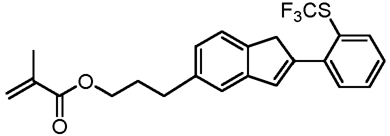 | A-013 |
| 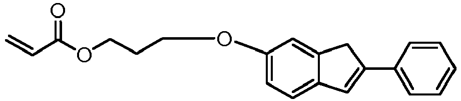 | A-014 |
| 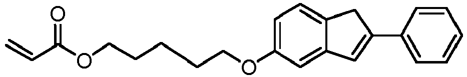 | A-015 |
| 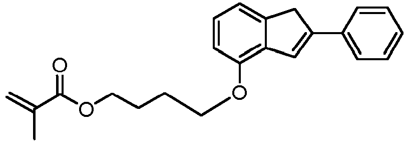 | A-016 |
| 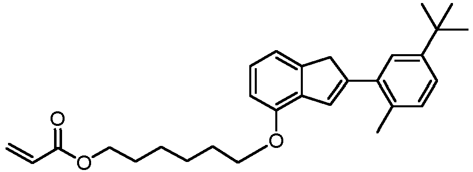 | A-017 |
| 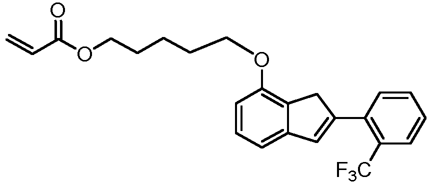 | A-018 |
| 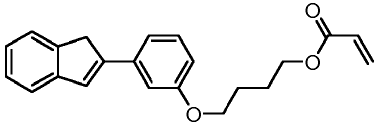 | A-019 |
| 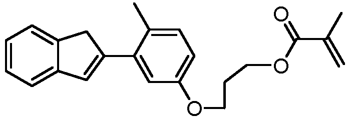 | A-020 |
| 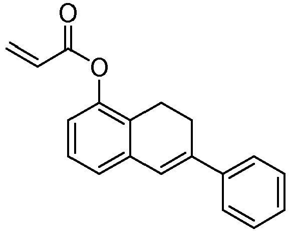 | A-021 |
| 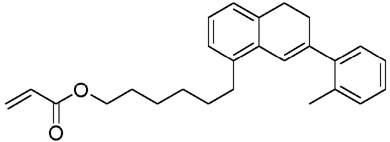 | A-022 |
| 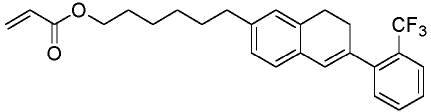 | A-023 |
| 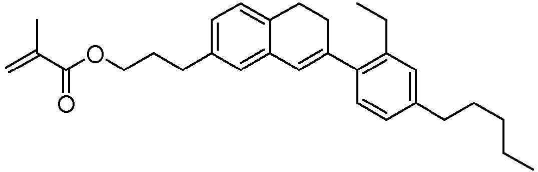 | A-024 |
| 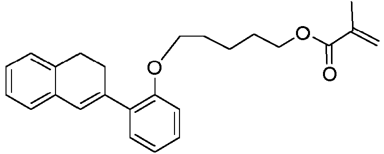 | A-025 |
| 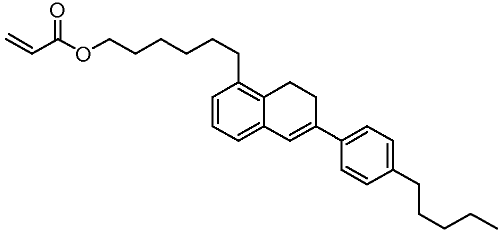 | A-026 |
| 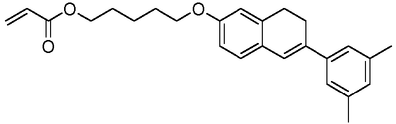 | A-027 |
| 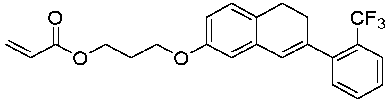 | A-028 |
| 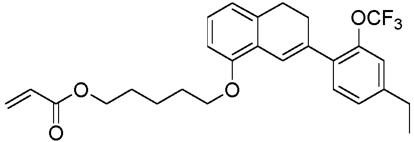 | A-029 |
| 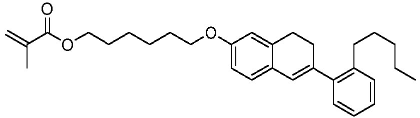 | A-030 |
| 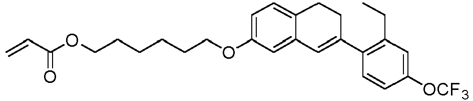 | A-031 |
| 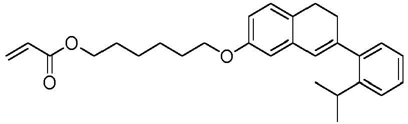 | A-032 |
| 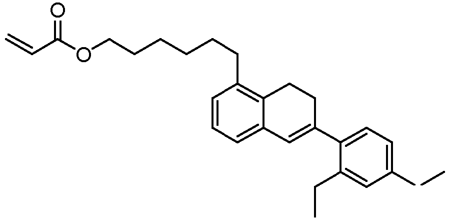 | A-033 |
| 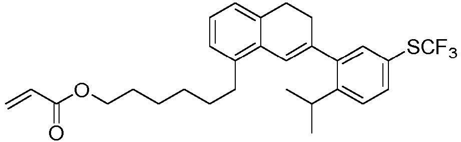 | A-034 |
| 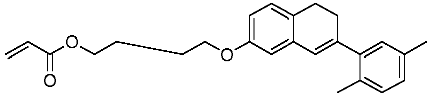 | A-035 |
| 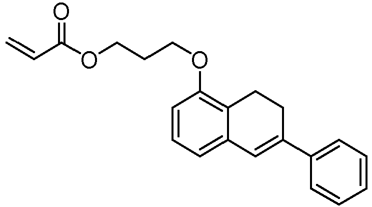 | A-036 |
| 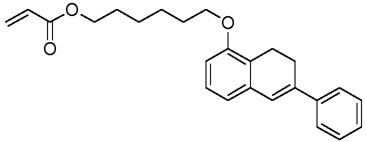 | A-037 |
| 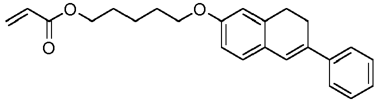 | A-038 |
| 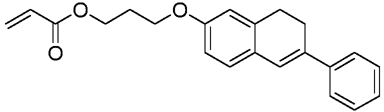 | A-039 |
| 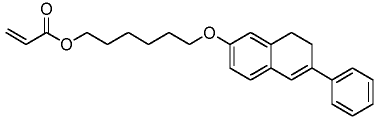 | A-040 |
| 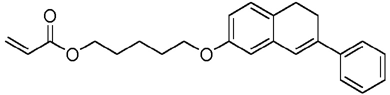 | A-041 |
| 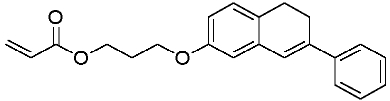 | A-042 |
| 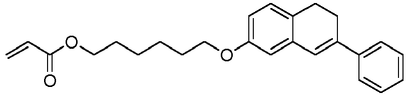 | A-043 |
| 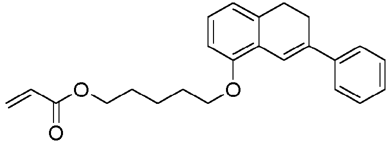 | A-044 |
| 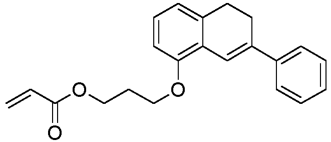 | A-045 |
| 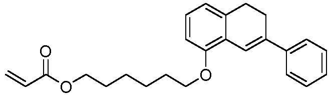 | A-046 |
| 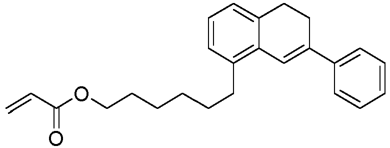 | A-047 |
| 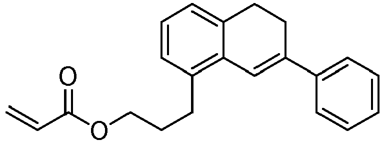 | A-048 |
| 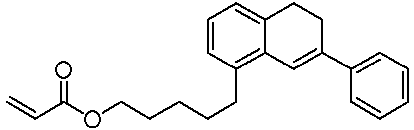 | A-049 |
| 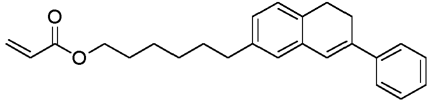 | A-050 |
| 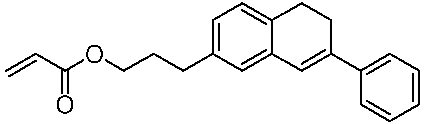 | A-051 |
| 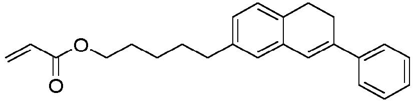 | A-052 |
| 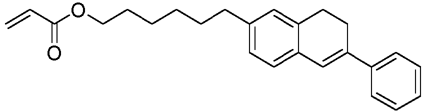 | A-053 |
| 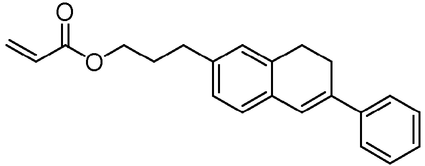 | A-054 |
| 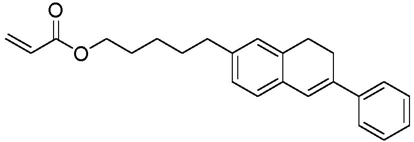 | A-055 |
| 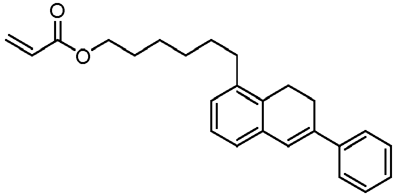 | A-056 |
| 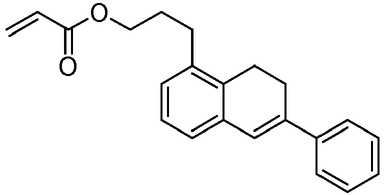 | A-057 |
| 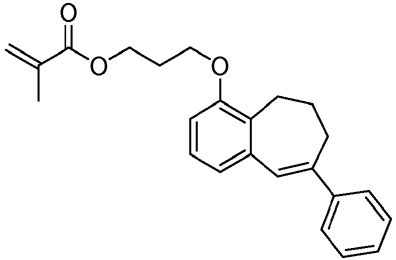 | A-058 |
| 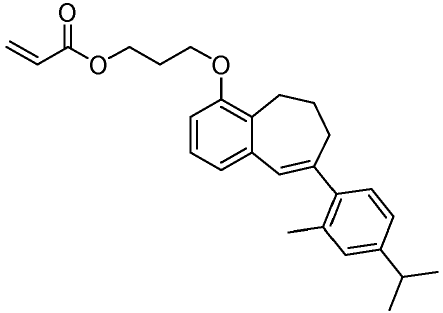 | A-059 |
| 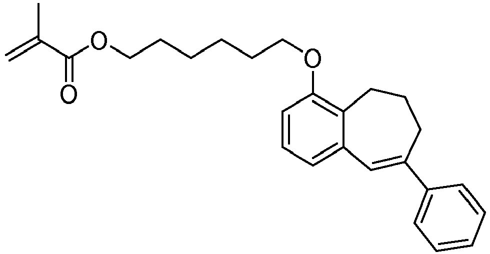 | A-060 |
| 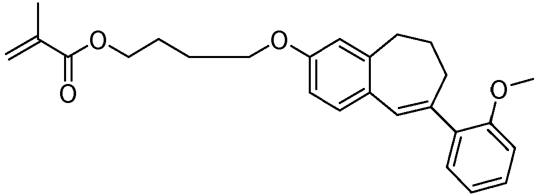 | A-061 |
| 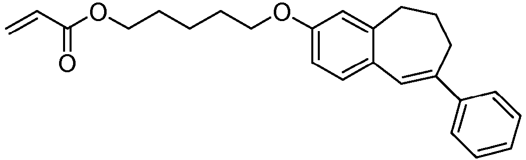 | A-062 |
| 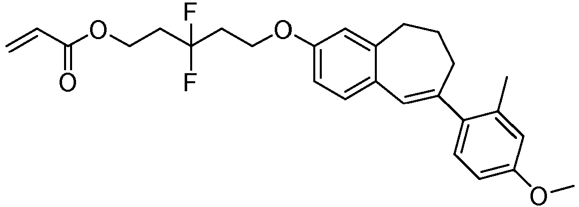 | A-063 |
| 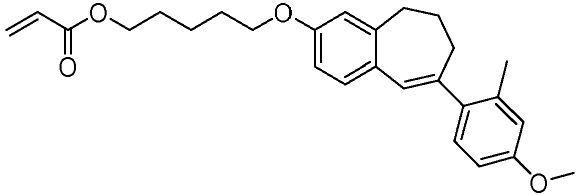 | A-064 |
| 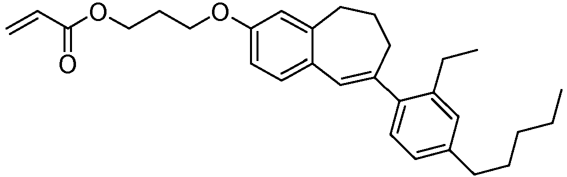 | A-065 |
| 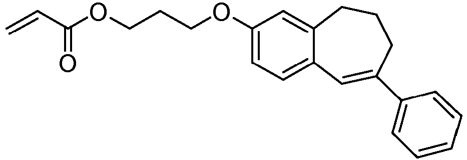 | A-066 |
| 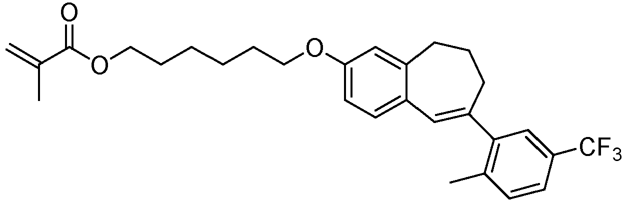 | A-067 |
| 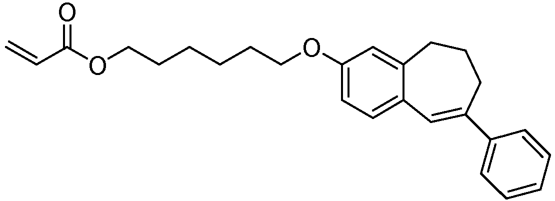 | A-068 |
| 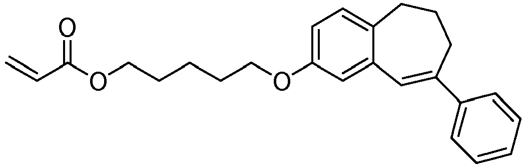 | A-069 |
| 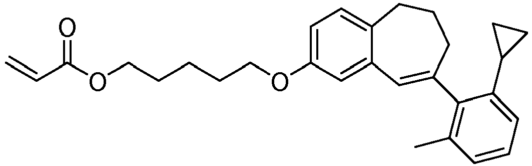 | A-070 |
| 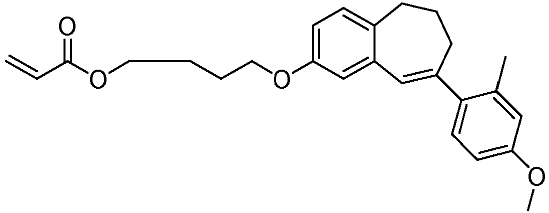 | A-071 |
| 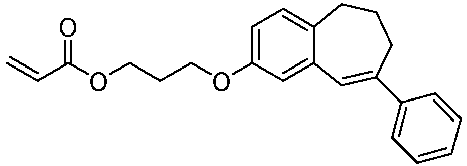 | A-072 |
| 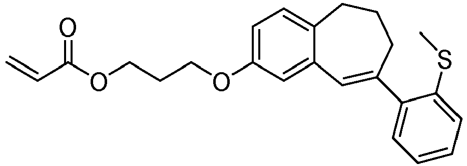 | A-073 |
| 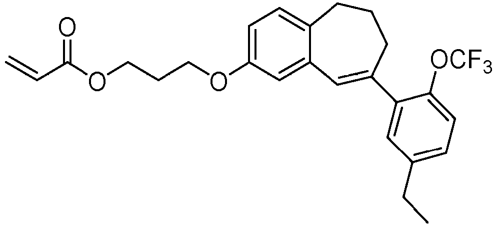 | A-074 |
| 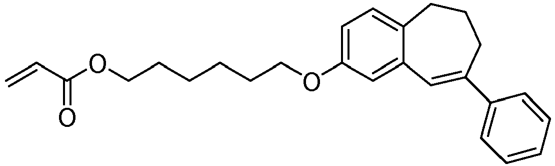 | A-075 |
| 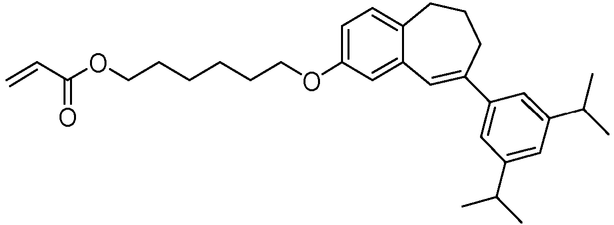 | A-076 |
| 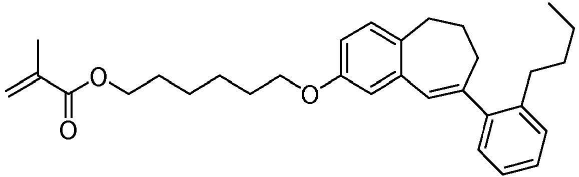 | A-077 |
| 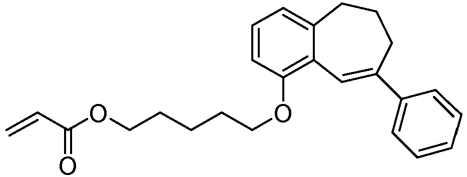 | A-078 |
| 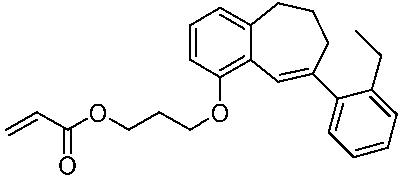 | A-079 |
| 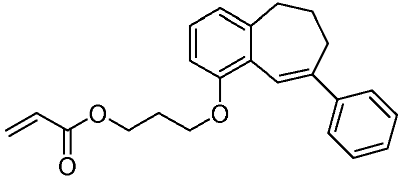 | A-080 |
| 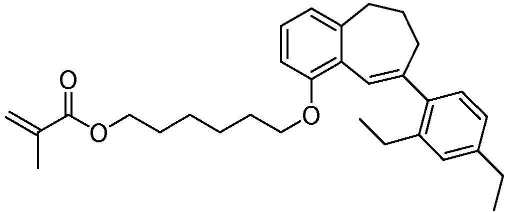 | A-081 |
| 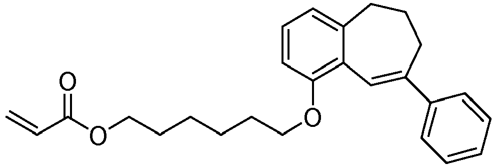 | A-082 |
| 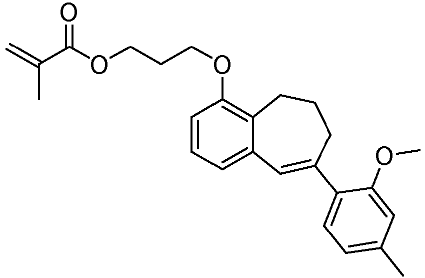 | A-083 |
| 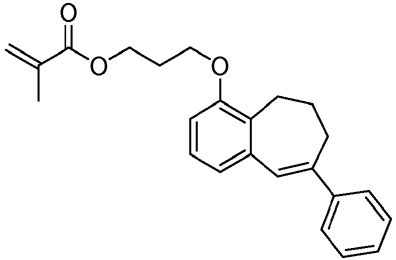 | A-084 |
| 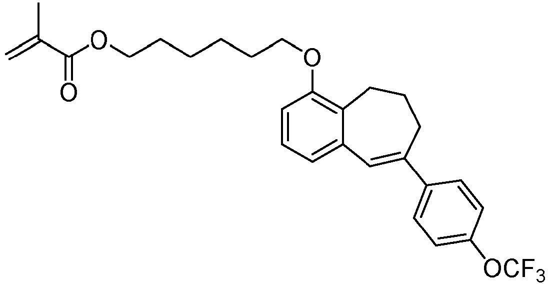 | A-085 |
| 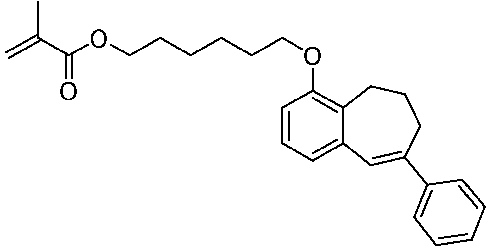 | A-086 |
| 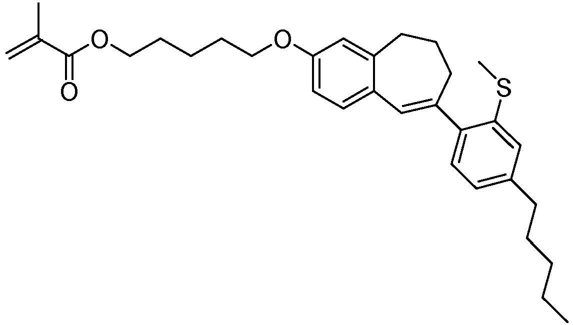 | A-087 |
| 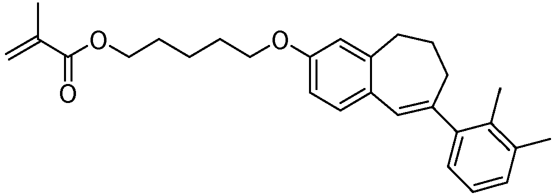 | A-088 |
| 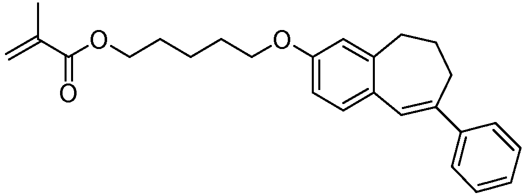 | A-089 |
| 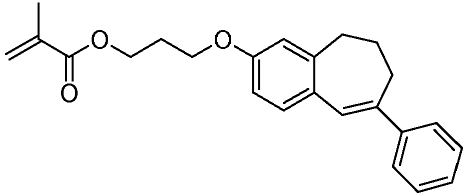 | A-090 |
| 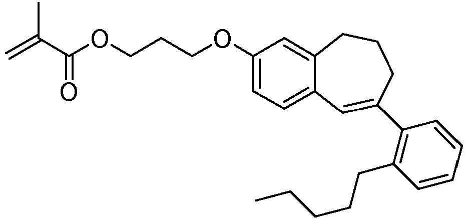 | A-091 |
| 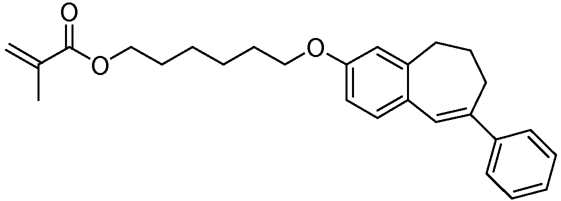 | A-092 |
| 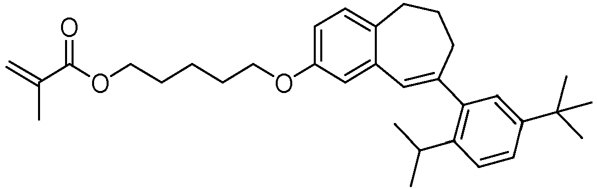 | A-093 |
| 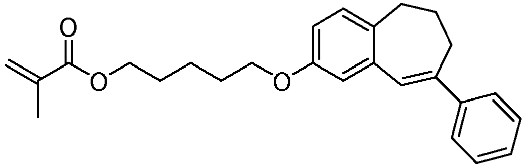 | A-094 |
| 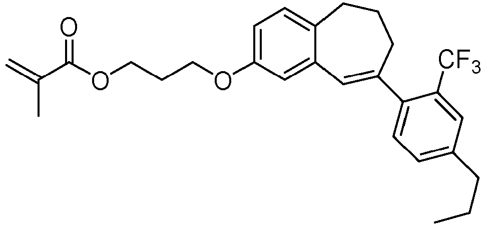 | A-095 |
| 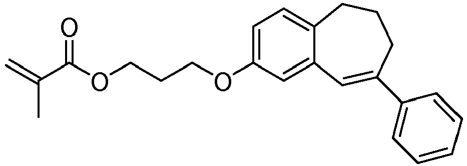 | A-096 |
| 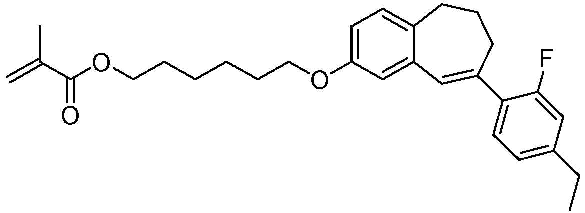 | A-097 |
| 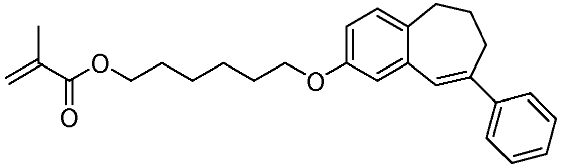 | A-098 |
| 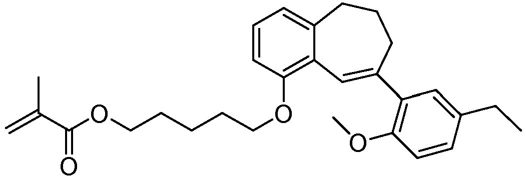 | A-099 |
| 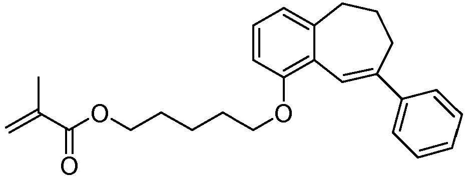 | A-100 |
| 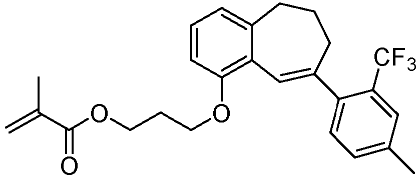 | A-101 |
| 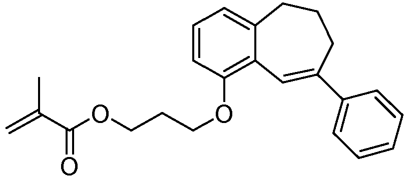 | A-102 |
| 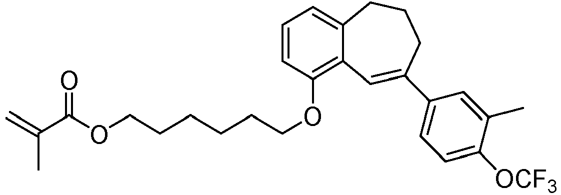 | A-103 |
| 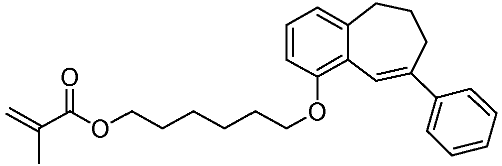 | A-104 |
| 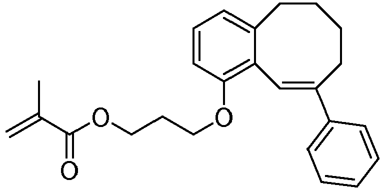 | A-105 |
| 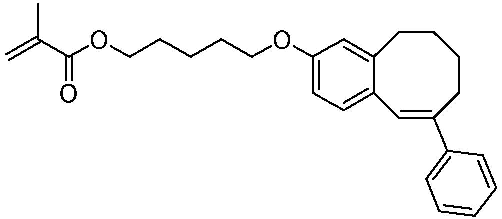 | A-106 |
| 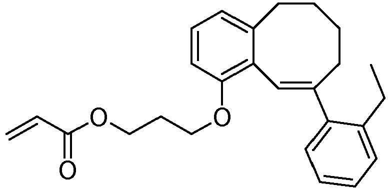 | A-107 |
| 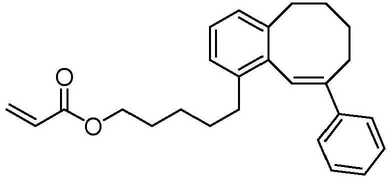 | A-108 |
| 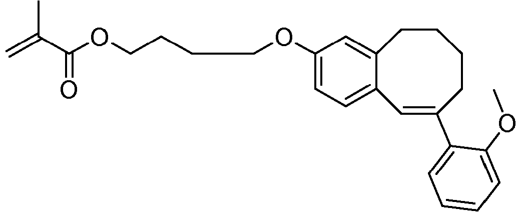 | A-109 |
| 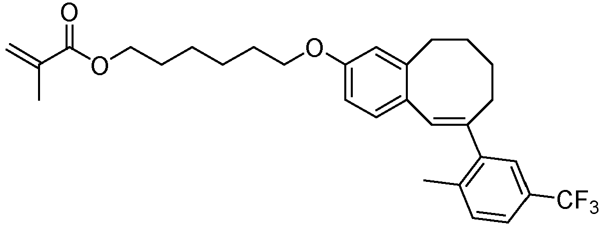 | A-110 |
| 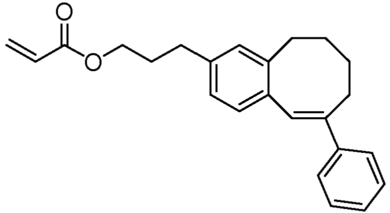 | A-111 |
| 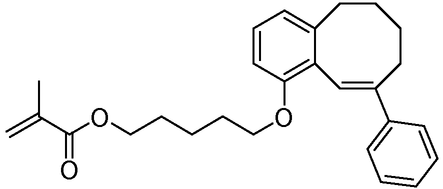 | A-112 |
| 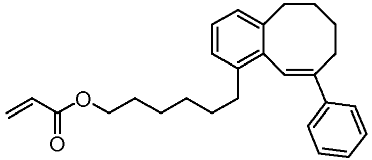 | A-113 |
| 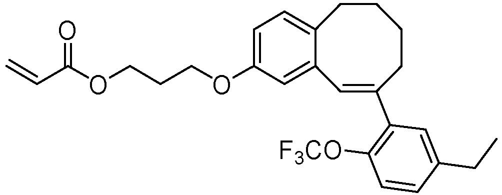 | A-114 |
| 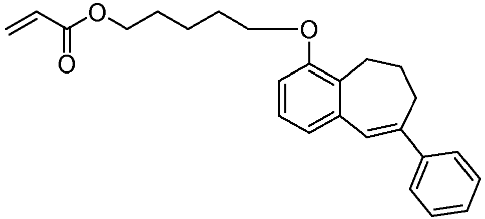 | A-115 |
| 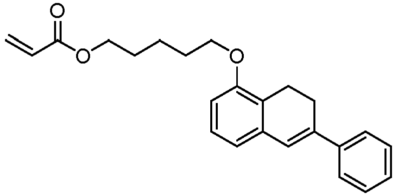 | A-116 |
| 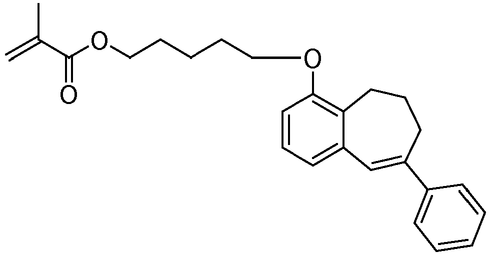 | A-117 |
| 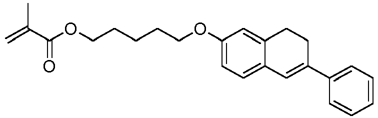 | A-118 |
| 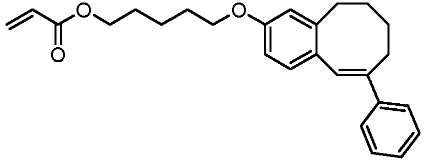 | A-119 |
| 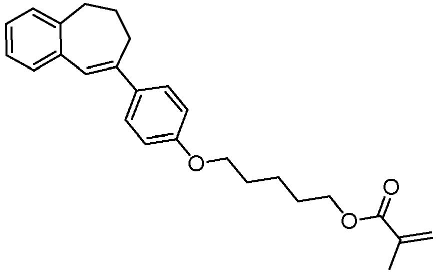 | A-120 |
| 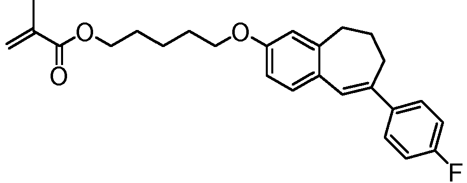 | A-121 |
| 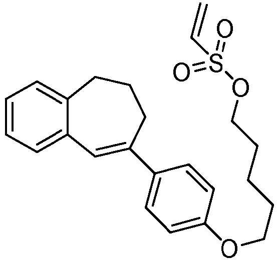 | A-122 |
| 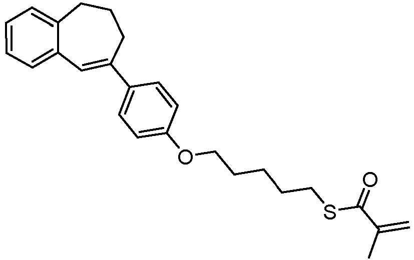 | A-123 |
| 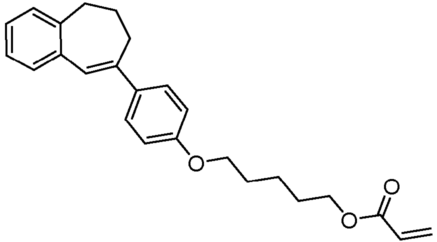 | A-124 |
| 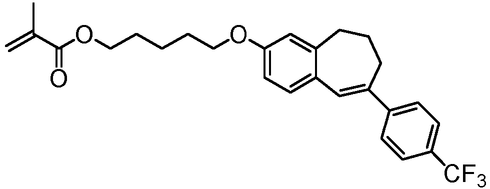 | A-125 |
| 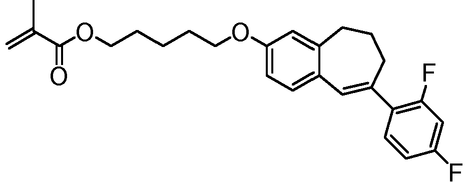 | A-126 |
| 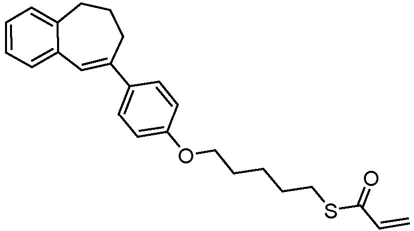 | A-127 |
| 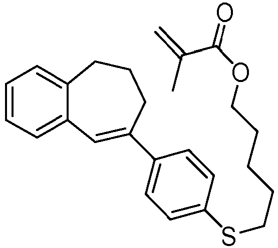 | A-128 |
| 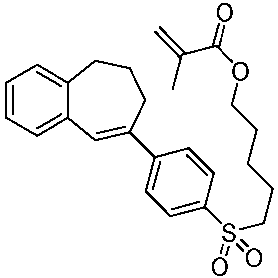 | A-129 |
| 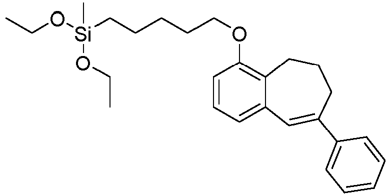 | A-130 |
| 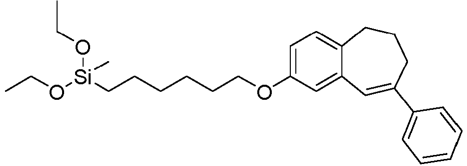 | A-131 |
| 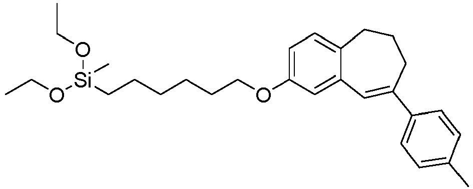 | A-132 |
| 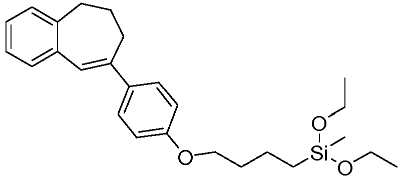 | A-133 |
| 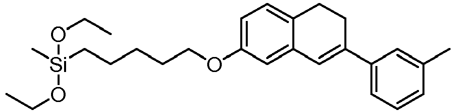 | A-134 |
| 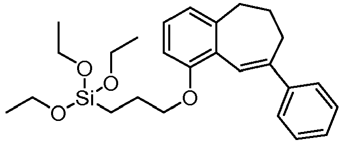 | A-135 |
| 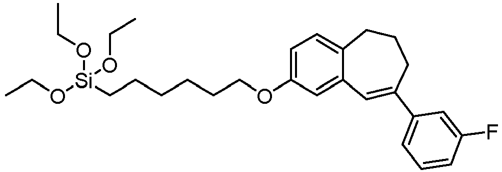 | A-136 |
| 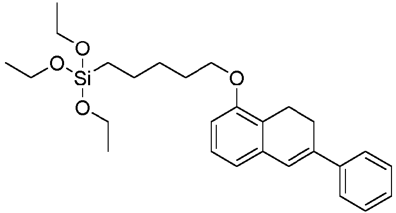 | A-137 |
| 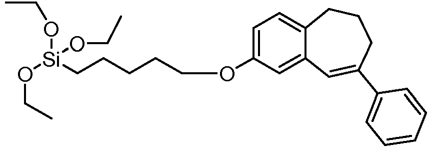 | A-138 |
| 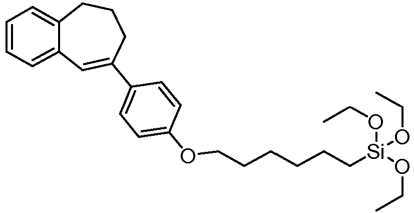 | A-139 |
| 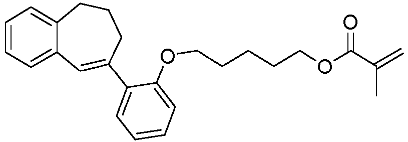 | A-140 |
| 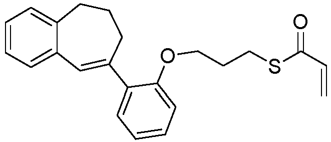 | A-141 |
| 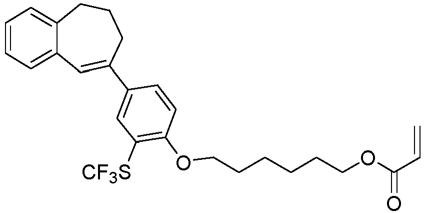 | A-142 |
| 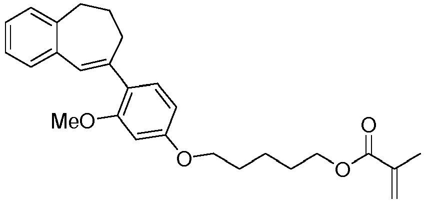 | A-143 |
| 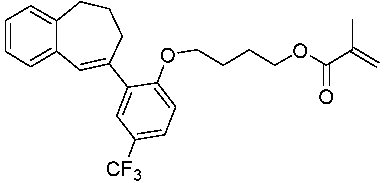 | A-144 |
| 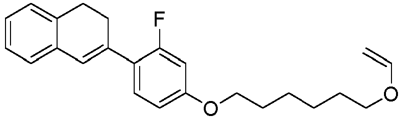 | A-145 |
| 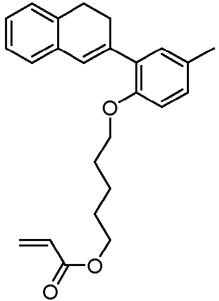 | A-146 |
| 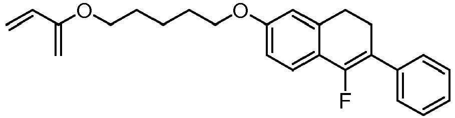 | A-147 |
| 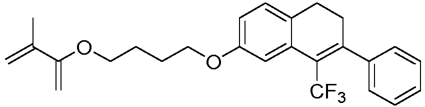 | A-148 |
| 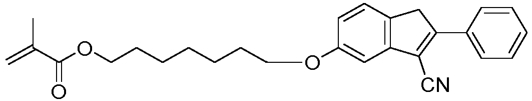 | A-149 |
| 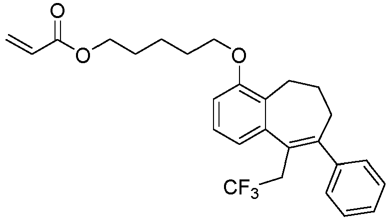 | A-150 |
| 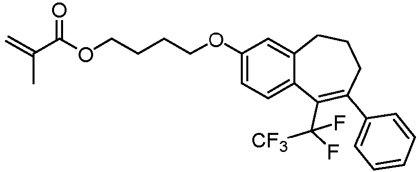 | A-151 |
| 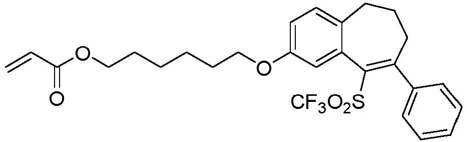 | A-152 |
| 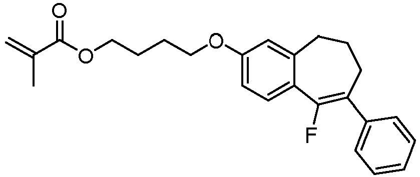 | A-153 |
| 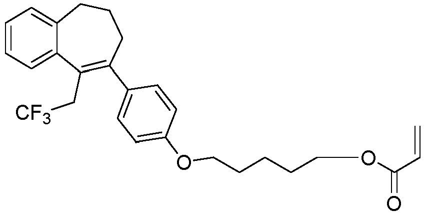 | A-154 |
| 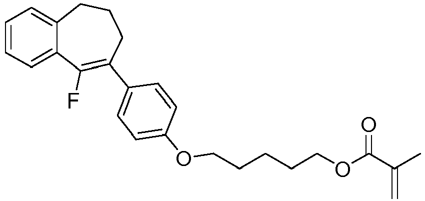 | A-155 |
| 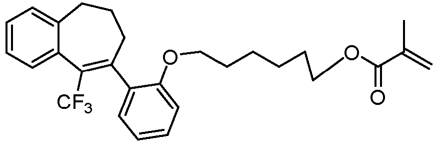 | A-156 |
| 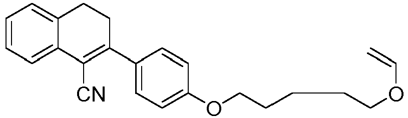 | A-157 |
| 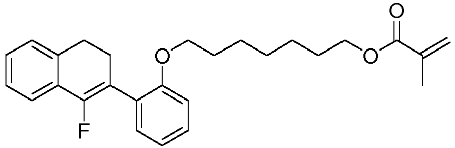 | A-158 |
| 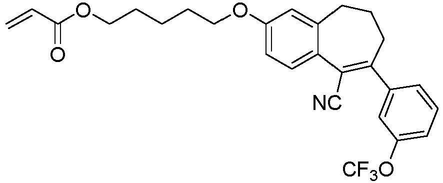 | A-159 |
| 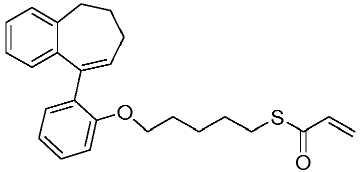 | A-160 |
| 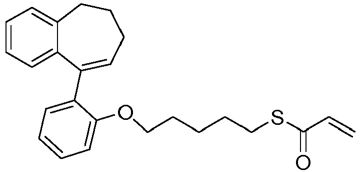 | A-161 |
| 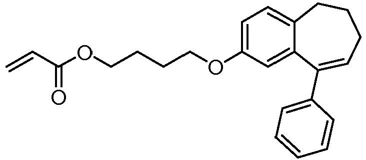 | A-162 |
| 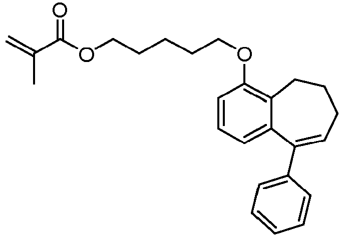 | A-163 |
| 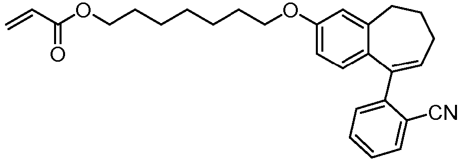 | A-164 |
| 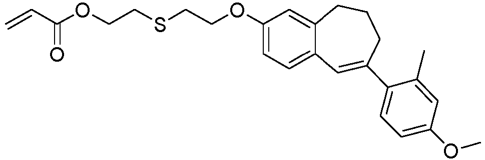 | A-165 |
| 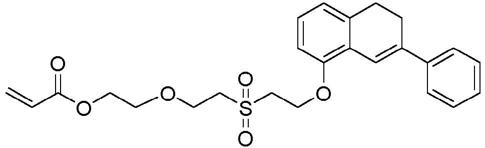 | A-166 |
| 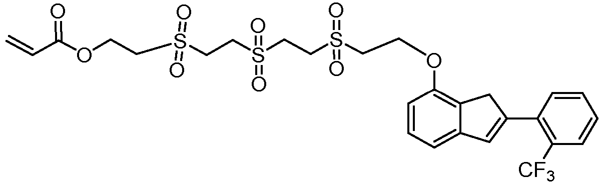 | A-167 |
| 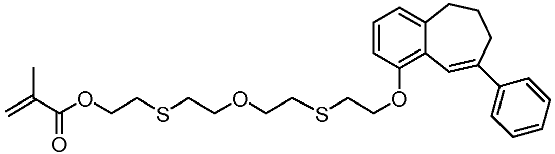 | A-168 |
| 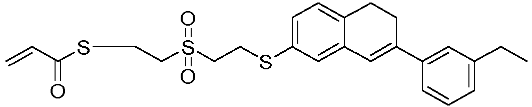 | A-169 |
| 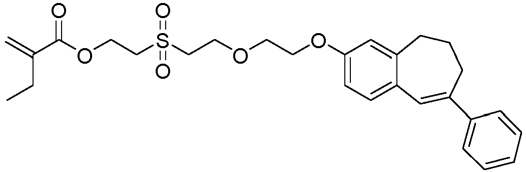 | A-170 |
| 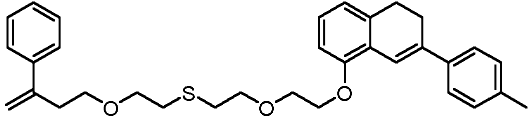 | A-171 |
| 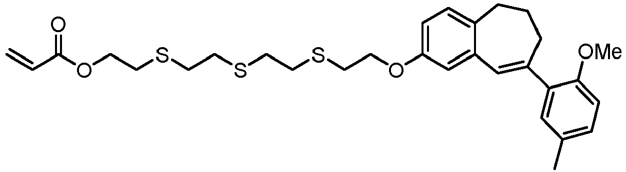 | A-172 |
| 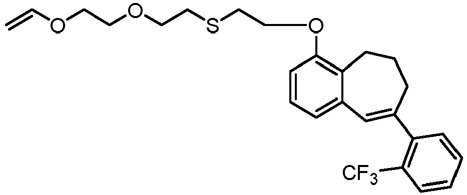 | A-173 |
| 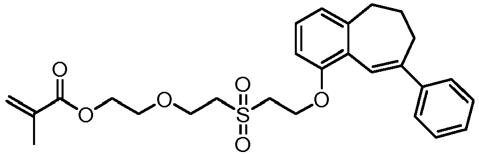 | A-174 |
| 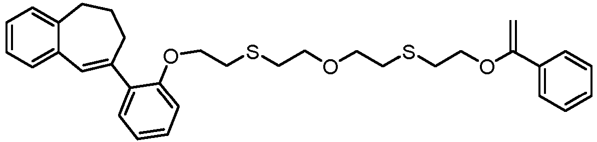 | A-175 |
| 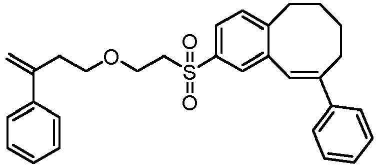 | A-176 |
| 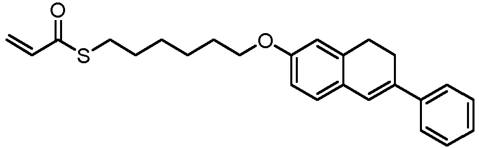 | A-177 |
| 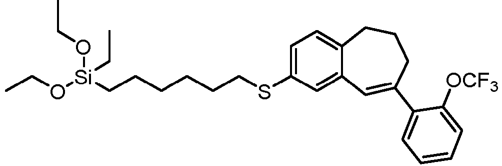 | A-178 |
| 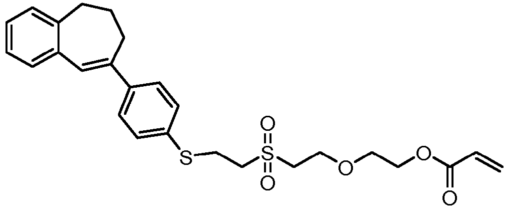 | A-179 |
| 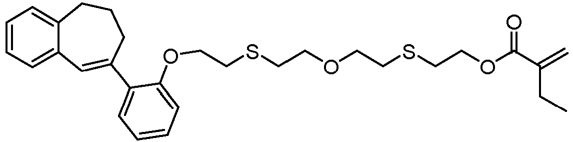 | A-180. |

Preferred examples for compounds/monomers of formulae (I), (I#), (1-1), (I-2), (I-2H), (I-3), (I-3-H), (I-4), (I-4-H), (I'), (I'-a), (I'-b), (I'-c), (I'-d), (I'-1), (I'-2), (I'-2-H), (I'-3), (I'-3-H), (I'-4), (I'-4-H) (I"), (I"-1), (I"-2), (I"-2-H), (I"-3), (I"-3-H), (I"-4) and/or (I"-4-H) are the following compounds as shown in table 1-1.

**Table 1-1:**

| | |
|---|---|
| 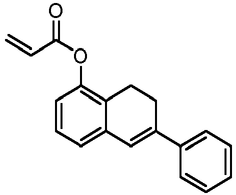 | A-021 |
| 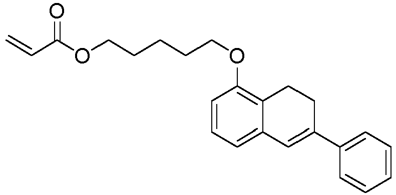 | A-116 |
| 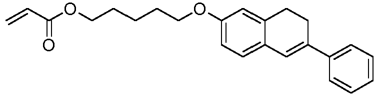 | A-038 |
| 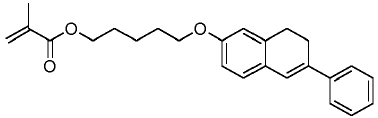 | A-118 |
| 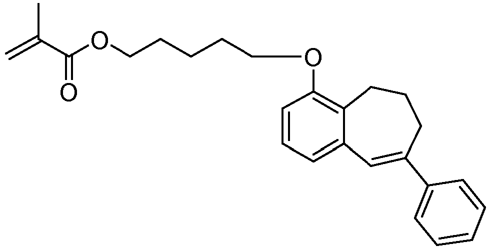 | A-117 |
| 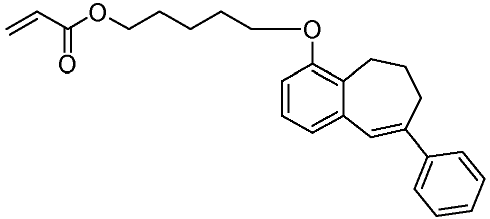 | A-115 |
| 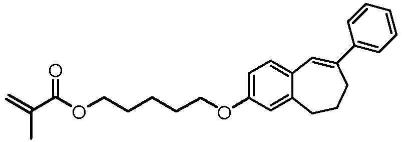 | A-089 |
| 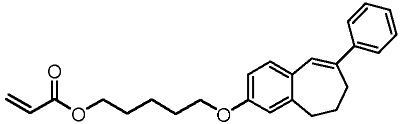 | A-062 |
| 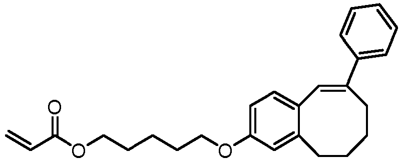 | A-119 |
| 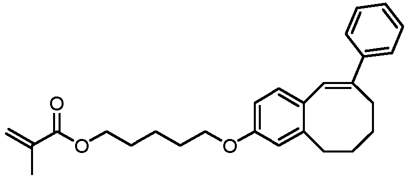 | A-106 |
| 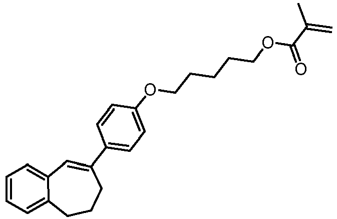 | A-120 |
| 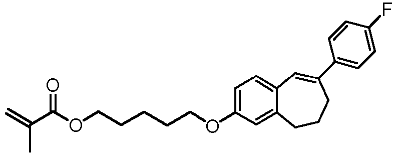 | A-121 |
| 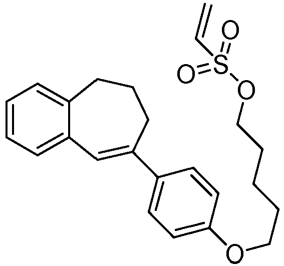 | A-122 |
| 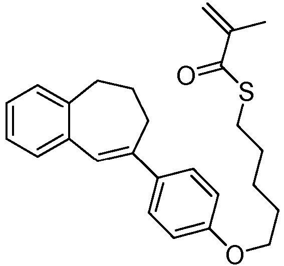 | A-123 |
| 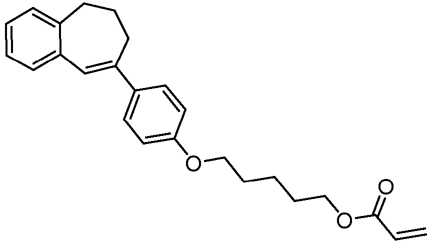 | A-124 |
| 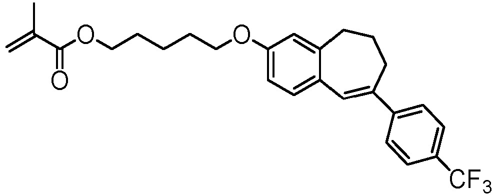 | A-125 |
| 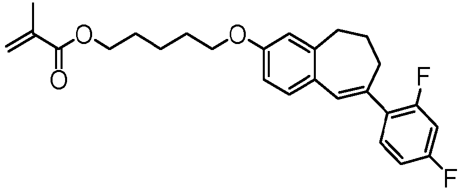 | A-126 |
| 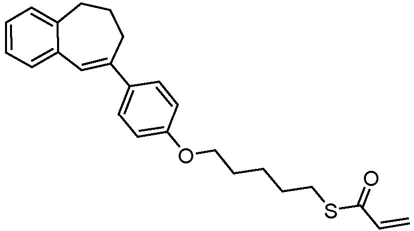 | A-127 |
| 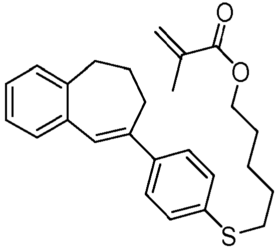 | A-128 |
| 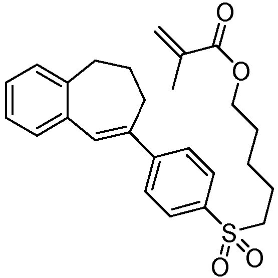 | A-129. |

The compounds of the present application may be synthesized by methods well known to the skilled person. Preferably, all syntheses are carried out under an inert atmosphere using dried solvents.

An exemplary reaction sequence is shown in Scheme 1 for the compounds of formula (I#) where A is CH₂, m is 1, Y is O, -R₂- is -(CH₂)ₒ-, m1 is 0, R₆ and R₇ are H and R₁ is an alkenyl of formula (4-2) and R₃, R₄, R₅, R₈, R₉, R' and n1 have a meaning as described or preferably described before.

In all schemes, Me is methyl.

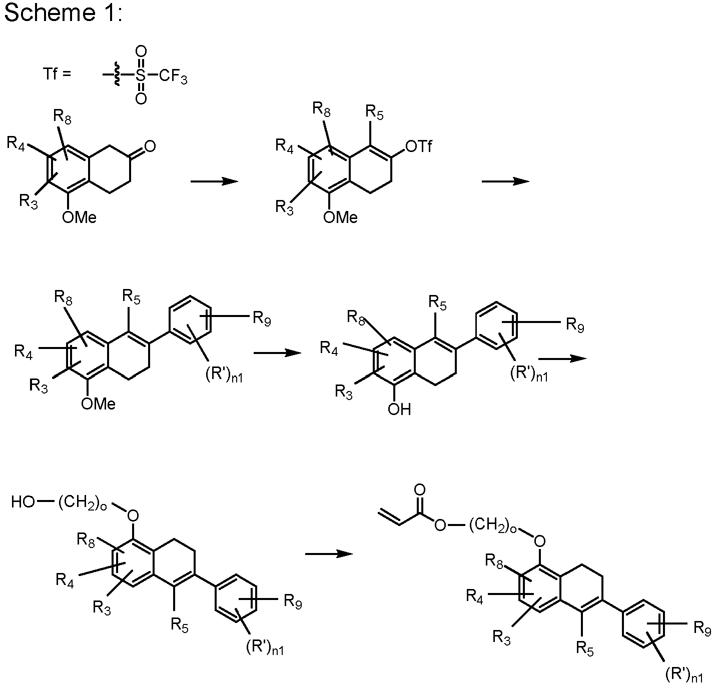

The first type of reaction is the formation of a stable enol equivalent.

The second type of reaction is a Suzuki reaction.

The third type of reaction is a methoxy-deprotection reaction.

The fourth type of reaction is a Williamson ether synthesis reaction.

The fifth type of reaction is an esterification reaction.

All these types of reaction and their reaction conditions are well known to a skilled person and can be easily optimized for the specific starting materials forming the compounds of formula (I). More details can be found in the experimental section.

A representative synthesis according to Scheme 1 is described in Scheme 1-1.

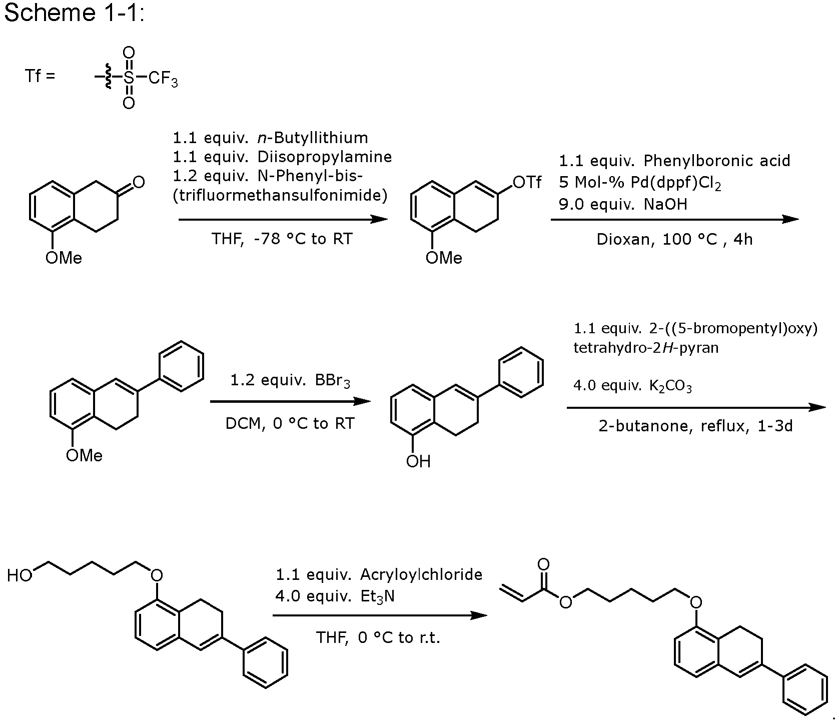

Another exemplary reaction sequence is shown in Scheme 2 for the compounds of formula (I#) where A is CH₂, m is 1, Y is a bond, -R₂- is - (CH₂)ₒ-, m1 is 0, R₆ and R₇ are H and R₁ is an alkenyl of formula (4-2) and R₃, R₄, R₅, R₈, R₉, R' and n1 have a meaning as described or preferably described before.

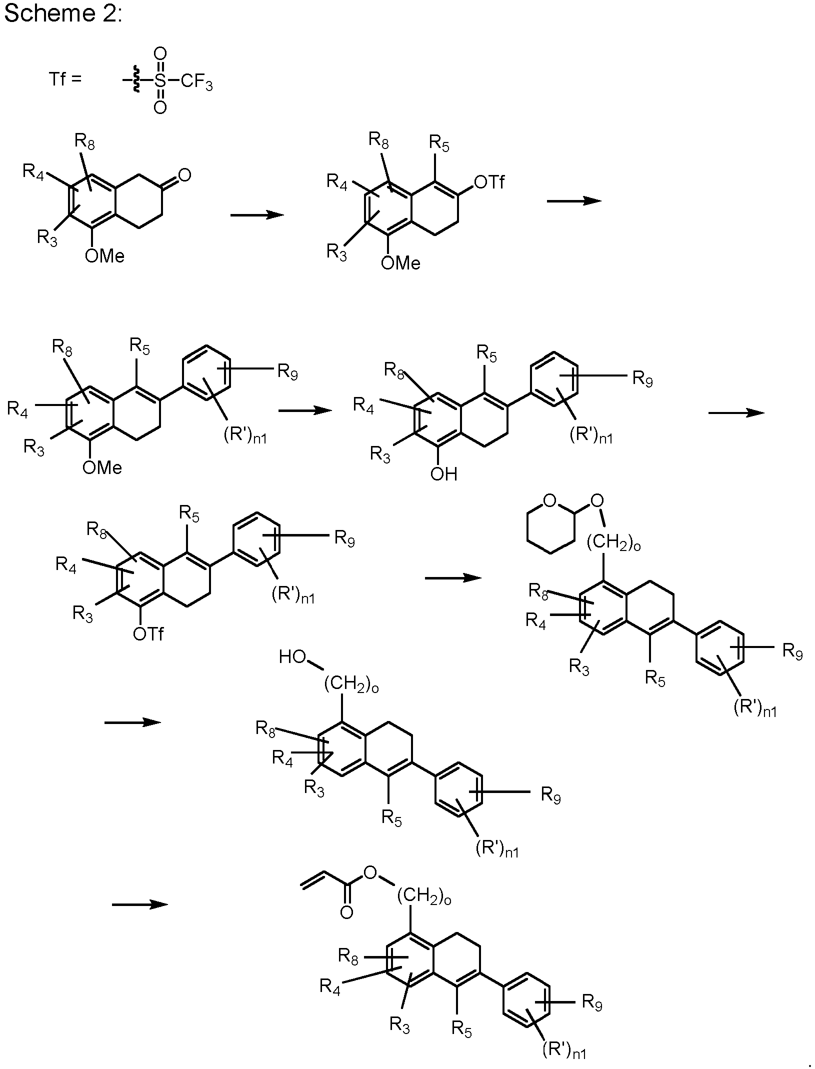

The first type of reaction is the formation of a stable enol equivalent.

The second type of reaction is Suzuki reaction.

The third type of reaction is a methoxy-deprotection reaction.

The fourth type of reaction is triflation reaction.

The fifth type of reaction is a cross-coupling reaction.

The sixth type of reaction is an acid-catalyzed deprotection reaction. The seventh type of reaction is an esterification reaction. All these types of reaction and their reaction conditions are well known to a skilled person and can be easily optimized for the specific starting materials forming the compounds of formula (I). More details can be found in the experimental section.

A representative synthesis according to Scheme 1 is described in Scheme 2-1.

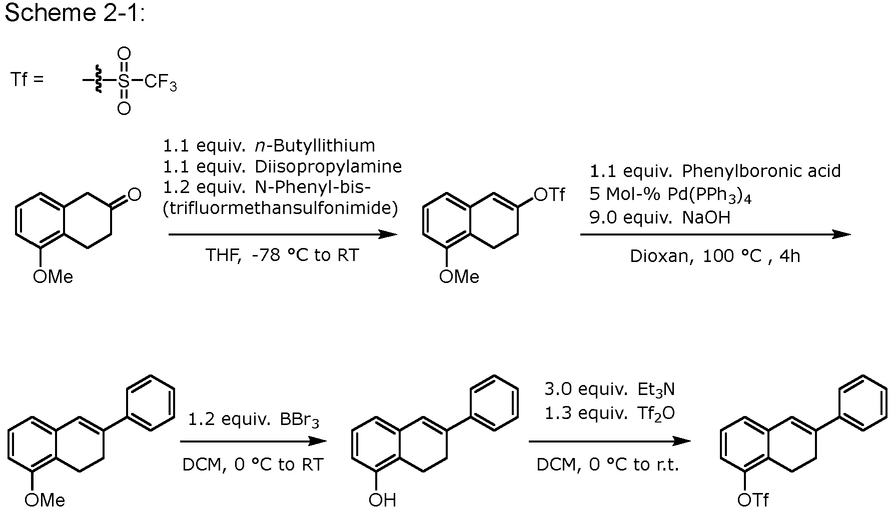

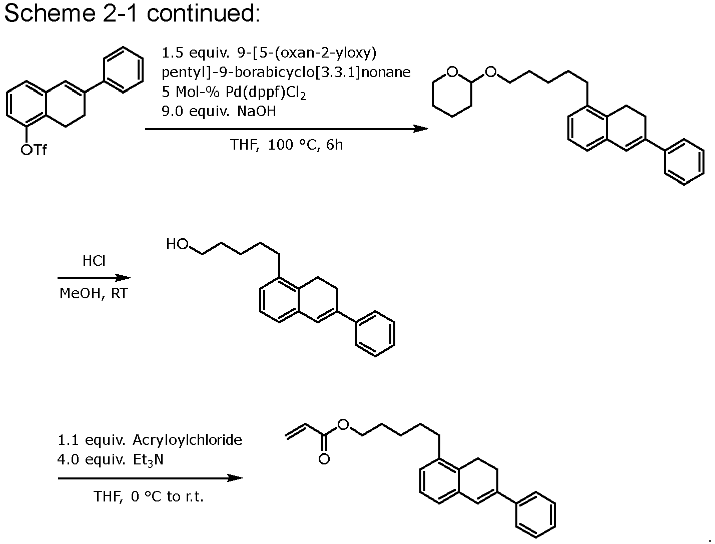

Another exemplary reaction sequence is shown in Scheme 3 for the compounds of formula (I#) where A is CH₂, m is 2, Y is O, -R₂- is -(CH₂)ₒ-, m1 is 0, R₆ and R₇ are H and R₁ is an alkenyl of formula (4-2) and R₃, R₄, R₅, R₈, R₉, R' and n1 have a meaning as described or preferably described before.

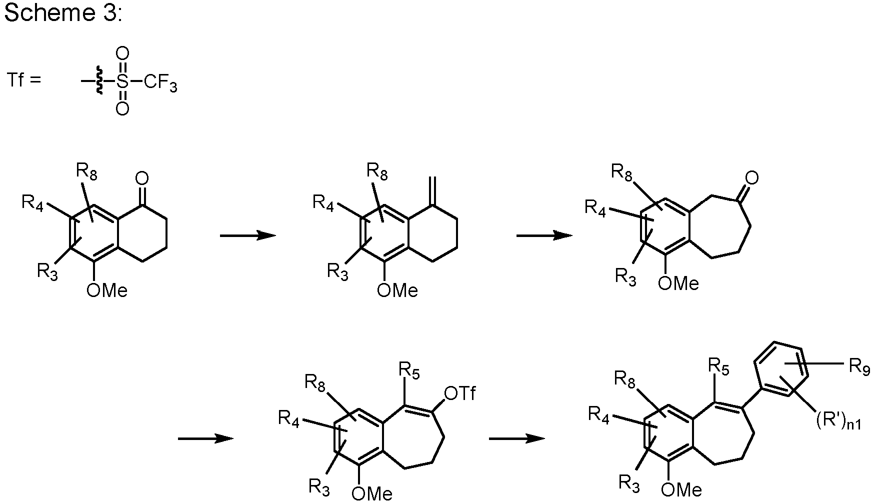

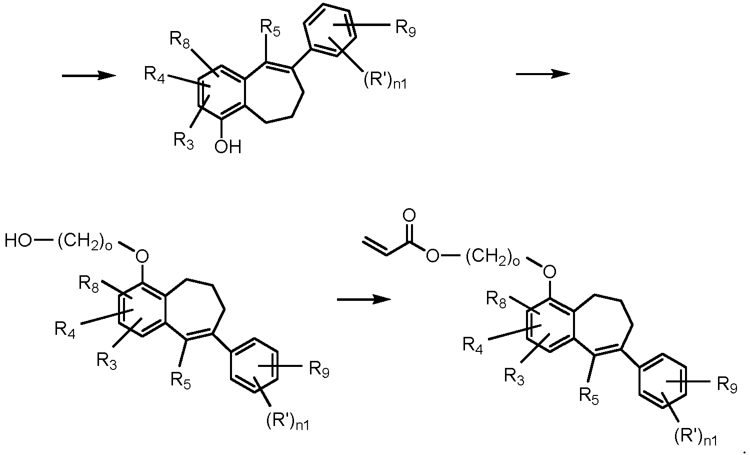

The first type of reaction is a Wittig reaction.

The second type of reaction is a hypervalent iodine-mediated ring-expansion reaction. The third type of reaction is the formation of a stable enol equivalent. The fourth type of reaction is a Suzuki reaction.

The fifth type of reaction is a methoxy-deprotection reaction.

The sixth type of reaction is an esterification reaction.

All these types of reaction and their reaction conditions are well known to a skilled person and can be easily optimized for the specific starting materials forming the compounds of formula (I). More details can be found in the experimental section.

A representative synthesis according to Scheme 3 is described in Scheme 3-1.

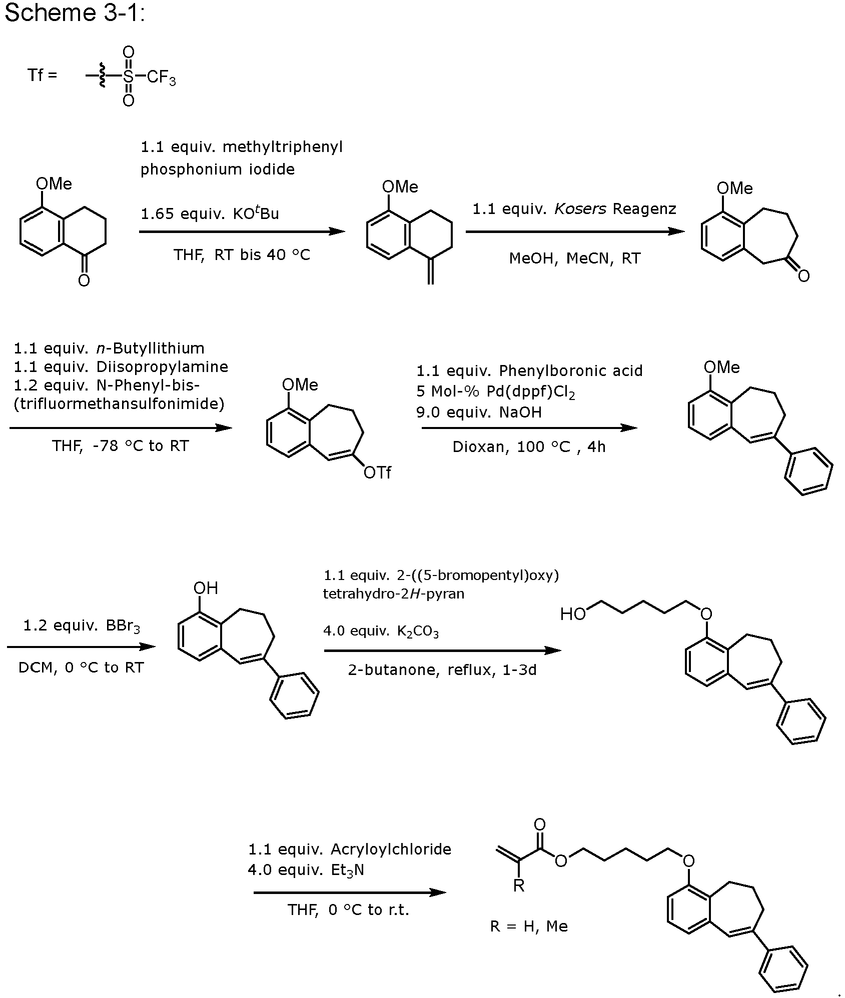

Another exemplary reaction sequence is shown in Scheme 4 for the compounds of formula (I#) where A is CH₂, m is 2, Y is a bond, -R₂- is - (CH₂)ₒ-, m1 is 0, R₆ and R₇ are H and R₁ is an alkenyl of formula (4-2) and R₃, R₄, R₅, R₈, R₉, R' and n1 have a meaning as described or preferably described before.

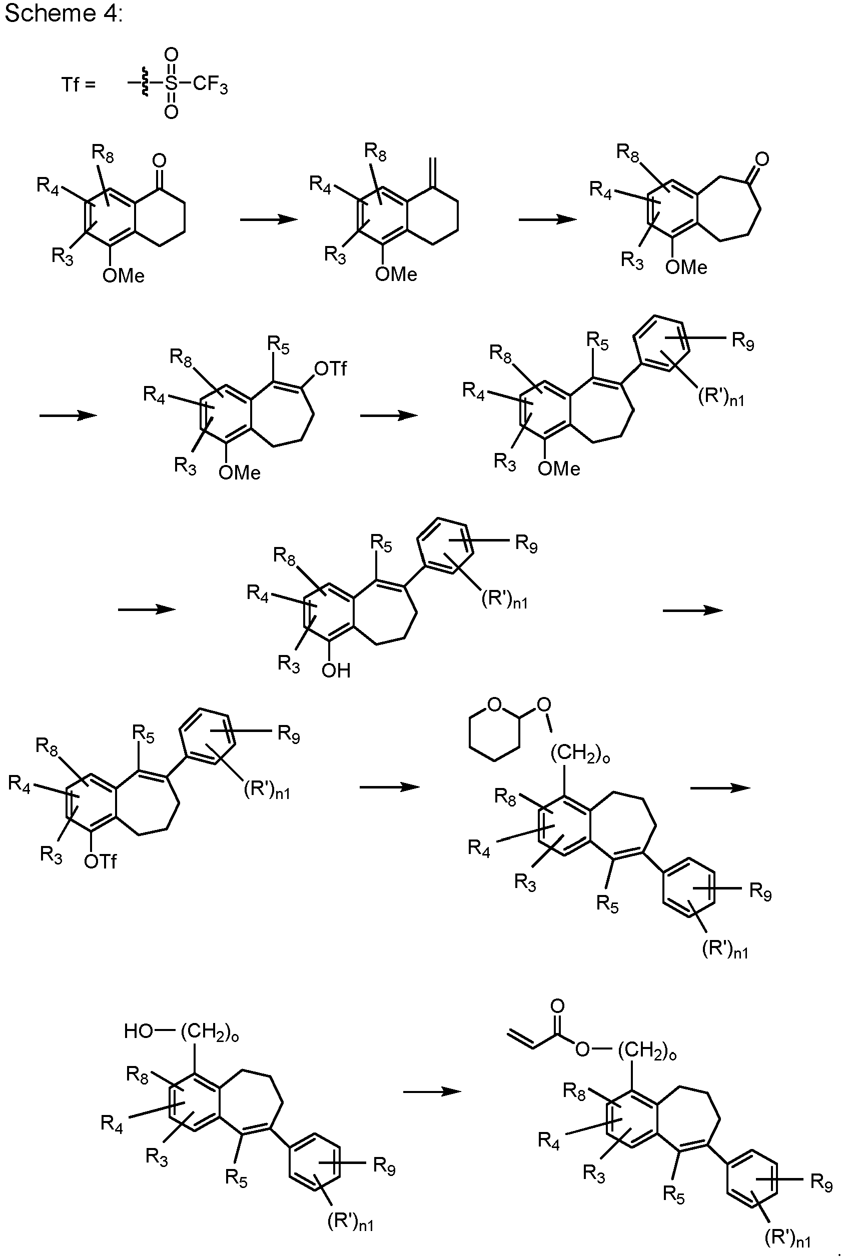

The first type of reaction is a Wittig reaction.

The second type of reaction is a hypervalent iodine-mediated ring-expansion reaction. The third type of reaction is the formation of a stable enol equivalent. The fourth type of reaction is a Suzuki reaction.

The fifth type of reaction is a methoxy-deprotection reaction. The sixth type of reaction is a triflation reaction. The seventh type of reaction is a cross-coupling reaction. The eight type of reaction is an acid-catalyzed deprotection reaction. The ninth type of reaction is an esterification reaction. All these types of reaction and their reaction conditions are well known to a skilled person and can be easily optimized for the specific starting materials forming the compounds of formula (I). More details can be found in the experimental section.

A representative synthesis according to Scheme 4 is described in Scheme

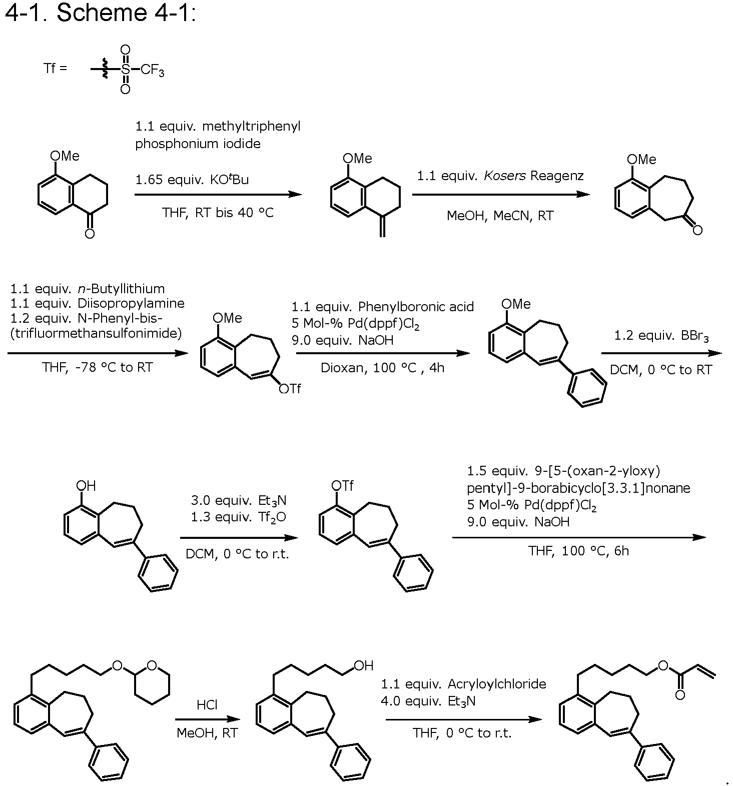

Another exemplary reaction sequence is shown in Scheme 5-1 for the compounds of formula (I#) where A is CH₂, m is 2, Y is O, -R₂- is -(CH₂)₅-, m1 is 1, n is 0, R₆ and R₇ are H and R₉ is an alkenyl of formula (4-11) or (4-12) and R₃, R₄, R₅, R₈, R₉, R' and n1 have a meaning as described or preferably described before.

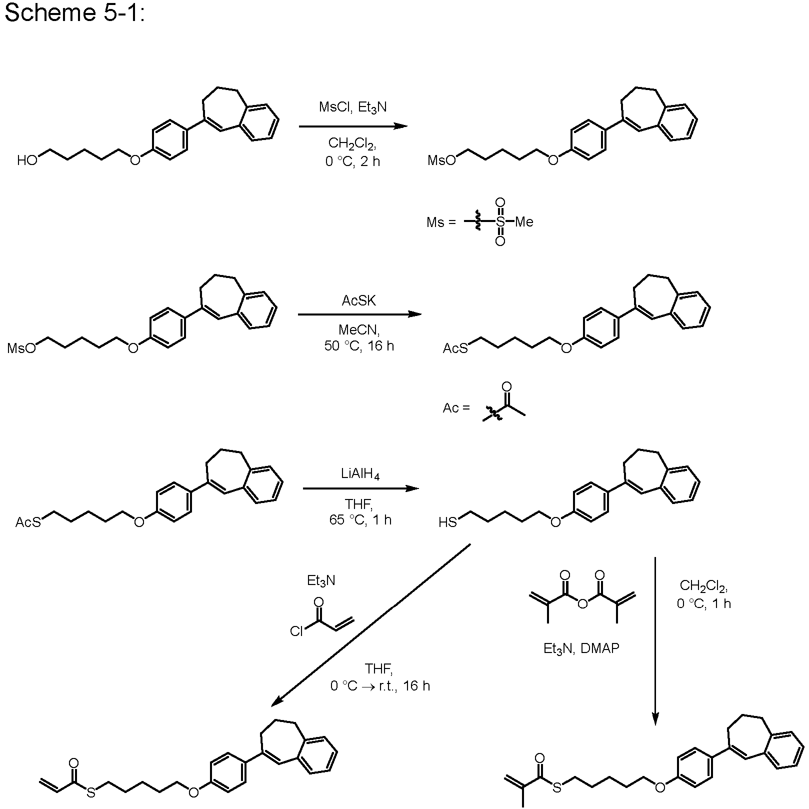

Another exemplary reaction sequence is shown in Scheme 6 for the compounds of formula (I#) where A is CH₂, m is 2, Y is O, -R₂- is -(CH₂)ₒ-, m1 is 0, n is 1, R₆ and R₇ are H, R₉ is H or R' and R₁ is a methyl-diethoxysilyl group and R₃, R₄, R₅, R₈, R' and n1 have a meaning as described or preferably described before.

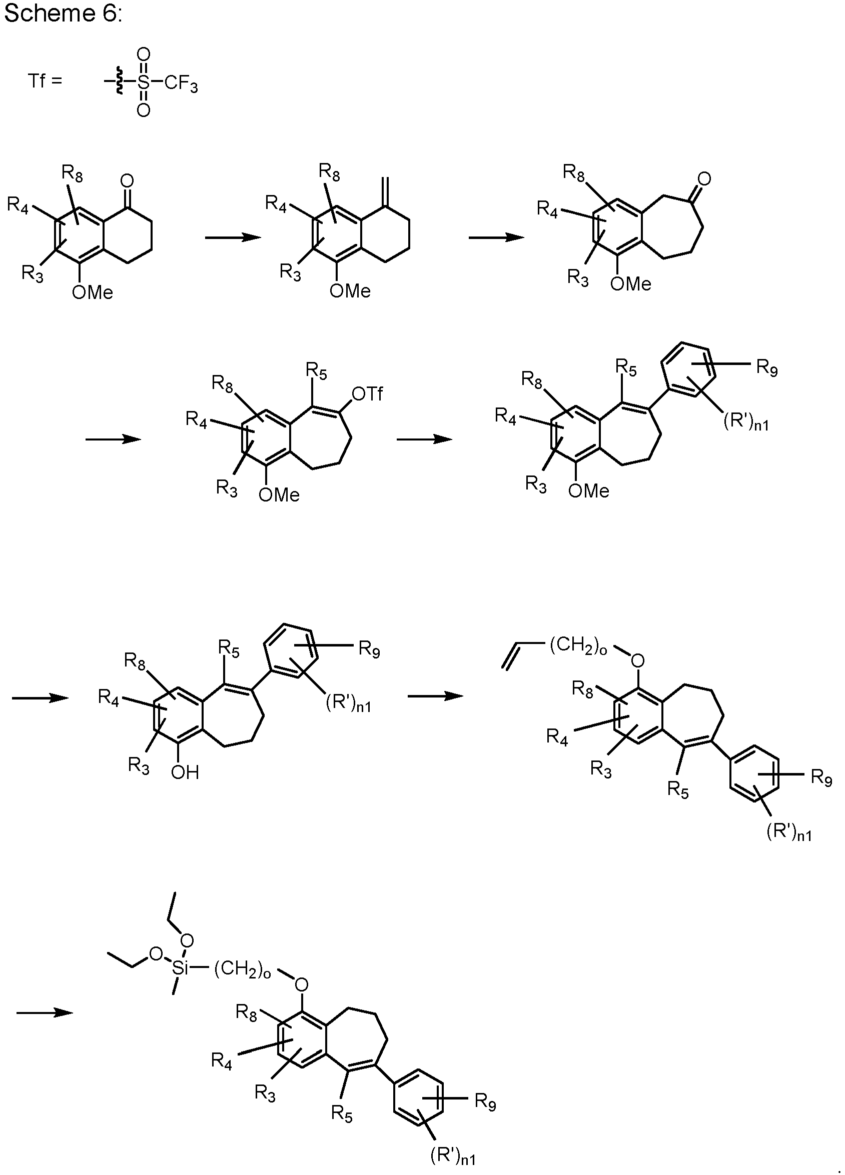

The first type of reaction is a Wittig reaction. The second type of reaction is a hypervalent iodine-mediated ring-expansion reaction. The third type of reaction is the formation of a stable enol equivalent. The fourth type of reaction is a Suzuki reaction. The fifth type of reaction is a methoxy-deprotection reaction. The sixth type of reaction is a Mitsunobu reaction. The seventh type is a hydrosylilation reaction.

A representative synthesis according to Scheme 6 is described in Scheme 6-1.

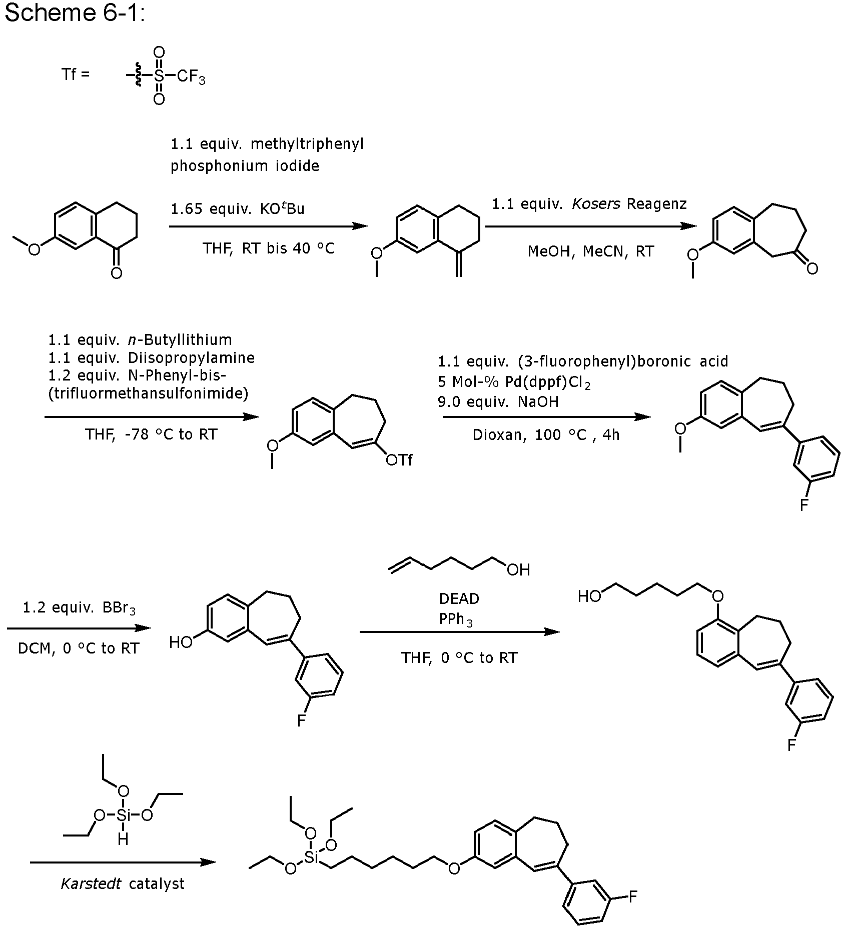

As described before, the compounds/monomers of formula (I), (I#), (1-1), (I-2), (I-2H), (I-3), (I-3-H), (I-4), (I-4-H), (I'), (I'-a), (I'-b), (I'-c), (I'-d), (I'-1), (I'-2), (I'-2-H), (I'-3), (I'-3-H), (I'-4), (I'-4-H) (I"), (I"-1), (I"-2), (I"-2-H), (I"-3), (I"-3-H), (I"-4) and (I"-4-H) as described before or preferably described before contain a polymerizable group and are predestinated as monomers for an oligomerization or a polymerization.

The invention is therefore further directed to an oligomer, polymer or copolymer comprising at least one polymerized compound of formulae (I), (I#), (1-1), (I-2), (I-2H), (I-3), (I-3-H), (I-4), (I-4-H), (I'), (I'-a), (I'-b), (I'-c), (I'-d), (I'-1), (I'-2), (I'-2-H), (I'-3), (I'-3-H), (I'-4), (I'-4-H) (I"), (I"-1), (I"-2), (I"-2-H), (I"-3), (I"-3-H), (I"-4) or (I"-4-H) as described before or preferably described before where oligomers, polymers and copolymers of 2-(4-vinylphenyl)indene and 2-[4-(2-propen-1-yl)phenyl-1H-indene are excluded.

The term "polymer" generally means a molecule of high relative molecular mass, the structure of which essentially comprises the multiple repetition of units derived, actually or conceptually, from molecules of low relative molecular mass (PAC, 1996, 68, 2291). The term "polymer" includes homopolymers and copolymers if not mentioned otherwise within the description. The term "oligomer" generally means a molecule of intermediate relative molecular mass, the structure of which essentially comprises a small plurality of units derived, actually or conceptually, from molecules of lower relative molecular mass (PAC, 1996, 68, 2291). In a preferred sense according to the present invention a polymer means a compound having ≥ 30 repeating units, and an oligomer means a compound with > 1 and < 30 repeating units.

Above and below, in formulae showing a polymer, an oligomer, a compound of formulae (I), (I#), (I-1), (I-2), (I-2H), (I-3), (I-3-H), (I-4), (I-4-H), (I'), (I'-a), (I'-b), (I'-c), (I'-d), (I'-1), (I'-2), (I'-2-H), (I'-3), (I'-3-H), (I'-4), (I'-4-H) (I"), (I"-1), (I"-2), (I"-2-H), (I"-3), (I"-3-H), (I"-4) or (I"-4-H) or a monomeric unit or a polymer formed from a compound of formulae (I), (I#), (1-1), (I-2), (I-2H), (I-3), (I-3-H), (I-4), (I-4-H), (I'), (I'-a), (I'-b), (I'-c), (I'-d), (I'-1), (I'-2), (I'-2-H), (I'-3), (I'-3-H), (I'-4), (I'-4-H) (I"), (I"-1), (I"-2), (I"-2-H), (I"-3), (I"-3-H), (I"-4) or (I"-4-H), an asterisk ("*") denotes a linkage to the adjacent repeating unit in the polymer chain or oligomer chain or to a terminal end group.

Suitable terminal end groups are known to the skilled artisan and depend on the polymerization method used.

The terms "repeating unit" and "monomeric unit" mean the constitutional repeating unit (CRU), which is the smallest constitutional unit the repetition of which constitutes a regular macromolecule, a regular oligomer molecule, a regular block or a regular chain (PAC, 1996, 68, 2291).

Unless stated otherwise, the molecular weight is given as the number average molecular weight Mₙ or weight average molecular weight M_{W}, which is determined by gel permeation chromatography (GPC) against polystyrene standards in eluent solvents such as tetrahydrofuran, trichloromethane (TCM, chloroform), chlorobenzene or 1,2,4-trichlorobenzene. Unless stated otherwise, tetrahydrofuran is used as solvent. The degree of polymerization (n) means the number average degree of polymerization given as n = Mₙ/M_{U}, wherein M_{U} is the molecular weight of the single repeating unit as described in J. M. G. Cowie, Polymers: Chemistry & Physics of Modern Materials, Blackie, Glasgow, 1991.

In the polymers including copolymers according to the present invention, the total number of repeating units n is preferably ≥ 30, very preferably ≥ 100, most preferably ≥ 200, and preferably up to 5000, very preferably up to 3000, most preferably up to 2000, including any combination of the aforementioned lower and upper limits of n.

The polymers of the present invention include homopolymers, statistical copolymers, random copolymers, alternating copolymers and block copolymers, and combinations of the aforementioned.

Throughout the description and claims of this specification, the words "comprise" and "contain" and variations of the words, for example "comprising" and "comprises", mean "including but not limited to", and are not intended to (and do not) exclude other components

Preferably the polymerizable group R₁ forms the regioregular, alternated, regiorandom, statistical, block or random homopolymer or copolymer backbone or is part of the polymer backbone where R₁ has a meaning as described or preferably described before.

Preferably, such oligomer, polymer or copolymer according to the invention comprises a constitutional unit M⁰ based on formulae (I), (I') or (I") 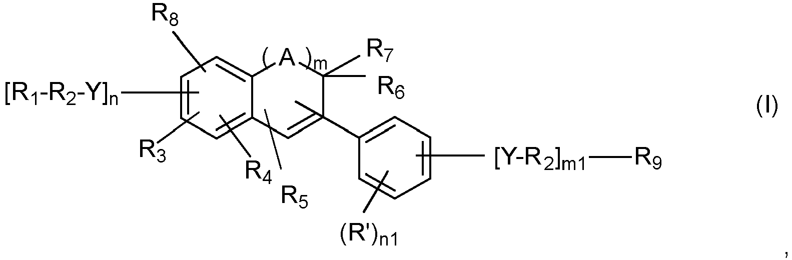 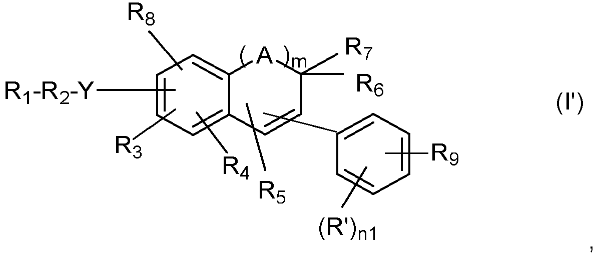 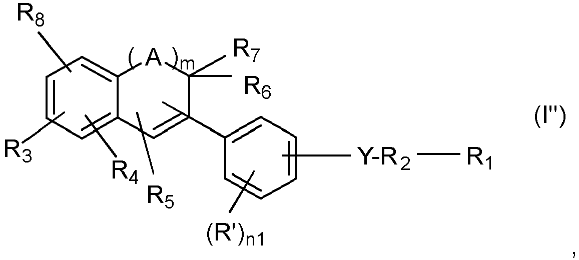 where the polymerizable group R₁ on each occurrence is polymerized and forms the regioregular, alternated, regiorandom, statistical, block or random oligomer or polymer backbone or is part of the copolymer backbone.

The invention is furthermore directed to an ophthalmic device or a precursor article for an ophthalmic device as described before or preferably described below comprising an oligomer, polymer or copolymer comprising a constitutional unit M⁰ based on formulae (I), (I') or (I") as described before or preferably described before where R₁ on each occurrence is polymerized and forms the regioregular, alternated, regiorandom, statistical, block or random oligomer or polymer backbone or is part of the copolymer backbone.

Preferably, such polymerized groups R₁ are of formulae (1-p), (2-p), (3-p) or (4-p) 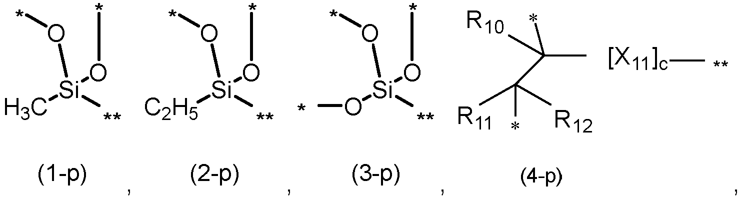 where the asterisk "*" within formulae (1-p) to (4-p) denotes a linkage to the adjacent repeating unit in the polymer chain or oligomer chain or to a terminal end group, the asterisk "**" within formulae (1-p) to (4-p) denotes the linkage to the remainder of formula (I) as described before or preferably described before and R₁₀, R₁₁, R₁₂, X₁₁ and c has a meaning as described before or preferably described before.

The invention is furthermore directed to an ophthalmic device or a precursor article for an ophthalmic device as described before or preferably described below where said polymerized group R₁ is of formulae (1-p), (2-p), (3-p) or (4-p) as described before.

The invention is furthermore directed to an oligomer, polymer or copolymer as described before or preferably described below where said polymerized group R₁ is of formulae (1-p), (2-p), (3-p) or (4-p) as described before.

Particularly preferably, such oligomer, polymer or copolymer according to the invention comprises a constitutional unit M⁰ of formulae (M⁰-I'), (M⁰-I"), (M⁰-I'-1), (M⁰-I"-1), (M⁰-I'-2-H), (M⁰-I"-2-H), (M⁰-I'-3-H), (M⁰-I"-3-H), (M⁰-I'-4-H) or (M⁰-I"-4-H), 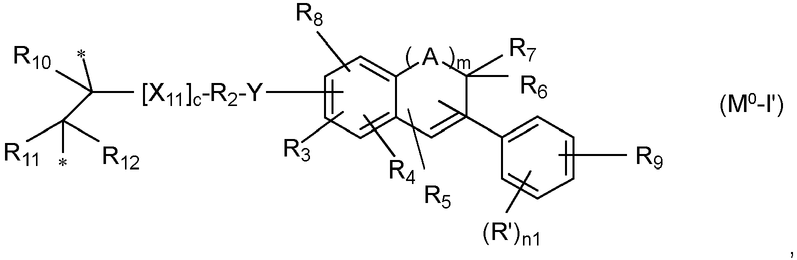 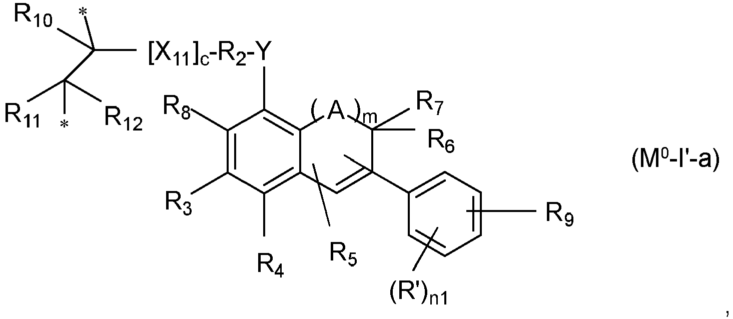 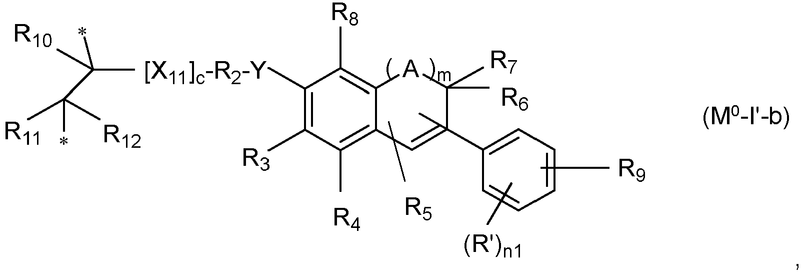 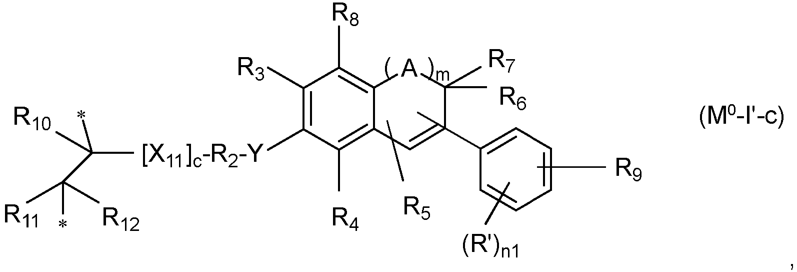 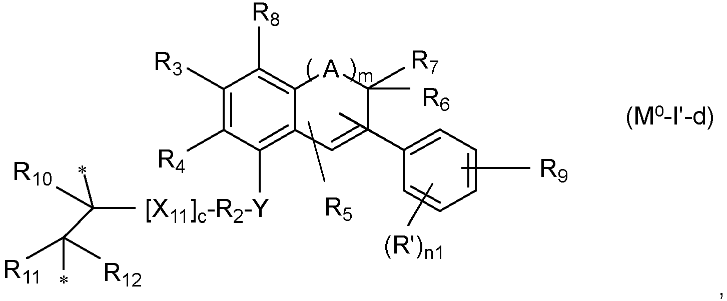 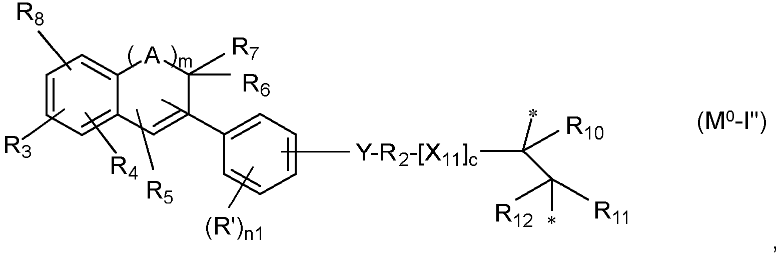 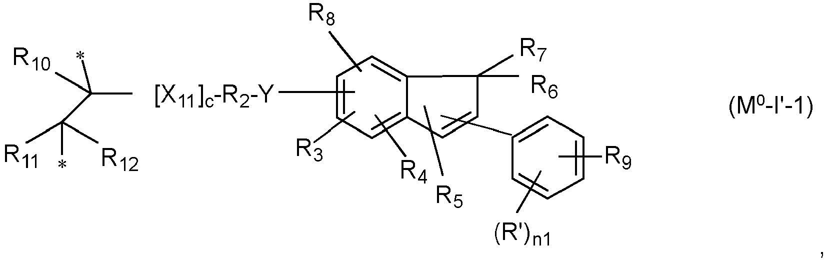 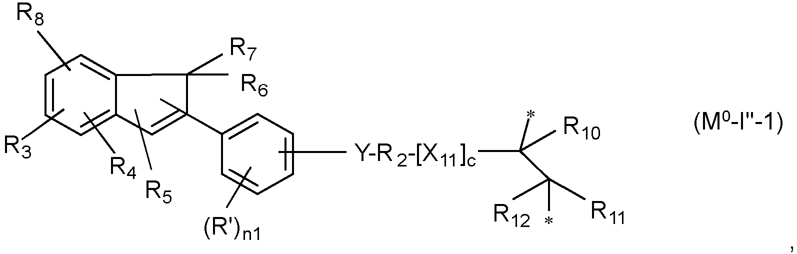 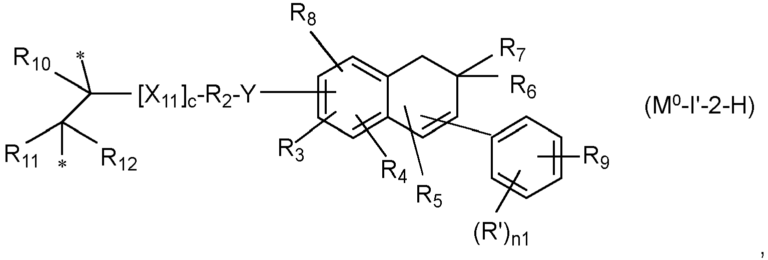 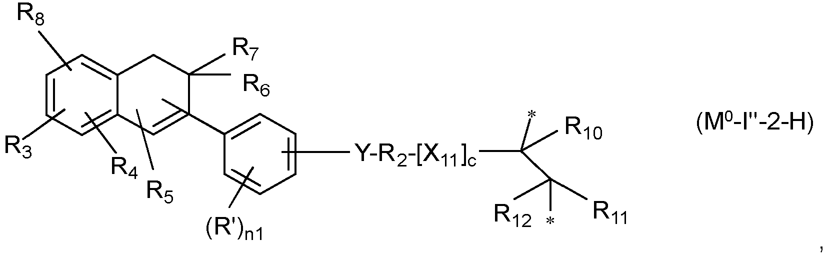 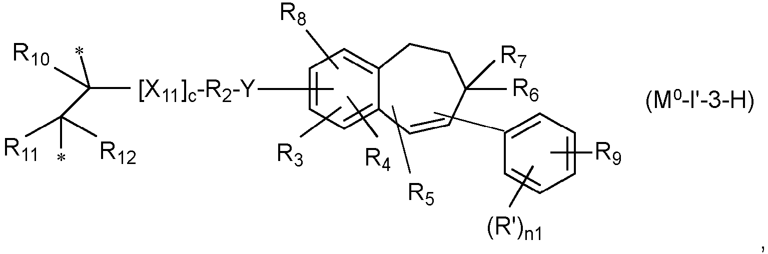 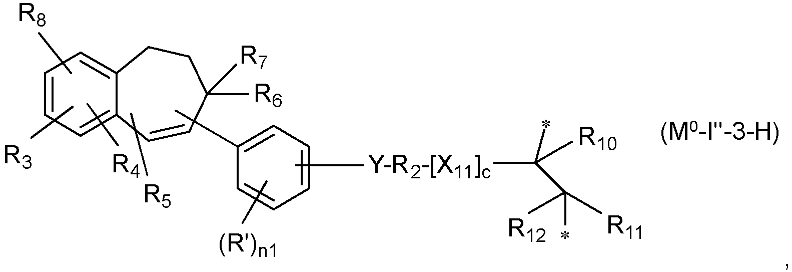 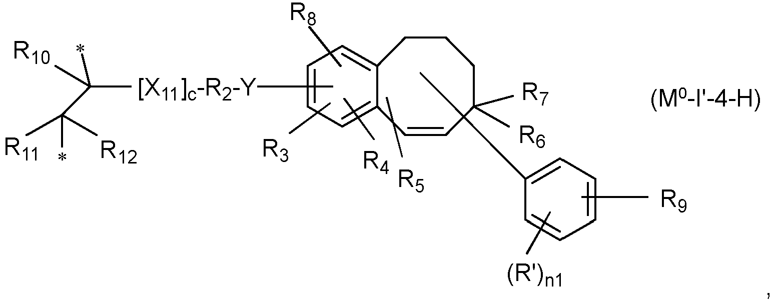 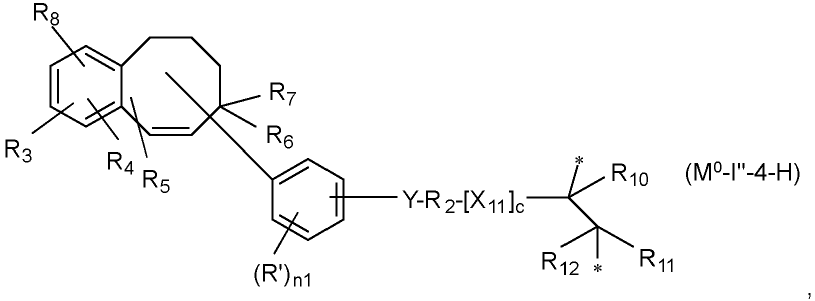 wherein -R₂-, Y, R₃, R₄, R₅, R₆, R₇, R₈, A, m, R', n1, X₁₁, R₁₀, R₁₁, R₁₂ and c have a meaning as described before or preferably described before or below for the compounds of formulae (I), (I#), (I-1), (I-2), (I-2H), (I-3), (I-3-H), (I-4), (I-4-H), (I'), (I'-a), (I'-b), (I'-c), (I'-d), (I'-1), (I'-2), (I'-2-H), (I'-3), (I'-3-H), (I'-4), (I'-4-H) (I"), (I"-1), (I"-2), (I"-2-H), (I"-3), (I"-3-H), (I"-4) and (I"-4-H) and R₉ is H or R' for compounds of formulae (M⁰-I'), (M⁰-I'-1), (M⁰-I'-2-H), (M⁰-I'-3-H) or (M⁰-I'-4-H). Combinations are excluded where two O atoms or an O atom and a S atom are directly linked to each other as known for a skilled artisan in the field of organic chemistry.

The invention is furthermore directed to an ophthalmic device or a precursor article for an ophthalmic device as described before or preferably described below wherein the constitutional unit M⁰ is of formulae (M⁰-I'-a), (M⁰-I'-b), (M⁰-I'-c), (M⁰-I'-d) or (M⁰-I") as described before and where the asterisk "*" denotes at each occurrence a linkage to the adjacent repeating unit in the polymer chain or oligomer chain or to a terminal end group.

Preferably, such oligomer, polymer or copolymer according to the invention comprises a constitutional unit M⁰ of formulae (M⁰-I'), (M⁰-I"), (M⁰-I'-1), (M⁰-I"-1), (M⁰-I'-2-H), (M⁰-I"-2-H), (M⁰-I'-3-H), (M⁰-I"-3-H), (M⁰-I'-4-H) or (M⁰-I"-4-H), as described before, wherein
-R₂- is selected from -(CH₂)₃-, -(CH₂)₄-, -(CH₂)₅-, -(CH₂)₆-, -(CH₂)₇-, -(CH₂)₈-, -(CH₂)₉-, -(CH₂)₁₀-, -(CH₂)₁₁-, -(CH₂)₂-S-(CH₂)₂-, -(CH₂)₂-SO₂-(CH₂)₂-, - (CH₂)-S-(CH₂)₂-O-(CH₂)-, -(CH₂)-SO₂-(CH₂)₂-O-(CH₂)-, -(CH₂)-SO₂-(CH₂)₂-S-(CH₂)-, -(CH₂)-O-(CH₂)₂-S-(CH₂)₂-O-(CH₂)-, -(CH₂)-S-(CH₂)₂-O-(CH₂)₂-S-(CH₂)-, -(CH₂)-SO₂-(CH₂)₂-O-(CH₂)₂-SO₂-(CH₂)-, -(CH₂)-S-(CH₂)₂-S-(CH₂)₂-S-(CH₂)-, -(CH₂)-SO₂-(CH₂)₂-SO₂-(CH₂)₂-SO₂-(CH₂)-, -(CH₂)-O-(CH₂)₂-SO₂-(CH₂)₂-O-(CH₂)-, -(CH₂)₁₂, -(CH₂)₃-(CF₂)-(CH₂)₃-, -(CH₂)-(CF₂)₃-(CH₂)-, - (CH₂)₂-(CF₂)₄-(CH₂)₂-, -(CH₂)-[CH(CF₃)]-(CH₂)-, -(CH₂)-[C(CH₃)CF₃]-(CH₂)-, -(CH₂)-[CH(CH₂CF₃)]-(CH₂)-, -(CH₂)-[C(CH₃)(CH₂CF₃)]-(CH₂)-, -(CH₂)₂-(CF₂)-O-(CF₂)-O-(CF₂)-(CH₂)₂- and -(CH₂)-(CF₂)-O-(CF₂)-O-(CF₂)-O-(CF₂)-(CH₂)- or has a meaning as preferably described before;
Y is O, S, SO₂, or a bond or has a meaning as preferably described before;
R₃, R₄ and R₈ are H, F, a straight-chain alkyl group with 1 to 4 C atoms or a straight-chain alkoxy group with 1 to 4 C atoms or has a meaning as preferably described before;
R₅ is H, F, CN, SO₂CF₃, CF₃, CF₂CF₃, or CH₂CF₃ or has a meaning as preferably described before;
R₆ and R₇ are H, F, a linear or branched alkyl group having 1 to 4 C atoms or a linear or branched partially or fully fluorinated alkyl group having 1 to 4 C atoms or has a meaning as preferably described before;
A is CH₂ or CHR₀ and R₀ has a meaning as described before or preferably described before or A is preferably CH₂;
m is 0, 1, 2 or 3 or has a meaning as preferably described before;
R' is selected from the group consisting of F, a linear or branched partially or fully fluorinated alkyl group having 1 to 10 C atoms, a linear or branched alkyl group having 1 to 10 C atoms, a linear or branched alkoxy group having 1 to 10 C atoms, a linear or branched partially or fully fluorinated alkoxy group having 1 to 10 C atoms and a linear or branched thioalkyl group having 1 to 10 C atoms or has a meaning as preferably described before;
n₁ is 0, 1, 2, 3 or 4 or has a meaning as preferably described before;
X₁₁ is selected from the group consisting of O, S, O-SO₂, SO₂-O, C(=O), OC(=O), C(=O)O, S(C=O) and (C=O)S, or has a meaning as preferably described before;
R₁₁ and R₁₂ are H;
R₁₀ is H, methyl, ethyl or phenyl, or has a meaning as preferably described before; and
c is 1.

Preferably, such oligomer, polymer or copolymer according is comprised in the ophthalmic device or precursor article for an ophthalmic device according to the invention.

Particularly preferably, such oligomer, polymer or copolymer comprises a constitutional unit M⁰ of formulae (M⁰-I'), (M⁰-I"), (M⁰-I'-1), (M⁰-I"-1), (M⁰-I'-2-H), (M⁰-I"-2-H), (M⁰-I'-3-H), (M⁰-I"-3-H), (M⁰-I'-4-H) or (M⁰-I"-4-H), as described before, wherein
-R₂- is selected from -(CH₂)₃-, -(CH₂)₄-, -(CH₂)₅-, -(CH₂)₆-, -(CH₂)₇-, -(CH₂)₈-, -(CH₂)₉-, -(CH₂)₁₀-, -(CH₂)₁₁-, -(CH₂)₂-S-(CH₂)₂-, -(CH₂)₂-SO₂-(CH₂)₂-, - (CH₂)-S-(CH₂)₂-O-(CH₂)-, -(CH₂)-SO₂-(CH₂)₂-O-(CH₂)-, -(CH₂)-SO₂-(CH₂)₂-S-(CH₂)-, -(CH₂)-O-(CH₂)₂-S-(CH₂)₂-O-(CH₂)-, -(CH₂)-S-(CH₂)₂-O-(CH₂)₂-S-(CH₂)-, -(CH₂)-SO₂-(CH₂)₂-O-(CH₂)₂-SO₂-(CH₂)-, -(CH₂)-S-(CH₂)₂-S-(CH₂)₂-S-(CH₂)-, -(CH₂)-SO₂-(CH₂)₂-SO₂-(CH₂)₂-SO₂-(CH₂)-, -(CH₂)-O-(CH₂)₂-SO₂-(CH₂)₂-O-(CH₂)-, -(CH₂)₁₂, -(CH₂)₃-(CF₂)-(CH₂)₃-, -(CH₂)-(CF₂)₃-(CH₂)-, - (CH₂)₂-(CF₂)₄-(CH₂)₂-, -(CH₂)-[CH(CF₃)]-(CH₂)-, -(CH₂)-[C(CH₃)CF_{3]}-(CH₂)-, -(CH₂)-[CH(CH₂CF₃)]-(CH₂)-, -(CH₂)-[C(CH₃)(CH₂CF₃)]-(CH₂)-, -(CH₂)₂-(CF₂)-O-(CF₂)-O-(CF₂)-(CH₂)₂- and -(CH₂)-(CF₂)-O-(CF₂)-O-(CF₂)-O-(CF₂)-(CH₂)- or has a meaning as preferably described before;
Y is O, S, SO₂, or a bond or has a meaning as preferably described before; R₃, R₄ and R₈ are H;
R₅ is H, F or CF₃ or has a meaning as preferably described before;
R₆ and R₇ are H;
A is CH₂;
m is 0, 1, 2 or 3 or has a meaning as preferably described before;
R' is selected from the group consisting of F, a linear or branched partially or fully fluorinated alkyl group having 1 to 10 C atoms, a linear or branched alkyl group having 1 to 10 C atoms, a linear or branched alkoxy group having 1 to 10 C atoms, a linear or branched partially or fully fluorinated alkoxy group having 1 to 10 C atoms and a linear or branched thioalkyl group having 1 to 10 C atoms or has a meaning as preferably described before;
n₁ is 0, 1, 2, 3 or 4 or has a meaning as preferably described before;
X₁₁ is selected from the group consisting of O, S, O-SO₂, SO₂-O, C(=O), OC(=O), C(=O)O, S(C=O) and (C=O)S, or has a meaning as preferably described before;
R₁₁ and R₁₂ are H;
R₁₀ is H, methyl, ethyl or phenyl, or has a meaning as preferably described before;
c is 1, and the position of the phenyl group is at the C atom of the isolated double bond which is adjacent to C(R₆)(R₇).

Particularly preferably, such oligomer, polymer or copolymer is comprised in the ophthalmic device or precursor article for an ophthalmic device according to the invention.

Particularly preferably, such oligomer or polymer comprises a constitutional unit M⁰ of formulae (M⁰-I'), (M⁰-I"), (M⁰-I'-1), (M⁰-I''-1), (M⁰-I'-2-H), (M⁰-I"-2-H), (M⁰-I'-3-H), (M⁰-I"-3-H), (M⁰-I'-4-H) or (M⁰-I"-4-H), as described before, wherein
-R₂- is selected from -(CH₂)₃-, -(CH₂)₄-, -(CH₂)₅-, -(CH₂)₆-, -(CH₂)₇-, -(CH₂)₈-, -(CH₂)₉-, -(CH₂)₁₀-, -(CH₂)₁₁-, -(CH₂)₂-S-(CH₂)₂-, -(CH₂)₂-SO₂-(CH₂)₂-, - (CH₂)-S-(CH₂)₂-O-(CH₂)-, -(CH₂)-SO₂-(CH₂)₂-O-(CH₂)-, -(CH₂)-SO₂-(CH₂)₂-S-(CH₂)-, -(CH₂)-O-(CH₂)₂-S-(CH₂)₂-O-(CH₂)-, -(CH₂)-S-(CH₂)₂-O-(CH₂)₂-S-(CH₂)-, -(CH₂)-SO₂-(CH₂)₂-O-(CH₂)₂-SO₂-(CH₂)-, -(CH₂)-S-(CH₂)₂-S-(CH₂)₂-S-(CH₂)-, -(CH₂)-SO₂-(CH₂)₂-SO₂-(CH₂)₂-SO₂-(CH₂)-, -(CH₂)-O-(CH₂)₂-SO₂-(CH₂)₂-O-(CH₂)-, -(CH₂)₁₂, -(CH₂)₃-(CF₂)-(CH₂)₃-, -(CH₂)-(CF₂)₃-(CH₂)-, - (CH₂)₂-(CF₂)₄-(CH₂)₂-, -(CH₂)-[CH(CF₃)]-(CH₂)-, -(CH₂)-[C(CH₃)CF_{3]}-(CH₂)-, -(CH₂)-[CH(CH₂CF₃)]-(CH₂)-, -(CH₂)-[C(CH₃)(CH₂CF₃)]-(CH₂)-, -(CH₂)₂-(CF₂)-O-(CF₂)-O-(CF₂)-(CH₂)₂- and -(CH₂)-(CF₂)-O-(CF₂)-O-(CF₂)-O-(CF₂)-(CH₂)- or has a meaning as preferably described before;
Y is O, S, SO₂, or a bond or has a meaning as preferably described before; R₃, R₄ and R₈ are H;
R₅ is H, F or CF₃ or has a meaning as preferably described before;
R₆ and R₇ are H;
A is CH₂;
m is 0, 1, 2 or 3 or has a meaning as preferably described before;
R' is selected from the group consisting of F, a linear or branched partially or fully fluorinated alkyl group having 1 to 10 C atoms, a linear or branched alkyl group having 1 to 10 C atoms, a linear or branched alkoxy group having 1 to 10 C atoms, a linear or branched partially or fully fluorinated alkoxy group having 1 to 10 C atoms and a linear or branched thioalkyl group having 1 to 10 C atoms or has a meaning as preferably described before;
n₁ is 0, 1, 2, 3 or 4 or has a meaning as preferably described before;
the position of the phenyl group is at the C atom of the isolated double bond which is adjacent to C(R₆)(R₇); and 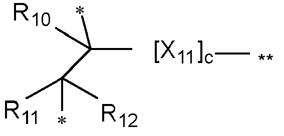 within formulae (M⁰-I'), (M⁰-I"), (M⁰-I'-1), (M⁰-I"-1), (M⁰-I'-2-H), (M⁰-I"-2-H), (M⁰-I'-3-H), (M⁰-I"-3-H), (M⁰-I'-4-H) and (M⁰-I"-4-H) is derived from any one of the preferred alkenyl groups of formulae (4-1), (4-2), (4-3), (4-4), (4-5), (4-6), (4-7), (4-8), (4-9), (4-10), (4-11) and (4-12) or any preferred embodiment thereof.

Particularly preferably, such oligomer, polymer or copolymer is comprised in the ophthalmic device or precursor article for an ophthalmic device according to the invention.

The copolymer may be an oligomer or polymer comprising one or more polymerized compounds of formulae (I), (I#), (I-1), (I-2), (I-2H), (I-3), (I-3-H), (I-4), (I-4-H), (I'), (I'-a), (I'-b), (I'-c), (I'-d), (I'-1), (I'-2), (I'-2-H), (I'-3), (I'-3-H), (I'-4), (I'-4-H) (I"), (I"-1), (I"-2), (I"-2-H), (I"-3), (I"-3-H), (I"-4) or (I"-4-H) as described before or preferably described before or one or more constitutional units M⁰ of formulae (M⁰-I'), (M⁰-I'-a), (M⁰-I'-b), (M⁰-I'-c), (M⁰-I'- d), (M⁰-I"), (M⁰-I'-1), (M⁰-I"-1), (M⁰-I'-2-H), (M⁰-I"-2-H), (M⁰-I'-3-H), (M⁰-I"-3-H), (M⁰-I'-4-H) and/or (M⁰-I"-4-H) as described before or preferably described before or one or more constitutional units (M⁰-001) to (M⁰-180) as described below, which may be the same or different from one another, and one or more constitutional units M², which may be the same or different from one another. Said one or more constitutional units M² are chemically different from the units M⁰. Preferably, said one or more constitutional units M² are derived by polymerization of one or more monomers selected from the group consisting of styrene, ethoxyethyl methacrylate (EOEMA), methyl methacrylate (MMA), methyl acrylates, n-alkyl acrylates (the n-alkyl group comprising 2-20 C-atoms), n-alkyl methacrylates (the n-alkyl group comprising 2-20 C-atoms), ethoxyethoxy ethylacrylate (EEEA), 2-hydroxyethyl methacrylate (HEMA), tetrahydrofuryl methacrylate (THFMA), glycidylmethacrylate (GMA), 16-hydroxyhexadecyl acrylate, 16-hydroxyhexadecyl methacrylate, 18-hydroxyoctadecyl acrylate, 18-hydroxyoctadecyl methacrylate, 2-phenoxyethyl acrylate (EGPEA), heptafluorobutyl acrylate, heptafluorobutyl methacrylate, hexafluorobutyl acrylate, hexafluorobutyl methacrylate, hexafluoroisopropyl acrylate, hexafluoroisopropyle methacrylate, petanfluoropropyl acrylate, pentafluoropropyl methacrylate, tetrafluoropropyl methacrylate, trifluoroethyl acrylate, and trifluoroethyl methacrylate.

The invention therefore relates further to an ophthalmic device or a precursor article for the ophthalmic device as described or preferably described before comprising beside of the at least one polymerized compound of formulae (I), (I') or (I") or the constitutional unit M⁰ of formulae (M⁰-I'), (M⁰-I'-a), (M⁰-I'-b), (M⁰-I'-c), (M⁰-I'-d), (M⁰-I"), (M⁰-I'-1), (M⁰-I"-1), (M⁰-I'-2-H), (M⁰-I"-2-H), (M⁰-I'-3-H), (M⁰-I"-3-H), (M⁰-I'-4-H) and/or (M⁰-I"-4-H) as described before or preferably described before or one or more constitutional units (M⁰-001) to (M⁰-180) as described below at least one further polymerized monomer selected from the group consisting of styrene, ethoxyethyl methacrylate (EOEMA), methyl methacrylate (MMA), methyl acrylate, n-alkyl acrylates (the n-alkyl group comprising 2-20 C-atoms), n-alkyl methacrylates (the n-alkyl group comprising 2-20 C-atoms), ethoxyethoxy ethylacrylate (EEEA), 2-hydroxyethyl methacrylate (HEMA), tetrahydrofuryl methacrylate (THFMA), glycidylmethacrylate (GMA), 16-hydroxyhexadecyl acrylate, 16-hydroxyhexadecyl methacrylate, 18-hydroxyoctadecyl acrylate, 18-hydroxyoctadecyl methacrylate, 2-phenoxyethyl acrylate (EGPEA), Bisphenol A diacrylate-1 EO/Phenol (BPADA), 2-[3'-2'H-benzotriazol- 2'-yl)-4'-hydroxyphenyl]ethyl methacrylate (BTPEM) or ehtyleneglycoldimethacrylate.

Particularly preferably, the at least one further polymerized monomer is selected from methyl methacrylate, 2-hydroxyethyl methacrylate, 2-phenoxyethyl acrylate, ethoxyethoxy ethylacrylate, 8-methylnonyl methacrylate, n-butyl methacrylate, 2-ethyl hexylmethacrylate or a mixture thereof.

Particularly preferably, such copolymer is comprised in the ophthalmic device or precursor article for an ophthalmic device according to the invention.

Alternatively the oligomer or polymer, preferably the polymer, according to the invention is a homopolymer, i.e. an oligomer or polymer, preferably a polymer, comprising one or more constitutional units M⁰ of formula (M⁰-I'), (M⁰-I'-a), (M⁰-I'-b), (M⁰-I'-c), (M⁰-I'-d), (M⁰-I"), (M⁰-I'-1), (M⁰-I"-1), (M⁰-I'-2-H), (M⁰-I"-2-H), (M⁰-I'-3-H), (M⁰-I"-3-H), (M⁰-I'-4-H) or (M⁰-I"-4-H) as described before or preferably described before or (M⁰-001) to (M⁰-180) as described below and wherein all constitutional units M⁰ are the same.

Exemplary homopolymeric compounds based on compounds of formulae (I), (I#), (I-1), (I-2), (I-2H), (I-3), (I-3-H), (I-4), (I-4-H), (I'), (I'-a), (I'-b), (I'-c), (I'-d), (I'-1), (I'-2), (I'-2-H), (I'-3), (I'-3-H), (I'-4), (I'-4-H) (I"), (I"-1), (I"-2), (I"-2-H), (I"-3), (I"-3-H), (I"-4) and/or (I"-4-H) are the following compounds (P-001) to (P-180) as shown in table 2.

**Table 2:**

| | |
|---|---|
| 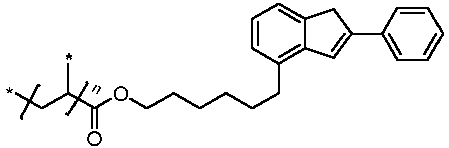 | P-001 |
| 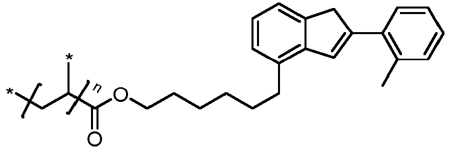 | P-002 |
| 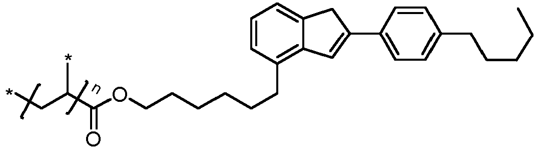 | P-003 |
| 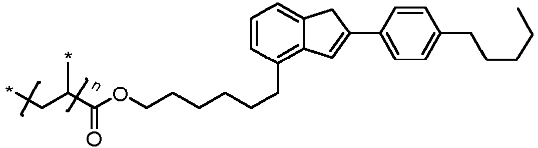 | P-004 |
| 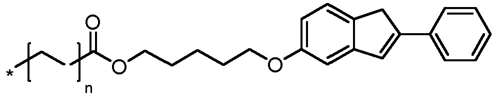 | P-005 |
| 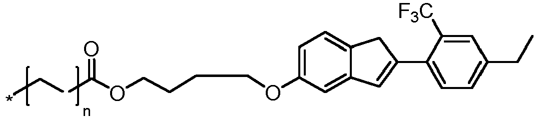 | P-006 |
| 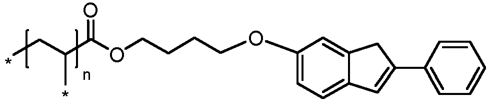 | P-007 |
| 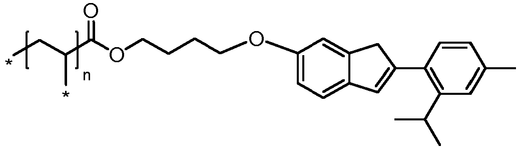 | P-008 |
| 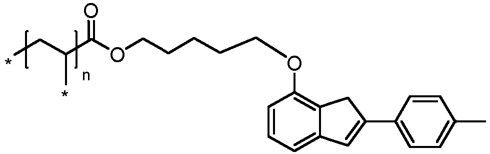 | P-009 |
| 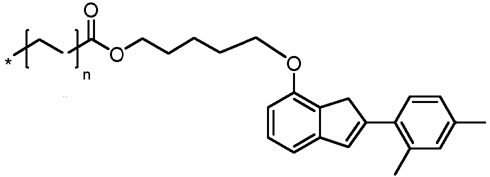 | P-010 |
| 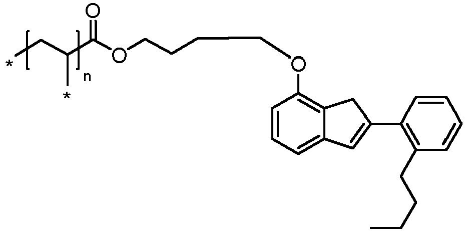 | P-011 |
| 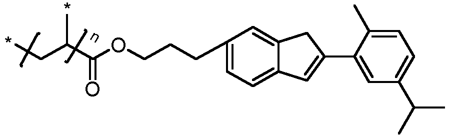 | P-012 |
| 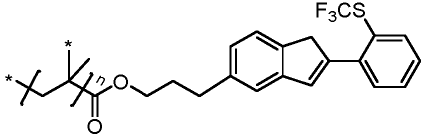 | P-013 |
| 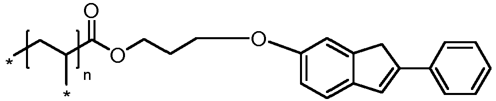 | P-014 |
| 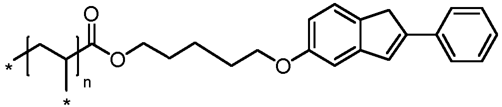 | P-015 |
| 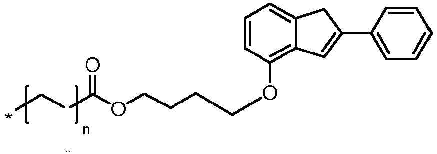 | P-016 |
| 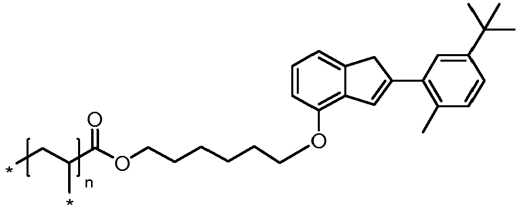 | P-017 |
| 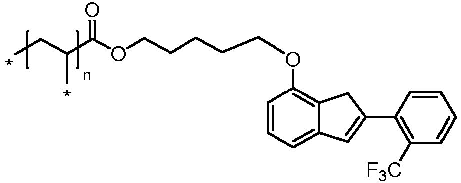 | P-018 |
| 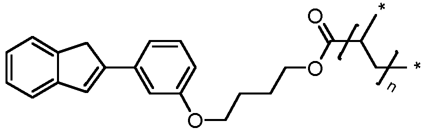 | P-019 |
| 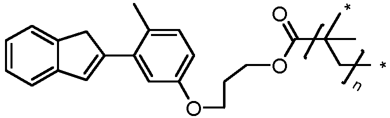 | P-020 |
| 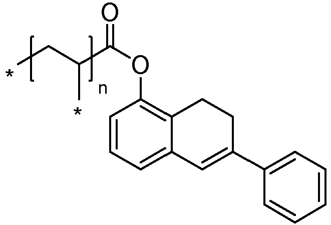 | P-021 |
| 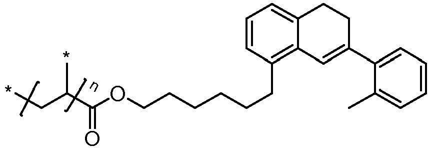 | P-022 |
| 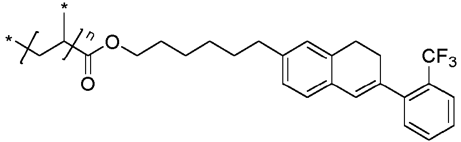 | P-023 |
| 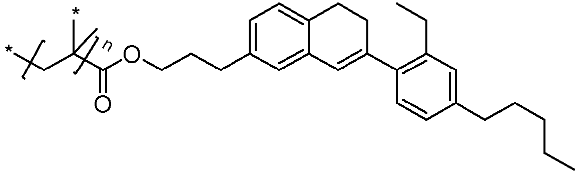 | P-024 |
| 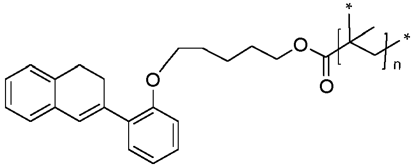 | P-025 |
| 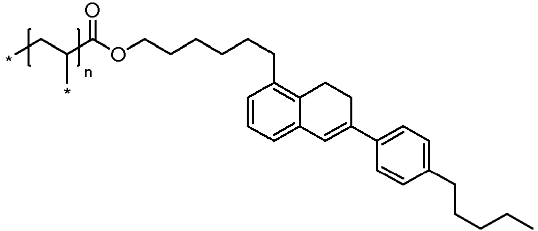 | P-026 |
| 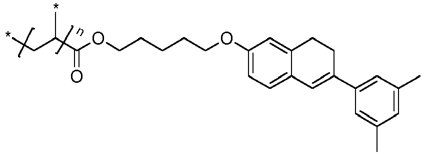 | P-027 |
| 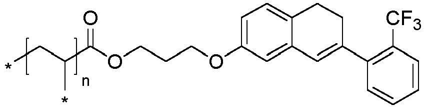 | P-028 |
| 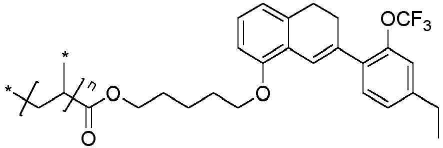 | P-029 |
| 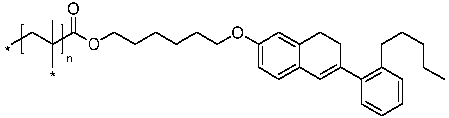 | P-030 |
| 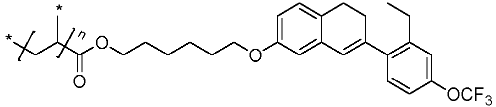 | P-031 |
| 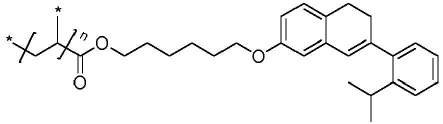 | P-032 |
| 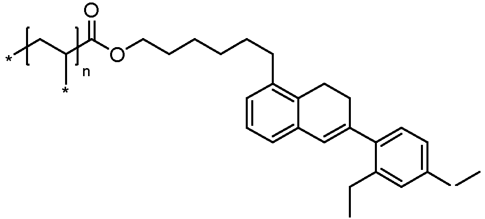 | P-033 |
| 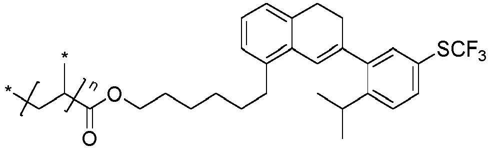 | P-034 |
| 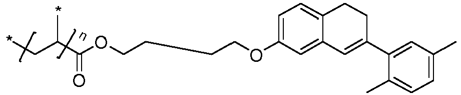 | P-035 |
| 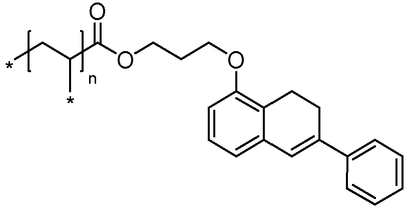 | P-036 |
| 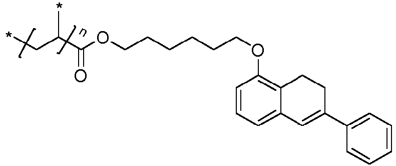 | P-037 |
| 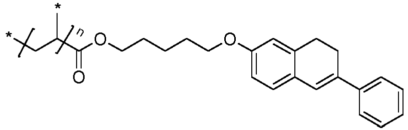 | P-038 |
| 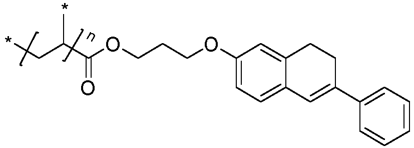 | P-039 |
| 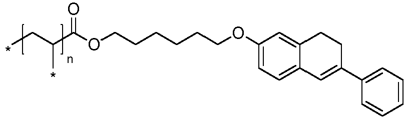 | P-040 |
| 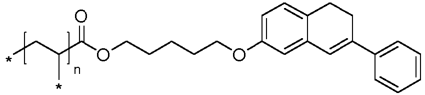 | P-041 |
| 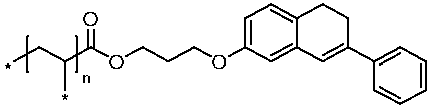 | P-042 |
| 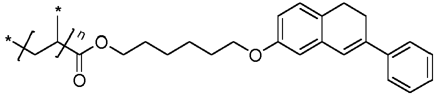 | P-043 |
| 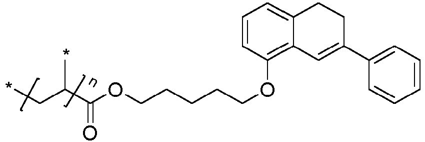 | P-044 |
| 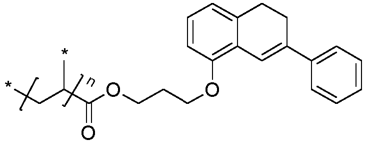 | P-045 |
| 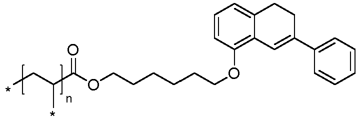 | P-046 |
| 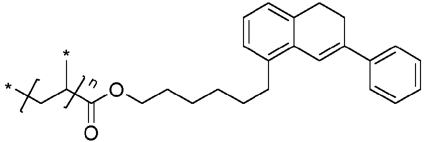 | P-047 |
| 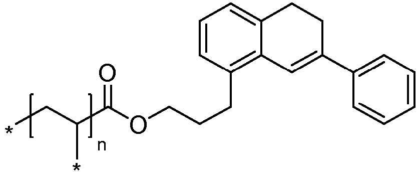 | P-048 |
| 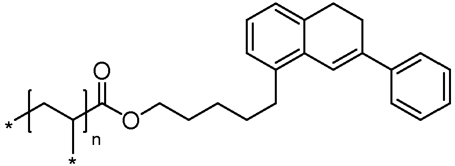 | P-049 |
| 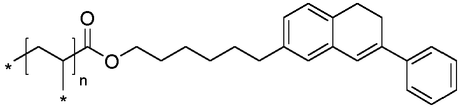 | P-050 |
| 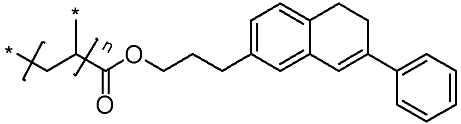 | P-051 |
| 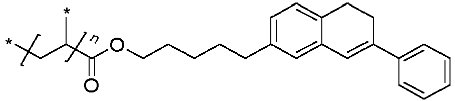 | P-052 |
| 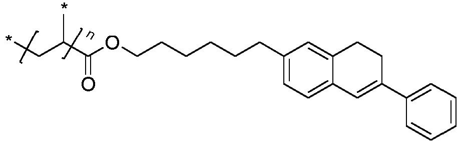 | P-053 |
| 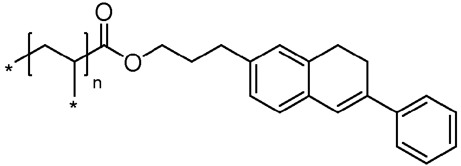 | P-054 |
| 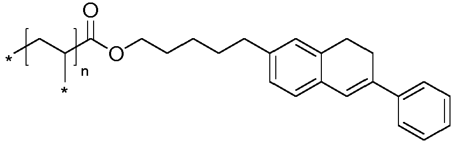 | P-055 |
| 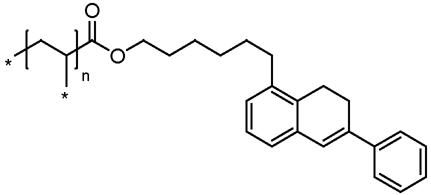 | P-056 |
| 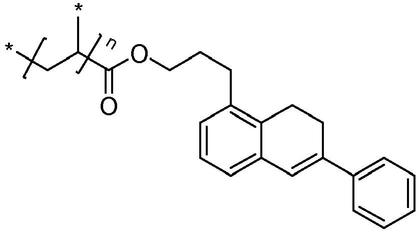 | P-P-057 |
| 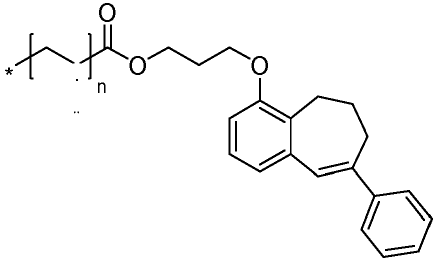 | P-058 |
| 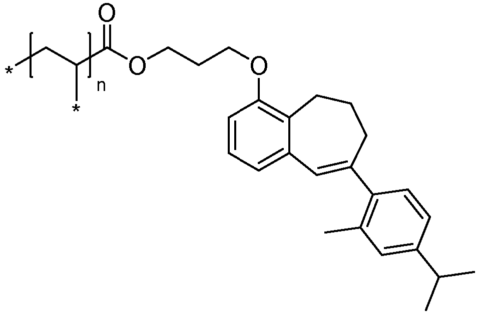 | P-059 |
| 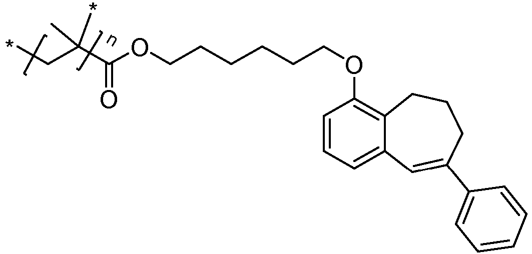 | P-060 |
| 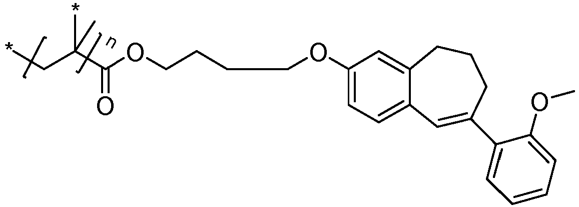 | P-061 |
| 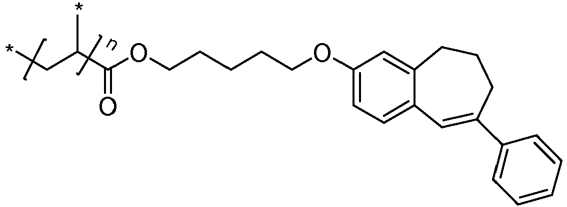 | P-062 |
| 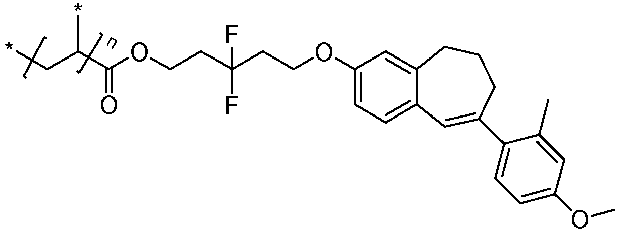 | P-063 |
| 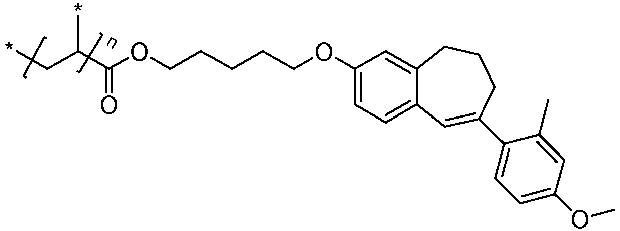 | P-064 |
| 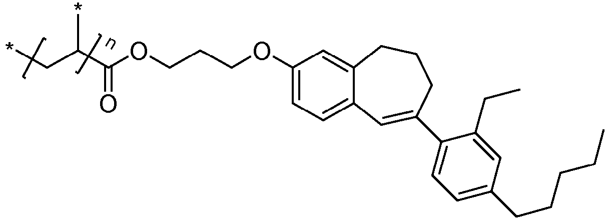 | P-065 |
| 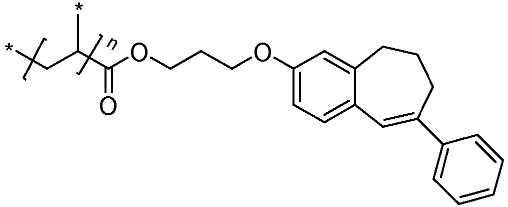 | P-066 |
| 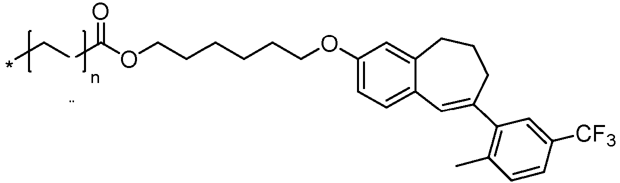 | P-067 |
| 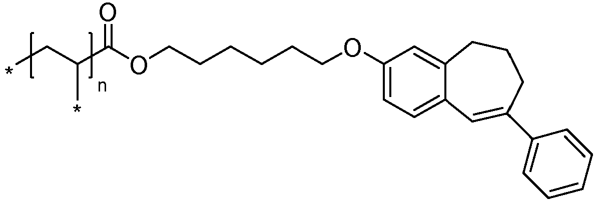 | P-068 |
| 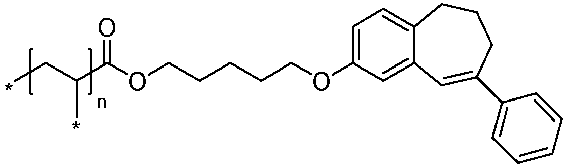 | P-069 |
| 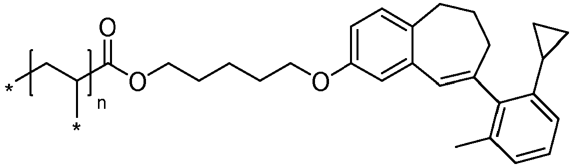 | P-070 |
| 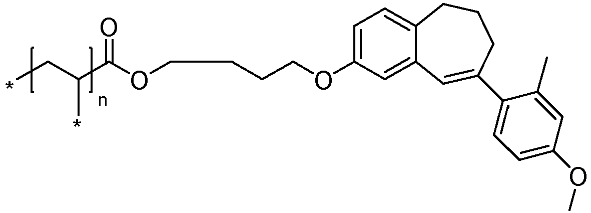 | P-071 |
| 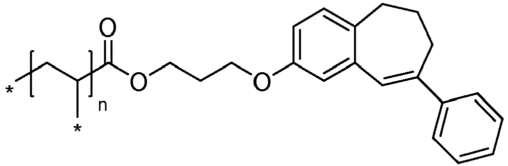 | P-072 |
| 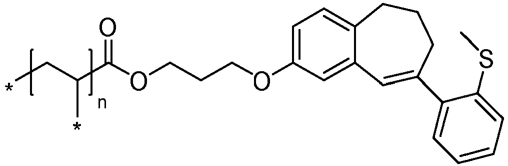 | P-073 |
| 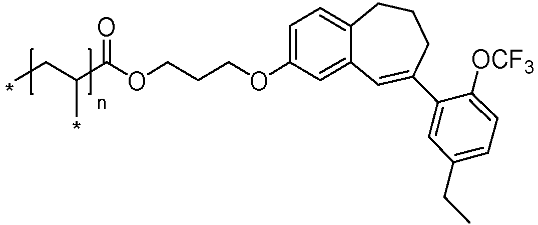 | P-074 |
| 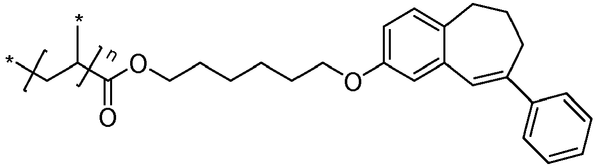 | P-075 |
| 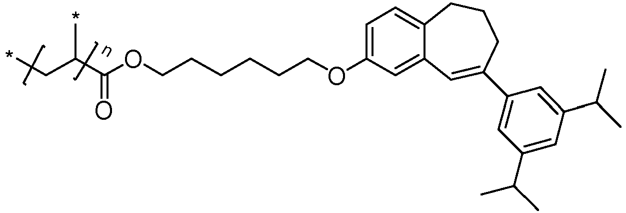 | P-076 |
| 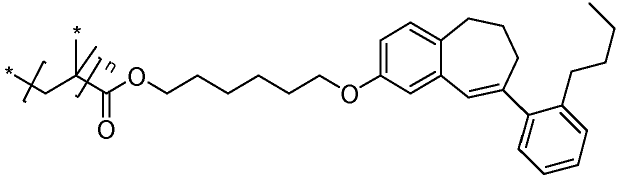 | P-077 |
| 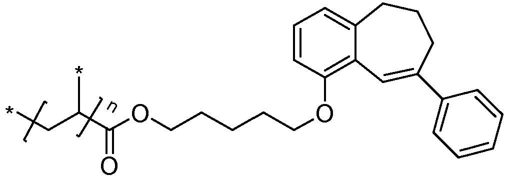 | P-078 |
| 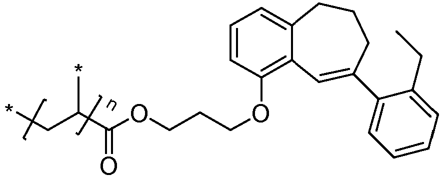 | P-079 |
| 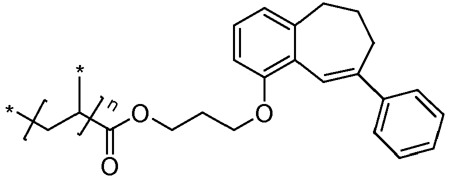 | P-080 |
| 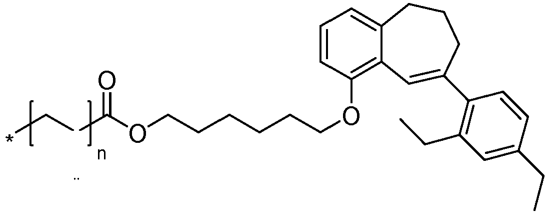 | P-081 |
| 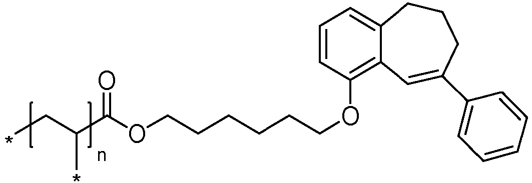 | P-082 |
| 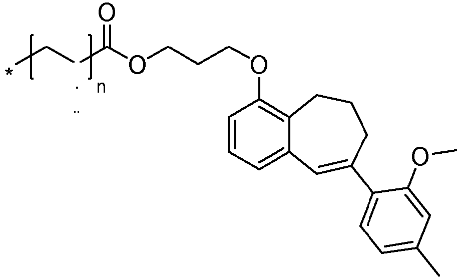 | P-083 |
| 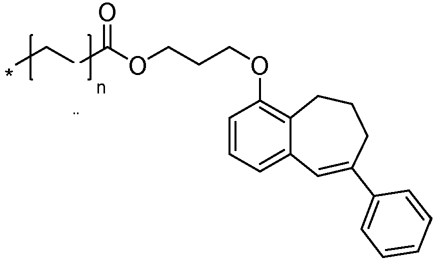 | P-084 |
| 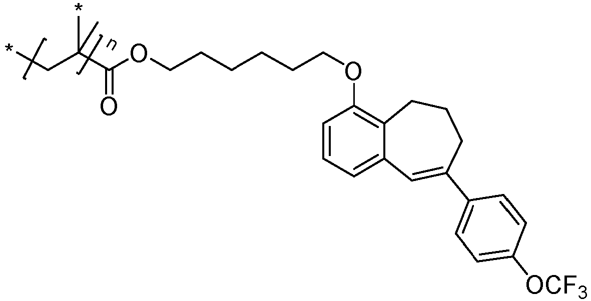 | P-085 |
| 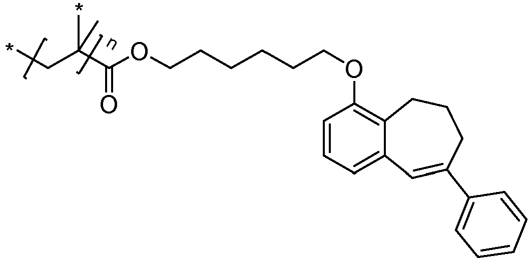 | P-086 |
| 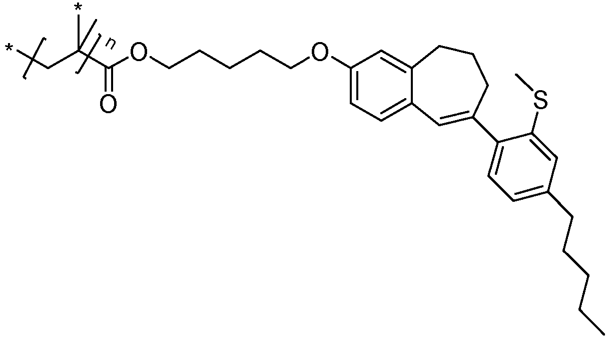 | P-087 |
| 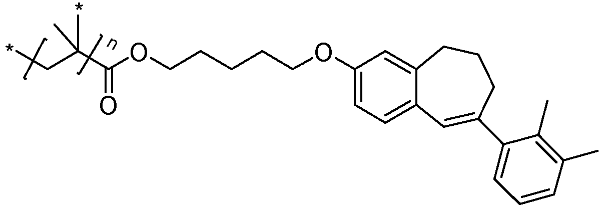 | P-088 |
| 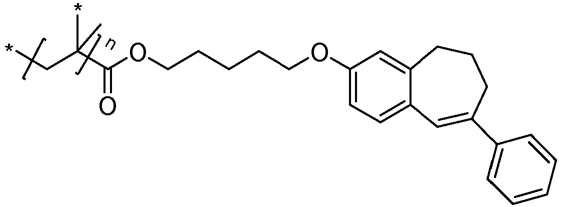 | P-089 |
| 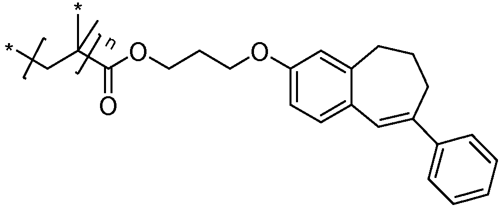 | P-090 |
| 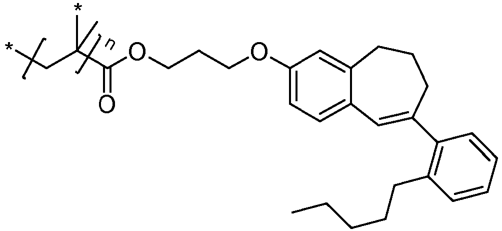 | P-091 |
| 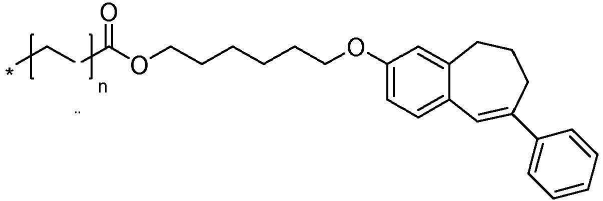 | P-092 |
| 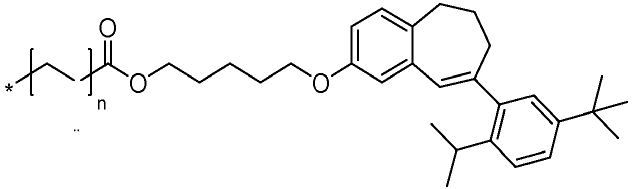 | P-093 |
| 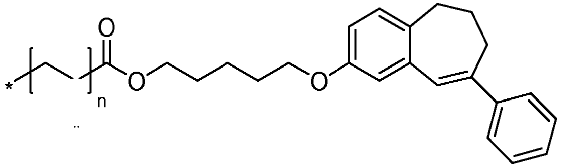 | P-094 |
| 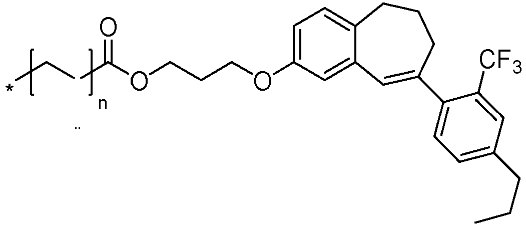 | P-095 |
| 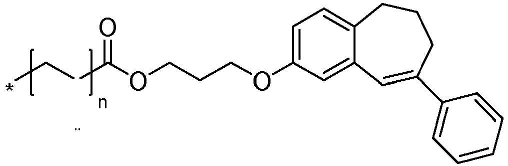 | P-096 |
| 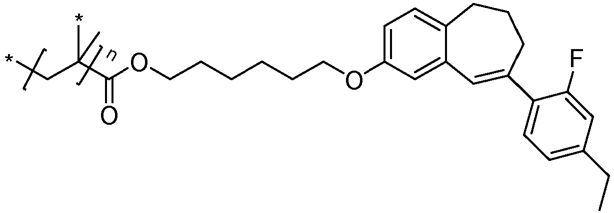 | P-097 |
| 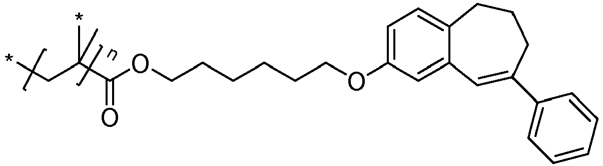 | P-098 |
| 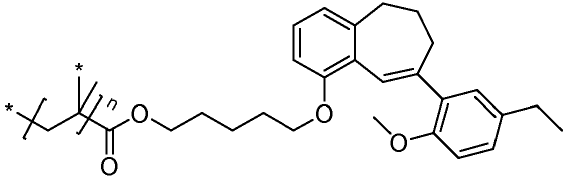 | P-099 |
| 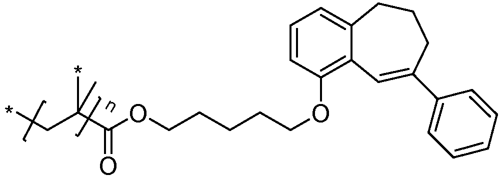 | P-100 |
| 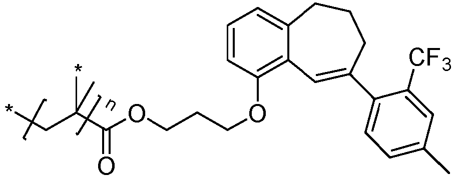 | P-101 |
| 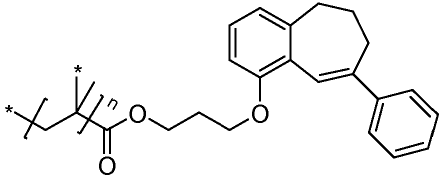 | P-102 |
| 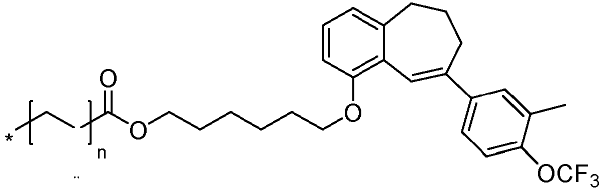 | P-103 |
| 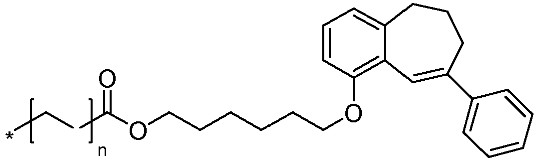 | P-104 |
| 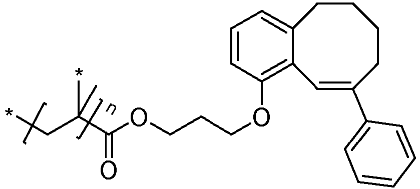 | P-105 |
| 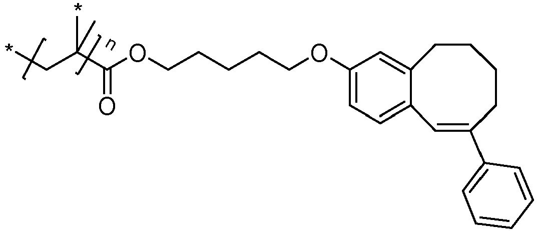 | P-106 |
| 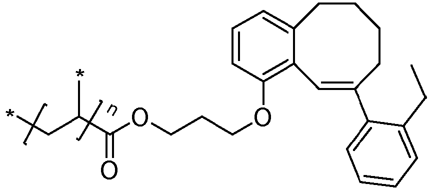 | P-107 |
| 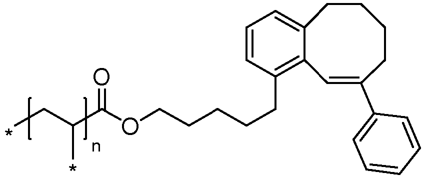 | P-108 |
| 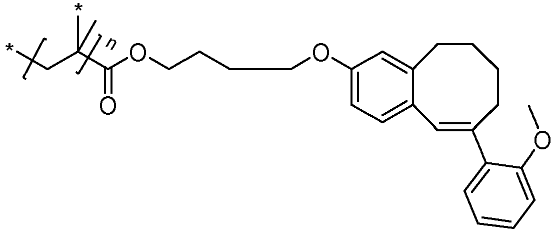 | P-109 |
| 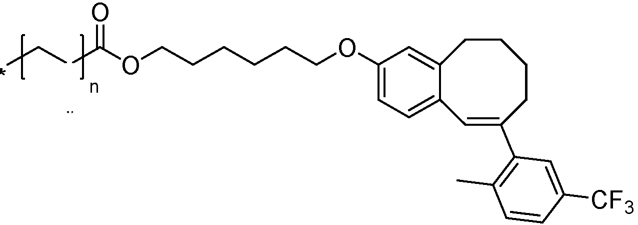 | P-110 |
| 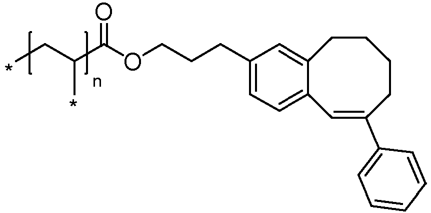 | P-111 |
| 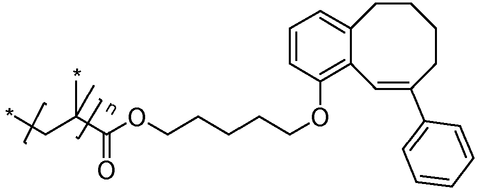 | P-112 |
| 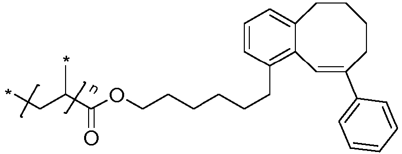 | P-113 |
| 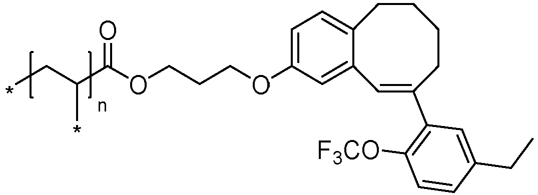 | P-114 |
| 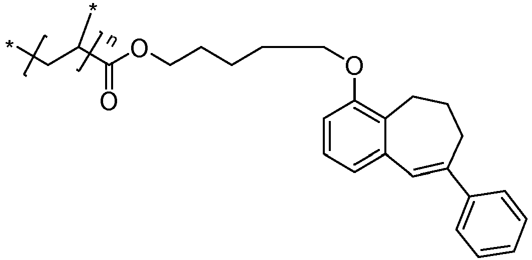 | P-115 |
| 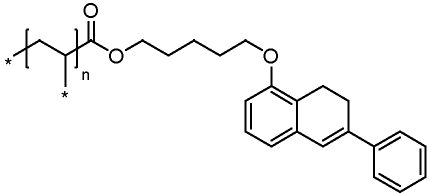 | P-116 |
| 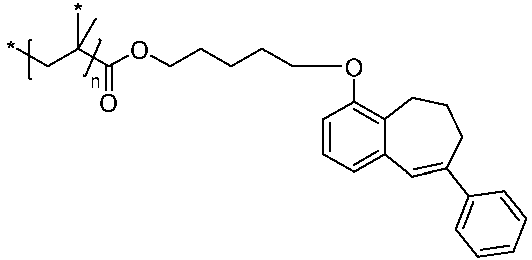 | P-117 |
| 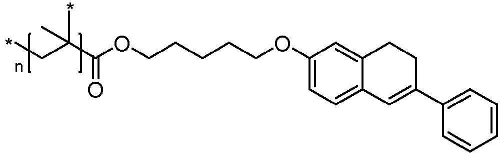 | P-118 |
| 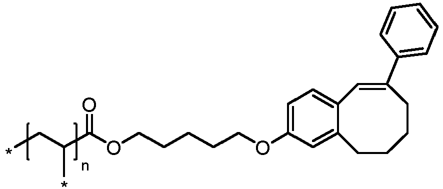 | P-119 |
| 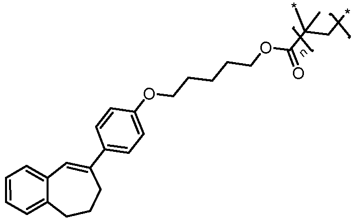 | P-120 |
| 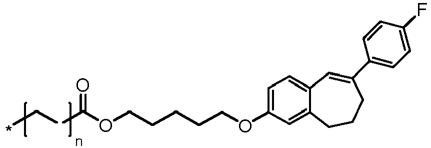 | P-121 |
| 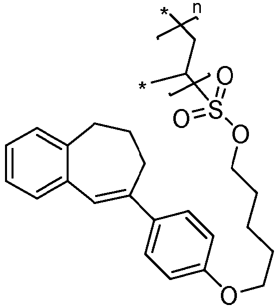 | P-122 |
| 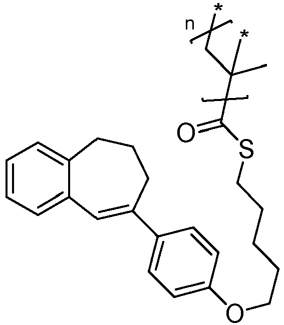 | P-123 |
| 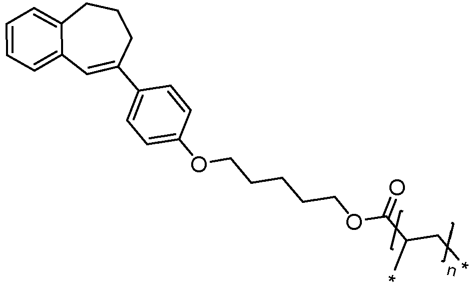 | P-124 |
| 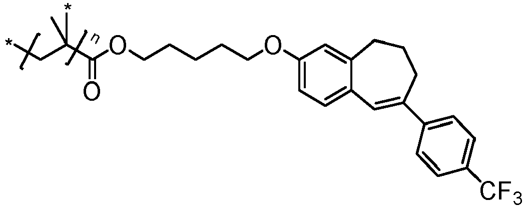 | P-125 |
| 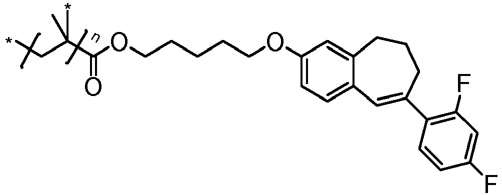 | P-126 |
| 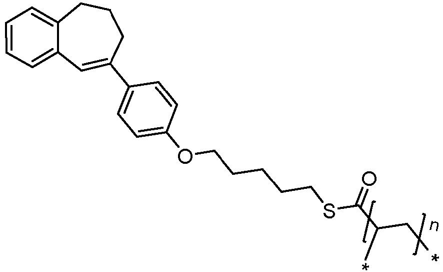 | P-127 |
| 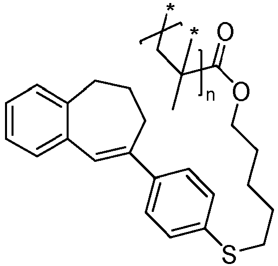 | P-128 |
| 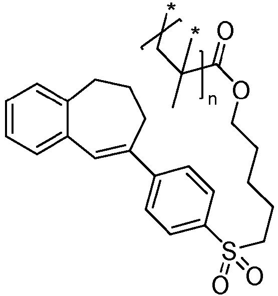 | P-129 |
| 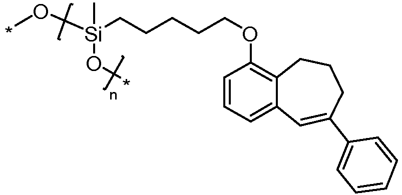 | P-130 |
| 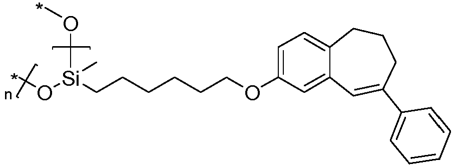 | P-131 |
| 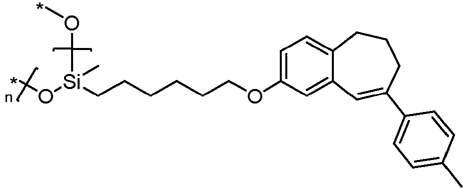 | P-132 |
| 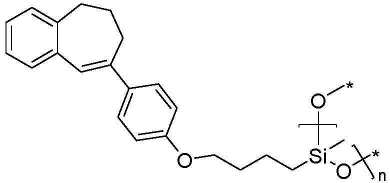 | P-133 |
| 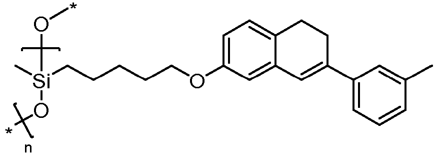 | P-134 |
| 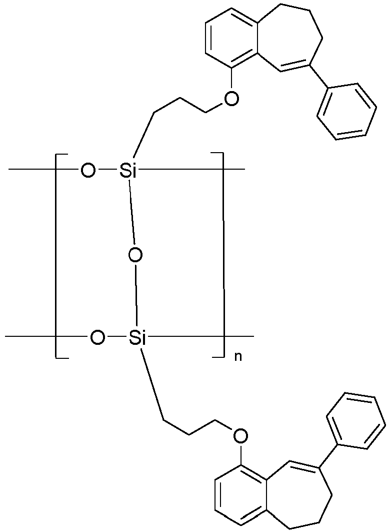 | P-135 |
| 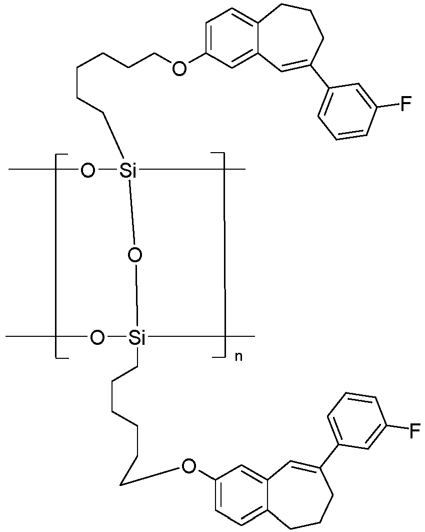 | P-136 |
| 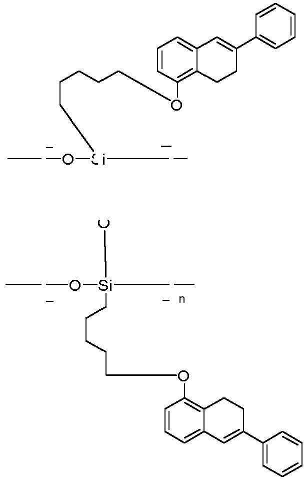 | P-137 |
| 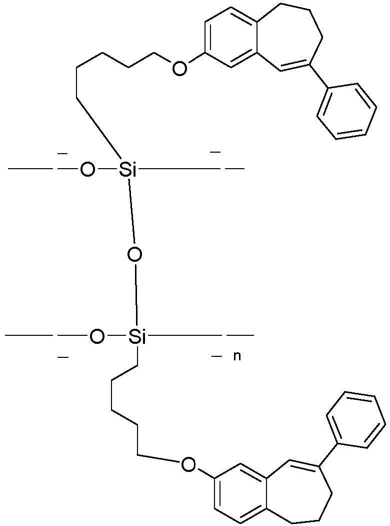 | P-138 |
| 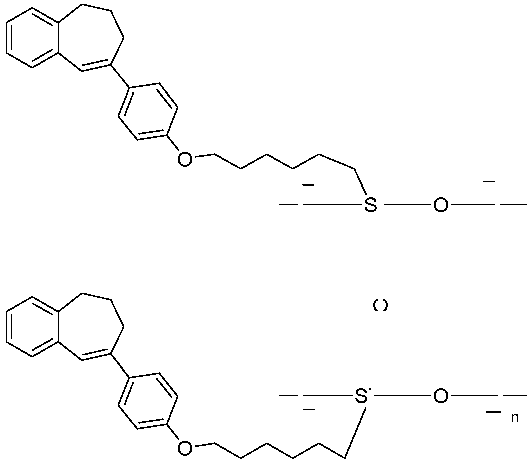 | P-139 |
| 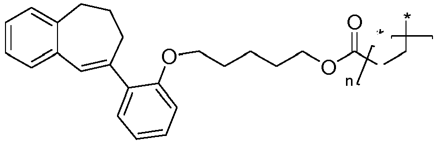 | P-140 |
| 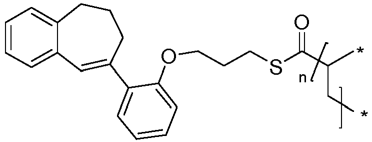 | P-141 |
| 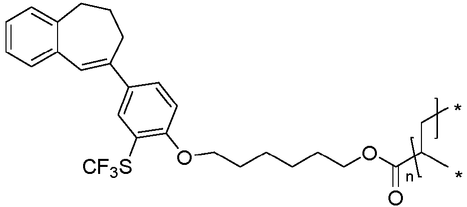 | P-142 |
| 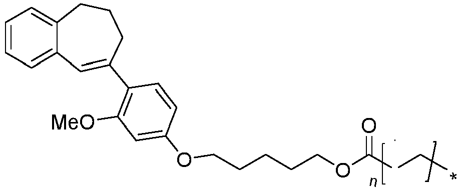 | P-143 |
| 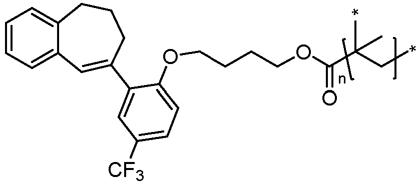 | P-144 |
| 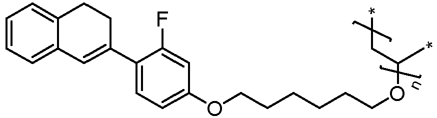 | P-145 |
| 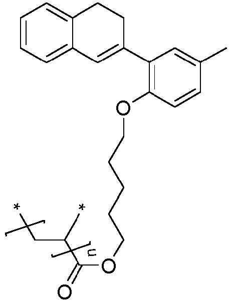 | P-146 |
| 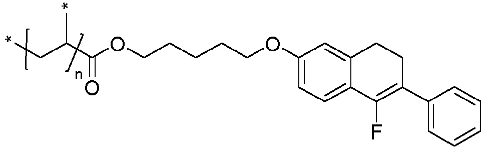 | P-147 |
| 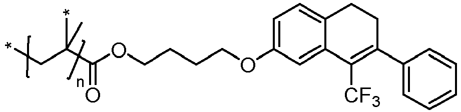 | P-148 |
| 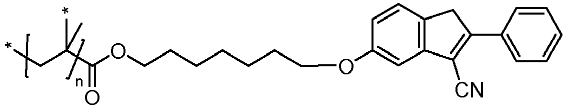 | P-149 |
| 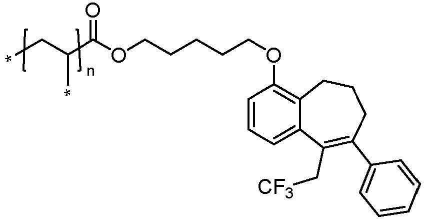 | P-150 |
| 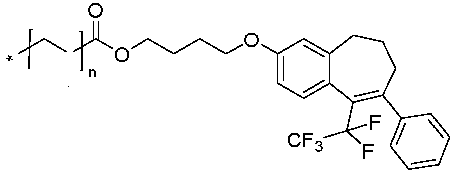 | P-151 |
| 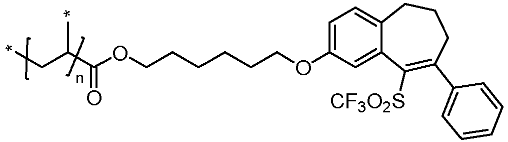 | P-152 |
| 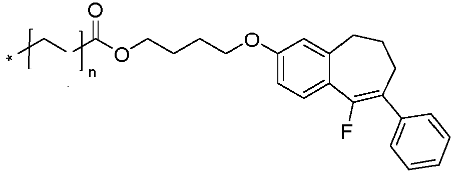 | P-153 |
| 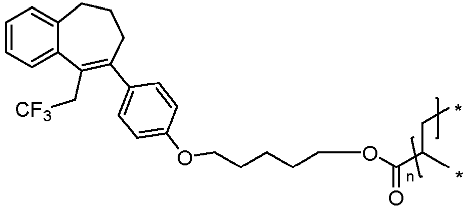 | P-154 |
| 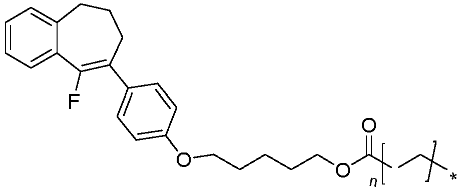 | P-155 |
| 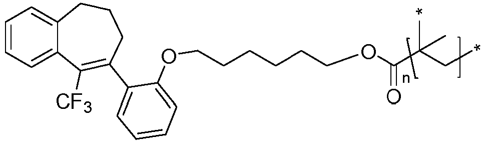 | P-156 |
| 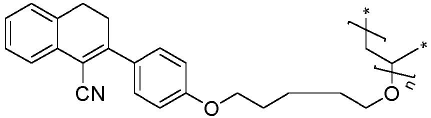 | P-157 |
| 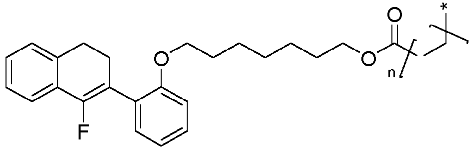 | P-158 |
| 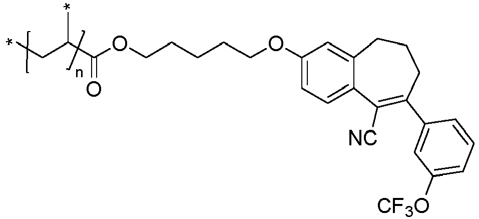 | P-159 |
| 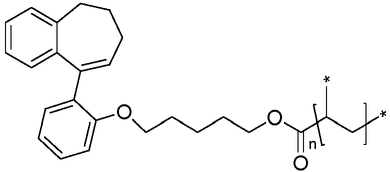 | P-160 |
| 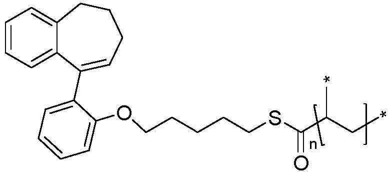 | P-161 |
| 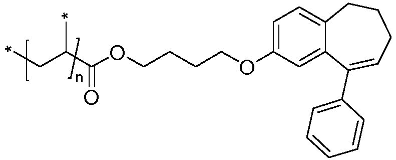 | P-162 |
| 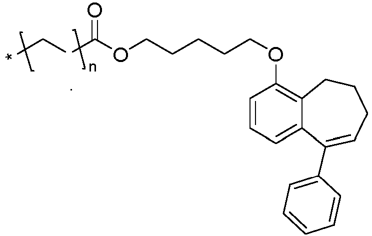 | P-163 |
| 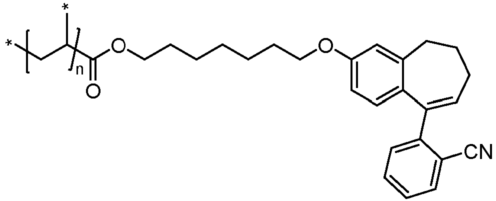 | P-164 |
| 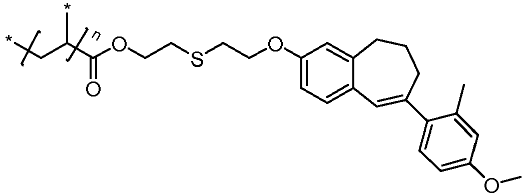 | P-165 |
| 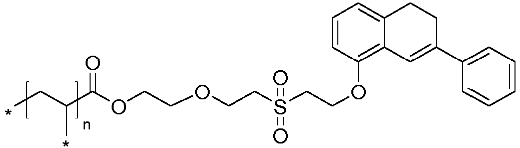 | P-166 |
| 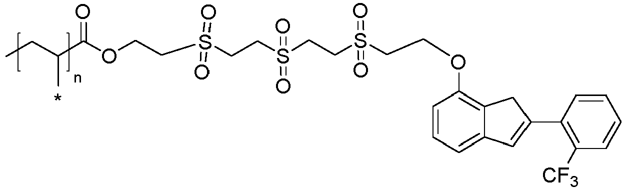 | P-167 |
| 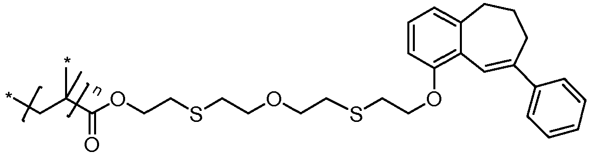 | P-168 |
| 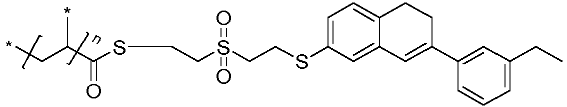 | P-169 |
| 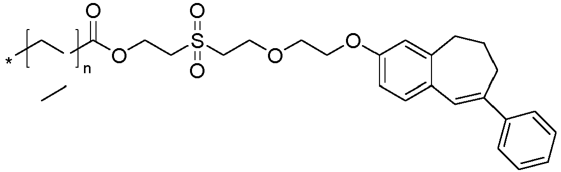 | P-170 |
| 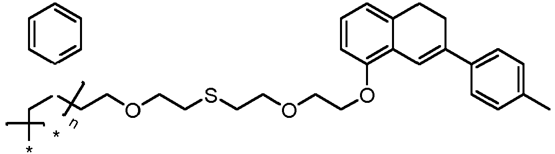 | P-171 |
| 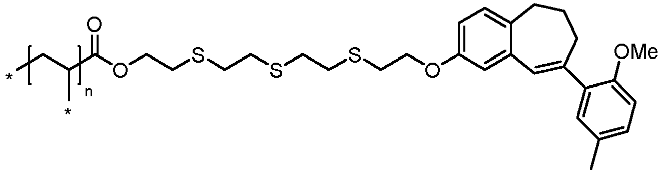 | P-172 |
| 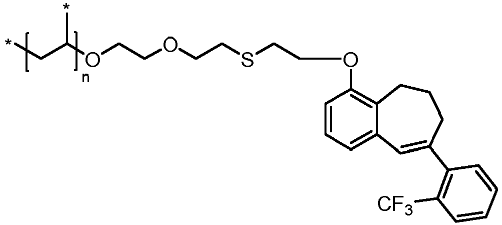 | P-173 |
| 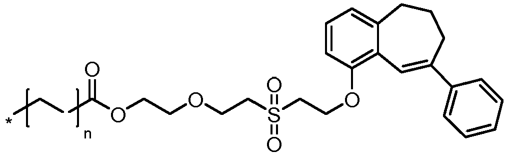 | P-174 |
| 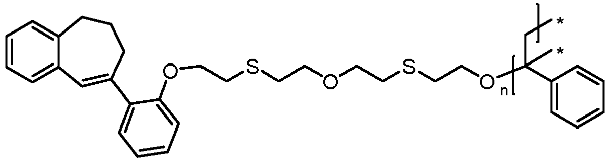 | P-175 |
| 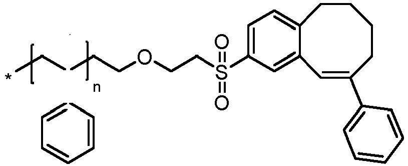 | P-176 |
| 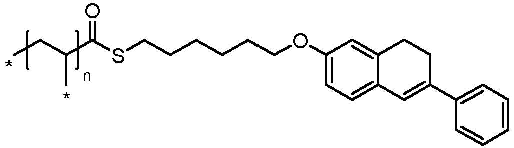 | P-177 |
| 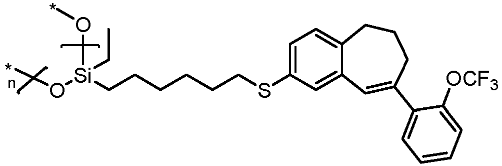 | P-178 |
| 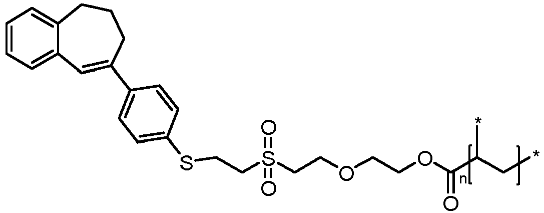 | P-179 |
| 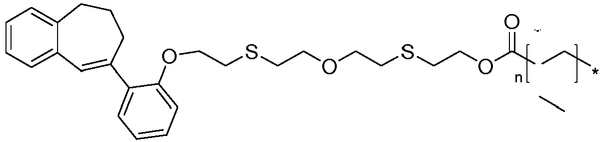 | P-180. |

| | |
|---|---|
| The letter n gives the degree of polymerization as explained before. | |

Exemplary constitutional units M⁰ based on compounds of formulae (I), (I#), (I-1), (I-2), (I-2H), (I-3), (I-3-H), (I-4), (I-4-H), (I'), (I'-a), (I'-b), (I'-c), (I'-d), (I'-1), (I'-2), (I'-2-H), (I'-3), (I'-3-H), (I'-4), (I'-4-H) (I"), (I"-1), (I"-2), (I"-2-H), (I"-3), (I"-3-H), (I"-4) and/or (I"-4-H) or constitutional unit M⁰ of formulae (M⁰-I'), (M⁰-I"), (M⁰-I'-1), (M⁰-I"-1), (M⁰-I'-2-H), (M⁰-I"-2-H), (M⁰-I'-3-H), (M⁰-I"-3-H), (M^{o}-I'-4-H) or (M⁰-I"-4-H) are the following compounds (M⁰-001) to (M⁰-180) as shown in table 2-1.

**Table 2-1:**

| | |
|---|---|
| 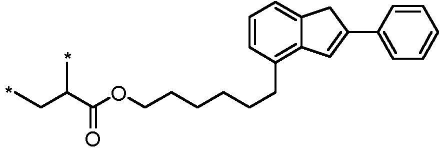 | M⁰-001 |
| 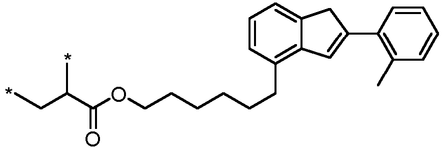 | M⁰-002 |
| 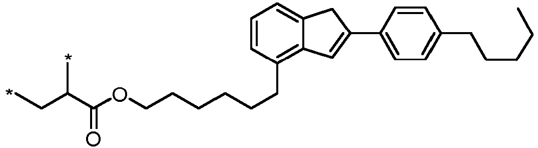 | M⁰-003 |
| 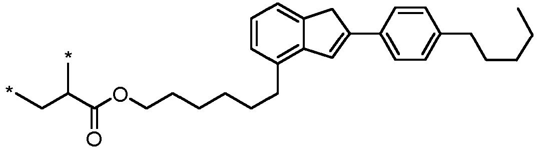 | M⁰-004 |
| 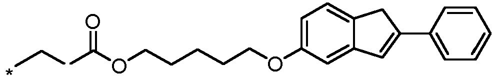 | M⁰-005 |
| 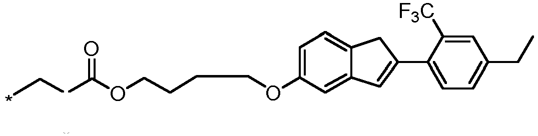 | M⁰-006 |
| 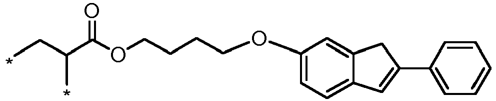 | M⁰-007 |
| 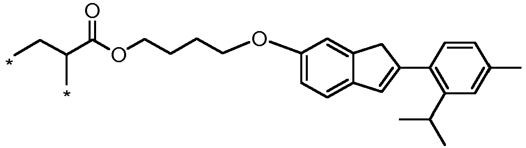 | M⁰-008 |
| 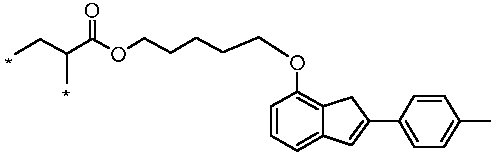 | M⁰-009 |
| 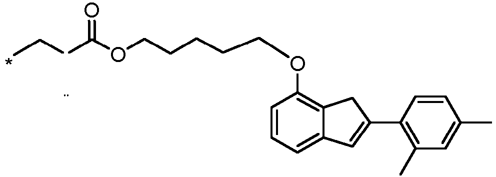 | M⁰-010 |
| 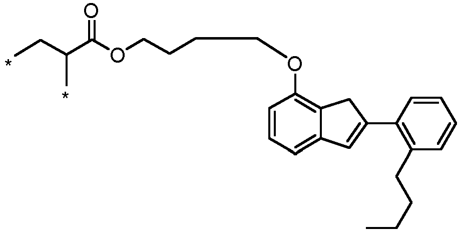 | M⁰-011 |
| 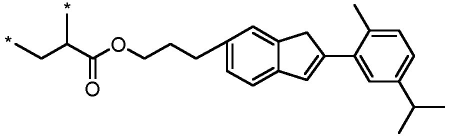 | M⁰-012 |
| 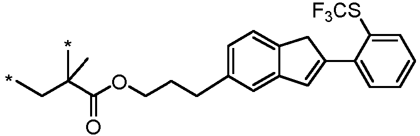 | M⁰-013 |
| 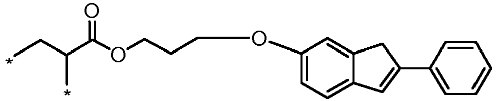 | M⁰-014 |
| 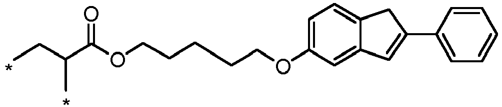 | M⁰-015 |
| 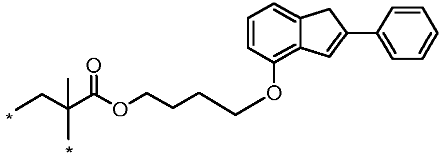 | M⁰-016 |
| 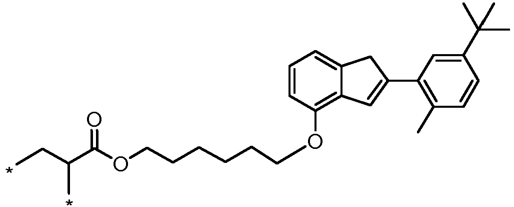 | M⁰-017 |
| 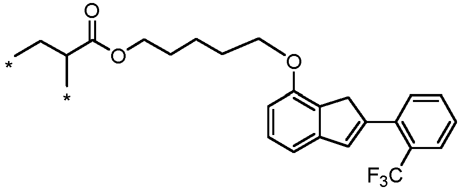 | M⁰-018 |
| 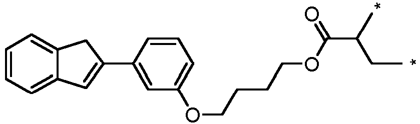 | M⁰-019 |
| 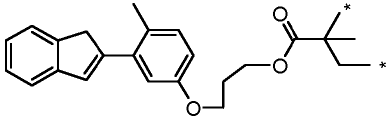 | M⁰-020 |
| 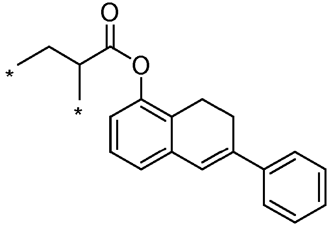 | M⁰-021 |
| 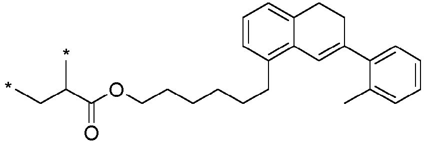 | M⁰-022 |
| 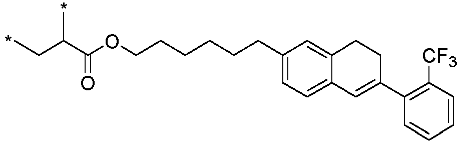 | M⁰-023 |
| 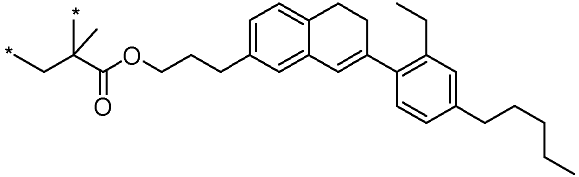 | M⁰-024 |
| 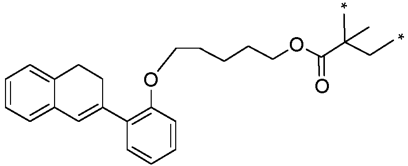 | M⁰-025 |
| 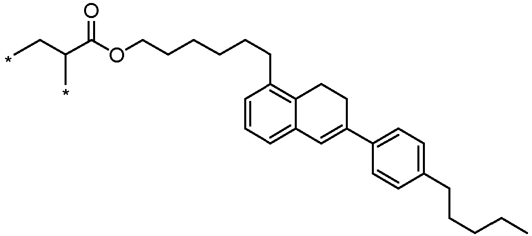 | M⁰-026 |
| 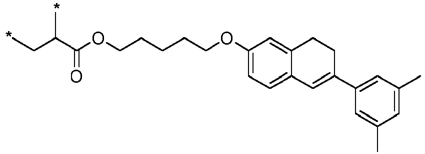 | M⁰-027 |
| 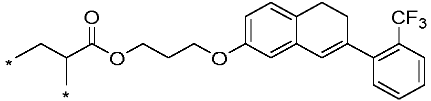 | M⁰-028 |
| 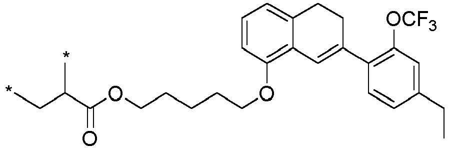 | M⁰-029 |
| 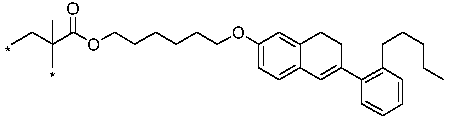 | M⁰-030 |
| 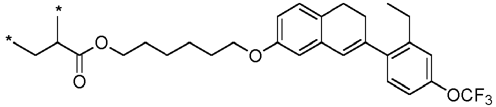 | M⁰-031 |
| 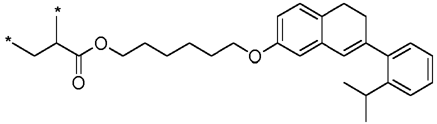 | M⁰-032 |
| 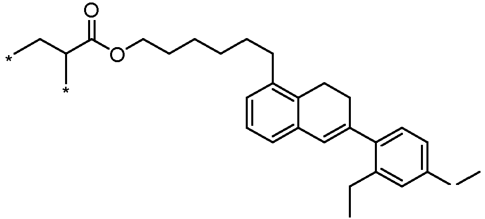 | M⁰-033 |
| 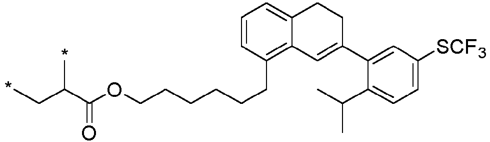 | M⁰-034 |
| 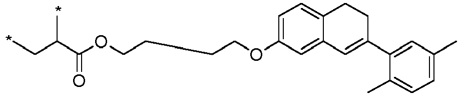 | M⁰-035 |
| 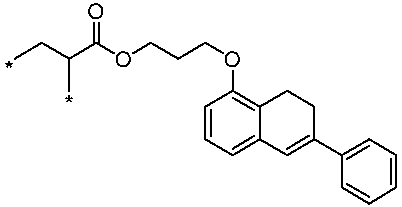 | M⁰-036 |
| 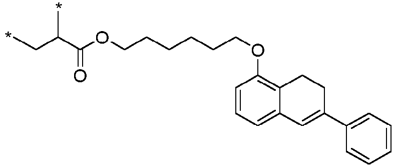 | M⁰-037 |
| 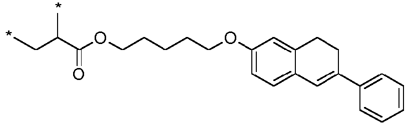 | M⁰-038 |
| 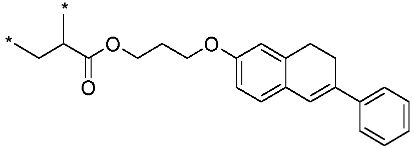 | M⁰-039 |
| 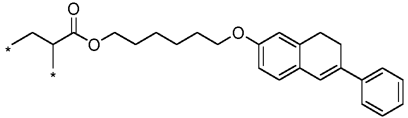 | M⁰-040 |
| 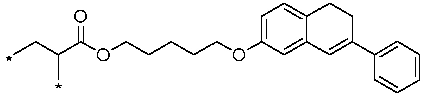 | M⁰-041 |
| 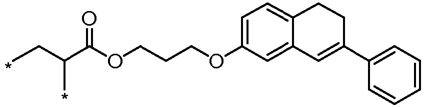 | M⁰-042 |
| 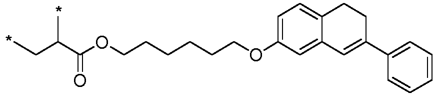 | M⁰-043 |
| 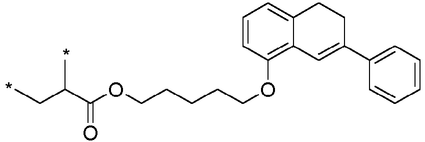 | M⁰-044 |
| 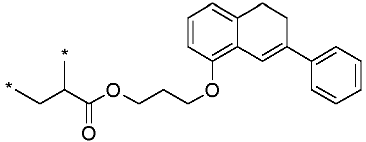 | M⁰-045 |
| 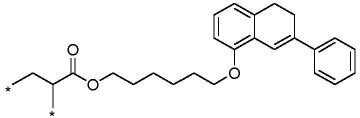 | M⁰-046 |
| 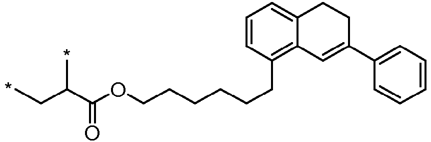 | M⁰-047 |
| 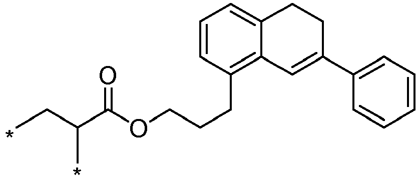 | M⁰-048 |
| 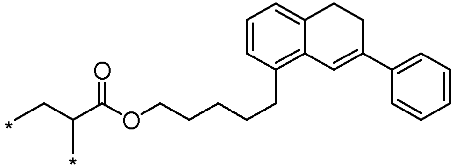 | M⁰-049 |
| 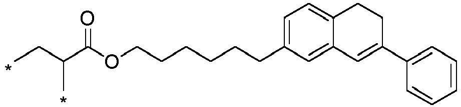 | M⁰-050 |
| 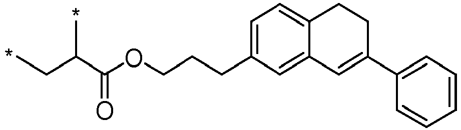 | M⁰-051 |
| 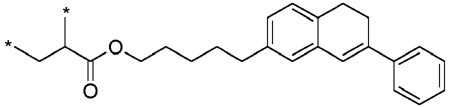 | M⁰-052 |
| 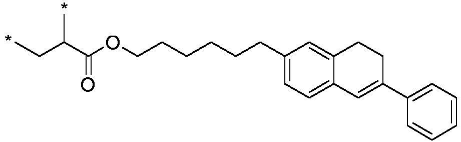 | M⁰-053 |
| 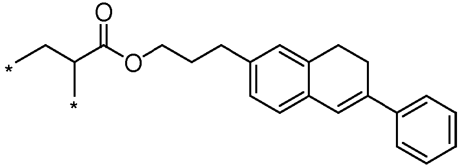 | M⁰-054 |
| 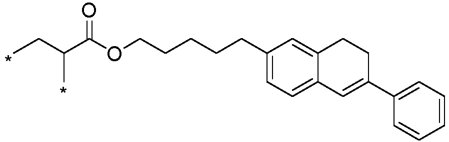 | M⁰-055 |
| 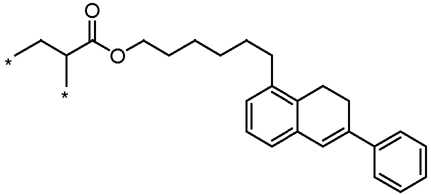 | M⁰-056 |
| 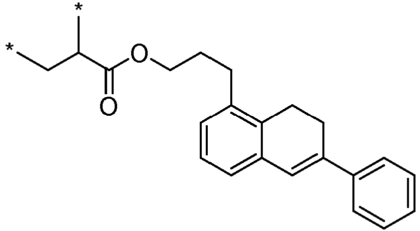 | M⁰-057 |
| 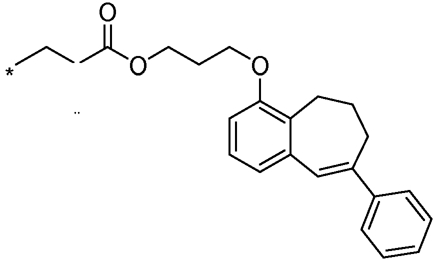 | M⁰-058 |
| 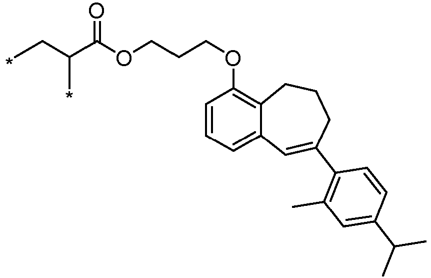 | M⁰-059 |
| 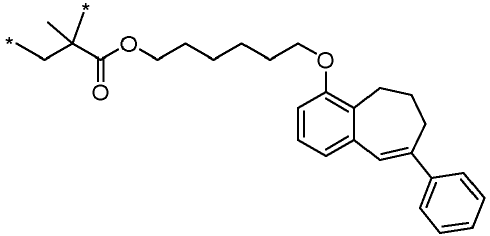 | M⁰-060 |
| 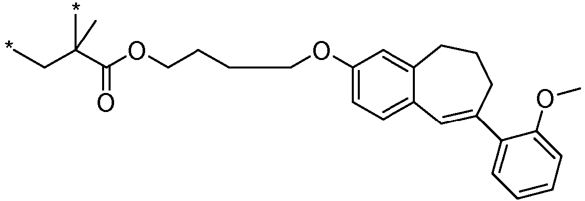 | M⁰-061 |
| 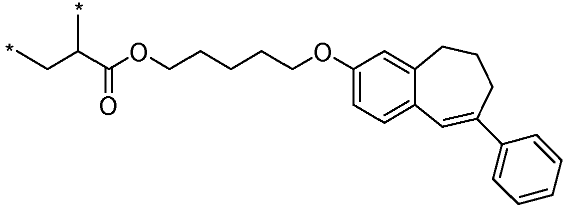 | M⁰-062 |
| 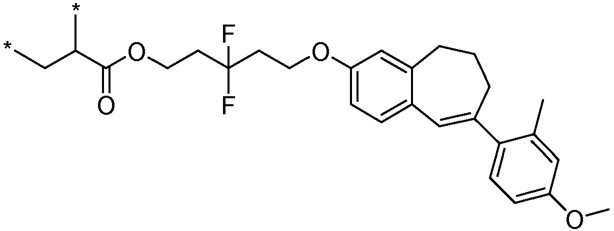 | M⁰-063 |
| 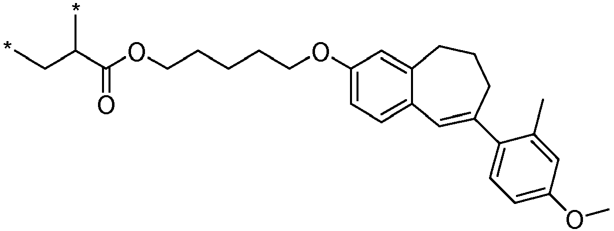 | M⁰-064 |
| 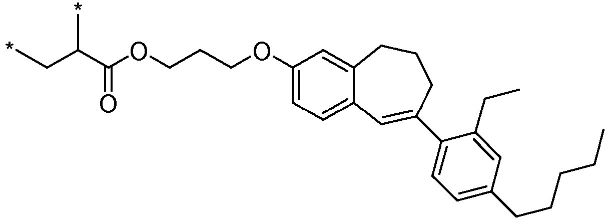 | M⁰-065 |
| 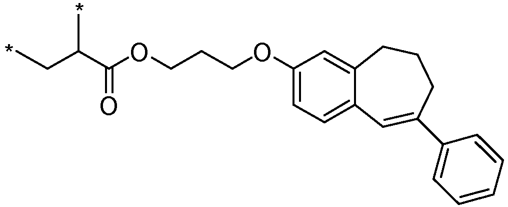 | M⁰-066 |
| 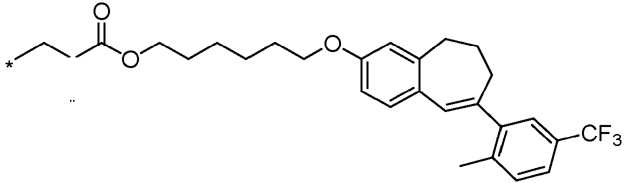 | M⁰-067 |
| 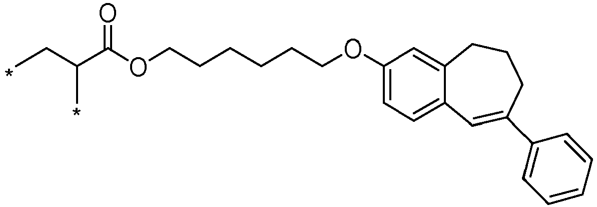 | M⁰-068 |
| 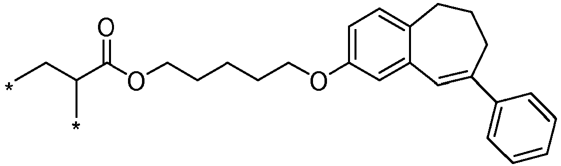 | M⁰-069 |
| 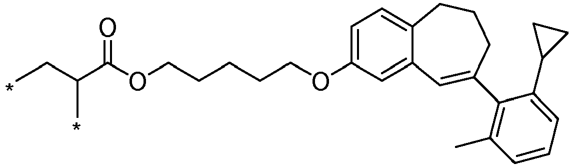 | M⁰-070 |
| 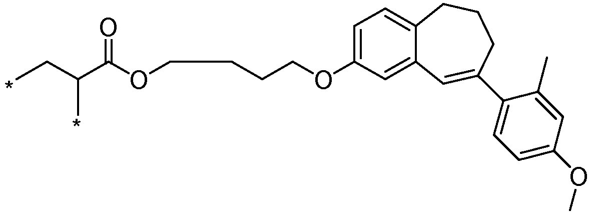 | M⁰-071 |
| 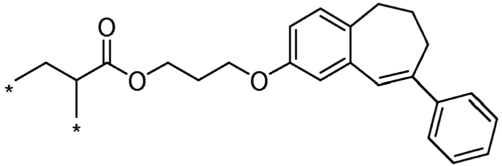 | M⁰-072 |
| 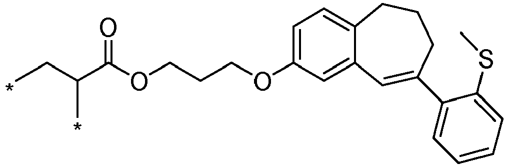 | M⁰-073 |
| 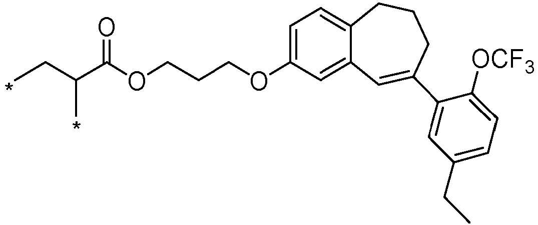 | M⁰-074 |
| 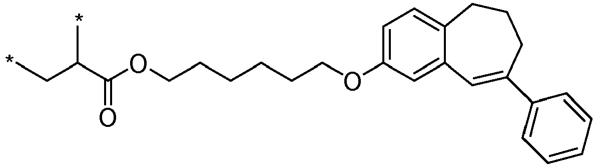 | M⁰-075 |
| 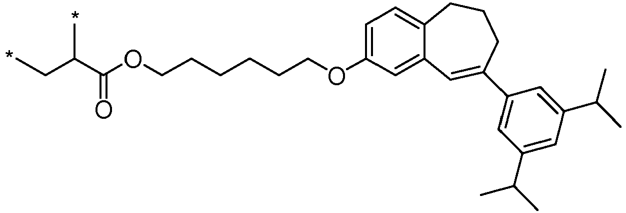 | M⁰-076 |
| 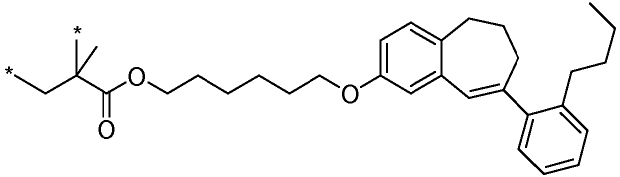 | M⁰-077 |
| 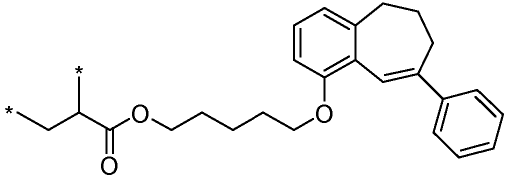 | M⁰-078 |
| 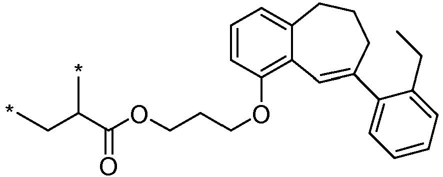 | M⁰-079 |
| 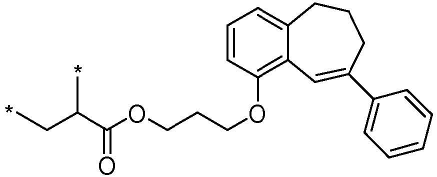 | M⁰-080 |
| 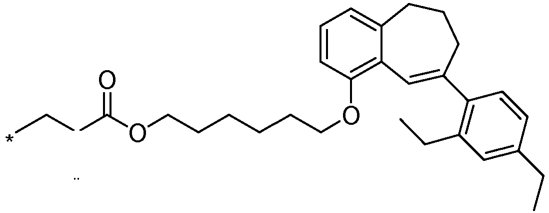 | M⁰-081 |
| 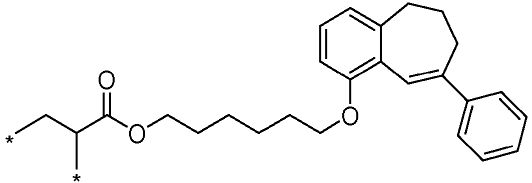 | M⁰-082 |
| 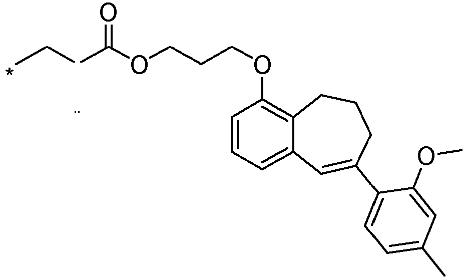 | M⁰-083 |
| 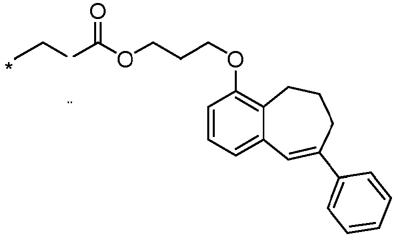 | M⁰-084 |
| 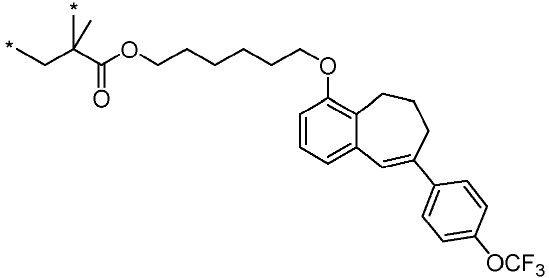 | M⁰-085 |
| 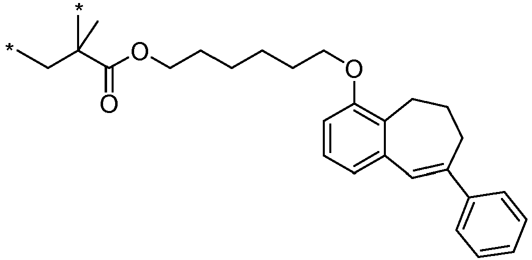 | M⁰-086 |
| 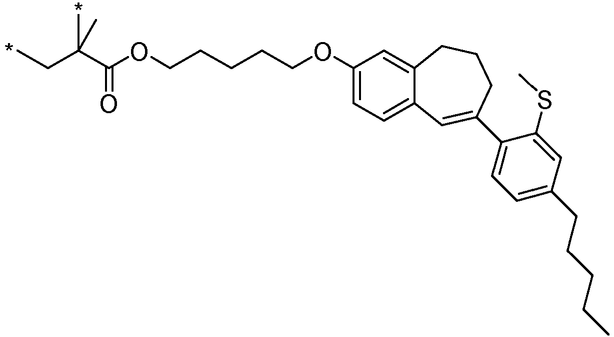 | M⁰-087 |
| 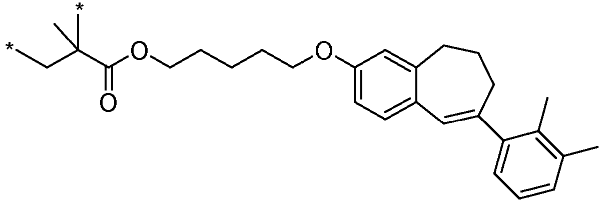 | M⁰-088 |
| 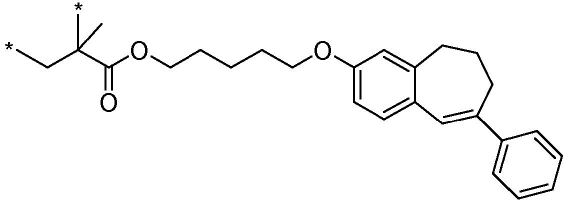 | M⁰-089 |
| 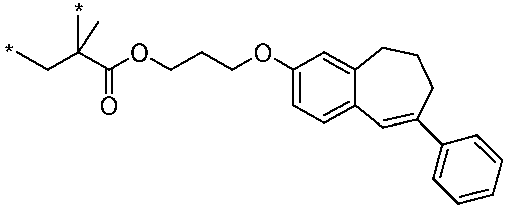 | M⁰-090 |
| 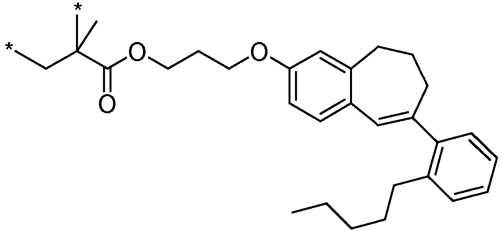 | M⁰-091 |
| 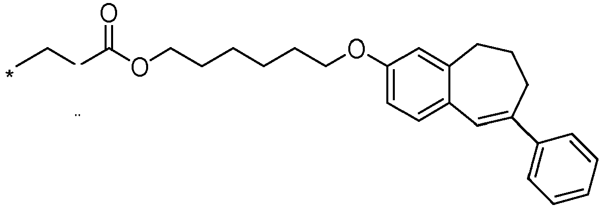 | M⁰-092 |
| 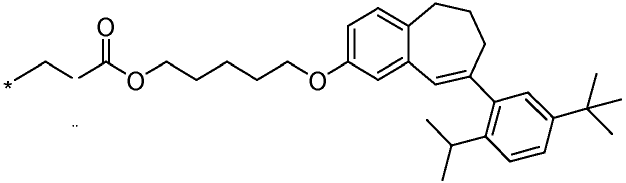 | M⁰-093 |
| 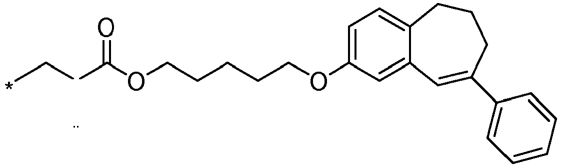 | M⁰-094 |
| 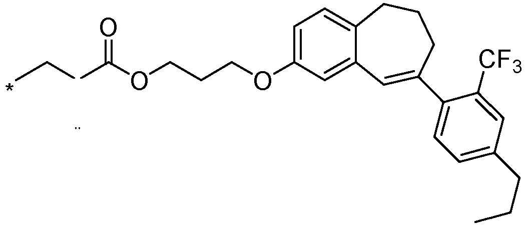 | M⁰-095 |
| 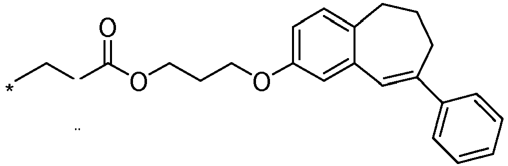 | M⁰-096 |
| 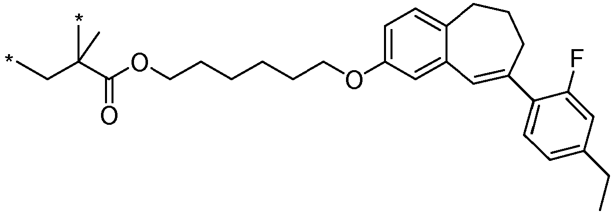 | M⁰-097 |
| 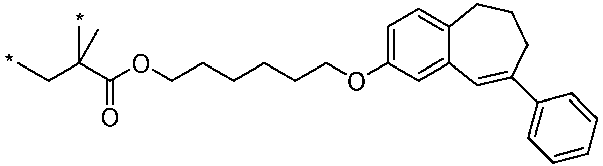 | M⁰-098 |
| 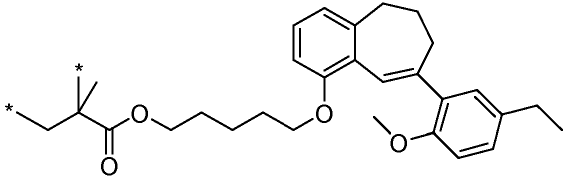 | M⁰-099 |
| 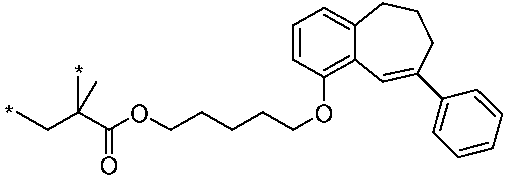 | M⁰-100 |
| 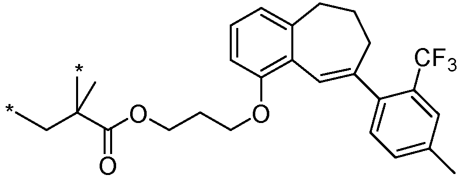 | M⁰-101 |
| 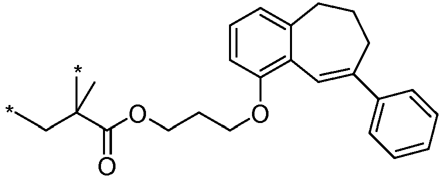 | M⁰-102 |
| 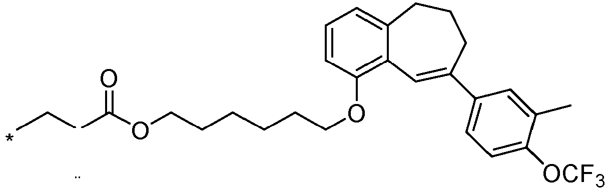 | M⁰-103 |
| 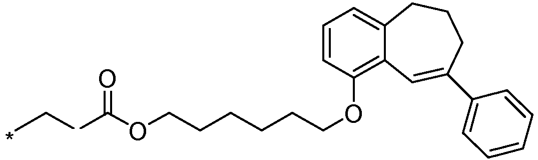 | M⁰-104 |
| 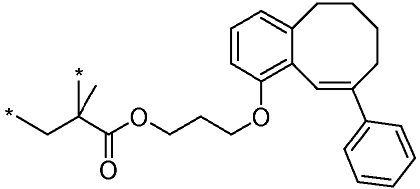 | M⁰-105 |
| 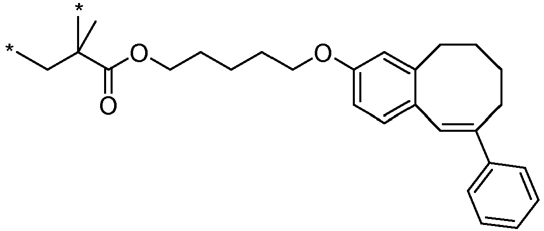 | M⁰-106 |
| 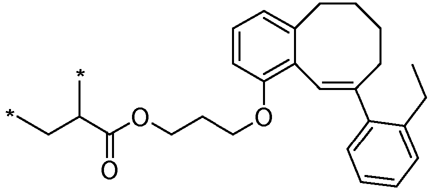 | M⁰-107 |
| 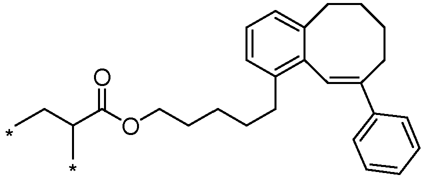 | M⁰-108 |
| 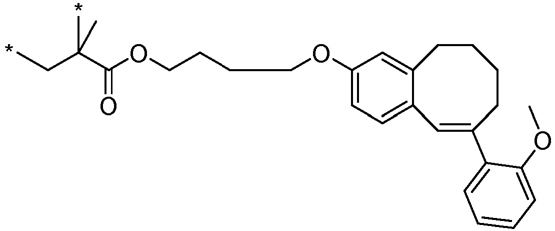 | M⁰-109 |
| 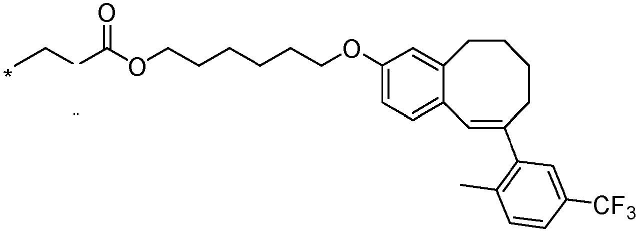 | M⁰-110 |
| 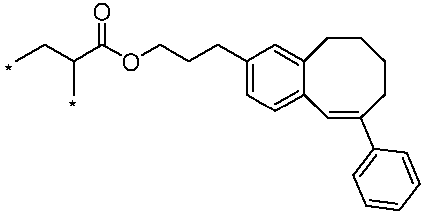 | M⁰-111 |
| 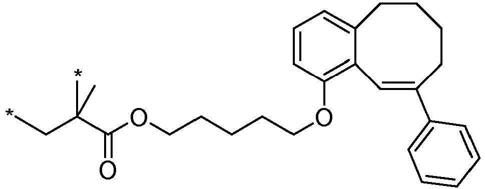 | M⁰-112 |
| 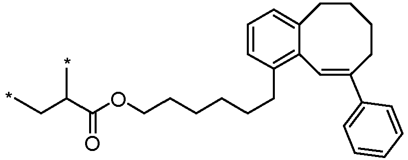 | M⁰-113 |
| 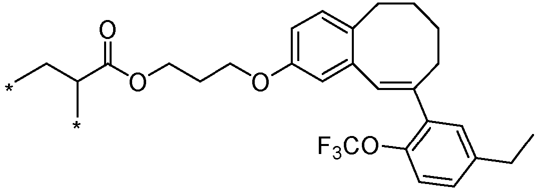 | M⁰-114 |
| 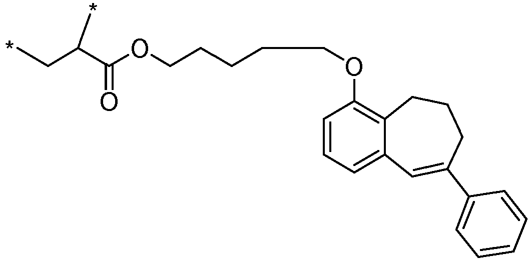 | M⁰-115 |
| 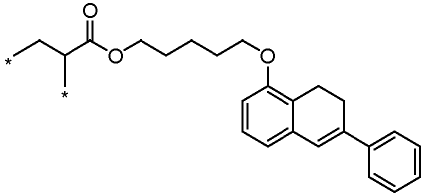 | M⁰-116 |
| 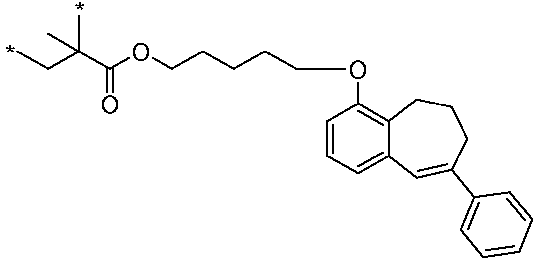 | M⁰-117. |
| 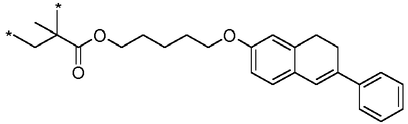 | M⁰-118 |
| 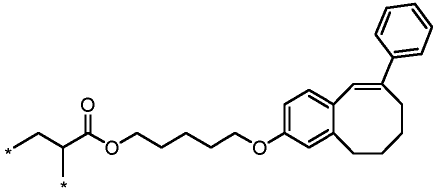 | M⁰-119 |
| 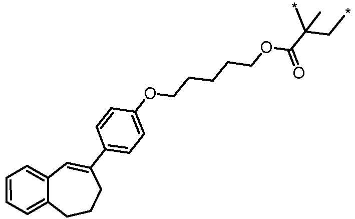 | M⁰-120 |
| 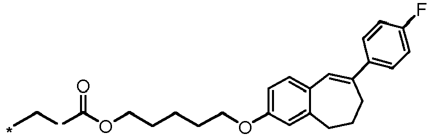 | M⁰-121 |
| 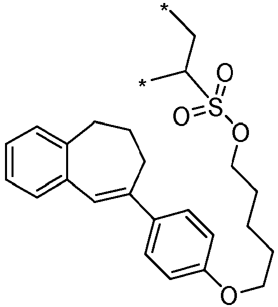 | M⁰-122 |
| 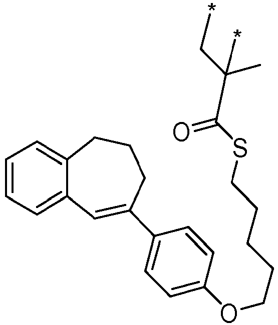 | M⁰-123 |
| 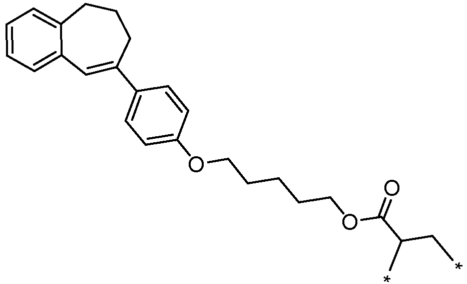 | M⁰-124 |
| 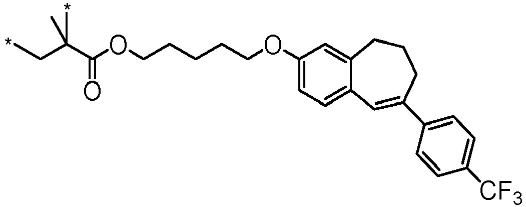 | M⁰-125 |
| 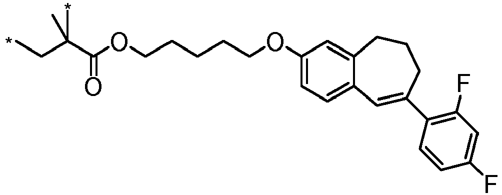 | M⁰-126 |
| 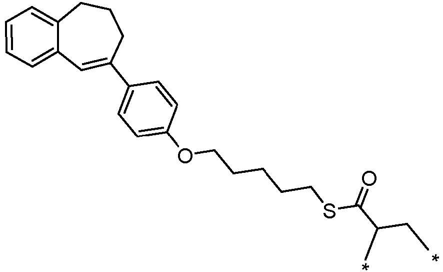 | M⁰-127 |
| 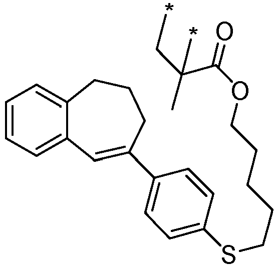 | M⁰-128 |
| 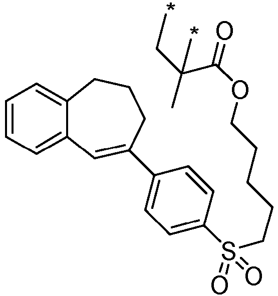 | M⁰-129 |
| 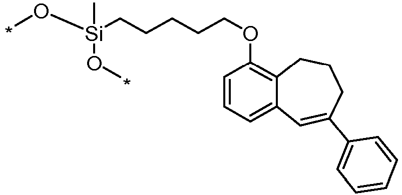 | M⁰-130 |
| 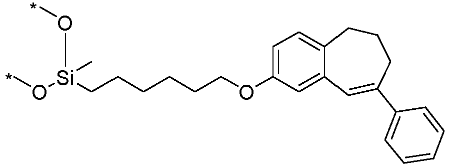 | M⁰-131 |
| 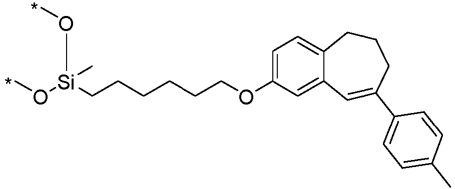 | M⁰-132 |
| 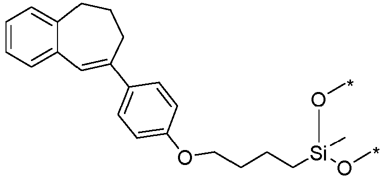 | M⁰-133 |
| 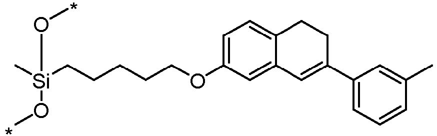 | M⁰-134 |
| 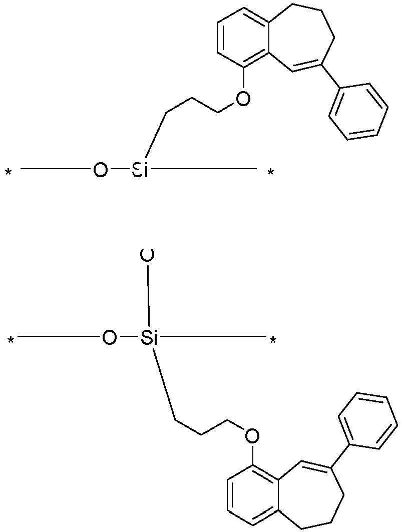 | M⁰-135 |
| 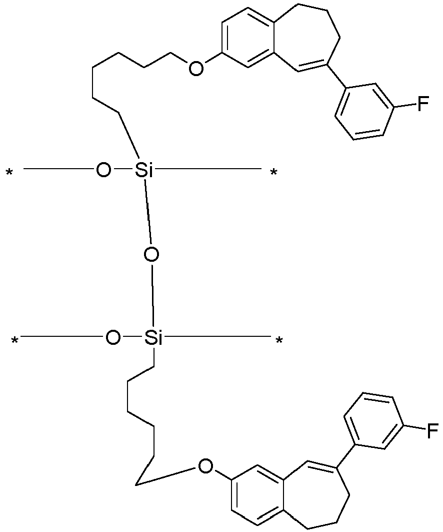 | M⁰-136 |
| 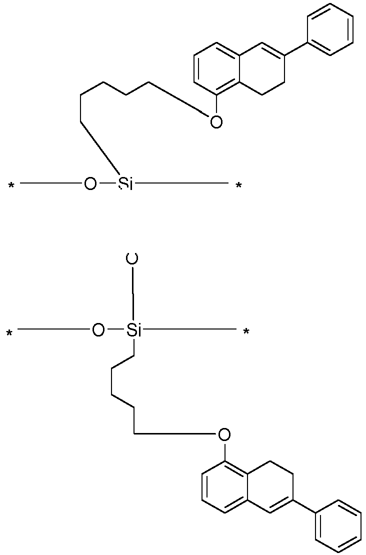 | M⁰-137 |
| 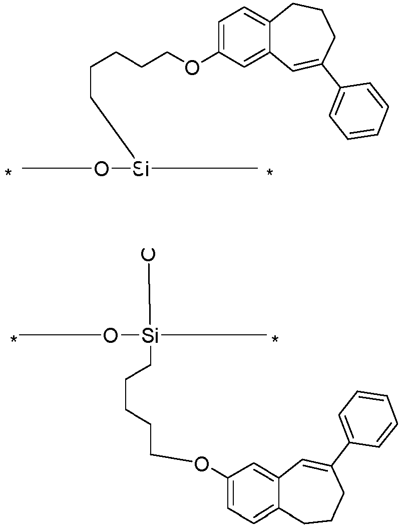 | M⁰-138 |
| 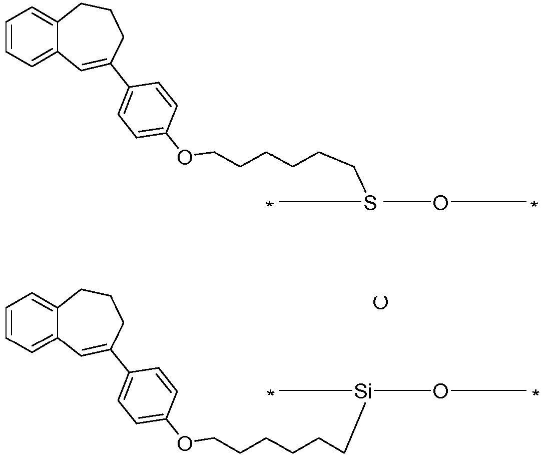 | M⁰-139 |
| 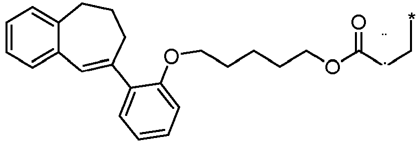 | M⁰-140 |
| 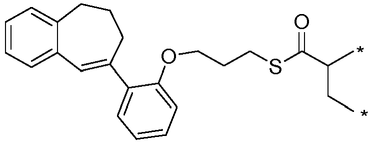 | M⁰-141 |
| 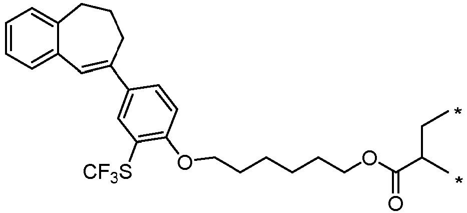 | M⁰-142 |
| 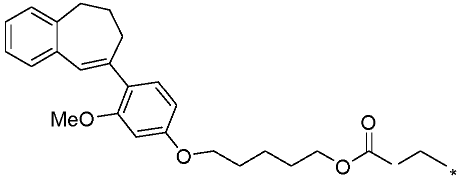 | M⁰-143 |
| 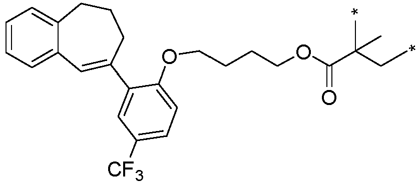 | M⁰-144 |
| 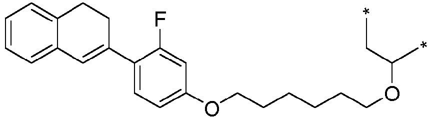 | M⁰-145 |
| 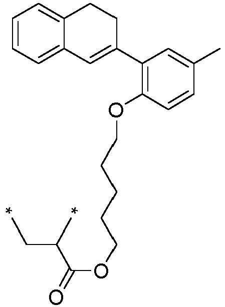 | M⁰-146 |
| 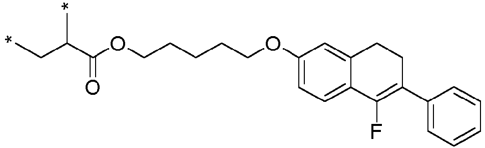 | M⁰-147 |
| 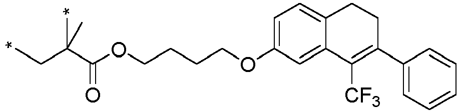 | M⁰-148 |
| 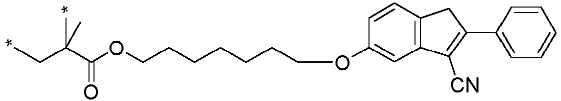 | M⁰-149 |
| 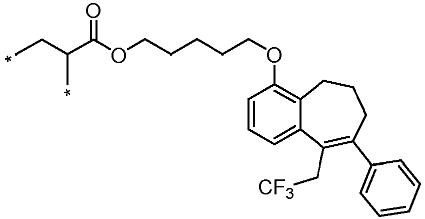 | M⁰-150 |
| 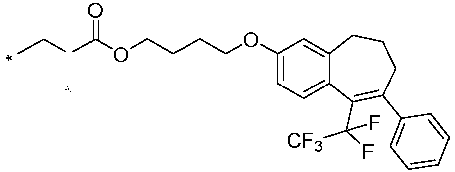 | M⁰-151 |
| 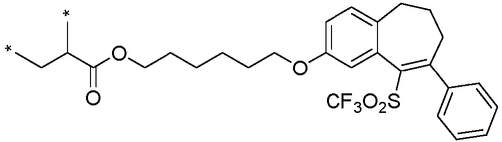 | M⁰-152 |
| 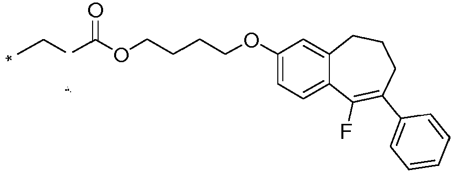 | M⁰-153 |
| 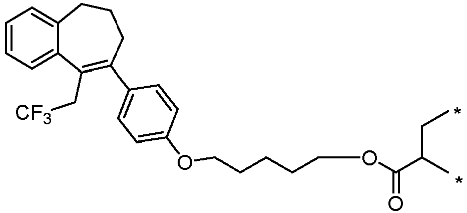 | M⁰-154 |
| 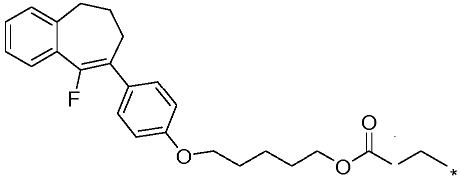 | M⁰-155 |
| 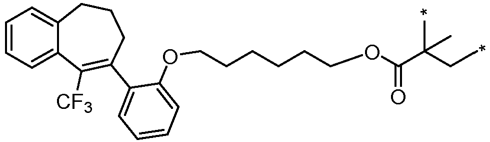 | M⁰-156 |
| 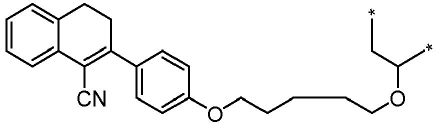 | M⁰-157 |
| 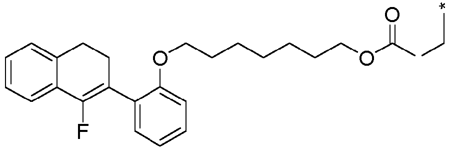 | M⁰-158 |
| 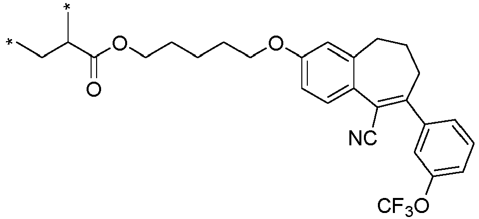 | M⁰-159 |
| 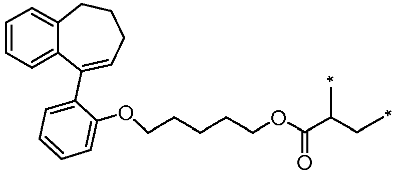 | M⁰-160 |
| 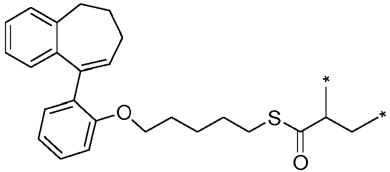 | M⁰-161 |
| 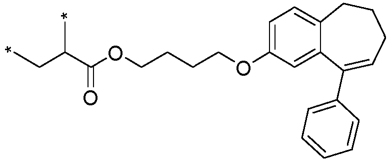 | M⁰-162 |
| 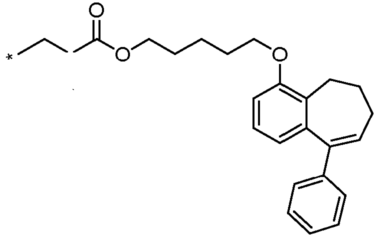 | M⁰-163 |
| 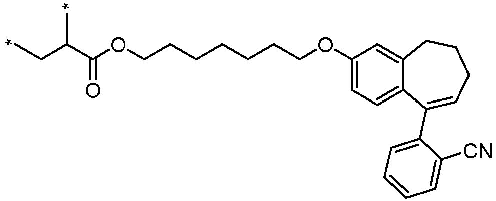 | M⁰-164 |
| 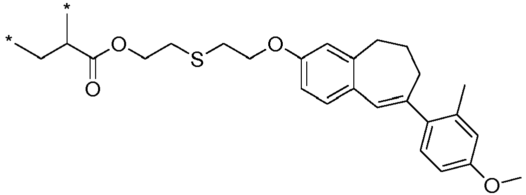 | M⁰-165 |
| 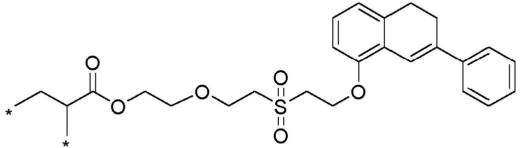 | M⁰-166 |
| 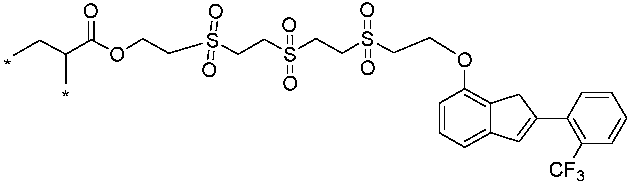 | M⁰-167 |
| 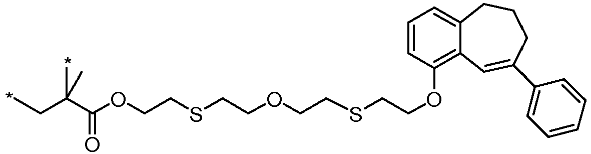 | M⁰-168 |
| 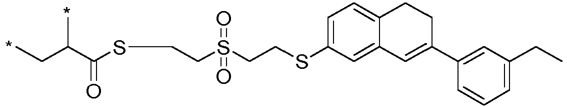 | M⁰-169 |
| 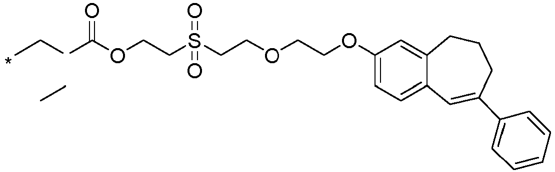 | M⁰-170 |
| 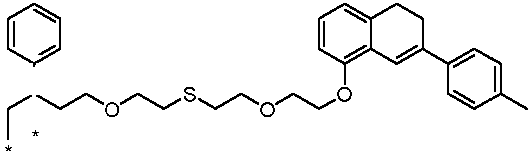 | M⁰-171 |
| 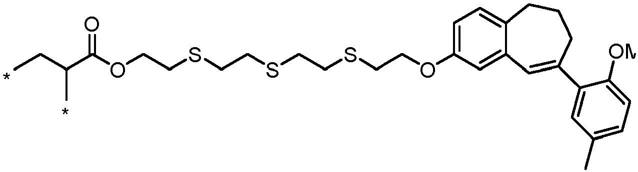 | M⁰-172 |
| 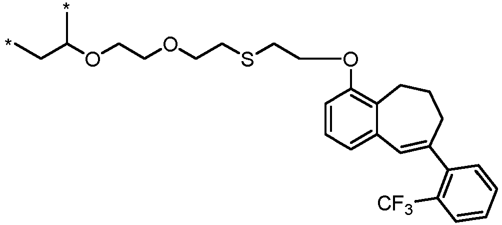 | M⁰-173 |
| 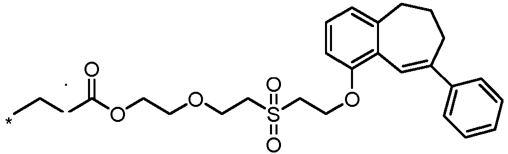 | M⁰-174 |
| 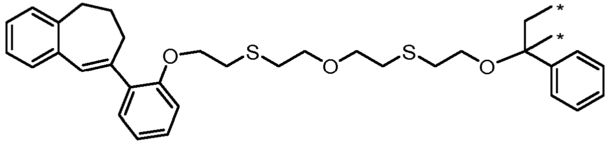 | M⁰-175 |
| 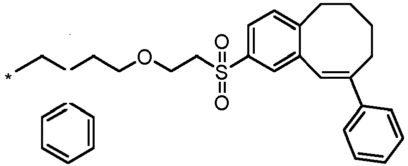 | M⁰-176 |
| 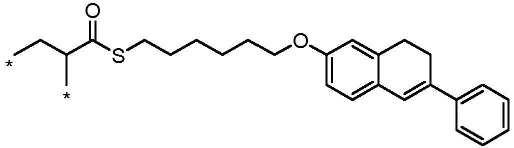 | M⁰-177 |
| 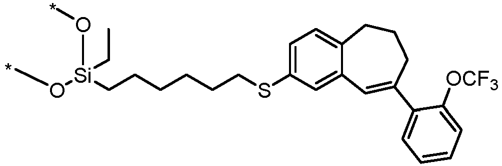 | M⁰-178 |
| 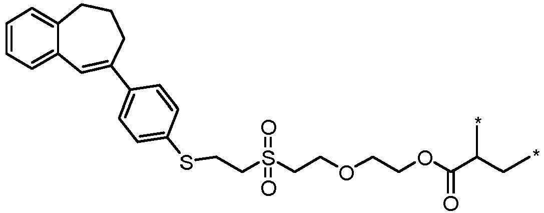 | M⁰-179 |
| 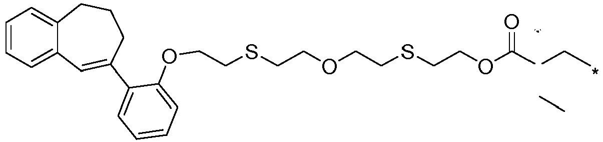 | M⁰-180. |

Preferred examples for monomeric units M⁰ derived from compounds of formulae (I), (I#), (1-1), (I-2), (I-2H), (I-3), (I-3-H), (I-4), (I-4-H), (I'), (I'-a), (I'-b), (I'-c), (I'-d), (I'-1), (I'-2), (I'-2-H), (I'-3), (I'-3-H), (I'-4), (I'-4-H) (I"), (I"-1), (I"-2), (I"-2-H), (I"-3), (I"-3-H), (I"-4) and/or (I"-4-H) or for constitutional units M⁰ of formulae (M⁰-I'), (M⁰-I"), (M⁰-I'-1), (M⁰-I"-1), (M⁰-I'-2-H), (M⁰-I"-2-H), (M⁰-I'-3-H), (M⁰-I"-3-H), (M⁰-I'-4-H) or (M⁰-I"-4-H) are the following monomeric units as shown in table 2-2.

**Table 2-2:**

| | |
|---|---|
| 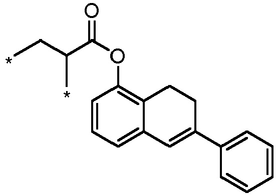 | M⁰-021 |
| 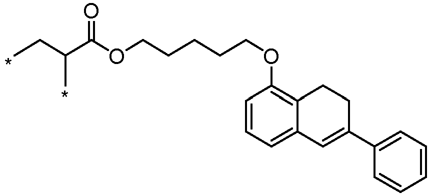 | M⁰-116 |
| 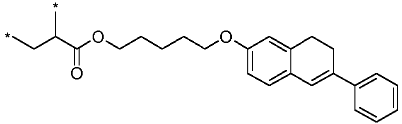 | M⁰-038 |
| 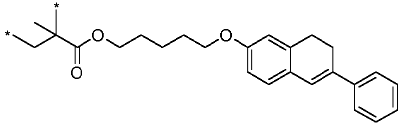 | M⁰-118 |
| 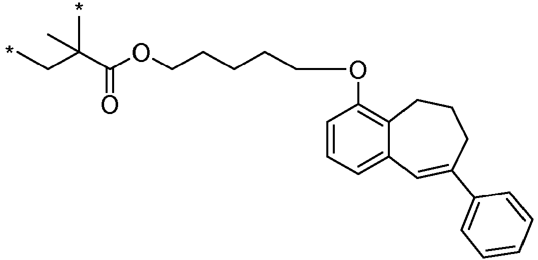 | M⁰-117 |
| 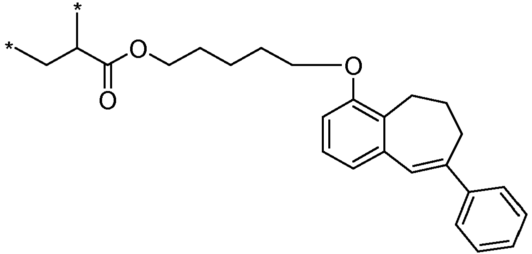 | M⁰-115 |
| 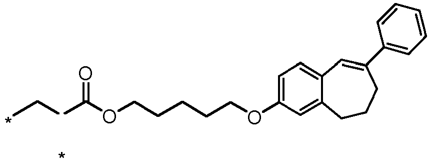 | M⁰-089 |
| 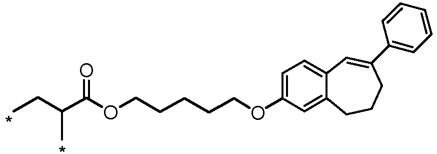 | M⁰-062 |
| 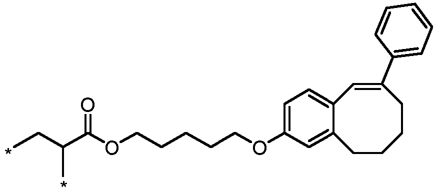 | M⁰-119 |
| 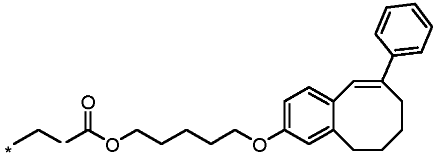 | M⁰-106 |
| 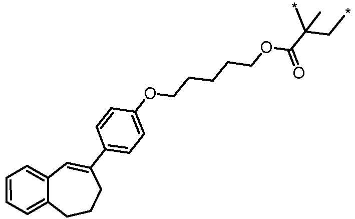 | M⁰-120 |
| 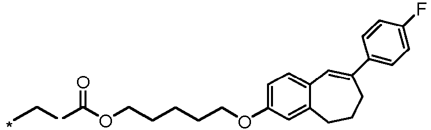 | M⁰-121 |
| 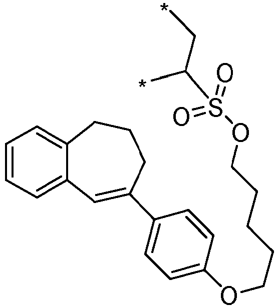 | M⁰-122 |
| 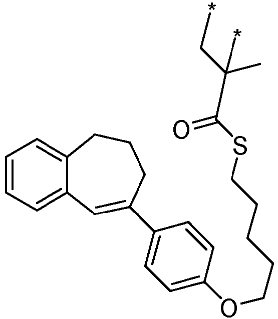 | M⁰-123 |
| 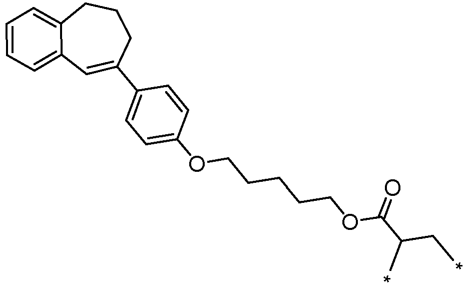 | M⁰-124 |
| 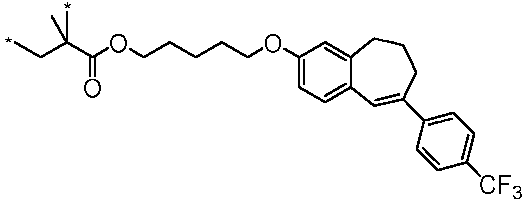 | M⁰-125 |
| 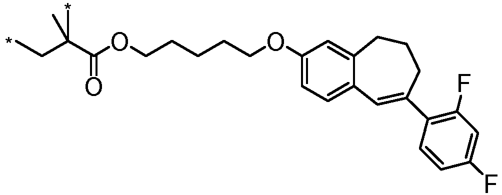 | M⁰-126 |
| 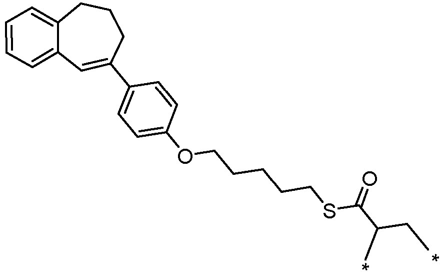 | M⁰-127 |
| 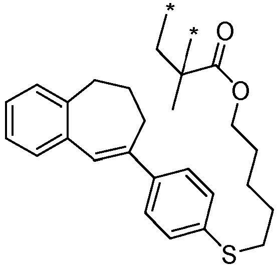 | M⁰-128 |
| 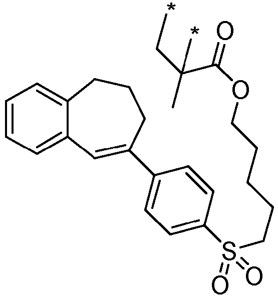 | M⁰-129. |

Preferably a copolymer according to the invention as described before or preferably described before comprises the one or more constitutional units M⁰ as described before with substituents as described before or preferably described before in a molar ratio m1 and the one or more constitutional units M² in a molar ratio m2, wherein the ratio m1 : m2 is at least 0.01 and at most 100.

Particularly preferably, such copolymer is comprised in the ophthalmic device or precursor article for an ophthalmic device according to the invention.

The oligomers, polymers or copolymers, preferably polymers or polymers, according to the invention as described before or preferably described may be cross-linked. Particularly preferably, such polymer or copolymer is comprised in the ophthalmic device or precursor article for an ophthalmic device according to the invention.

The oligomers or polymers of the present invention may be made by any suitable method. It is, however, preferred that the present oligomers, polymers and copolymers are made by radical polymerization, wherein the polymerization reaction is started by means of a suitable radical polymerization initiator. For the purposes of the present invention the type of radical polymerization initiator is not particularly limited and may be any suitable radical generating compound. Such compounds are well known to the skilled person. Suitable polymerization initiators may be selected from thermal initiators or photoinitiators, i.e. compounds that generate radicals by exposure to heat or irradiation with light of a suitable wavelength. Examples of suitable thermal polymerization initiators may be selected from the groups of compounds comprising one or more peroxide groups, i.e. compounds comprising a group -O-O-, and/or compounds comprising one or more azo groups, i.e. compounds comprising a group -N≡N-.

Suitable polymerization initiators comprising one or more peroxide groups may, for example, be selected from the groups consisting of t-butyl(peroxy-2-ethyl-hexanoate), di-(tert-butylcyclohexyl)peroxydicarbonate and benzoyl peroxide.

Suitable polymerization initiators comprising one or more azo groups may, for example, be selected from the group consisting of 1,1'-azobis(cyclohexancarbonitrile) and 2,2'azobis(cyclohexanecarbonitrile) (AIBN).

Suitable examples of a photoinitiator are dimethylaminobenzoate /camphorquinone, diphenyl(2,4,6-trimethylbenzoyl)phosphine oxide (TPO) or phenylbis(2,4,6-trimethylbenzoyl)phosphine oxide (BAPO).

If a photoinitiator is used as polymerization initiator, it is preferred that the wavelength required to decompose said photoinitiator is different from the wavelength needed to irradiate the compound of the present application so as to change its optical properties.

Preferably, the radical initiators are used in an amount of at least 0.0001 eq and of at most 0.1 eq of the main monomer. Such radical initiators could be thermal initiators, e.g. azobisisobutyronitrile (AIBN) or photochemical initiators like dimethylaminobenzoate/camphorquinone, diphenyl(2,4,6-trimethylbenzoyl)phosphine oxide (TPO) or phenylbis(2,4,6-trimethylbenzoyl)phosphine oxide (BAPO).

The present invention is also directed to a composition for polymerization. Depending upon the intended use such composition as described or preferably described before may comprise further different components. Such further components may, for example, be selected from the group consisting of UV absorbers, antioxidants and cross-linkers.

Cross-linkers may also be referred to as crosslinking agents.

The present invention is also directed to a composition for polymerization comprising at least one compound of formulae (I), (I#), (1-1), (I-2), (I-2H), (I-3), (I-3-H), (I-4), (I-4-H), (I'), (I'-a), (I'-b), (I'-c), (I'-d), (I'-1), (I'-2), (I'-2-H), (I'-3), (I'-3-H), (I'-4), (I'-4-H) (I"), (I"-1), (I"-2), (I"-2-H), (I"-3), (I"-3-H), (I"-4) or (I"-4-H) or compounds (A-001) to (A-180) as described or preferably described before and/or an oligomer or polymer as described before or preferably described before but having at least one reactive group left for polymerization excluding 2-(vinylphenyl)indene, 2-[4-(2-propen-1-yl)phenyl-1H-indene and any oligomer, polymer or copolymer derived from 2-(vinylphenyl)indene and 2-[4-(2-propen-1-yl)phenyl-1H-indene and/or a crosslinking agent and/or a UV absorber and/or a radical initiator and optionally further monomers different from compounds of formulae (I), (I#), (I-1), (I-2), (I-2H), (I-3), (I-3-H), (I-4), (I-4-H), (I'), (I'-a), (I'-b), (I'-c), (I'-d), (I'-1), (I'-2), (I'-2-H), (I'-3), (I'-3-H), (I'-4), (I'-4-H) (I"), (I"-1), (I"-2), (I"-2-H), (I"-3), (I"-3-H), (I"-4) or (I"-4-H) or compounds (A-001) to (A-180).

The present invention is also directed to a preferred composition for polymerization comprising at least one compound of formulae (I), (I#), (1-1), (I-2), (I-2H), (I-3), (I-3-H), (I-4), (I-4-H), (I'), (I'-a), (I'-b), (I'-c), (I'-d), (I'-1), (I'-2), (I'-2-H), (I'-3), (I'-3-H), (I'-4), (I'-4-H) (I"), (I"-1), (I"-2), (I"-2-H), (I"-3), (I"-3-H), (I"-4) or (I"-4-H) or compounds (A-001) to (A-180) as described or preferably described before excluding 2-(vinylphenyl)indene, 2-[4-(2-propen-1-yl)phenyl-1H-indene and/or a crosslinking agent and/or a UV absorber and/or a radical initiator and optionally further monomers different from compounds of formulae (I), (I#), (1-1), (I-2), (I-2H), (I-3), (I-3-H), (I-4), (I-4-H), (I'), (I'-a), (I'-b), (I'-c), (I'-d), (I'-1), (I'-2), (I'-2-H), (I'-3), (I'-3-H), (I'-4), (I'-4-H) (I"), (I"-1), (I"-2), (I"-2-H), (I"-3), (I"-3-H), (I"-4) or (I"-4-H) or compounds (A-001) to (A-180).

A composition comprising at least one compound of formulae (I), (I#), (1-1), (I-2), (I-2H), (I-3), (I-3-H), (I-4), (I-4-H), (I'), (I'-a), (I'-b), (I'-c), (I'-d), (I'-1), (I'-2), (I'-2-H), (I'-3), (I'-3-H), (I'-4), (I'-4-H) (I"), (I"-1), (I"-2), (I"-2-H), (I"-3), (I"-3-H), (I"-4) or (I"-4-H) or compounds (A-001) to (A-180) as described or preferably described before and an oligomer or polymer according to the invention as described before is primarily used for the synthesis of block copolymers with the condition that the oligomer or polymer has at least one reactive group left which may react with the monomers.

The compositions may include or comprise, essentially consist of or consist of the said requisite or optional constituents. All compounds or components which can be used in the compositions are either known and commercially available or can by synthesized by known processes.

The UV absorber that may be used in the present composition is not particularly limited and can easily be selected from those generally known to the skilled person. Generally suitable UV absorbers are characterized by being unsaturated compounds, preferably compounds comprising one or more selected from group consisting of olefinic groups, aryl groups and heteroaryl groups; these groups may be present in any combination.

Suitable UV-absorber for use in the present composition may, for example, be selected from those comprising a group selected from benzotriazole, benzophenone and triazine. Suitable UV-absorbers are, for example, disclosed in U.S. Pat. Nos. 5,290,892; 5,331,073 and 5,693,095.

Suitable UV-absorber are 2-(3-(t-butyl)-4-hydroxy-5-(5-methoxy-2-benzotriazolyl)phenoxy)ethyl methacrylate, 3-(3-(t-butyl)-4-hydroxy-5-(5-methoxy-2-benzotriazolyl)phenoxy)propyl methacrylate, 3-(3-t-Butyl-5-(5-chlorobenzotriazol-2-yl)-4-hydroxyphenyl)propyl methacrylate3-(3-(tert-Butyl)-4-hydroxy-5-(5-methoxy-2H-benzo[d][1,2,3]triazol-2-yl)phenoxy)propylmethacrylat, 2-(2-Hydroxy-5-vinylphenyl)-2H-benzotriazol, Allyl-2-hydroxybenzophenon, 2-Allyl-6-(2H-benzotriazol-2-yl)-p-cresol, 4-Methacryloxy-2-hydroxybenzophenon, 2-(2'-Hydroxy-3'-methallyl-5'-methylphenyl)benzotriazol, 2-Hydroxy-4-methacryloyloxybenzophenon, 4-Acryloylethoxy-2-hydroxybenzophenon, 2-[3-(2H-Benzotriazol-2-yl)-4-hydroxyphenyl]ethylmethacrylat, 2-(2'-Hydroxy-5'-methacrylamidophenyl)-5-methoxybenzotriazol, 2-(2'-Hydroxy-5'-methacrylamidophenyl)-5-chlorobenzotriazol, 2-(2'-Hydroxy-5'-methacryloxypropylphenyl)benzotriazol, 2-(2'-Hydroxy-5'-methacryloylpropyl-3'-tert-butyl-phenyl)-5-methoxy-2H-benzotriazol, 2-(3-(tert-Butyl)-4-hydroxy-5-(5-methoxy-2H-benzo[d][1,2,3]triazol-2-yl)phenoxy)ethylmethacrylat, 2-[3'-tert-Butyl-2'-hydroxy-5'-(3"-methacryloyloxypropyl)phenyl]-5-chlorbenzotriazol, 2-{2'-Hydroxy-3'-tert-butyl-5'-[3'-methacryloyloxypropoxy]phenyl}-5-methoxy-2H-benzotriazol, 2-[3'tert-Butyl-5'-(3"-dimethylvinylsilylpropoxy)-2'-hydroxyphenyl]-5-methoxybenzotriazol, 2-(tert-Butyl)-6-(5-chloro-2H-benzo[d][1,2,3]triazol-2-yl)-4-vinylphenol, 2-(2H-1,2,3-benzotriazol-2-yl)-4-methyl-6-(2-methylprop-2-enyl)phenol, 2-(3-acetyl-2-aminophenoxy)ethyl methacrylat, 2-(4-benzoyl-3-hydroxyphenoxy)ethyl acrylat or a combination of this compounds.

Suitable cross-linker may be used to impart elastomeric properties to the present composition and the ophthalmic devices or precursor articles produced therewith. Typically any suitable di- or tri-functional monomer may be used as crosslinker. Such monomers are generally well known to the skilled person and may be selected from poly(ethylene glycol) diacrylate, poly(ethylene glycol) dimethacrylate, ethyleneglycoldimethacrylate (EGDMA), ethyleneglycoldiacrylate, 1,3-propanedioldiacrylat, 1,6-Hexanedioldiacrylate, 1,8-Octanedioldiacrylate, 1,15-Pentadecandioldiacrylate, 1,16-Hexadecanedioldiacrylate, 1,18-Octadecanedioldiacrylate, 1,3-Propanedioldimethacrylate, 1,6-Hexanedioldimethacrylate, 1,8-Octanedioldimethacrylate, 1,15-Pentadecanedioldimethacrylate, 1,16-Hexadecanedioldimethacrylate, 1,18-Octadecanedioldimethacrylate.

Preferred cross-linker may be selected from the following group of compounds

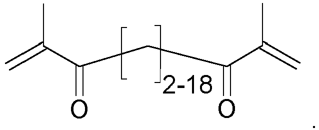

Ethylene glycol dimethacrylate (EGDMA) is particularly preferred.

Suitable antioxidants are phenyl acrylate derivatives bearing a hindered phenol moiety. A preferred antioxidant is

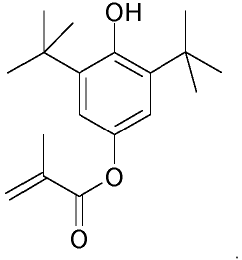

The compounds of formulae (I), (I#), (I-1), (I-2), (I-2H), (I-3), (I-3-H), (I-4), (I-4-H), (I'), (I'-a), (I'-b), (I'-c), (I'-d), (I'-1), (I'-2), (I'-2-H), (I'-3), (I'-3-H), (I'-4), (I'-4-H) (I"), (I"-1), (I"-2), (I"-2-H), (I"-3), (I"-3-H), (I"-4) or (I"-4-H) or compounds (A-001) to (A-180) according to the invention as described or preferably described before and their oligomers, polymers or copolymers as described before or preferably described before comprising one or more constitutional units M⁰ of formulae (M⁰-I'), (M⁰-I"), (M⁰-I'-1), (M⁰-I"-1), (M⁰-I'-2-H), (M⁰-I"-2-H), (M⁰-I'-3-H), (M⁰-I"-3-H), (M⁰-I'-4-H) or (M⁰-I"-4-H), or one or more constitutional units (M⁰-001) to (M⁰-127) as described before or preferably described before are particularly well suited for use in optically active devices as described before.

The compounds of formulae (I), (I#), (I-1), (I-2), (I-2H), (I-3), (I-3-H), (I-4), (I-4-H), (I'), (I'-a), (I'-b), (I'-c), (I'-d), (I'-1), (I'-2), (I'-2-H), (I'-3), (I'-3-H), (I'-4), (I'-4-H) (I"), (I"-1), (I"-2), (I"-2-H), (I"-3), (I"-3-H), (I"-4) or (I"-4-H) or compounds (A-001) to (A-180) according to the invention as described or preferably described before and their oligomers, polymers or copolymers as described before or preferably described before comprising one or more constitutional units M⁰ of formulae (M⁰-I'), (M⁰-I"), (M⁰-I'-1), (M⁰-I"-1), (M⁰-I'-2-H), (M⁰-I"-2-H), (M⁰-I'-3-H), (M⁰-I"-3-H), (M⁰-I'-4-H) or (M⁰-I"-4-H), or one or more constitutional units (M⁰-001) to (M⁰-180) as described before or preferably described before are particularly sensitive to two-photon or multiphoton absorption. Hence the ophthalmic device and the precursor article for the ophthalmic device are sensitive to two-photon or multiphoton absorption.

Hence the present invention is also directed to precursor articles wherein said precursor article is a blank which may be transformed into optically active ophthalmic devices comprising at least one oligomer, polymer or copolymer as described before or preferably described before comprising one or more constitutional units M⁰ of formulae (M⁰-I'), (M⁰-I"), (M⁰-I'-1), (M⁰-I"-1), (M⁰-I'-2-H), (M⁰-I"-2-H), (M⁰-I'-3-H), (M⁰-I"-3-H), (M⁰-I'-4-H) or (M⁰-I"-4-H), or one or more constitutional units (M⁰-001) to (M⁰-180) as described before or preferably described before.

Preferred ophthalmic devices are optically active ophthalmic devices. Examples of such ophthalmic devices or eye-implants include lenses, keratoprostheses, and corneal inlays or rings. More preferably, said ophthalmic device or eye-implant is a lens article. Most preferably, such ophthalmic device is a lens. The type of lens is not restricted and may comprise a contact lens or an intraocular lens. Most preferably, such ophthalmic device is an intraocular lens article, which may, for example, be a posterior chamber intraocular lens or an anterior chamber intraocular lens.

A blank of this invention may be produced as a step in the manufacturing process used to create a lens, preferably an intraocular lens. For example, without limitation, a manufacturing process may include the steps of polymer synthesis, polymer sheet casting, blank cutting, optic lathe cutting, optic milling, haptic milling or attachment, polishing, solvent extraction, sterilization and packaging while the term polymer is used as described before or preferably described before.

The present ophthalmic devices or precursor articles for an ophthalmic device according to the invention as described before or preferably described before may be formed by a process comprising the steps of
- providing a composition comprising at least one compound of formulae (I), (I#), (1-1), (I-2), (I-2H), (I-3), (I-3-H), (I-4), (I-4-H), (I'), (I'-a), (I'-b), (I'-c), (I'-d), (I'-1), (I'-2), (I'-2-H), (I'-3), (I'-3-H), (I'-4), (I'-4-H) (I"), (I"-1), (I"-2), (I"-2-H), (I"-3), (I"-3-H), (I"-4) or (I"-4-H) or compounds (A-001) to (A-180) as described herein or preferably described herein and/or an oligomer or polymer as described herein or preferably described herein but having at least one reactive group left for polymerization and optionally further monomers different from compounds of formulae (I), (I') or (I") and/or crosslinking agents and/or UV absorbers and/or radical initiators; and
- subsequently forming the ophthalmic device or precursor article of said composition.

The present ophthalmic devices or precursor articles according to the invention as described before or preferably described before may be formed by a process comprising the steps of
- providing a composition comprising at least one compound of formulae (I), (I#), (1-1), (I-2), (I-2H), (I-3), (I-3-H), (I-4), (I-4-H), (I'), (I'-a), (I'-b), (I'-c), (I'-d), (I'-1), (I'-2), (I'-2-H), (I'-3), (I'-3-H), (I'-4), (I'-4-H) (I"), (I"-1), (I"-2), (I"-2-H), (I"-3), (I"-3-H), (I"-4) or (I"-4-H) or compounds (A-001) to (A-127) as described herein or preferably described herein and optionally further monomers different from compounds of formulae (I), (I') or (I") and/or crosslinking agents and/or UV absorbers and/or radical initiators; and
- subsequently forming the ophthalmic device or precursor article of said composition.

Intraocular lenses in accordance with the present invention are believed to show particularly advantageous properties in that they are flexible enough so as to be rolled or folded and consequently requiring a much smaller incision for them to be inserted into the eye. It is believed that this will allow for improved healing of the eye, particularly in respect to the time for the eye to heal.

The type of intraocular lens is not limited in any way. It may, for example, be a pseudo-phakic intraocular lens or a phakic intraocular lens. The former type replaces the eye's natural, crystalline lens, usually to replace a cataractous lens that has been removed. The latter type is used to supplement an existing lens and functions as a permanent corrective lens, which is implanted in the anterior or posterior chamber to correct refractive errors of the eye. It may, for example, comprise one or more optic and one or more haptic components, wherein the one or more optic components serve as lens and the one or more haptic components are attached to the one or more optic components and hold the one or more optic components in place in the eye. The present intraocular lens may be of a one-piece design or of multi-piece design, depending on whether the one or more optic components and the one or more haptic components are formed from a single piece of material (one-piece design) or are made separately and then combined (multi-piece design). The present intraocular lens is also designed in such a way that it allows to be, for example, rolled up or folded small enough so that it fits through an incision in the eye, said incision being as small as possible, for example, at most 3 mm in length.

Additionally, intraocular lenses in accordance with the present invention allow for the non-invasive adjustment of the optical properties, particularly the refractive power, after implantation of the lens into the eye, thus reducing the need for post-surgery vision aids or reducing or totally avoiding follow-up surgery.

In order to change the optical properties and particularly the refractive power of the intraocular lens it is exposed to irradiation having a wavelength of at least 200 nm and of at most 1500 nm.

Hence, the present invention is also directed to a process of changing the optical properties of an ophthalmic device or a precursor article for an ophthalmic device as defined or preferably defined herein, said process comprising the steps of
- providing an ophthalmic device or a precursor article for an ophthalmic device as defined herein; and
- subsequently exposing said ophthalmic device or precursor article to irradiation having a wavelength of at least 200 nm and at most 1500 nm.

Preferably, said irradiation has a wavelength of at least 250 nm or 300 nm, more preferably of at least 350 nm, even more preferably of at least 400 nm, still even more preferably of at least 450 nm, and most preferably of at least 500 nm. Preferably, said irradiation has a wavelength of at most 1400 nm or 1300 nm or 1200 nm or 1100 nm or 1000 nm, more preferably of at most 950 nm or 900 nm, even more preferably of at most 850 nm, still even more preferably of at most 800 nm and most preferably of at most 750 nm.

Alternatively, you may describe the change of refractive power as a modification of the index of refraction of said ophthalmic device as described before or preferably described before. Alternatively, you may describe the change of refractive power as a modification of the index of refraction of said intraocular lens article as described before or preferably described before. Irradiation within the focal volume results in refractive optical structures characterized by a change in refractive index relative to the index of refraction of the bulk of said ophthalmic device or alternatively the non-irradiated portion of said ophthalmic device. Irradiation within the focal volume results in refractive optical structures characterized by a change in refractive index relative to the index of refraction of the bulk of said intraocular lens article or alternatively the non-irradiated portion of said intraocular lens article. The change in refractive index can in other words be used to form patterned desired refractive structures in the optical ophthalmic device as described or preferably described before, preferably in the intraocular lens article as described or preferably described before.

It is preferred to provide refractive structures that exhibit a change in refractive index, and exhibit little or no scattering loss in such a way that ablation or removal of the optical ophthalmic device, preferably the intraocular lens article is not observed in the irradiated region.

In such processes, the irradiated regions of the ophthalmic device as described before or preferably described before can take the form of two- or three-dimensional, area or volume filled refractive structures that can provide spherical, aspherical, toroidal, or cylindrical correction. In fact, any optical structure can be formed to yield power correction in both physical directions. Moreover, the optical structures can be stacked vertically or written in separate planes in the ophthalmic device as described before or preferably described before to act as a single lens element.

### Examples

The following examples are intended to show the advantages of the present compounds in a non-limiting way.

Unless indicated otherwise, all syntheses are carried out under an inert atmosphere using dried (i.e. water-free) solvents. Solvents and reagents are purchased from commercial suppliers.

DCM is used to denote dichloromethane. DMF is used to denote dimethylformamide. EE is used to denote ethyl acetate. THF is used to denote tetrahydrofuran. RT means room temperature.

Copolymer-properties can be investigated on blanks, prepared by bulk polymerization of the monomers. Co-monomers, cross-linkers and initiators therefore can be purchased from commercial sources. All chemicals are of highest purity available and can be used as received.

### Synthesis of precursor materials:

### Example 1:

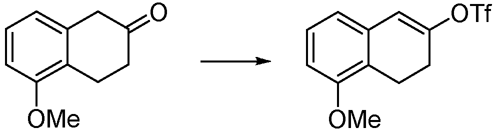

CAS: 32940-15-1

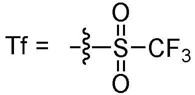

To a solution of diisopropylamine (4.25 ml; 30.28 mmol; 1.10 eq.) in THF (30 ml) n-butyllithium (2.5M in hexane) (12.11 ml; 30.28 mmol; 1.10 eq.) is added dropwise at -78 °C. The mixture is stirred for 10 min at this temperature and 30 min at 0 °C. 5-methoxy-1,2,3,4-tetrahydronaphthalen-2-one (5.00 g; 27.52 mmol; 1.00 eq.), dissolved in THF (30 ml) is added to the LDA solution at - 78 °C and stirred for 2h. Then N-phenyl-bis-(trifluoromethanesulfonimide) (11.80 g; 33.03 mmol; 1.20 eq.) is added and stirred overnight while the cooling bath warmed to RT. The solvent is removed, and the crude product is cleaned by column chromatography (2% EtOAc/heptane). The synthesis yield 8.52 g of trifluoromethanesulfonic acid 5-methoxy-3,4-dihydro-naphthalen-2-yl ester (27.6 mmol, quant.).

**¹H NMR** (500 MHz, Chloroform-d) δ 7.16 (t, J = 7.9 Hz, 1H), 6.81 (dd, J = 8.3, 1.0 Hz, 1H), 6.71 (dd, J = 7.6, 0.9 Hz, 1H), 6.44 (t, J = 1.3 Hz, 1H), 3.83 (s, 3H), 3.05 (t, J = 8.6 Hz, 2H), 2.66 (td, J = 8.7, 1.4 Hz, 2H).

Analogously, other derivatives are prepared in the same manner:

| **No.** | **Reactant** | **Product** | **Yield [%]** |
|---|---|---|---|
| **1a** | 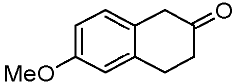 CAS: 2472-22-2 | 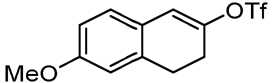 | 95 |
| **1b** | 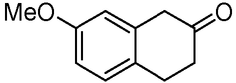 CAS: 4133-34-0 | 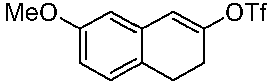 | 92 |
| **1c** | 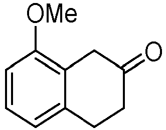 CAS: 5309-19-3 | 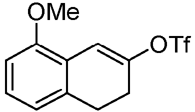 | 98 |

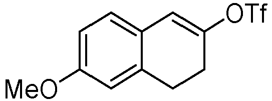

**¹H NMR** (500 MHz, Chloroform-d) δ 7.00 (d, *J* = 7.9 Hz, 1H), 6.71 (d, *J =* 8.0 Hz, 2H), 6.43 (d, *J* = 1.4 Hz, 1H), 3.80 (s, 3H), 3.03 (t, *J* = 8.4 Hz, 2H), 2.67 (td, J = 8.4, 1.4 Hz, 2H).

### Example 2.1:

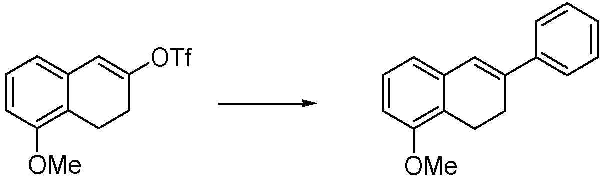

To a solution of trifluoromethanesulfonic acid 5-methoxy-3,4-dihydro-naphthalen-2-yl ester (10.0 g; 32.4 mmol, 1.00 eq.) and Iron(III) acetylacetonate (572 mg, 1.62 mmol, 0.05 eq.) in 1-methyl-2-pyrrolidinone (NMP) (29.7 mL) and tetrahydrofuran (650 ml) phenylmagnesium bromide is added fastly at -30 °C. The mixture is stirred for 45 min and quenched with sat. NH₄Cl-solution. After dilution with water, the aqueous layer is extracted four times with diethylether. The organic layers are dried over Na₂SO₄ and the solvent is evaporated. The crude product is cleaned by column chromatography (0-5% EtOAc/heptane). The synthesis yield 5.00 g of 8-methoxy-3-phenyl-1,2-dihydronaphthalene (21.2 mmol, 65% of theory). **¹H NMR** (500 MHz, Chloroform-d) δ 7.60 - 7.56 (m, 2H), 7.40 (t, *J* = 7.7 Hz, 2H), 7.33 - 7.28 (m, 1H), 7.18 (t, *J = 7.9* Hz, 1H), 6.86 (d, *J* = 1.5 Hz, 1H), 6.84 - 6.79 (m, 2H), 3.89 (s, 3H), 2.99 (dd, *J* = 9.2, 7.4 Hz, 2H), 2.77 (td, J = 8.4, 1.3 Hz, 2H).

Analogously, other derivatives are prepared with phenylmagnesium bromide in the same manner:

| No. | Reactant | Product | Yield **[%]** |
|---|---|---|---|
| 2a | 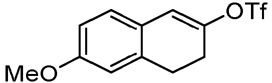 | 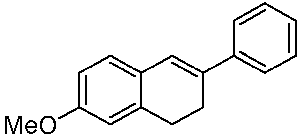 CAS: 22117-57-3 | 80 |
| 2b | 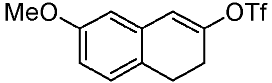 | 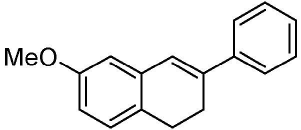 | 61 |
| 2c | 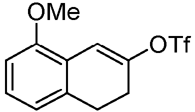 | 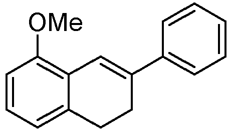 CAS: 109037-04-9 | 65 |

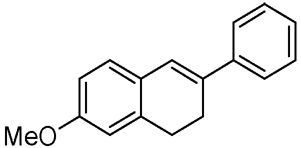

**¹H NMR** (500 MHz, Chloroform-d) δ 7.48 - 7.43 (m, 2H), 7.29 (dd, J = 8.4, 7.1 Hz, 2H), 7.18 (tt, J = 6.8, 1.2 Hz, 1H), 7.00 (d, *J* = 8.0 Hz, 1H), 6.69 - 6.64 (m, 2H), 3.75 (s, 3H), 2.86 (dd, J = 9.1, 7.0 Hz, 2H), 2.69 - 2.63 (m, 2H).

### Example 2.2:

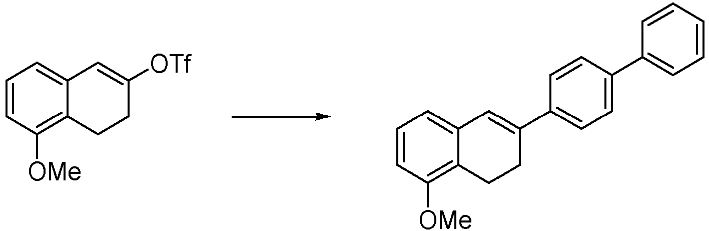

4-Biphenylboronic acid (1.00 g, 5.05 mmol, 1.00 eq.), Pd(dppf)Cl₂ (0.092 g, 0.126 mmol, 0.025 eq.) and 4-methoxy-6,7-dihydro-5H-benzo[7]annulen-8-yl trifluoromethane-sulfonate (1.56 g, 1.00 eq.) are dissolved in 1,4-dioxane (80 mL). Then 3 M NaOH-solution (20 mL) is added the mixture is stirred under reflux for 2 h. The reaction is quenched with sat. NH₄Cl-solution and diluted with EtOAc. The phases are separated, and the aqueous phase is extracted with EtOAc. The organic layers are dried over MgSO₄ and the crude product is cleaned by column chromatography (5-10% EtOAc/cyclohexane). The synthesis yield 1.45 g 4-(5-methoxy-3,4-dihydronaphthalen-2-yl)-1,1'-biphenyl (88% yield of theory).

**¹H NMR** (500 MHz, Chloroform-d) δ 7.68 - 7.58 (m, 6H), 7.45 (dd, J = 8.5, 7.0 Hz, 2H), 7.39 - 7.32 (m, 1H), 7.17 (t, *J* = 7.9 Hz, 1H), 6.90 (d, *J = 1.5* Hz, 1H), 6.86 - 6.72 (m, 2H), 3.87 (s, 3H), 2.98 (dd, J = 9.1, 7.5 Hz, 2H), 2.78 (td, J = 8.4, 1.3 Hz, 2H).

### Example 3:

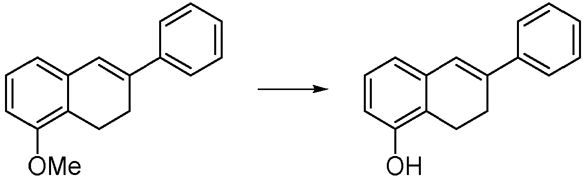

A solution of boron tribromide (1.79 mL, 18.6 mmol, 1.10 eq.) in DCM (75 mL) is added to 8-methoxy-3-phenyl-1,2-dihydronaphthalene (4.00 g, 16.9 mmol, 1.00 eq.) in DCM (70 mL) at 0 °C. The cooling bath is removed and stirred for 5 h. The reaction is quenched with sat. NaHCO₃-Solution and extracted with DCM three times. The organic layers are dried over MgSO₄ and the solvent is removed. The synthesis yield 3.80 g 6-phenyl-7,8-dihydronaphthalen-1-ol (17.10 mmol, quant.).

**¹H NMR** (500 MHz, Chloroform-d) δ 7.58 - 7.54 (m, 2H), 7.39 (dd, J = 8.5, 7.0 Hz, 2H), 7.32 - 7.28 (m, 1H), 7.07 (t, *J* = 7.8 Hz, 1H), 6.84 (s, 1H), 6.79 (dd, J = 7.6, 1.0 Hz, 1H), 6.68 (dd, J = 8.1, 1.0 Hz, 1H), 4.65 (s, 1H), 2.94 (dd, J = 9.0, 7.3 Hz, 2H), 2.85 - 2.72 (m, 2H).

Analogously, other derivatives are prepared in the same manner:

| **No.** | **Reactant** | **Product** | Yield **[%]** |
|---|---|---|---|
| **3a** | 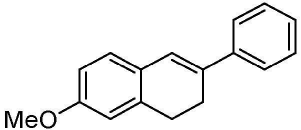 | 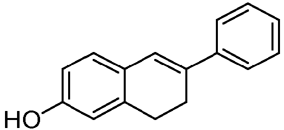 | 86 |
| **3b** | 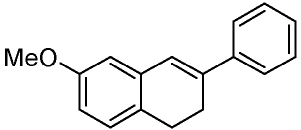 | 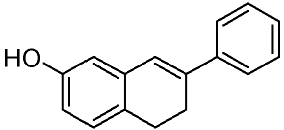 | 85 |
| **3c** | 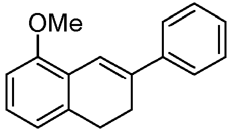 | 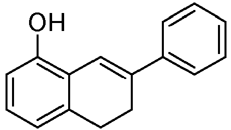 | 96 |
| 3d | 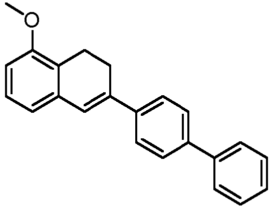 | 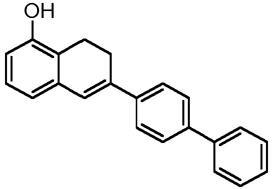 | 70 |

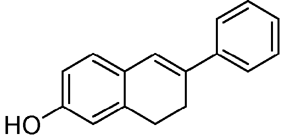

**¹H NMR** (500 MHz, Chloroform-d) δ 7.52 (dd, *J* = 8.3, 1.3 Hz, 2H), 7.36 (t, *J* = 8.4, 7.0 Hz, 2H), 7.27-7.24 (m, 1H), 7.02 (d, *J* = 7.9 Hz, 1H), 6.80 (s, 1H), 6.67 - 6.63 (m, 2H), 4.66 (s, 1H), 2.90 (dd, J = 9.1, 7.1 Hz, 2H), 2.73 (td, J = 8.2, 1.2 Hz, 2H).

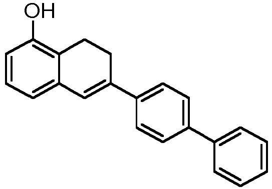

**¹H NMR** (500 MHz, Chloroform-d) δ 7.67 - 7.60 (m, 6H), 7.46 (dd, J = 8.3, 7.1 Hz, 2H), 7.40 - 7.32 (m, 1H), 7.08 (t, *J* = 7.8 Hz, 1), 6.90 (d, *J* = 1.4 Hz, 1H), 6.80 (d, *J* = 7.5 Hz, 1H), 6.69 (dd, *J* = 8.1, 1.0 Hz, 1H), 2.96 (dd, *J* = 9.1, 7.3 Hz, 2H), 2.86 - 2.76 (m, 2H).

### Example 4:

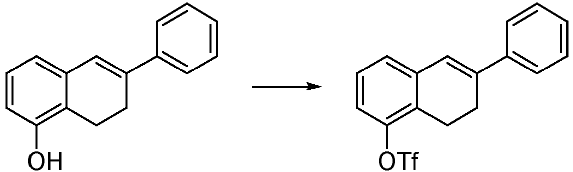

6-Phenyl-7,8-dihydronaphthalen-1-ol is dissolved in DCM (30 mL) and triethylamine (7.18 mL, 51.3 mmol, 3.00 eq.) is added followed by the addition of trifluoromethanesulfonic anhydride (3.67 mL, 22.2 mmol, 1.30 eq., dissolved in 5.50ml DCM) at 0 °C. The solution is stirred overnight while warming to room temperature. The reaction is washed with water two times and the aqueous layers are extracted two times with DCM. The organic layers are dried over MgSO₄ and the solvent is evaporated. The crude product is cleaned by column chromatography (0-10% EtOAc/heptane). The synthesis yield 4.30 g 6-phenyl-7,8-dihydronaphthalen-1-yl trifluoromethanesulfonate (12.1 mmol, 71% of theory).

**¹H NMR** (500 MHz, Chloroform-d) δ 7.55 (dd, J = 8.4, 1.3 Hz, 2H), 7.41 (t, *J* = 7.6 Hz, 2H), 7.36 - 7.31 (m, 1H), 7.27 - 7.23 (m, 1H), 7.16 (d, *J =* 7.5 Hz, 1H), 7.10 (d, *J* = 8.2 Hz, 1H), 6.87 (s, 1H), 3.09 - 3.02 (m, 2H), 2.80 (ddd, J = 9.3, 7.2, 1.4 Hz, 2H).

Analogously, other derivatives are prepared in the same manner:

| **No.** | **Reactant** | **Product** | **Yield [%]** |
|---|---|---|---|
| **4a** | 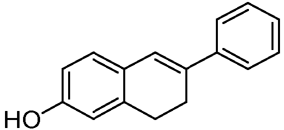 | 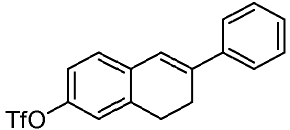 | 88 |
| **4b** | 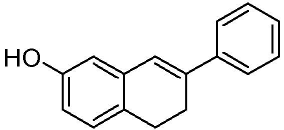 | 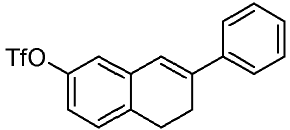 | 77 |
| **4c** | 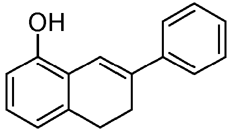 | 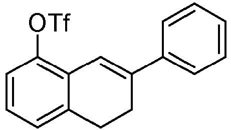 | 78 |

### Example 5a:

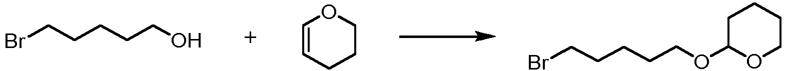

5-Bromopentan-1-ol (5.47 g; 29.47 mmol; 1.00 eq.) is dissolved in dichloromethane (75.28 ml; 1178.85 mmol; 40.00 eq.) and *para*toluenesulfonic-monohydrate (7.40 mg; 0.04 mmol; 0.001 eq.) is added at 0 °C. 3,4-Dihydro-2H-pyran, 97% (3.33 ml; 35.37 mmol; 1.20 eq.) is added over a period of 1h and warmed overnight to r.t.. The organic phase is washed with sat. NaHCO₃-Lsg. and the phases are separated. The organic phase is dried over MgSO₄ and the solvent is removed. The crude product is purified via column chromatography (0-5% EtOAC/heptane) and yield 6.4g 2-((5-bromopentyl)oxy)tetrahydro-2H-pyran (25.48mmol; 87% of theory).

**¹H NMR** (500 MHz, Chloroform-d) δ 4.57 (dd, J = 4.5, 2.8 Hz, 1H), 3.86 (ddd, J = 11.1, 7.6, 3.3 Hz, 1H), 3.75 (dt, J = 9.7, 6.6 Hz, 1H), 3.54 - 3.47 (m, 1H), 3.45 - 3.36 (m, 3H), 1.90 (dt, J = 14.3, 7.0 Hz, 2H), 1.83 (qd, J = 7.9, 3.5 Hz, 1H), 1.75 - 1.68 (m, 1H), 1.66 - 1.60 (m, 2H), 1.54 (dddd, J = 19.2, 14.5, 7.6, 3.2 Hz, 6H).

The other THP-ether derivatives 2-(3-bromopropoxy)tetrahydro-2H-pyran and 2-((6-bromohexyl)oxy)tetrahydro-2H-pyran are prepared in the same manner using 3-bromopropan-1-ol or 6-bromohexan-1-ol using the above-mentioned reaction procedure.

### Example 5b:

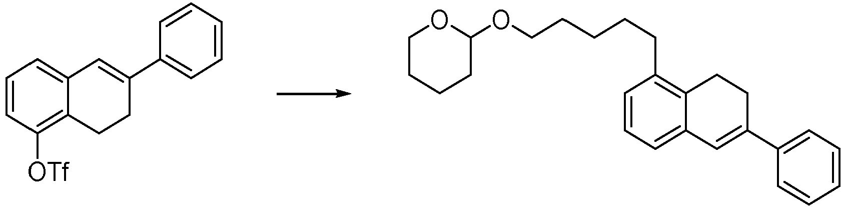

To a solution of 9-[5-(oxan-2-yloxy)pentyl]-9-borabicyclo[3.3.1 ]nonane (0.3M in THF, 33.9 mL, 8.47 mmol, 1.50 eq.) and Pd(dppf)Cl₂ (207 mg, 0.28 mmol, 0.05 eq.) in THF (80 mL) is added 6-phenyl-7,8-dihydronaphthalen-1-yl trifluoromethanesulfonate (2.00 g, 5.64 mmol, 1.00 eq.) and 3 M NaOH-solution (17.0 mL, 50.8 mmol, 9.00 eq.) and stirred for 6 hat reflux. The reaction is quenched with sat. NH₄Cl-solution and the phases are separated. The aqueous layer is extracted with 2-Methyl-THF two times. The organic layers are dried over MgSO₄ and the solvent is removed. The crude product is cleaned by column chromatography (0-10% EtOAc/heptane). The synthesis yield 1.08 g 2-{[5-(6-phenyl-7,8-dihydronaphthalen-1-yl)pentyl]oxy}oxane (2.87 mmol, 51% of theory).

**¹H NMR** (500 MHz, Chloroform-d) δ 7.57 - 7.53 (m, 2H), 7.38 (t, *J* = 7.7 Hz, 2H), 7.30 - 7.27 (m, 1H), 7.11 (t, *J = 7.5* Hz, 1H), 7.01 (ddd, J = 11.5, 7.5, 1.3 Hz, 2H), 6.85 (s, 1H), 4.58 (dd, J = 4.5, 2.8 Hz, 1H), 3.86 (ddd, J = 11.0, 7.6, 3.2 Hz, 1H), 3.76 (dt, J = 9.7, 6.9 Hz, 1H), 3.53 - 3.47 (m, 1H), 3.40 (dt, *J* = 9.5, 6.6 Hz, 1H), 2.93 (dd, J = 9.1, 7.0 Hz, 2H), 2.74 (t, *J =* 7.7 Hz, 2H), 2.70 - 2.63 (m, 2H), 1.83 (ddt, J = 11.5, 8.4, 4.6 Hz, 1H), 1.76 - 1.44 (m, 11H).

Analogously, other derivatives are prepared in the same manner: R1 means reactant, R2 means reactant 2, [P] means product.

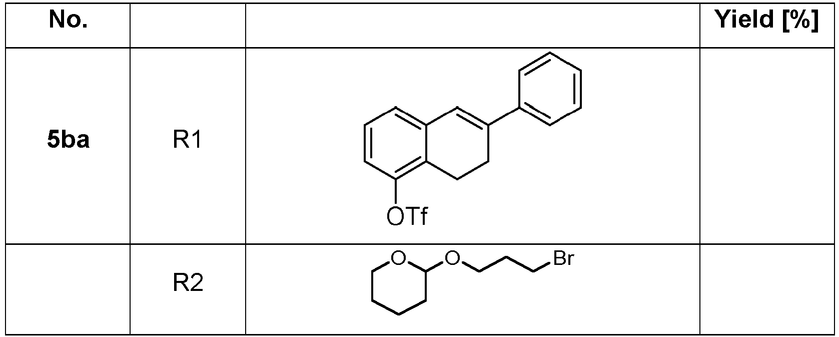

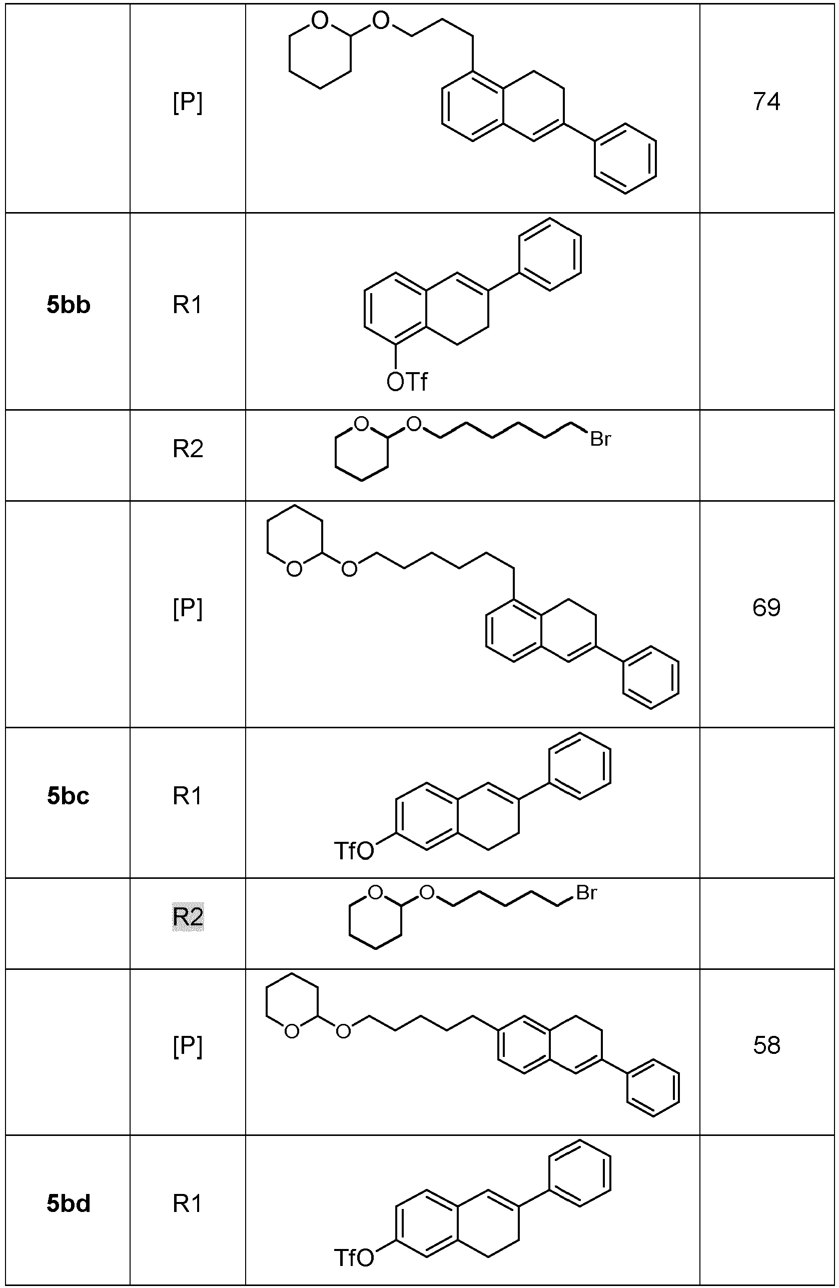

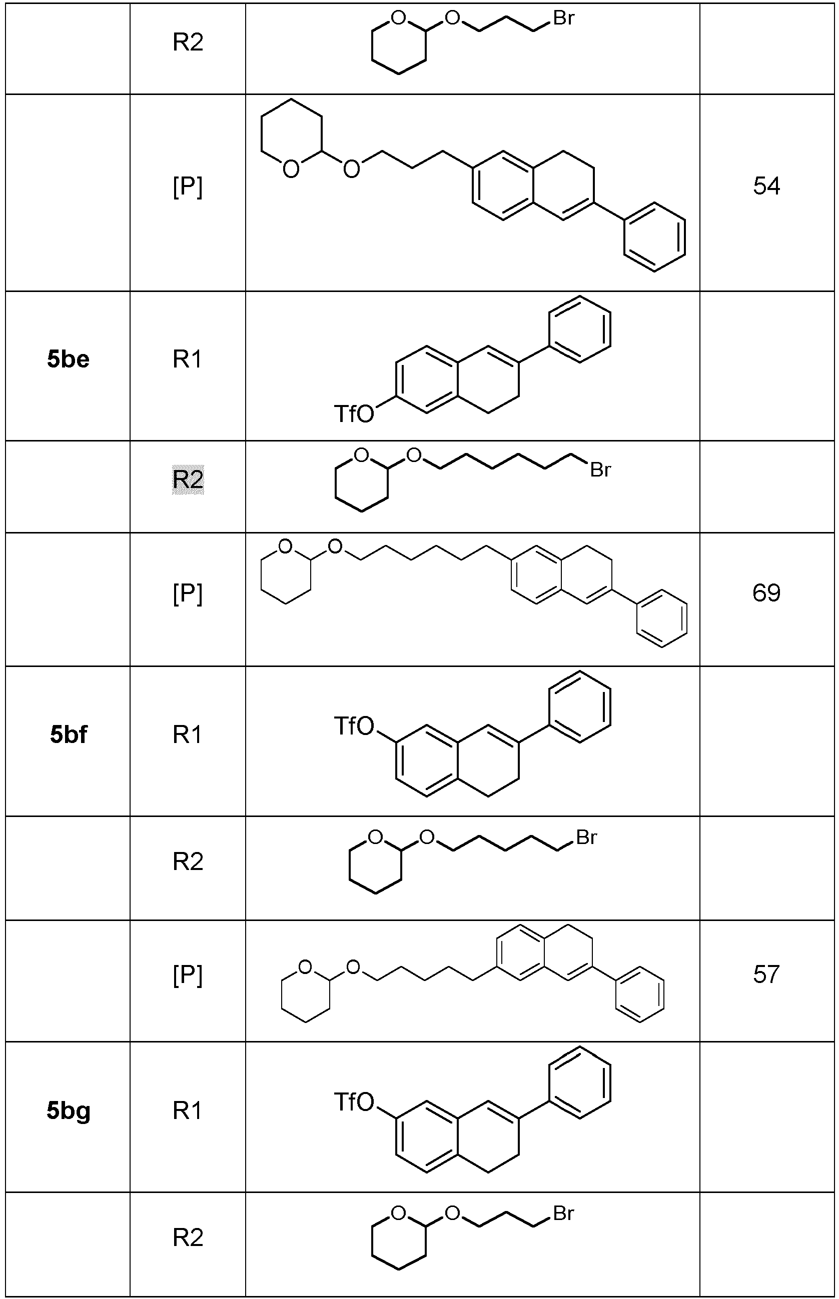

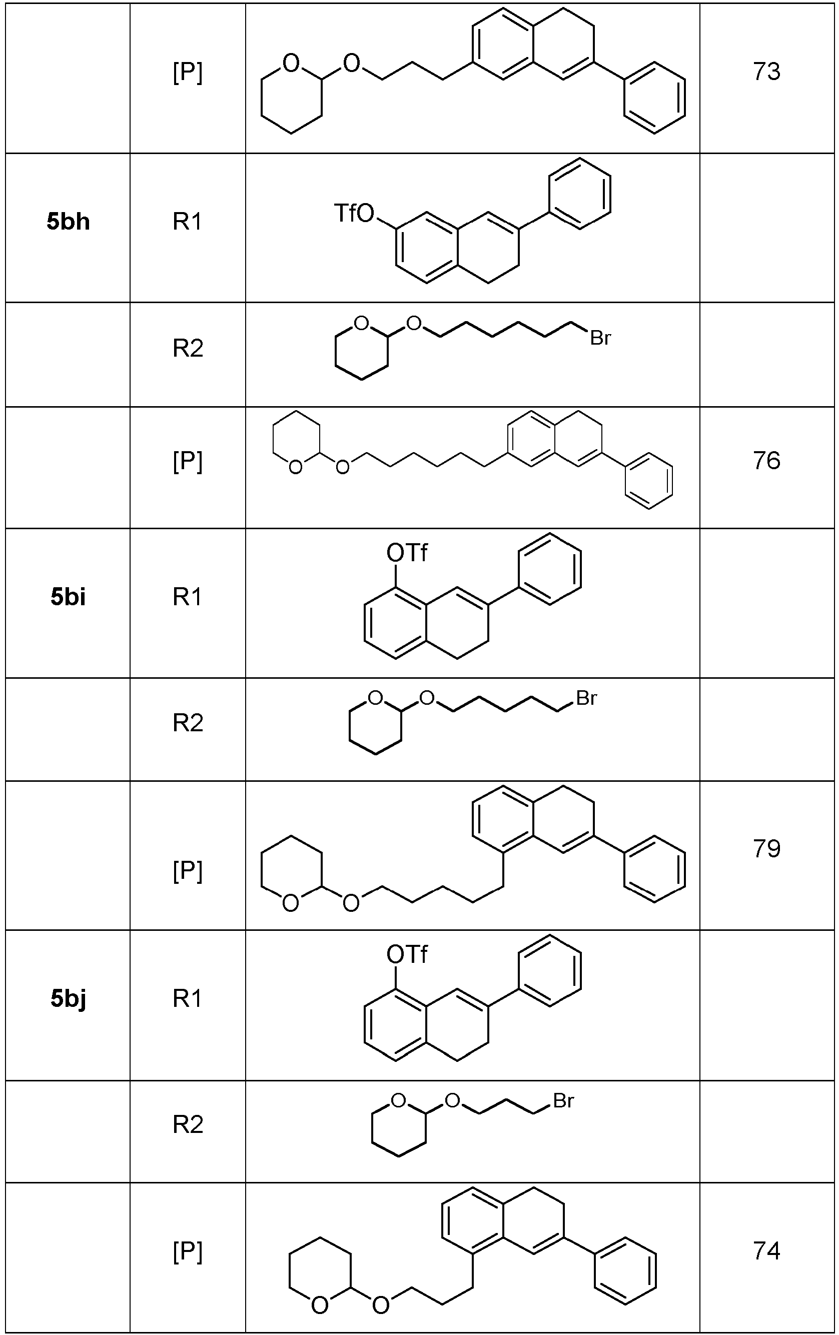

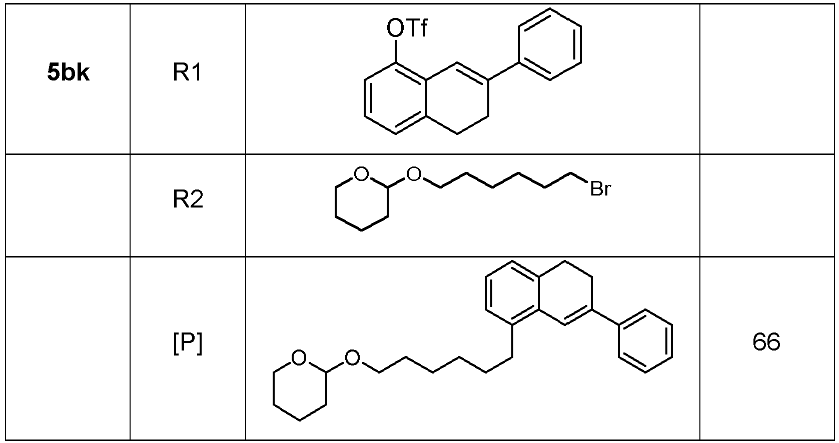

### Example 6:

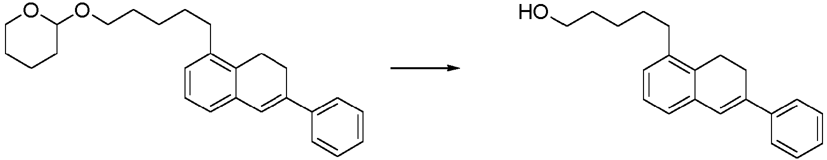

2-{[5-(6-phenyl-7,8-dihydronaphthalen-1-yl)pentyl]oxy}oxane (980 mg, 2.60 mmol, 1.00 eq.) is dissolved in DCM (15 mL) and MeOH (15 mL). Then conc. HCl (0.325 mL, 3.90 mmol, 1.50 eq.) is added and stirred for 45 min. The reaction is quenched with sat. NaHCO₃-Solution and diluted with DCM. The phases are separated, and the aqueous phase is extracted with DCM three times. The organic layers are dried over MgSO₄ and the solvent is removed. The crude product is cleaned by column chromatography (20-40% EtOAc/heptane). The synthesis yield 730 mg 5-(6-phenyl-7,8-dihydronaphthalen-1-yl)pentan-1-ol (2.50 mmol, 96% of theory).

**¹H NMR** (500 MHz, Chloroform-d) δ 7.58 - 7.52 (m, 2H), 7.38 (dd, J = 8.4, 7.0 Hz, 2H), 7.31 = 7.26 (m, 1H), 7.12 (t, *J* = 7.5 Hz, 1H), 7.05 - 7.00 (m, 2H), 6.86 (d, *J* = 1.4 Hz, 1H), 3.67 (t, *J* = 6.6 Hz, 2H), 2.94 (dd, J = 9.1, 7.0 Hz, 2H), 2.78 - 2.72 (m, 2H), 2.69 - 2.62 (m, 2H), 1.70 - 1.56 (m, 4H), 1.51 - 1.42 (m, 2H), 1.26 (s, 1H).

Analogously, other derivatives are prepared in the same manner:

| **No.** | **Reactant** | **Product** | **Yield [%]** |
|---|---|---|---|
| **6a** | 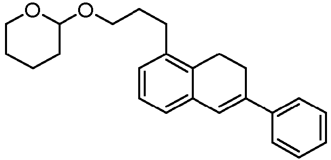 | 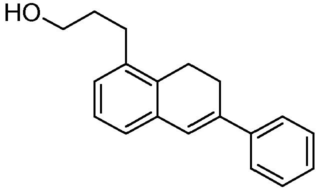 | 92 |
| **6b** | 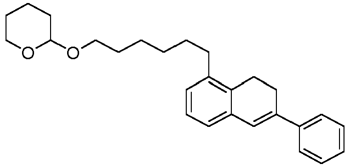 | 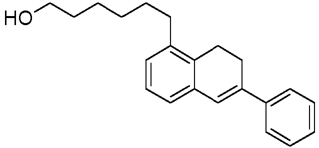 | 94 |
| **6c** | 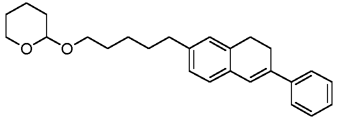 | 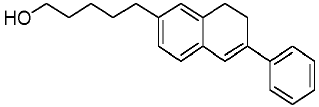 | 99 |
| **6d** | 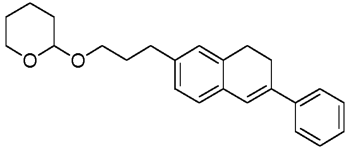 | 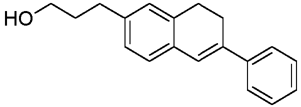 | 96 |
| **6e** | 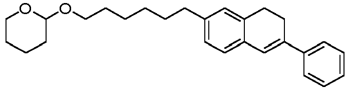 | 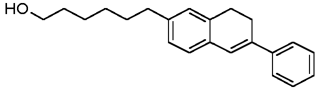 | 95 |
| **6f** | 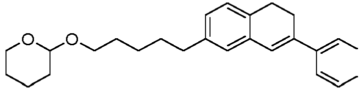 | 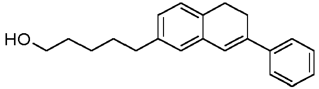 | 91 |
| **6g** | 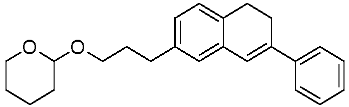 | 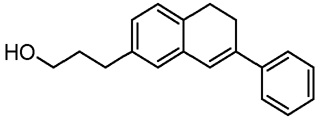 | 93 |
| **6h** | 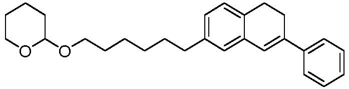 | 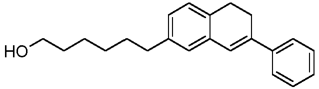 | 90 |
| **6i** | 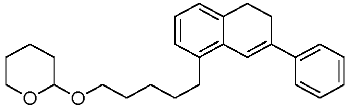 | 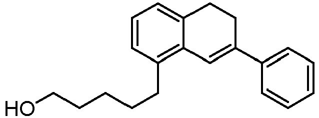 | 94 |
| **6j** | 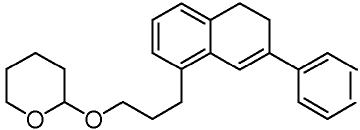 | 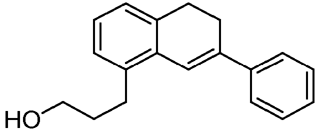 | 97 |
| **6k** | 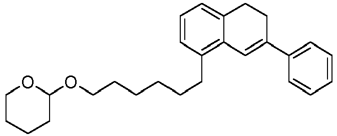 | 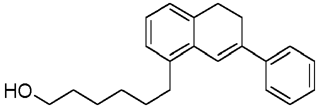 | 98 |

### Preparation of compounds according to the invention:

### Example 7:

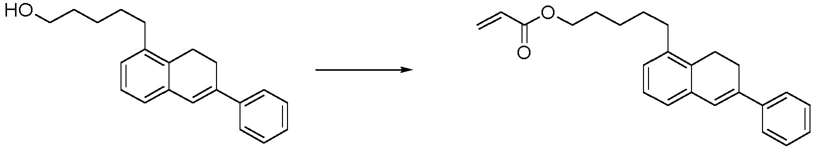

5-(6-phenyl-7,8-dihydronaphthalen-1-yl)pentan-1-ol (640 mg, 2.19 mmol, 1.00 eq.), 4-(Dimethylamino)-pyridine (13.4 mg, 0.11 mmol, 0.05 eq.) are dissolved in THF (25 mL) and triethylamine (1.21 mL, 8.75 mmol, 4.00 eq.) is added. Acryloyl chloride (0.240 mL, 2.85 mmol, 1.30 eq.) is added at 0 °C and stirred at room temperature for 5 h. The reaction is quenched with 2-propanol (0.25 mL). The suspension is filtered, and the solvent of the filtrate is removed. The crude product is cleaned by column chromatography (20-40% EtOAc/heptane). The synthesis yield 644 mg 5-(6-phenyl-7,8-dihydronaphthalen-1-yl)pentyl prop-2-enoate (1.86 mmol, 85% of theory).

**¹H NMR** (500 MHz, Chloroform-*d*) δ 7.58 - 7.53 (m, 2H), 7.38 (t, *J* = 7.7 Hz, 2H), 7.28 (t, *J* = 7.4 Hz, 1H), 7.12 (t, *J* = 7.5 Hz, 1H), 7.02 (ddd, *J* = 6.8, 5.4, 1.3 Hz, 2H), 6.86 (s, 1H), 6.40 (dd, *J* = 17.3, 1.5 Hz, 1H), 6.13 (dd, *J* = 17.3, 10.4 Hz, 1H), 5.82 (dd, *J* = 10.4, 1.5 Hz, 1H), 4.18 (t, *J* = 6.7 Hz, 2H), 2.93 (dd, *J* = 9.1, 7.0 Hz, 2H), 2.79 - 2.72 (m, 2H), 2.71 - 2.63 (m, 2H), 1.77 - 1.70 (m, 2H), 1.67 - 1.59 (m, 2H), 1.52 - 1.44 (m, 2H).

Analogously, other derivatives are prepared in the same manner with acryloyl chloride:

| **No.** | **Reactant** | **Product** | **Yield [%]** |
|---|---|---|---|
| **7a** | 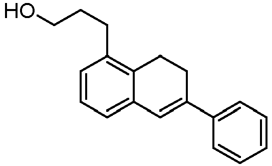 | 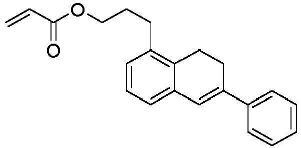 | 94 |
| **7b** | 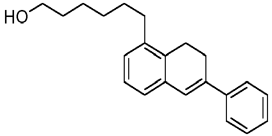 | 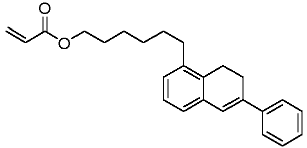 | 95 |
| **7c** | 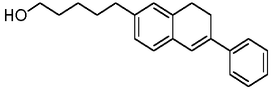 | 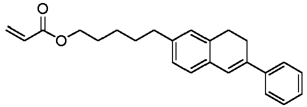 | 92 |
| **7d** | 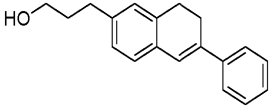 | 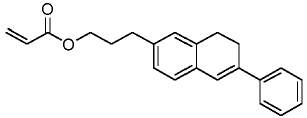 | 91 |
| **7e** | 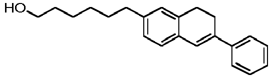 | 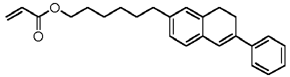 | 88 |
| **7f** | 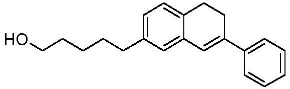 | 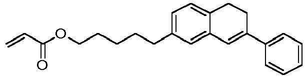 | 90 |
| **7g** | 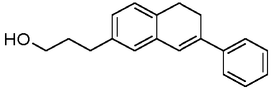 | 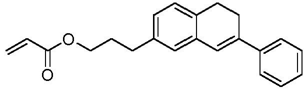 | 89 |
| **7h** | 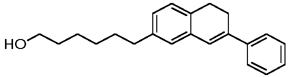 | 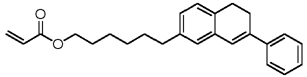 | 81 |
| **7i** | 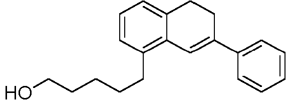 | 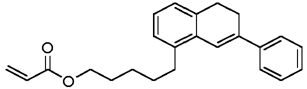 | 87 |
| **7j** | 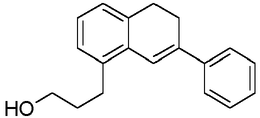 | 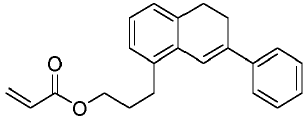 | 89 |
| **7k** | 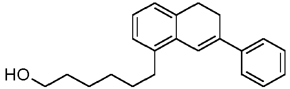 | 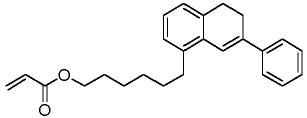 | 90 |

### Example 8:

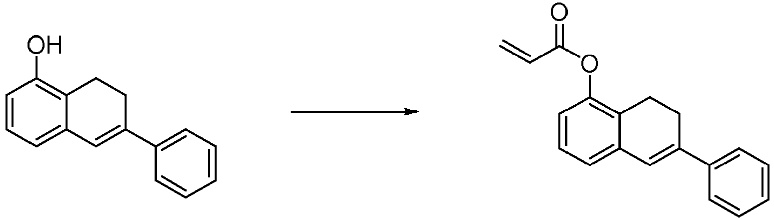

6-phenyl-7,8-dihydronaphthalen-1-ol (775 mg, 3.49 mmol, 1.00 eq.) is dissolved in THF (40 mL) and triethylamine (1.95 mL, 14.0 mmol, 4.00 eq.) and acryloyl chloride (0.341 mL, 4.01 mmol, 1.15 eq.) is added at 0 °C. After stirring at r.t. for 3 h the suspension is filtered. The solvent of the filtrate is evaporated, and the crude product is cleaned by column chromatography (10-30% 2-Methyl-THF/heptane). The synthesis yield 600 mg 6-phenyl-7,8-dihydronaphthalen-1-yl prop-2-enoate (2.17 mmol, 62% of theory).

**¹H NMR** (500 MHz, Chloroform-*d*) δ 7.55 - 7.52 (m, 2H), 7.38 (t, *J* = 7.7 Hz, 2H), 7.32 - 7.28 (m, 1H), 7.21 (t, *J* = 7.8 Hz, 1H), 7.06 (dd, *J* = 7.5, 1.1 Hz, 1H), 6.94 (dd, *J* = 8.1, 1.1 Hz, 1H), 6.87 (d, *J* = 1.5 Hz, 1H), 6.65 (dd, *J =* 17.3, 1.2 Hz, 1H), 6.38 (dd, *J* = 17.3, 10.5 Hz, 1H), 6.05 (dd, *J* = 10.5, 1.2 Hz, 1H), 2.82 (dd, *J* = 8.7, 6.4 Hz, 2H), 2.73 (ddt, *J* = 8.8, 7.5, 1.9 Hz, 2H).

Analogously, other derivatives are prepared in the same manner with acryloyl chloride:

| **No.** | **Reactant** | **Product** | **Yield [%]** |
|---|---|---|---|
| **8a** | 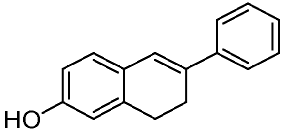 | 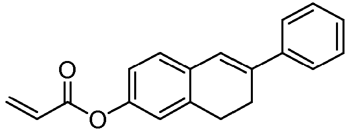 | 71 |
| **8b** | 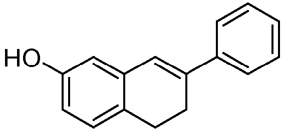 | 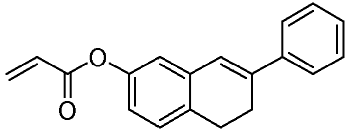 | 84 |
| **8c** | 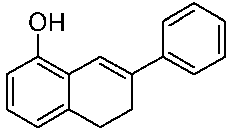 | 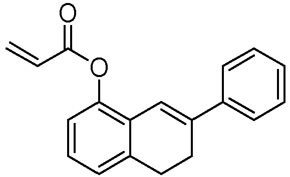 | 77 |

### Preparation of precursor materials:

### Example 9.

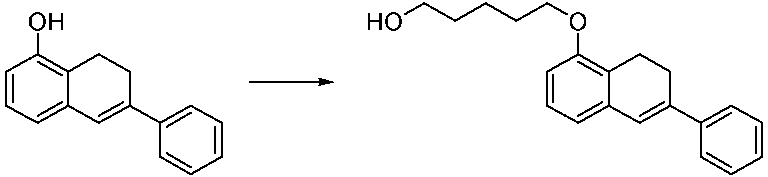

6-phenyl-7,8-dihydronaphthalen-1-ol (866 mg, 3.90 mmol, 1.00 eq.) is refluxed with potassium carbonate (2.18 g, 15.6 mmol, 4.00 eq.) and 2-[(5-bromopentyl)oxy]oxane (1.03 g, 4.09 mmol, 1.05 mmol) in acetone (15 mL) for 2 d. The suspension is filtered, and the solvent of the filtrate is evaporated. The residue is dissolved in DCM (25 mL) and MeOH (25 mL) and conc. HCl (0.49 mL) is added. After stirring for 1 h the reaction is quenched with sat. NaHCO₃-solution and the phases are separated. The aqueous phase is extracted two times with DCM. The organic layers are dried over MgSO₄. The crude product is cleaned by column chromatography (12-30% 2-MTHF/Cyclohexane). The synthesis yield 1.06 g 5-[(6-phenyl-7,8-dihydronaphthalen-1-yl)oxy]pentan-1-ol (3.44 mmol, 88%).

**¹H NMR** (500 MHz, Chloroform-*d*) δ 7.57 - 7.53 (m, 2H), 7.38 (t, *J* = 7.7 Hz, 2H), 7.28 (t, *J* = 7.4 Hz, 1H), 7.13 (t, *J* = 7.9 Hz, 1H), 6.83 (s, 1H), 6.77 (dd, *J* = 14.7, 7.8 Hz, 2H), 4.01 (t, *J* = 6.3 Hz, 2H), 3.70 (td, *J* = 6.3, 5.2 Hz, 2H), 3.00 - 2.93 (m, 2H), 2.74 (td, *J* = 8.3, 7.8, 1.4 Hz, 2H), 1.89 - 1.82 (m, 2H), 1.71 - 1.63 (m, 2H), 1.63 - 1.56 (m, 2H), 1.43 (s, 1H).

Analogously, other derivatives are prepared in the same manner: R1 means reactant 1, R2 means reactant 2 and [P] means product.

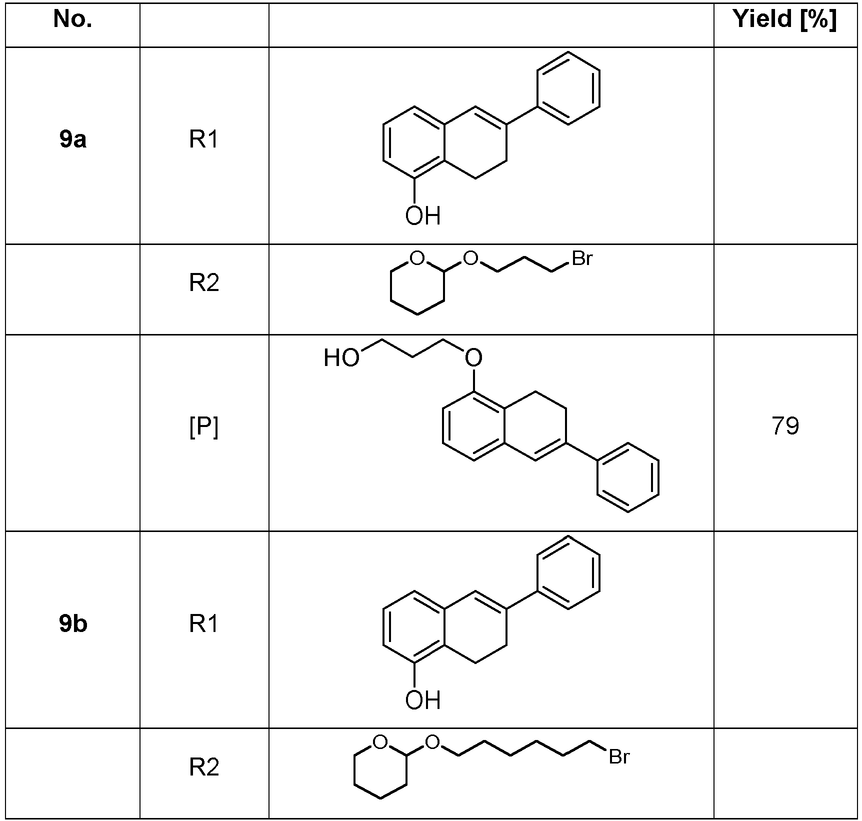

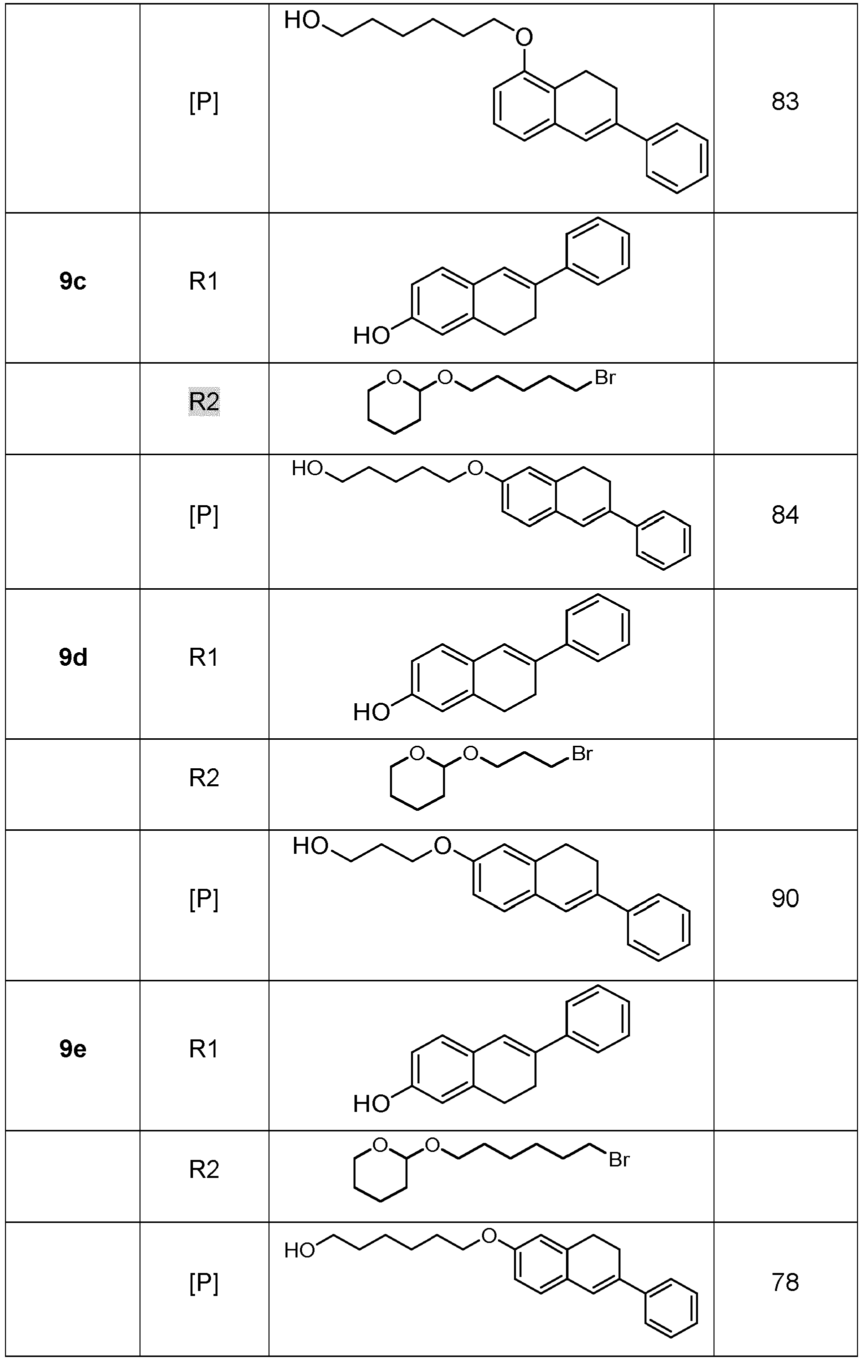

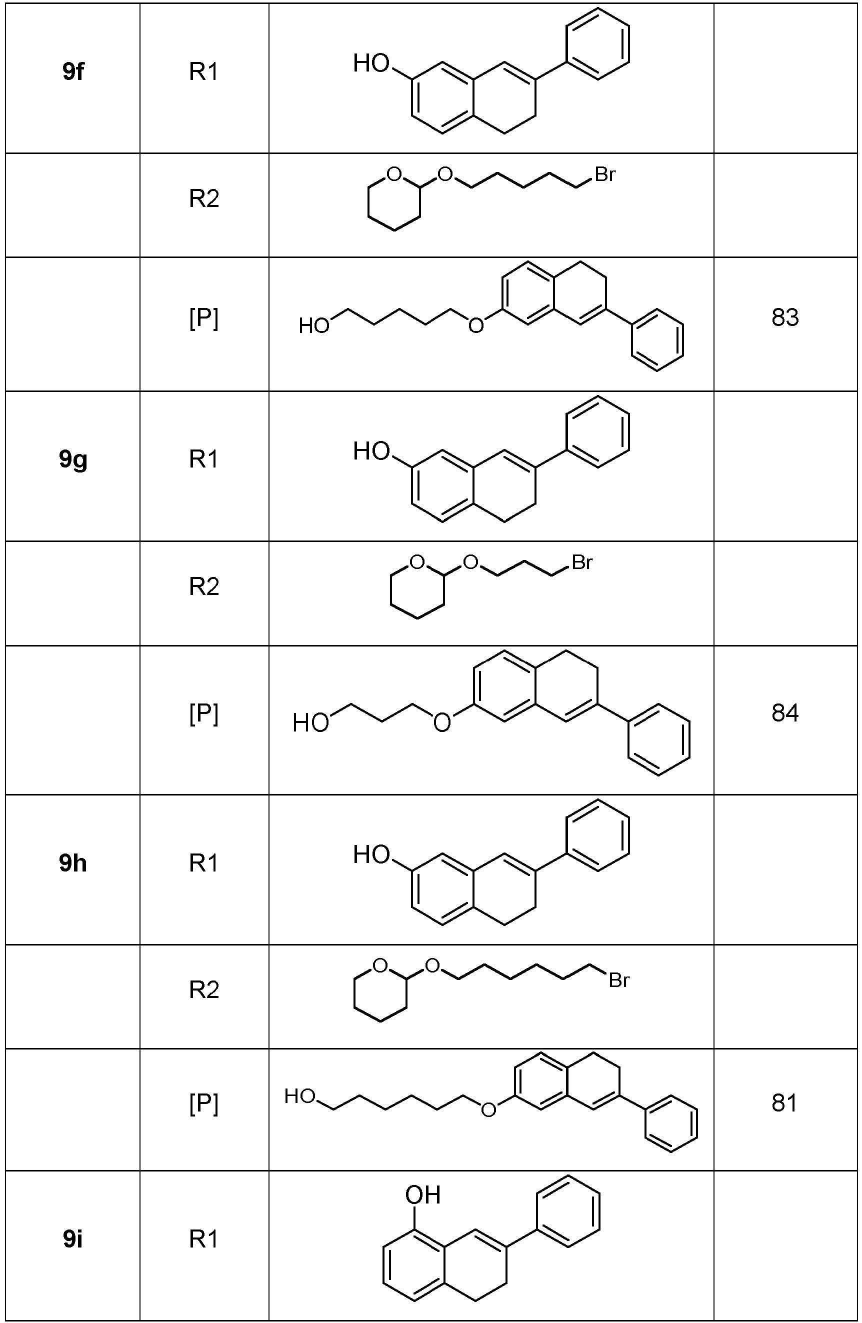

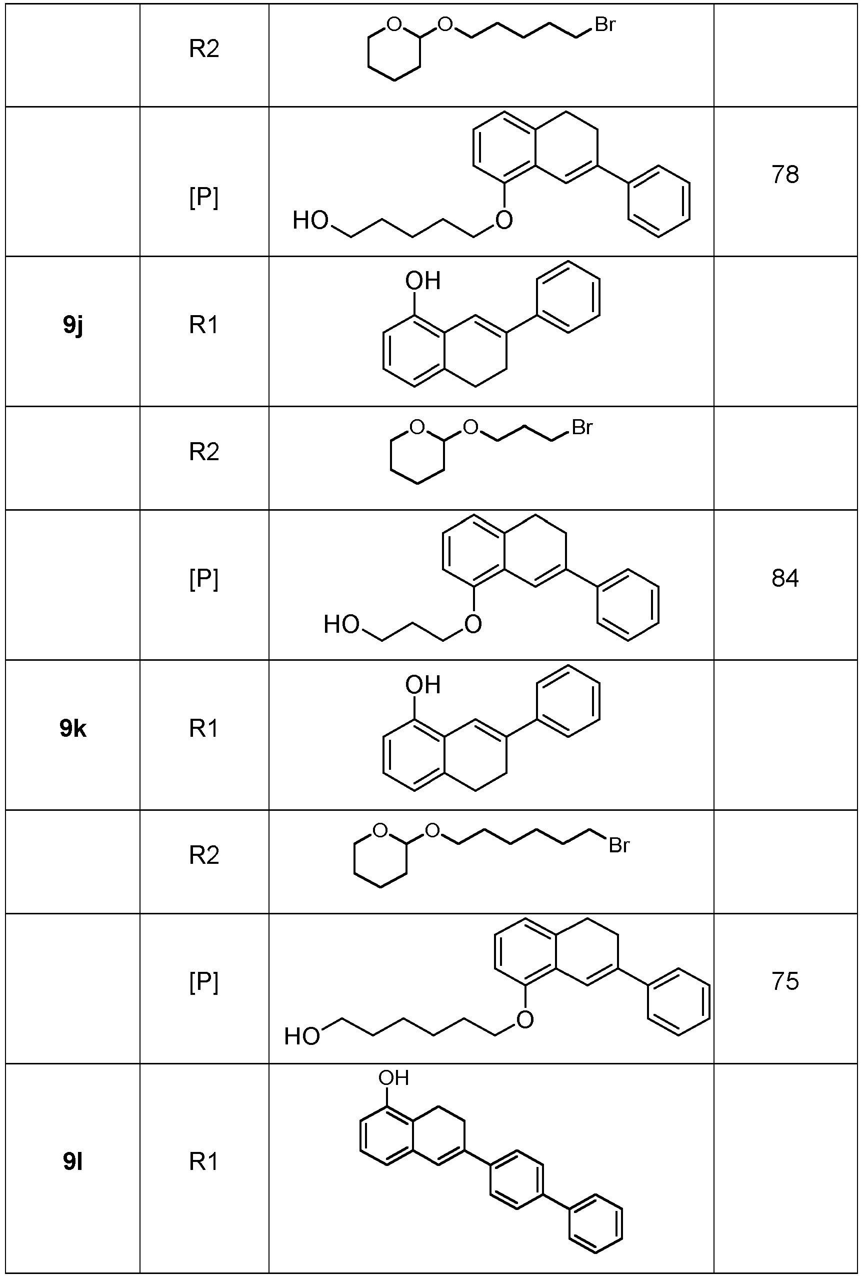

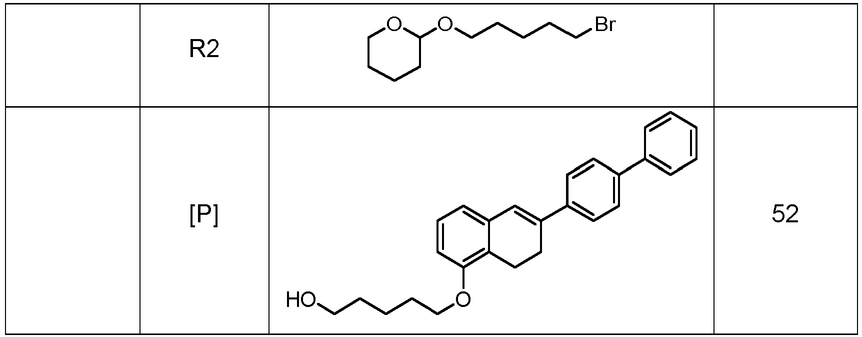

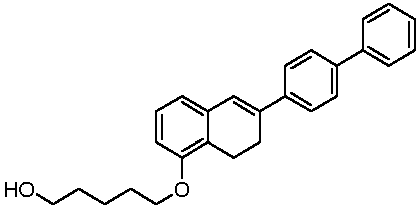

**¹H NMR** (500 MHz, Chloroform-*d*) δ 7.69 - 7.58 (m, 6H), 7.45 (dd, *J* = 8.4, 7.0 Hz, 2H), 7.39 - 7.31 (m, 1H), 7.14 (t, *J =* 7.9 Hz, 1H), 6.90 (d, *J* = 1.4 Hz, 1H), 6.81 (d, *J* = 7.5 Hz, 1H), 6.77 (dd, *J* = 8.2, 1.0 Hz, 1H), 4.02 (t, *J =* 6.3 Hz, 2H), 3.71 (q, *J* = 6.2 Hz, 2H), 2.99 (dd, *J* = 9.1, 7.5 Hz, 2H), 2.77 (td, *J* = 8.3, 1.3 Hz, 2H), 1.92 - 1.84 (m, 2H), 1.73 - 1.65 (m, 2H), 1.64 - 1.58 (m, 2H), 1.27 (q, *J* = 4.0, 2.8 Hz, 1H).

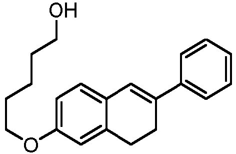

**¹H NMR** (500 MHz, Chloroform-*d*) δ 7.54 - 7.51 (m, 2H), 7.36 (t, *J* = 7.7 Hz, 2H), 7.27-7.24 (m, 1H), 7.05 (d, *J* = 8.2 Hz, 1H), 6.81 (s, 1H), 6.73- 6.70 (m, 2H), 3.99 (t, *J* = 6.4 Hz, 2H), 3.73 - 3.65 (m, 2H), 2.92 (dd, *J* = 9.1, 7.0 Hz, 2H), 2.74 - 2.71 (m, 2H), 1.83 (p, *J* = 6.7 Hz, 2H), 1.66 (dq, *J* = 8.7, 6.2 Hz, 2H), 1.60-1.53 (d, *J* = 5.7 Hz, 2H), 1.29 (t, *J* = 5.4 Hz, 1H).

### Example 10:

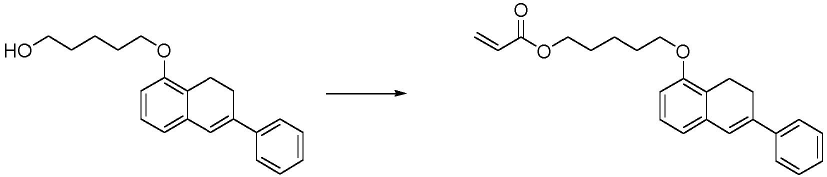

5-[(6-phenyl-7,8-dihydronaphthalen-1-yl)oxy]pentan-1-ol (1.06 g mg, 3.44 mmol, 1.00 eq.) is dissolved in THF (40 mL) and triethylamine (1.91 mL, 13.8 mmol, 4.00 eq.) and acryloyl chloride (0.371 mL, 4.45 mmol, 1.30 eq.) is added at 0 °C. After stirring at r.t. for 3 h the suspension is filtered. The solvent of the filtrate is evaporated, and the crude product is cleaned by column chromatography (1-10% 2-Methyl-THF/cyclohexane). The synthesis yield 996 mg 5-[(6-phenyl-7,8-dihydronaphthalen-1-yl)oxy]pentyl prop-2-enoate (2.75 mmol, 80% of theory).

**¹H NMR** (500 MHz, Chloroform-*d*) δ 7.57 - 7.52 (m, 2H), 7.38 (t, *J* = 7.7 Hz, 2H), 7.28 (t, *J* = 7.4 Hz, 1H), 7.15 - 7.10 (m, 1H), 6.82 (d, *J* = 1.5 Hz, 1H), 6.79 (d, *J* = 7.5 Hz, 1H), 6.75 (dd, *J* = 8.2, 1.0 Hz, 1H), 6.41 (dd, *J* = 17.4, 1.5 Hz, 1H), 6.13 (dd, *J* = 17.4, 10.4 Hz, 1H), 5.82 (dd, *J* = 10.4, 1.5 Hz, 1H), 4.21 (t, *J* = 6.6 Hz, 2H), 4.01 (t, *J* = 6.3 Hz, 2H), 2.99 - 2.91 (m, 2H), 2.76 - 2.69 (m, 2H), 1.91 - 1.83 (m, 2H), 1.82 - 1.74 (m, 2H), 1.65 - 1.57 (m, 2H).

Analogously, other derivatives are prepared in the same manner:

| **No.** | **Reactant** | **Product** | **Yield [%]** |
|---|---|---|---|
| **10a** | 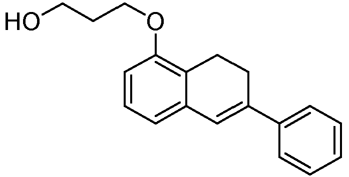 | 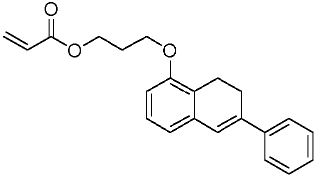 | 81 |
| **10b** | 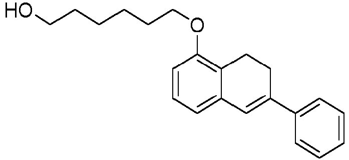 | 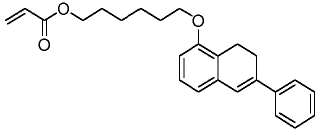 | 89 |
| **10c** | 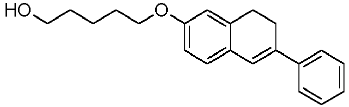 | 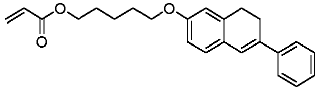 | 83 |
| **10d** | 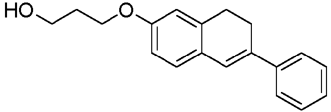 | 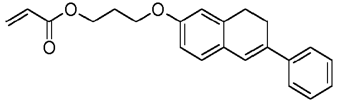 | 86 |
| **10e** | 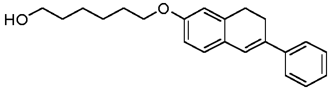 | 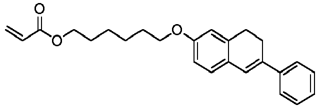 | 94 |
| **10f** | 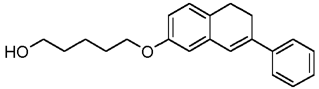 | 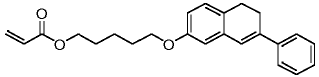 | 89 |
| **10g** | 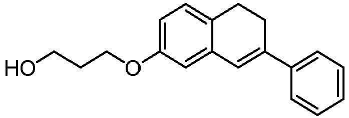 | 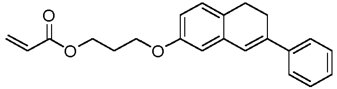 | 90 |
| **10h** | 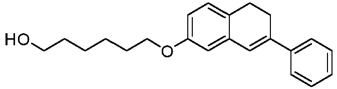 | 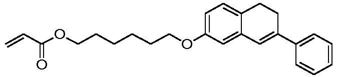 | 86 |
| **10i** | 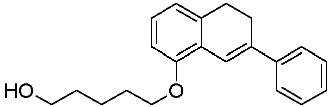 | 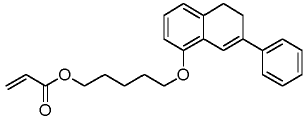 | 87 |
| **10j** | 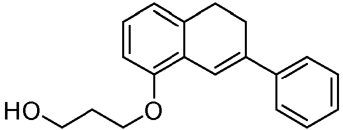 | 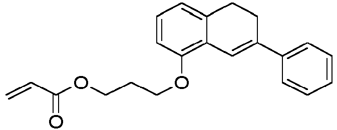 | 82 |
| **10k** | 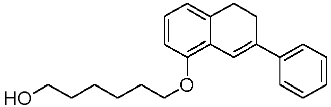 | 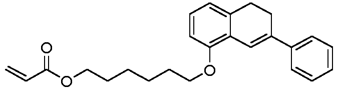 | 88 |
| **10I** | 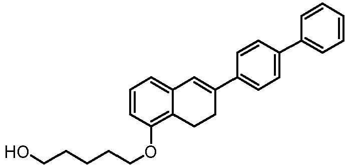 | 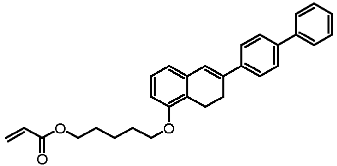 | 62 |
| **10m** | 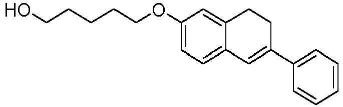 | 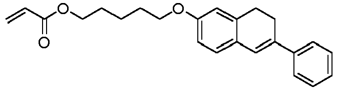 | 42 |

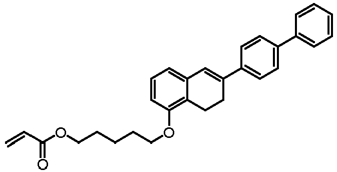

**¹H NMR** (500 MHz, Chloroform-*d*) δ 7.70 - 7.55 (m, 6H), 7.45 (t, *J* = 7.7 Hz, 2H), 7.38 - 7.32 (m, 1H), 7.14 (t, *J* = 7.9 Hz, 1H), 6.90 (d, *J* = 1.5 Hz, 1H), 6.81 (d, *J* = 7.5 Hz, 1H), 6.76 (dd, *J* = 8.2, 1.0 Hz, 1H), 6.42 (dd, *J* = 17.4, 1.4 Hz, 1H), 6.14 (dd, *J* = 17.3, 10.4 Hz, 1H), 5.83 (dd, *J* = 10.4, 1.5 Hz, 1H), 4.22 (t, *J* = 6.6 Hz, 2H), 4.02 (t, *J* = 6.3 Hz, 2H), 2.98 (dd, *J* = 9.1, 7.5 Hz, 2H), 2.84 - 2.72 (m, 2H), 1.92 - 1.84 (m, 2H), 1.83 - 1.75 (m, 2H), 1.67 - 1.58 (m, 2H).

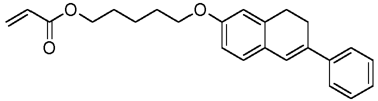

**¹H NMR** (500 MHz, Chloroform-d) δ 7.54 - 7.51 (m, 2H), 7.36 (dd, *J* = 8.5, 7.0 Hz, 2H), 7.27-7.24 (m, 1H), 7.05 (d, *J* = 8.2 Hz, 1H), 6.82 (s, *J* = 1.6 Hz, 1H), 6.74 - 6.69 (m, 2H), 6.44 - 6.38 (m, 1H), 6.13 (ddd, *J* = 17.3, 10.4, 3.2 Hz, 1H), 5.83 (dd, *J* = 10.4, 1.4 Hz, 1H), 4.20 (t, *J* = 6.6 Hz, 2H), 3.98 (t, *J =* 6.3 Hz, 2H), 2.92 (dd, *J* = 9.1, 7.0 Hz, 2H), 2.73 (ddd, *J* = 9.4, 7.1, 1.3 Hz, 2H), 1.83 (dq, *J* = 8.1, 6.5 Hz, 2H), 1.76 (dt, *J* = 15.0, 6.8 Hz, 2H), 1.63 - 1.55 (m, 2H).

### Example 11:

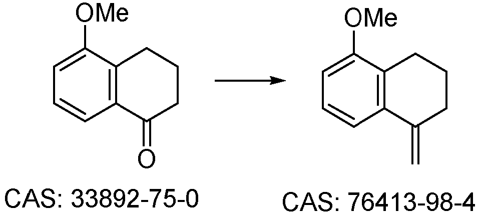

5-Methoxy-1-tetralone (6.63 g, 37.6 mmol, 1.00 eq.) and methyltriphenylphosphonium iodide is suspended in THF (35 mL). Potassium *tert*-butanolate (35.3 g, 0.489 mol, 13.00 eq., previously dissolved in THF (35 mL)) is added slowly. After addition, the mixture is stirred for 2 h. The reaction is quenched with sat. NH₄Cl-solution and diluted with methyl *tert-*butylether. The layers are separated, and the aqueous phase is extracted with methyl *tert*-butylether. The organic layers are dried over MgSO₄ and the crude product is cleaned by column chromatography (0-5% EtOAc/cyclohexane). The synthesis yield 5.90 g 5-methoxy-1-methylidene-1,2,3,4-tetrahydronaphthalene (33.9 mmol, 90% of theory).

**¹H NMR** (500 MHz, Chloroform-*d*) δ 7.30 - 7.23 (m, 1H), 7.14 (td, *J* = 7.9, 0.8 Hz, 1H), 6.75 (dd, *J* = 8.0, 1.0 Hz, 1H), 5.47 (d, *J* = 1.2 Hz, 1H), 4.96 (d, *J* = 1.5 Hz, 1H), 3.83 (s, 3H), 2.75 (t, *J* = 6.4 Hz, 2H), 2.52 - 2.47 (m, 2H), 1.92 - 1.84 (m, 2H).

Analogously, other derivatives are prepared in the same manner:

| **No.** | **Reactant** | **Product** | **Yield [%]** |
|---|---|---|---|
| **11a** | 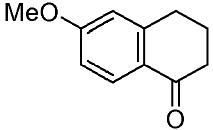 CAS: 1078-19-9 | 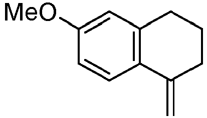 CAS: 13587-99-0 | 68 |
| **11b** | 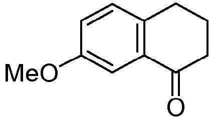 CAS: 6836-19-7 | 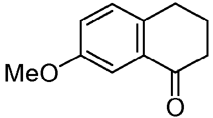 CAS: 6836-19-7 | 87 |
| **11c** | 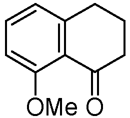 CAS: 13185-18-7 | 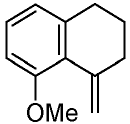 CAS: 52130-18-4 | 86 |
| **11d** | 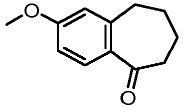 CAS: 6500-65-8 | 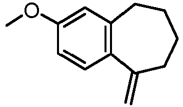 CAS: 64746-51-6 | 97 |

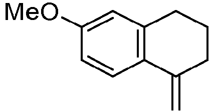

**¹H NMR** (500 MHz, DMSO-d6) δ 7.59 (d, *J* = 8.7 Hz, 1H), 6.73 (dd, *J* = 8.7, 2.8 Hz, 1H), 6.67 (dd, *J* = 2.7, 1H), 5.36 (d, *J =* 1.4 Hz, 1H), 4.81 (d, *J* = 1.5 Hz, 1H), 3.73 (s, 3H), 2.76 (t, *J* = 6.2 Hz, 2H), 2.46 - 2.43 (m, 2H), 1.75 (p, *J* = 6.2 Hz, 2H).

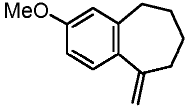

**¹H NMR** (500 MHz, DMSO-*d*₆) δ 7.07 (d, *J* = 9.1 Hz, 1H), 6.70 (d, *J* = 6.2 Hz, 2H), 5.03 (s, 1H), 4.89 (d, *J* = 2.3 Hz, 1H), 3.72 (s, 3H), 2.68 - 2.65 (m, 2H), 2.31 (t, *J* = 6.0 Hz, 2H), 1.74 (p, *J* = 6.1 Hz, 2H), 1.66 (p, *J* = 5.9 Hz, 2H).

### Example 12:

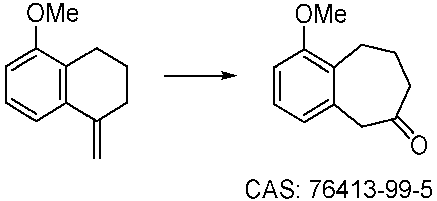

lodobenzene (4.34 mL, 38.9 mmol, 1.15 eq.), 3-chlorperoxybenzoic acid (8.73 g, 38.9 mmol, 1.15 eq.) and *para*-toluenesulfonic acid (7.56 g, 38.9 mmol, 1.15 eq.) are dissolved in DCM (250 mL). Then 1,1,1,3,3,3-hexafluoro-2-propanol (HFIP) (40 mL) is added and the reaction mixture is stirred for 45 min. After the addition of water (16 mL), 5-methoxy-1-methylidene-1,2,3,4-tetrahydronaphthalene (5.90 g, 33.9 mmol, 1.00 eq.) is added at 0 °C. After the reaction is complete, sat. NH₄Cl-solution is added. The layers are separated, and the aqueous layer is extracted with 2-methyl tetrahydrofuran two times. The organic layers are dried over MgSO₄ and the crude product is cleaned by column chromatography (10-25% EtOAc/cyclohexane). The synthesis yield 4.86 g 1-methoxy-6,7,8,9-tetrahydro-5H-benzo[7]annulen-6-one (25.5 mmol, 75% of theory).

**¹H NMR** (500 MHz, Chloroform-d) δ 7.12 (dd, *J* = 8.3, 7.5 Hz, 1H), 6.80 (dd, *J* = 8.3, 1.0 Hz, 1H), 6.74 (dd, *J* = 7.5, 1.0 Hz, 1H), 3.80 (s, 3H), 3.69 (s, 2H), 3.01 - 2.95 (m, 2H), 2.51 (t, *J* = 6.9 Hz, 2H), 1.98 - 1.89 (m, 2H).

Analogously, other derivatives are prepared in the same manner:

| **No.** | **Reactant** | **Product** | **Yield [%]** |
|---|---|---|---|
| **12a** | 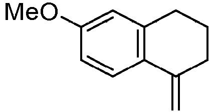 | 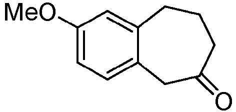 CAS: 40463-28-3 | 85 |
| **12b** | 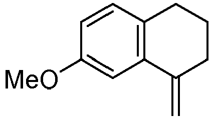 | 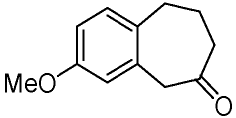 CAS: 91495-63-5 | 79 |
| **12c** | 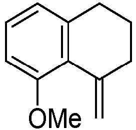 | 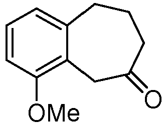 CAS: 122762-25-8 | 66 |
| **12d** | 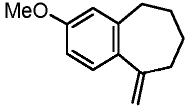 | 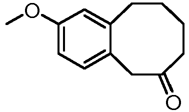 | 89 |

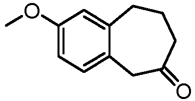

**¹H NMR** (500 MHz, DMSO-*d*₆) δ 7.07 (d, *J* = 8.2 Hz, 1H), 6.79 (d, *J* = 2.7 Hz, 1H), 6.72 (dd, *J* = 8.3, 2.8 Hz, 1H), 3.72 (s, 3H), 3.65 (s, 2H), 2.93 - 2.88 (m, 2H), 2.51 - 2.48 (m, 2H), 1.88 - 1.81 (m, 2H).

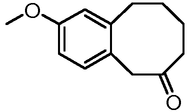

**¹H NMR** (500 MHz, DMSO-*d*₆) δ 7.03 (d, *J* = 8.4 Hz, 1H), 6.79 (d, *J* = 2.8 Hz, 1H), 6.73 (dd, *J* = 8.3, 2.7 Hz, 1H), 3.73 (s, 3H), 3.69 (s, 2H), 2.77 - 2.75 (m, 2H), 2.22 - 2.20 (m, 2H), 1.75 (ddd, *J* = 8.7, 7.4, 4.5 Hz, 2H), 1.61 - 1.56 (m, 2H).

### Example 13:

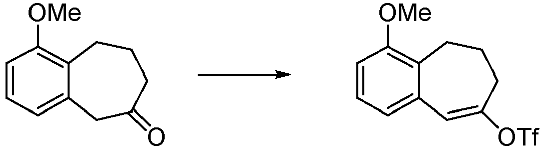

To a solution of diisopropylamine (3.94 mL, 28.1 mmol, 1.10 eq.) in THF (30 mL) is added n-butyllithium (2.5 M in hexanes, 11.2 mL, 28.1 mmol, 1.10 eq.) at -78 °C dropwise. The mixture is stirred at this temperature for 10 min and 30 min at 0 °C. 1-methoxy-6,7,8,9-tetrahydro-5H-benzo[7]annulen-6-one (4.86 g, 25.6 mmol, 1.00 eq.) is dissolved in THF (25 mL) and added to the LDA solution at -78 °C and stirred for 2 h. N-Phenyl-bis-(trifluormethanesulfonimide) (11.0 g, 30.6 mmol, 1.20 eq.) is added and stirred overnight while warming to room temperature. The solvent is evaporated und cleaned by column chromatography (2% EtOAc/cyclohexane). The synthesis yield 7.57 g 4-methoxy-6,7-dihydro-5H-benzo[7]annulen-8-yl trifluoro-methanesulfonate (23.5 mmol, 92% of theory).

**¹H NMR** (500 MHz, Chloroform-d) δ 7.15 (t, *J* = 8.0 Hz, 1H), 6.83 (d, *J* = 8.3 Hz, 1H), 6.79 (d, *J* = 7.6 Hz, 1H), 6.56 (s, 1H), 3.82 (s, 3H), 2.99 - 2.92 (m, 2H), 2.75 (t, *J* = 6.7 Hz, 1H), 1.99 - 1.89 (m, 2H).

Analogously, other derivatives are prepared in the same manner:

| **No.** | **Reactant** | **Product** | **Yield [%]** |
|---|---|---|---|
| **13a** | 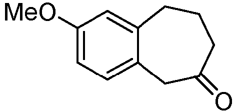 | 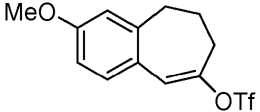 | 92 |
| **13b** | 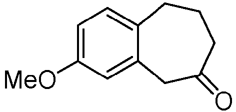 | 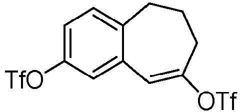 | 81 |
| **13c** | 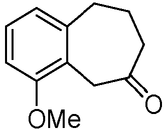 | 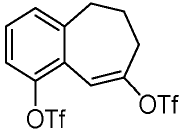 | 92 |
| **13d** | 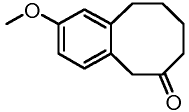 | 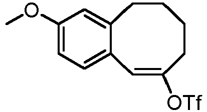 | 93 |
| **13e** | 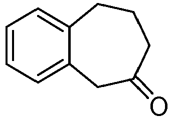 CAS: 34663-15-5 | 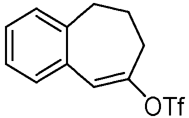 | 94 |

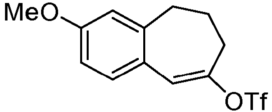

**¹H NMR** (500 MHz, DMSO-*d*₆) δ 7.28 (d, *J* = 8.1 Hz, 1H), 6.83 - 6.77 (m, 2H), 6.73 - 6.68 (m, 1H), 3.76 (s, 3H), 2.83 - 2.78 (m, 2H), 2.75 (dt, *J* = 6.5, 3.3 Hz, 2H), 1.87 (dt, *J* = 10.5, 6.3 Hz, 2H).

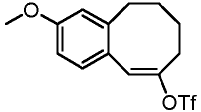

**¹H NMR** (500 MHz, DMSO-*d*₆) δ 7.20 (d, *J* = 8.5 Hz, 1H), 6.83 (dd, *J* = 8.5, 2.7 Hz, 1H), 6.80 (d, *J* = 2.6 Hz, 1H), 6.65 (s, 1H), 3.76 (s, 3H), 2.74 (dd, *J* = 7.4, 5.3 Hz, 2H), 2.56 - 2.53 (m, 2H), 1.67 (h, *J* = 5.7, 5.2 Hz, 2H), 1.50 (dq, *J* = 12.0, 5.9 Hz, 2H).

### Example 14:

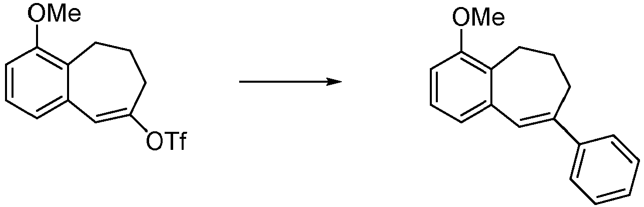

Benzeneboronic acid (3.51 g, 28.2 mmol, 1.20 eq.), Pd(dppf)Cl₂ (0.859 g, 1.17 mmol, 0.05 eq.) and 4-methoxy-6,7-dihydro-5H-benzo[7]annulen-8-yl trifluoromethane-sulfonate (7.57 g, 1.00 eq.) are dissolved in 1,4-dioxane (350 mL). Then 3 M NaOH-solution (70 mL) is added the mixture is stirred under reflux for 2 h. The reaction is quenched with sat. NH₄Cl-solution and diluted with EtOAc. The phases are separated, and the aqueous phase is extracted with EtOAc. The organic layers are dried over MgSO₄ and the crude product is cleaned by column chromatography (5-10% EtOAc/cyclohexane). The synthesis yield 5.16 g 4-methoxy-8-phenyl-6,7-dihydro-5H-benzo[7]annulene (20.6 mmol, 88% of theory).

**¹H NMR** (500 MHz, Chloroform-*d*) δ 7.54 - 7.49 (m, 2H), 7.36 (t, *J* = 7.7 Hz, 2H), 7.31 - 7.26 (m, 1H), 7.16 (t, *J* = 7.9 Hz, 1H), 6.85 (d, *J* = 7.7 Hz, 1H), 6.82 (s, 1H), 6.78 (dd, *J* = 8.3, 1.0 Hz, 1H), 3.85 (s, 3H), 2.88 - 2.83 (m, 2H), 2.57 (t, *J* = 6.9 Hz, 2H), 2.25 - 2.18 (m, 2H).

Analogously, other derivatives are prepared with benzeneboronic acid in the same manner:

| **No.** | | | **Yield [%]** |
|---|---|---|---|
| **14a** | 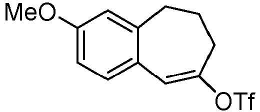 | 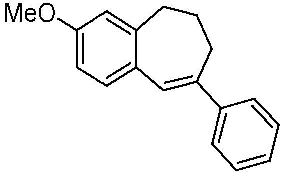 CAS: 109610-87-9 | 79 |
| **14b** | 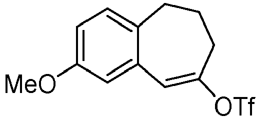 | 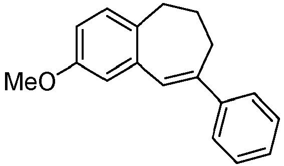 | 90 |
| **14c** | 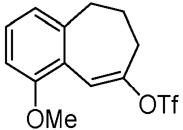 | 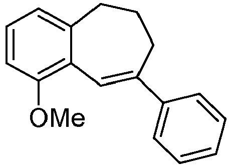 | 86 |
| **14d** | 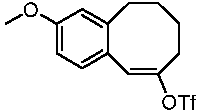 | 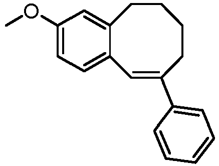 | 73 |

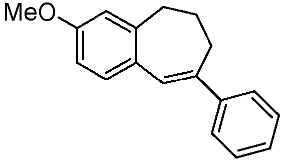

**¹H NMR** (500 MHz, Chloroform-*d*) δ 7.50 (d, *J* = 7.9 Hz, 2H), 7.36 (dd, *J* = 8.4, 6.9 Hz, 2H), 7.28-7.26 (t, *J* = 7.5 Hz, 1H), 7.16 (d, *J* = 8.1 Hz, 1H), 6.76 - 6.73 (m, 3H), 3.83 (s, 3H), 2.82 - 2.80 (m, 2H), 2.68 - 2.65 (m, 2H), 2.23 - 2.18 (m, 2H).

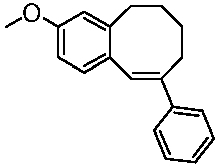

**¹H NMR** (500 MHz, Chloroform-*d*) δ 7.55 - 7.52 (m, 1H), 7.38 - 7.27 (m, 4H), 7.12 (d, *J* = 8.4 Hz, 1H), 6.82 (s, 1H), 6.79 (d, *J* = 2.7 Hz, 1H), 6.75 (dd, *J* = 8.4, 2.7 Hz, 1H), 3.83 (s, 3H), 2.73 - 2.70 (m, 2H), 2.42 - 2.40 (m, 2H), 1.79 - 1.72 (m, 2H), 1.59 (q, *J* = 5.9 Hz, 1H).

Analogously, additional derivatives are prepared with different boronic acids in the same manner:

| | | | |
|---|---|---|---|
| **No.** | | | **Yield [%]** |
| **14e** | R1 | 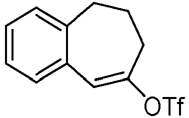 | |
| | R2 | 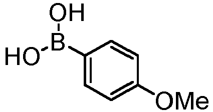 | |
| | [P] | 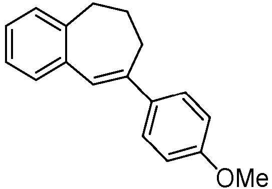 | 93 |
| **14f** | R1 | 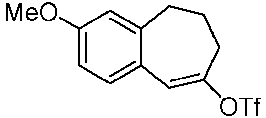 | |
| | R2 | 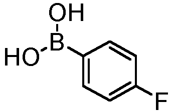 | |
| | [P] | 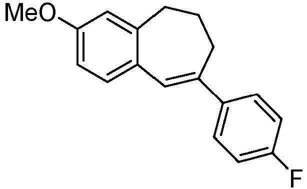 | 92 |
| **14g** | R1 | 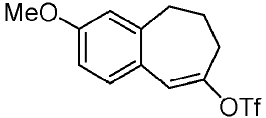 | |
| | R2 | 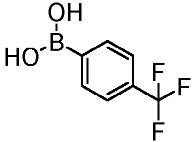 | |
| | [P] | 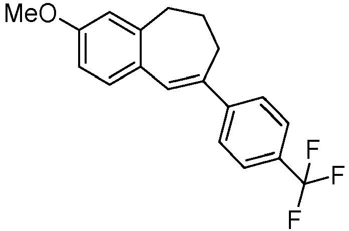 | 81 |
| **14h** | R1 | 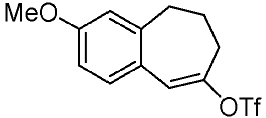 | |
| | R2 | 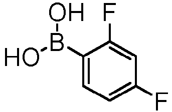 | |
| | [P] | 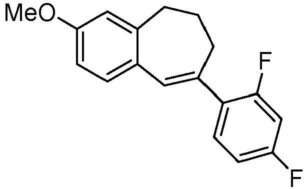 | 96 |
| **14i** | R1 | 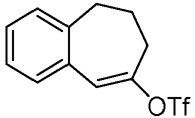 | |
| | R2 | 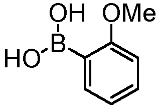 | |
| | [P] | 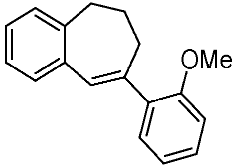 | 85 |
| **14j** | R1 | 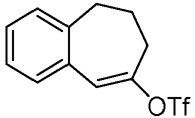 | |
| | R2 | 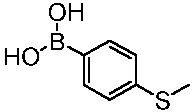 | |
| | [P] | 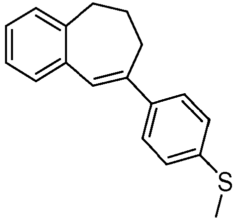 | 87 |

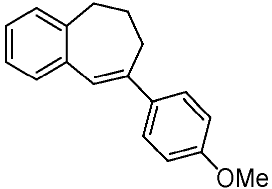

**¹H NMR** (500 MHz, Chloroform-*d*) δ 7.45 (d, *J =* 8.7 Hz, 2H), 7.23 - 7.09 (m, 4H), 6.91 (d, *J* = 8.7 Hz, 2H), 6.75 (s, 1H), 3.84 (s, 3H), 2.88 - 2.72 (m, 2H), 2.62 (t, *J* = 6.8 Hz, 2H), 2.27 - 2.12 (m, 2H).

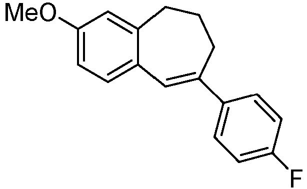

**¹H NMR** (500 MHz, Chloroform-*d*) δ 7.44 (dd, *J* = 8.6, 5.5 Hz, 2H), 7.14 (d, *J* = 8.2 Hz, 1H), 7.03 (t, *J* = 8.7 Hz, 2H), 6.74 (dd, *J* = 11.0, 2.7 Hz, 2H), 6.68 (s, 1H), 3.82 (s, 3H), 2.82 - 2.78 (m, 2H), 2.63 (t, *J* = 6.7 Hz, 2H), 2.18 (p, *J* = 6.5 Hz, 2H).

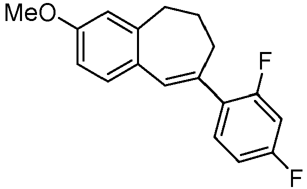

**¹H NMR** (500 MHz, Chloroform-*d*) δ 7.34 (td, *J* = 8.7, 6.5 Hz, 1H), 7.13 (d, *J* = 9.1 Hz, 1H), 6.90 - 6.81 (m, 2H), 6.76 (dd, *J* = 6.3, 2.7 Hz, 2H), 6.56 (s, 1H), 3.84 (s, 3H), 2.88 - 2.83 (m, 2H), 2.57 (t, *J* = 6.7 Hz, 2H), 2.20 (p, *J =* 6.6 Hz, 2H).

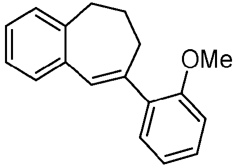

**¹H NMR** (500 MHz, Chloroform-*d*) δ 7.37 - 7.26 (m, 2H), 7.23 - 7.18 (m, 3H), 7.17 - 7.13 (m, 1H), 6.99 (td, *J* = 7.4, 1.1 Hz, 1H), 6.94 (dd, *J* = 8.1, 1.0 Hz, 1H), 6.58 (s, 1H), 3.87 (s, 3H), 2.93 - 2.82 (m, 2H), 2.57 (td, *J =* 6.8, 1.0 Hz, 2H), 2.30 - 2.16 (m, 2H).

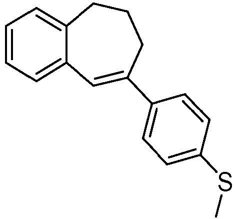

**¹H NMR** (500 MHz, Chloroform-*d*) δ 7.45 (d, *J* = 8.5 Hz, 2H), 7.33 - 7.24 (m, 2H), 7.23 - 7.19 (m, 2H), 7.19 - 7.13 (m, 2H), 6.80 (s, 1H), 2.86 - 2.71 (m, 2H), 2.67 - 2.56 (m, 2H), 2.52 (s, 3H), 2.27 - 2.15 (m, 2H).

### Example 15-1:

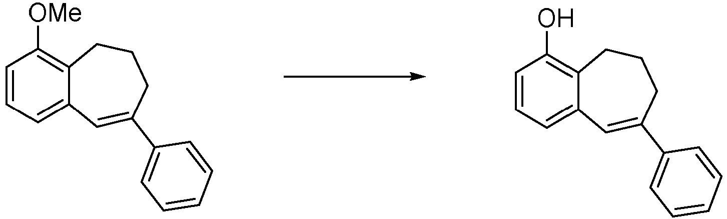

To 4-methoxy-8-phenyl-6,7-dihydro-5H-benzo[7]annulene (5.16 g, 20.6 mmol, 1.00 eq.) in DCM (180 mL) is added BBr₃ (2.18 mL, 22.7 mmol, 1.00 eq.) and stirred for 6 h. The mixture is quenched with sat. NaHCO₃-solution and extracted with DCM three times. The organic layers are dried over MgSO₄ and the crude product is cleaned by column chromatography (5-15% EtOAc/cyclohexane). The synthesis yield 4.50 g 8-phenyl-6,7-dihydro-5H-benzo[7]annulen-4-ol (19,0 mmol, 92% of theory).

**¹H NMR** (500 MHz, Chloroform-*d*) δ 7.54 - 7.48 (m, 2H), 7.40 - 7.34 (m, 2H), 7.31 - 7.26 (m, 1H), 7.06 (t, *J* = 7.8 Hz, 1H), 6.84 (d, *J* = 7.5 Hz, 1H), 6.81 (s, 1H), 6.68 (dd, *J* = 8.1, 1.1 Hz, 1H), 4.73 (d, *J* = 1.4 Hz, 1H), 2.85 - 2.79 (m, 2H), 2.59 (t, *J* = 6.9 Hz, 2H), 2.28 - 2.21 (m, 2H).

Analogously, other derivatives are prepared in the same manner:

| **No.** | | | **Yield [%]** |
|---|---|---|---|
| **15a** | 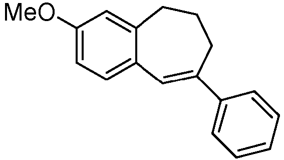 | 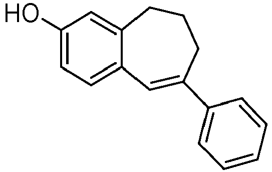 CAS: 1143508-09-1 | 87 |
| **15b** | 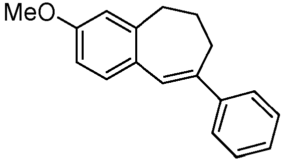 | 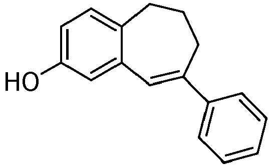 | 89 |
| **15c** | 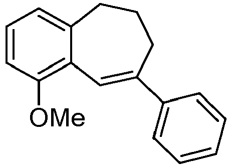 | 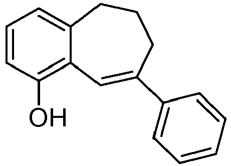 | 93 |
| **15d** | 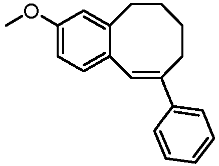 | 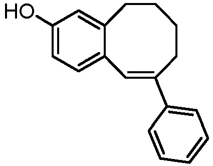 | 87 |
| **15e** | 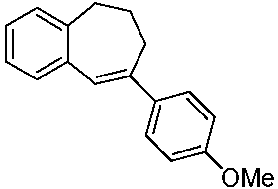 | 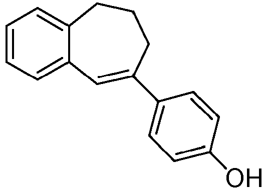 | 99 |
| **15 f** | 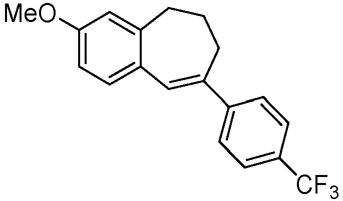 | 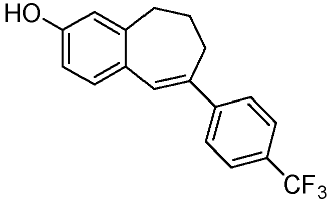 | quant . |
| **15g** | 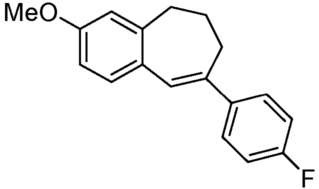 | 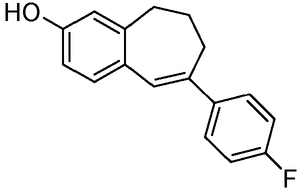 | 91 |
| **15h** | 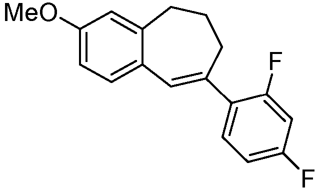 | 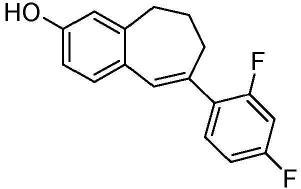 | 85 |
| **15i** | 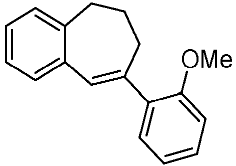 | 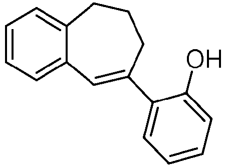 | 75 |

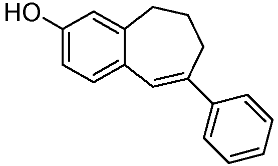

**¹H NMR** (500 MHz, Chloroform-*d*) δ 7.50 - 7.46 (m, 2H), 7.35 (dd, *J* = 8.4, 7.0 Hz, 2H), 7.29 - 7.25 (m, 1H), 7.11 - 7.09 (m, 1H), 6.73 (s, 1H), 6.68 - 6.66 (m, 2H), 4.66 (s, 1H), 2.79 - 2.76 (m, 2H), 2.67 - 2.64 (m, 2H), 2.19 (tt, *J* = 6.6, 5.3 Hz, 2H).

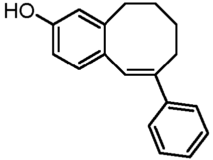

**¹H NMR** (500 MHz, Chloroform-*d*) δ 7.57 - 7.52 (m, 2H), 7.37 (t, *J* = 7.6 Hz, 2H), 7.33 - 7.28 (m, 1H), 7.08 (d, *J* = 8.2 Hz, 1H), 6.81 (s, 1H), 6.74 (d, *J =* 2.7 Hz, 1H), 6.68 (dd, *J* = 8.2, 2.6 Hz, 1H), 4.88 (s, 1H), 2.71 - 2.68 (m, 2H), 2.43 - 2.41 (m, 2H), 1.75 (p, *J* = 6.0 Hz, 2H), 1.59 (p, *J* = 5.9 Hz, 2H).

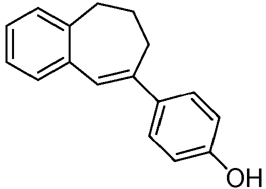

**¹H NMR** (500 MHz, Chloroform-*d*) δ 7.41 (d, *J* = 8.6 Hz, 2H), 7.23 - 7.06 (m, 4H), 6.83 (d, *J* = 8.6 Hz, 2H), 6.74 (s, 1H), 4.82 (t, *J* = 7.2 Hz, 1H), 2.88 - 2.70 (m, 2H), 2.67 - 2.52 (m, 2H), 2.27 - 2.15 (m, 2H).

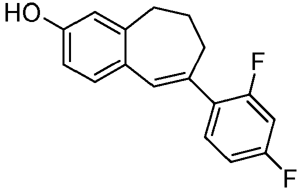

**¹H NMR** (500 MHz, Chloroform-*d*) δ 7.31 (td, *J* = 8.6, 6.5 Hz, 1H), 7.08 - 7.03 (m, 1H), 6.88 - 6.78 (m, 2H), 6.66 (d, *J* = 6.7 Hz, 2H), 6.52 (s, 1H), 4.66 (s, 1H), 2.82 - 2.78 (m, 2H), 2.54 (t, *J* = 6.7 Hz, 2H), 2.17 (p, *J* = 6.5 Hz, 2H).

**¹⁹F NMR** (470 MHz, Chloroform-d) δ -110.9 (q, *J* = 9.1 Hz), -112.6.

### Example 15-2:

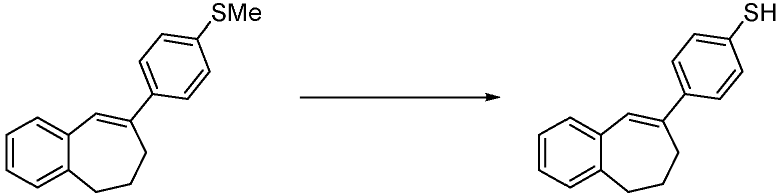

8-[4-(methylsulfanyl)phenyl]-6,7-dihydro-5H-benzo[7]annulene (44.48 mmol; 1,0 eq.; 11.85 g) is dissolved in 1-methylpyrrolidin-2-one (889.63 mmol; 20.0 eq.; 85.62 ml) and tert.-butylmercaptane sodium salt (133.44 mmol; 3.0 eq.; 16,63 g) is added at r.t.. The reaction mixture is heated to 180 °C overnight. The reaction mixture is then added to 1 L of water and extracted three times with EtOAc. Combined organics are washed with H₂O and brine before they are dried over Na₂SO₄ and filtered over a pad of silica which is rinsed with EtOAc. The crude product is purified by column chromatography on silica gel (cyclohexane/EtOAc 0-3%). The synthesis yield 9.96g 4-{6,7-dihydro-5H-benzo[7]annulen-8-yl}benzene-1-thiol (39.46 mmol, 89% of theory).

**¹H NMR** (500 MHz, Chloroform-*d*) δ 7.41 (d, *J* = 8.4 Hz, 2H), 7.29 (d, *J* = 8.4 Hz, 2H), 7.25 - 7.14 (m, 4H), 6.80 (s, 1H), 3.49 (s, 1H), 2.89 - 2.71 (m, 2H), 2.70 - 2.59 (m, 2H), 2.36 - 2.16 (m, 2H).

### Example 16-1:

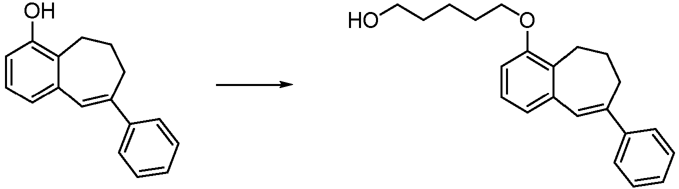

6-phenyl-7,8-dihydronaphthalen-1-ol (866 mg, 3.90 mmol, 1.00 eq.) is refluxed with potassium carbonate (2.18 g, 15.6 mmol, 4.00 eq.) and 2-[(5-bromopentyl)oxy]oxane (1.03 g, 4.09 mmol, 1.05 mmol) in acetone (15 mL) for 2 d. The suspension is filtered and the solvent of the filtrate is evaporated. The residue is dissolved in DCM (25 mL) and MeOH (25 mL) and conc. HCl (0.49 mL) is added. After stirring for 1 h the reaction is quenched with sat. NaHCO₃-solution and the phases are separated. The aqueous phase is extracted two times with DCM. The organic layers are dried over MgSO₄. The crude product is cleaned by column chromatography (12-30% 2-methyl tetrahydrofuran/cyclohexane). The synthesis yield 1.06 g 5-[(6-phenyl-7,8-dihydronaphthalen-1-yl)oxy]pentan-1-ol (3.44 mmol, 88% of theory).

**¹H NMR** (500 MHz, Chloroform-*d*) δ 7.54 - 7.49 (m, 2H), 7.37 (t, *J* = 7.7 Hz, 2H), 7.28 (t, *J* = 7.4 Hz, 1H), 7.13 (t, *J* = 7.9 Hz, 1H), 6.84 (d, *J* = 7.6 Hz, 1H), 6.82 (s, 1H), 6.76 (dd, *J* = 8.2, 1.0 Hz, 1H), 4.00 (t, *J* = 6.3 Hz, 2H), 3.73 - 3.68 (m, 2H), 2.89 - 2.84 (m, 2H), 2.57 (t, *J* = 6.9 Hz, 2H), 2.25 - 2.18 (m, 2H), 1.90 - 1.82 (m, 2H), 1.72 - 1.62 (m, 2H).

Analogously, other derivatives are prepared in the same manner: R1 means reactant and [P] means product.

| **No.** | | | **Yield [%]** |
|---|---|---|---|
| **16a** | R1 | 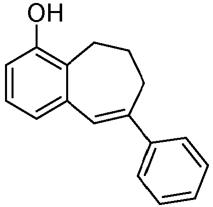 | |
| | R2 | 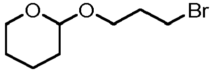 | |
| | [P] | 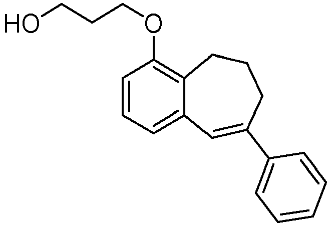 | 80 |
| **16b** | R1 | 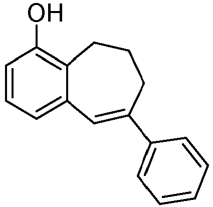 | |
| | R2 | 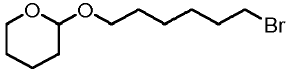 | |
| | [P] | 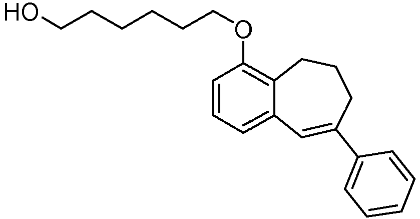 | 83 |
| **16c** | R1 | 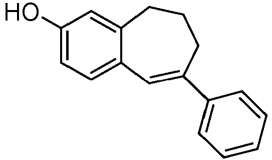 | |
| | R2 | 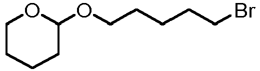 | |
| | [P] | 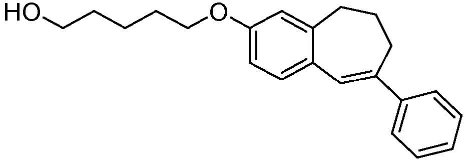 | 77 |
| **16d** | R1 | 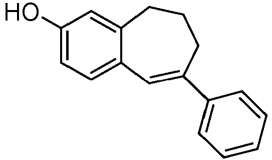 | |
| | R2 | 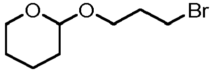 | |
| | [P] | 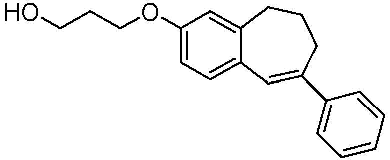 | 75 |
| **16e** | R1 | 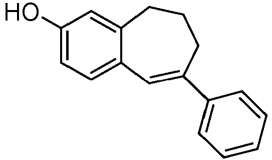 | |
| | R2 | 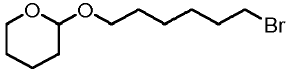 | |
| | [P] | 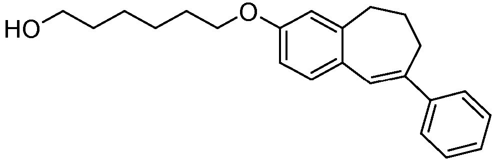 | 80 |
| **16f** | R1 | 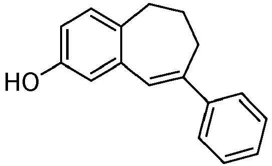 | |
| | R2 | 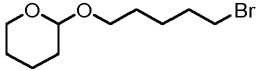 | |
| | [P] | 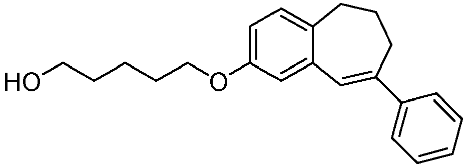 | 84 |
| **16g** | R1 | 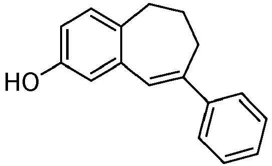 | |
| | R2 | 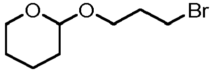 | |
| | [P] | 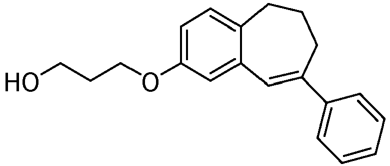 | 82 |
| **16h** | R1 | 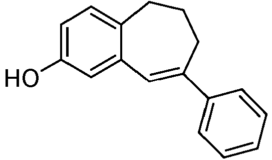 | |
| | R2 | 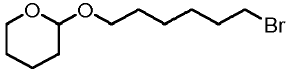 | |
| | [P] | 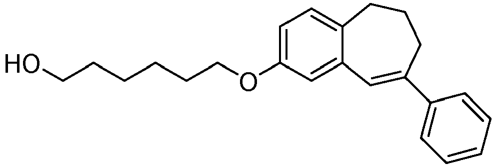 | 79 |
| **16i** | R1 | 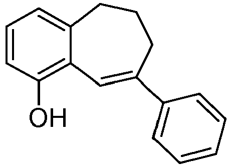 | |
| | R2 | 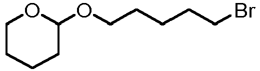 | |
| | [P] | 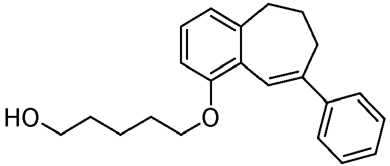 | 76 |
| **16j** | R1 | 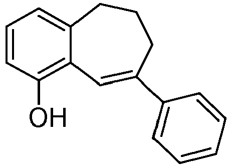 | |
| | R2 | 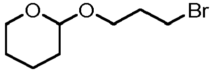 | |
| | [P] | 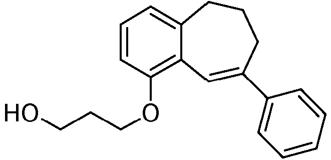 | 78 |
| **16k** | R1 | 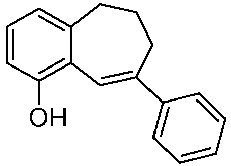 | |
| | R2 | 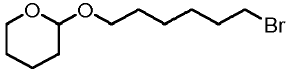 | |
| | [P] | 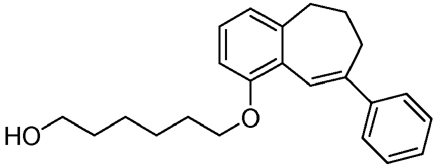 | 85 |
| **16l** | R1 | 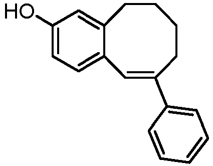 | |
| | R2 | 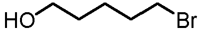 | |
| | [P] | 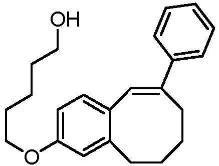 | 89 |
| **16m** | R1 | 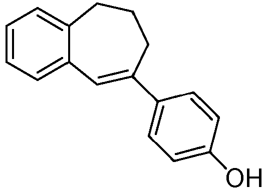 | |
| | R2 | 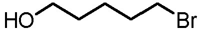 | |
| | [P] | 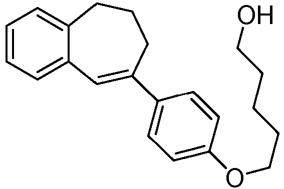 | 77 |
| **16n** | R1 | 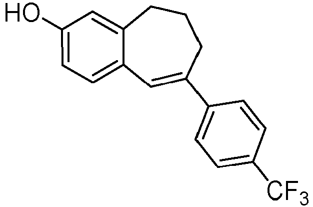 | |
| | R2 | 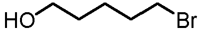 | |
| | [P] | 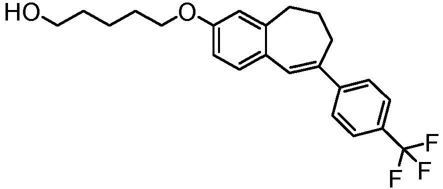 | 66 |
| **160** | R1 | 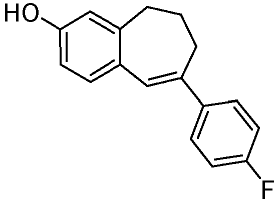 | |
| | R2 | 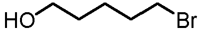 | |
| | [P] | 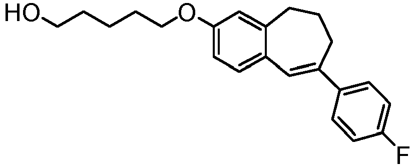 | 77 |
| **16p** | R1 | 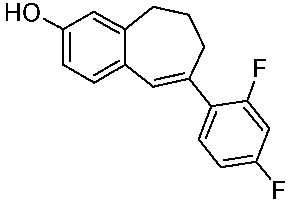 | |
| | R2 | 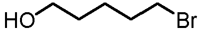 | |
| | [P] | 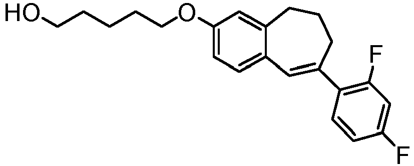 | 78 |
| **16q** | R1 | 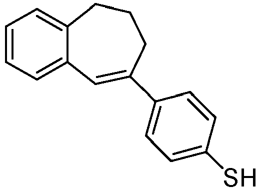 | |
| | R2 | 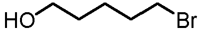 | |
| | | 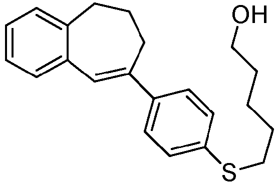 | 98 |

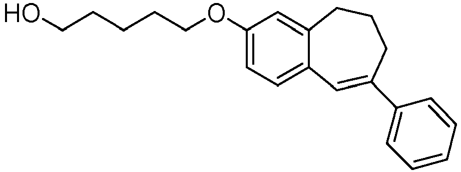

**¹H NMR** (500 MHz, Chloroform-*d*) δ 7.51 - 7.49 (m, 2H), 7.36 (t, *J* = 7.7 Hz, 2H), 7.28 (d, *J =* 7.6 Hz, 1H), 7.16 - 7.13 (m, 1H), 6.74 (dd, *J* = 6.9, 2.1 Hz, 3H), 4.00 (t, *J* = 6.4 Hz, 2H), 3.71 (q, *J* = 6.2 Hz, 2H), 2.82 - 2.79 (m, 2H), 2.67 (t, *J* = 6.7 Hz, 2H), 2.20 (qd, *J* = 6.6, 3.6 Hz, 2H), 1.84 (p, *J* = 6.7 Hz, 2H), 1.70 - 1.65 (m, 2H), 1.61 - 1.55 (m, 2H).

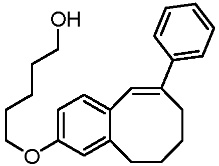

**¹H NMR** (500 MHz, DMSO-*d*₆) δ 7.57 - 7.55 (m, 2H), 7.37 (dd, *J* = 8.4, 6.9 Hz, 2H), 7.32 - 7.27 (m, 1H), 7.11 (d, *J* = 8.5 Hz, 1H), 6.82 (d, *J* = 2.2 Hz, 2H), 6.75 (dd, *J =* 8.4, 2.7 Hz, 1H), 4.38 (t, *J* = 5.1 Hz, 1H), 3.96 (t, *J* = 6.5 Hz, 2H), 3.42 (q, *J* = 5.8 Hz, 2H), 2.63 (dd, *J* = 7.1, 4.1 Hz, 2H), 2.37 - 2.31 (m, 2H), 1.70 (dt, *J* = 13.4, 7.0 Hz, 4H), 1.46 (dddd, *J* = 18.9, 15.3, 7.4, 2.3 Hz, 6H).

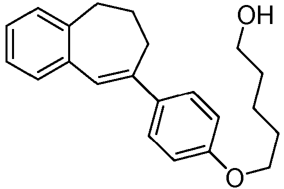

**¹H NMR** (500 MHz, Chloroform-*d*) δ 7.44 (d, *J* = 8.7 Hz, 2H), 7.23 - 7.09 (m, 4H), 6.89 (d, *J* = 8.7 Hz, 2H), 6.75 (s, 1H), 4.00 (t, *J* = 6.4 Hz, 2H), 3.70 (q, *J* = 6.0 Hz, 2H), 2.90 - 2.67 (m, 2H), 2.62 (t, *J* = 6.8 Hz, 2H), 2.21 (p, *J =* 6.7 Hz, 2H), 1.95 - 1.77 (m, 2H), 1.74 - 1.62 (m, 2H), 1.62 - 1.51 (m, 2H), 1.26 (t, *J* = 5.2 Hz, 1H).

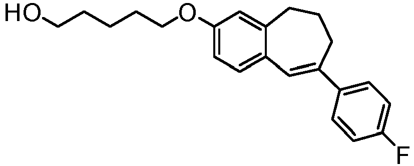

**¹H NMR** (500 MHz, DMSO-*d*₆) δ 7.55 (dd, *J* = 8.8, 5.5 Hz, 2H), 7.21 - 7.15 (m, 3H), 6.77 - 6.73 (m, 2H), 6.72 (s, 1H), 4.37 (t, *J* = 5.2 Hz, 1H), 3.96 (t, *J* = 6.5 Hz, 2H), 3.43 - 3.39 (m, 2H), 2.77 - 2.73 (m, 2H), 2.63 - 2.59 (m, 2H), 2.10 - 2.03 (m, 2H), 1.71 (p, *J* = 6.7 Hz, 2H), 1.50 - 1.42 (m, 4H).

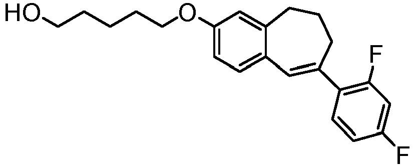

**¹H NMR** (500 MHz, Chloroform-*d*) δ 7.31 (td, *J* = 8.6, 6.5 Hz, 1H), 7.10 - 7.07 (m, 1H), 6.83 (dddd, *J* = 19.5, 11.2, 8.6, 2.6 Hz, 2H), 6.72 (d, *J* = 6.9 Hz, 2H), 6.53 (s, 1H), 3.99 (t, *J* = 6.4 Hz, 2H), 3.69 (td, *J* = 6.5, 2.6 Hz, 2H), 2.84 - 2.80 (m, 2H), 2.55 (t, *J* = 6.7 Hz, 2H), 2.17 (p, *J* = 6.5 Hz, 2H), 1.83 (p, *J* = 6.7 Hz, 2H), 1.66 (dt, *J* = 15.2, 6.6 Hz, 2H), 1.60 - 1.52 (m, 2H), 1.28 (s, *J* = 4.4 Hz, 1H).

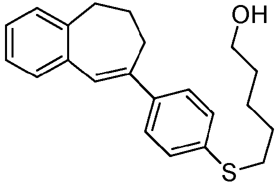

**¹H NMR** (500 MHz, Chloroform-*d*) δ 7.43 (d, *J* = 8.3 Hz, 2H), 7.31 (d, *J =* 8.3 Hz, 2H), 7.24 - 7.11 (m, 4H), 6.80 (s, 1H), 3.66 (q, *J* = 6.1 Hz, 2H), 2.95 (t, *J* = 7.3 Hz, 2H), 2.87 - 2.74 (m, 2H), 2.62 (t, *J* = 6.8 Hz, 2H), 2.21 (p, *J =* 6.7 Hz, 2H), 1.71 (p, *J* = 7.4 Hz, 2H), 1.66 - 1.42 (m, 4H), 1.23 (t, *J* = 5.3 Hz, 1H).

### Example 16-2-1:

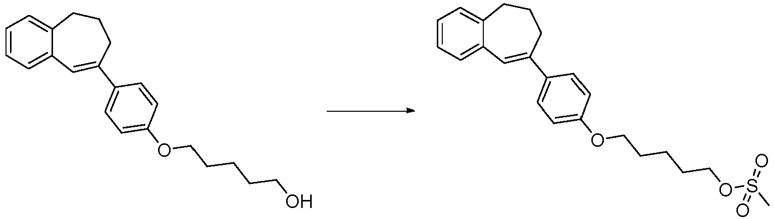

5-(4-(6,7-dihydro-5H-benzo[7]annulen-8-yl)phenoxy)pentan-1-ol (0.70 g, 2.17 mmol, 1.00 eq) is dissolved in CH₂Cl₂ (9.70 mL) before Et₃N (0.61 mL, 4.34 mmol, 2.00 eq) is added dropwise at r.t. The resulting reaction mixture is cooled to 0 °C before mesyl chloride (0.18 mL, 2.39 mmol, 1.10 eq) is added dropwise. After 1.5 h at 0 °C TLC shows complete conversion. The reaction mixture is added to water and extracted with CH₂Cl₂ (3×10 mL). Combined organic extracts are washed with brine (15 mL), dried over Na₂SO₄ and filtered over a pad of silica which is rinsed with CH₂Cl₂. After removal of the solvent under reduced pressure compound 5-(4-(6,7-dihydro-5H-benzo[7]annulen-8-yl)phenoxy)pentyl methanesulfonate (0.84 g, 2.10 mmol, 97%) is isolated as pale yellow oil and can be used for the next step without further purification.

**¹H NMR** (500 MHz, Chloroform-*d*) δ 7.44 (d, *J* = 8.8 Hz, 2H), 7.22 - 7.07 (m, 4H), 6.88 (d, *J* = 8.8 Hz, 2H), 6.75 (s, 1H), 4.27 (t, *J* = 6.5 Hz, 2H), 4.00 (t, *J* = 6.2 Hz, 2H), 3.01 (s, 3H), 2.85 - 2.76 (m, 2H), 2.68 - 2.56 (m, 2H), 2.21 (m, 2H), 1.95 - 1.78 (m, 4H), 1.63 (m, 2H).

### Example 16-2-2:

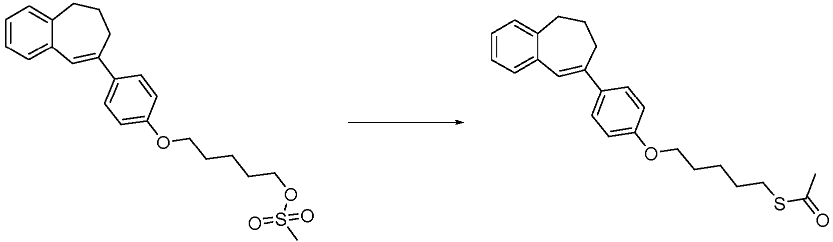

5-(4-(6,7-dihydro-5H-benzo[7]annulen-8-yl)phenoxy)pentyl methanesulfonate (0.84 g, 2.10 mmol, 1.00 eq) is dissolved in MeCN (8.22 mL) before potassium thioacetate (0.26 g, 2.31 mmol, 1.10 eq) is added portion wise at r.t. The resulting reaction mixture is heated to 50 °C for 16 h. The reaction mixture is added to water and extracted with EtOAc (3×10 mL). Combined organic extracts are washed with brine (15 mL), dried over Na₂SO₄ and filtered over a pad of silica which is rinsed with EtOAc. After removal of the solvent under reduced pressure the crude product is purified via column chromatography using Cyhex/EtOAc (10% to 15% EtOAc) to give compound S-(5-(4-(6,7-dihydro-5H-benzo[7]annulen-8-yl)phenoxy)pentyl) ethanethioate (0.61 g, 1.60 mmol, 76%) as pale yellow oil which crystallizes during storage in the fridge.

**¹H NMR** (500 MHz, Chloroform-*d*) δ 7.43 (d, *J* = 8.8 Hz, 2H), 7.23 - 7.10 (m, 4H), 6.88 (d, *J* = 8.8 Hz, 2H), 6.74 (s, 1H), 3.98 (t, *J* = 6.4 Hz, 2H), 2.91 (t, *J* = 7.2 Hz, 2H), 2.83 - 2.76 (m, 2H), 2.61 (t, *J* = 6.8 Hz, 2H), 2.33 (s, 3H), 2.21 (m, 2H), 1.81 (m, 2H), 1.73 - 1.60 (m, 2H), 1.62 - 1.52 (m, 2H).

### Example 16-2-3:

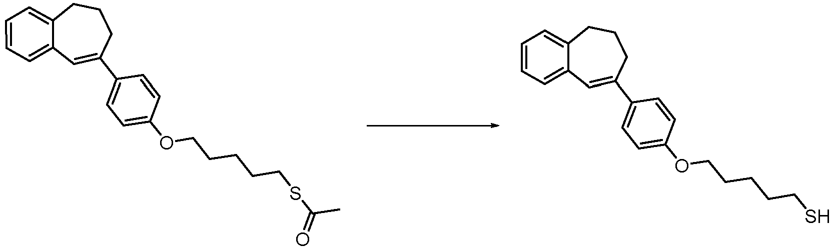

S-(5-(4-(6,7-dihydro-5H-benzo[7]annulen-8-yl)phenoxy)pentyl) ethanethioate (0.61 g, 1.60 mmol, 1.00 eq) is dissolved in THF (9.74 mL) and cooled to 0 °C before LiAlH₄ (70.5 mg, 1.76 mmol, 1.10 eq) is added carefully. The resulting reaction mixture is heated to 65 °C for 1 h. The reaction mixture is cooled to 0 °C before 1 m HCl (5 mL) is added. After gas evolution stopped, the organic layer is extracted with EtOAc (3×10 mL). Combined organic extracts are washed with brine (15 mL), dried over Na₂SO₄ and filtered over a pad of silica which is rinsed with EtOAc. After removal of the solvent compound 5-(4-(6,7-dihydro-5H-benzo[7]annulen-8-yl)phenoxy)pentane-1-thiol (0.54 g, 1.60 mmol, 99%) is isolated as colorless oil which crystallizes during storage in the fridge and can be used for the next step without further purification.

**¹H NMR** (500 MHz, Chloroform-*d*) δ 7.44 (d, *J* = 8.7 Hz, 2H), 7.21 - 7.10 (m, 4H), 6.89 (d, *J* = 8.7 Hz, 2H), 6.75 (s, 1H), 3.99 (t, *J* = 6.4 Hz, 2H), 2.90 - 2.68 (m, 2H), 2.68 - 2.46 (m, 4H), 2.21 (p, *J* = 6.7 Hz, 2H), 1.81 (m, 2H), 1.71 (p, *J* = 7.2 Hz, 2H), 1.59 (m, 2H), 1.36 (t, *J* = 7.8 Hz, 1H).

### Preparation of compounds according to the invention:

### Example 17:

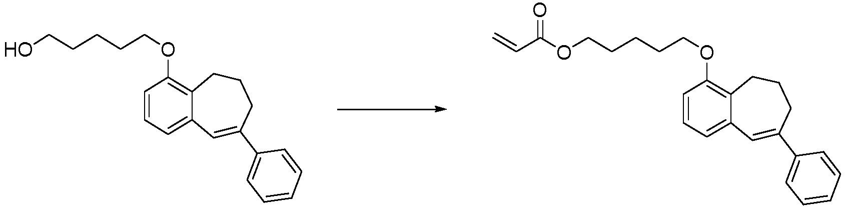

5-({8-phenyl-6,7-dihydro-5H-benzo[7]annulen-4-yl}oxy)pentan-1-ol (1.87 g, 5.80 mmol, 1.00 eq.) is dissolved in THF (70 mL) and triethylamine (3.22 mL, 23.2 mmol, 4.00 eq.) and acryloyl chloride (0.628 mL, 7.54 mmol,1.30 eq.) is added at 0 °C. After stirring at r.t. for 3 h the suspension is filtered. The solvent of the filtrate is evaporated and the crude product is cleaned by column chromatography (1-10% 2-methyl tetrahydrofuran/cyclohexane). The synthesis yield 1.94 g 5-({8-phenyl-6,7-dihydro-5H-benzo[7]annulen-4-yl}oxy)pentyl prop-2-enoate (5.14 mmol, 89% of theory).

**¹H NMR** (500 MHz, Chloroform-*d*) δ 7.53 - 7.49 (m, 2H), 7.37 (t, *J* = 7.7 Hz, 2H), 7.28 (t, *J* = 7.4 Hz, 1H), 7.13 (t, *J* = 7.9 Hz, 1H), 6.84 (d, *J* = 7.6 Hz, 1H), 6.82 (s, 1H), 6.75 (dd, *J* = 8.2, 1.0 Hz, 1H), 6.41 (dd, *J* = 17.4, 1.5 Hz, 1H), 6.13 (dd, *J* = 17.3, 10.4 Hz, 1H), 5.82 (dd, *J* = 10.4, 1.5 Hz, 1H), 4.21 (t, *J* = 6.6 Hz, 2H), 4.00 (t, *J* = 6.2 Hz, 2H), 2.89 - 2.83 (m, 2H), 2.56 (t, *J* = 6.9 Hz, 2H), 2.25 - 2.17 (m, 2H), 1.90 - 1.83 (m, 2H), 1.82 - 1.74 (m, 2H), 1.66 - 1.56 (m, 2H).

Analogously, other derivatives are prepared in the same manner with acryloyl chloride. R1 means reactant, [P] means product:

| **No.** | | | **Yield [%]** |
|---|---|---|---|
| **17a** | R1 | 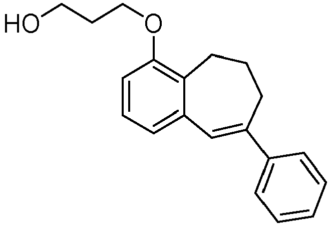 | |
| | [P] | 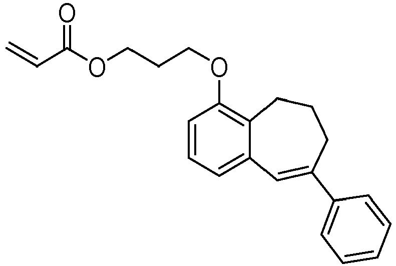 | 82 |
| **17b** | R1 | 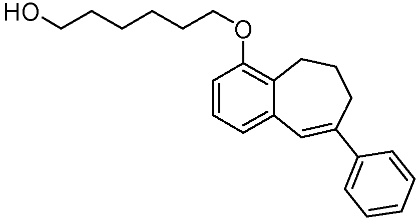 | |
| | [P] | 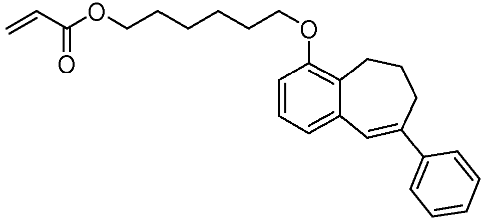 | 84 |
| **17c** | R1 | 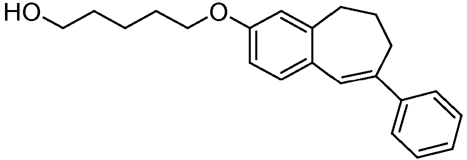 | |
| | [P] | 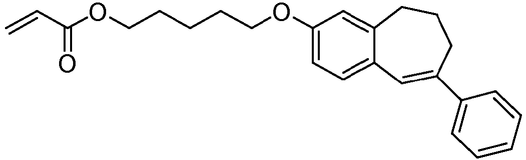 | 79 |
| **17d** | R1 | 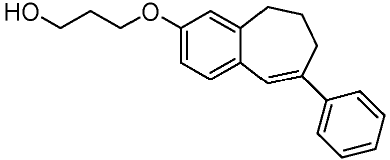 | |
| | [P] | 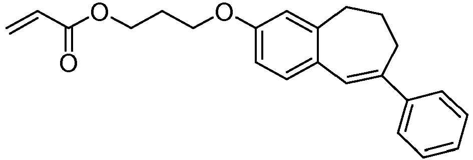 | 87 |
| **17e** | R1 | 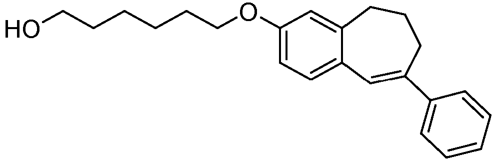 | |
| | [P] | 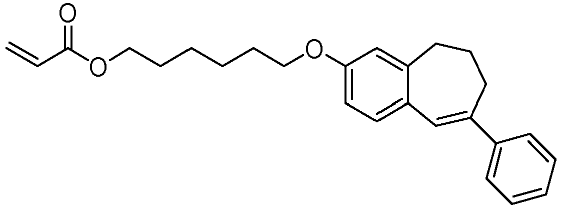 | 86 |
| **17f** | R1 | 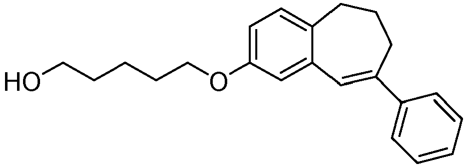 | |
| | [P] | 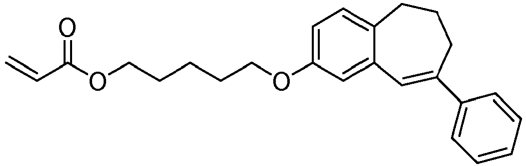 | 77 |
| **17g** | R1 | 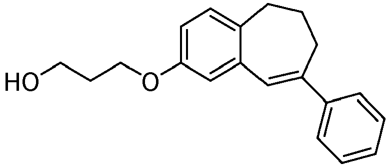 | |
| | [P] | 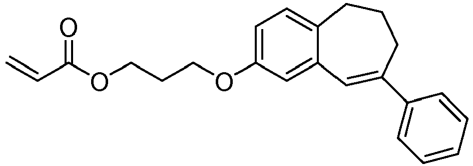 | 83 |
| **17h** | R1 | 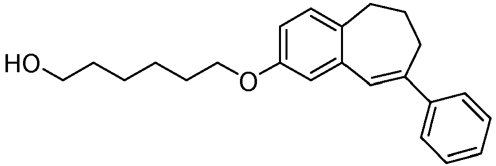 | |
| | [P] | 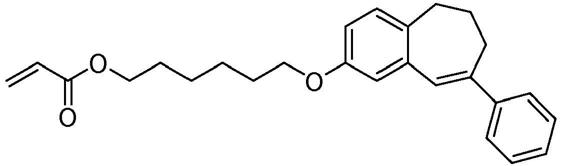 | 85 |
| **17i** | R1 | 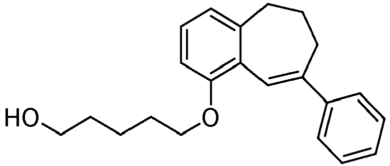 | |
| | [P] | 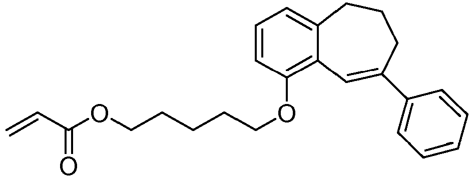 | 88 |
| **17j** | R1 | 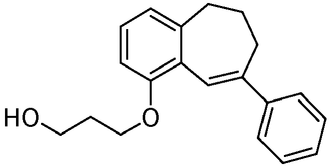 | |
| | [P] | 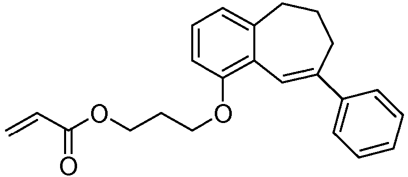 | 79 |
| **17k** | R1 | 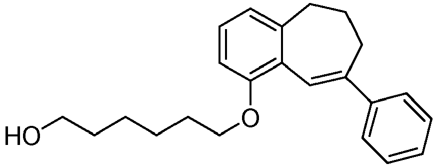 | |
| | [P] | 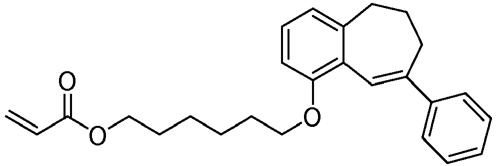 | 81 |
| **17l** | R1 | 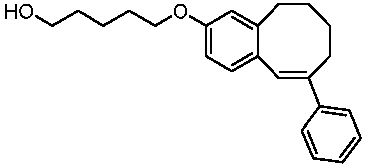 | |
| | [P] | 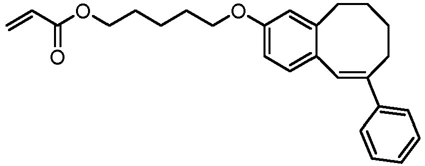 | 85 |
| **17m** | R1 | 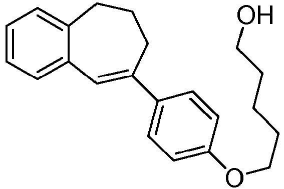 | |
| | [P] | 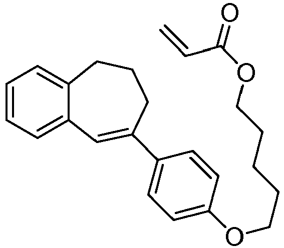 | 43 |
| **17n** | R1 | 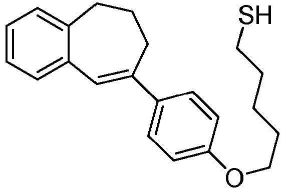 | |
| | [P] | 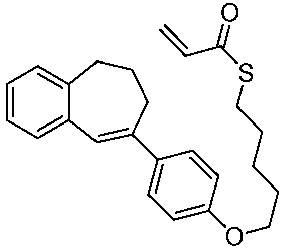 | <10 |
| **17o** | R1 | 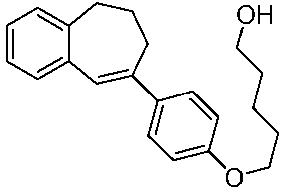 | |
| | [P] | 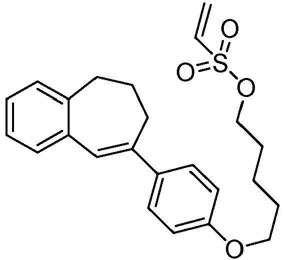 | 52 |

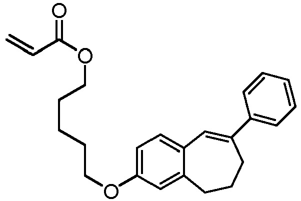

**¹H NMR** (500 MHz, Chloroform-*d*) δ 7.51 - 7.48 (m, 2H), 7.35 (t, *J* = 7.6 Hz, 2H), 7.28-7.25 (m, 1H), 7.15 - 7.12 (m, 1H), 6.75 - 6.71 (m, 3H), 6.41 (dd, *J* = 17.2, 1.4 Hz, 1H), 6.13 (dd, *J* = 17.4, 10.4 Hz, 1H), 5.83 (dd, *J* = 10.5, 1.4 Hz, 1H), 4.20 (t, *J* = 6.6 Hz, 2H), 3.99 (t, *J* = 6.3 Hz, 2H), 2.82 - 2.77 (m, 2H), 2.66 (t, *J* = 6.7 Hz, 2H), 2.19 (p, *J* = 6.5 Hz, 2H), 1.87 - 1.80 (m, 2H), 1.77 (dt, *J* = 14.4, 6.8 Hz, 2H), 1.62 - 1.54 (m, 2H).

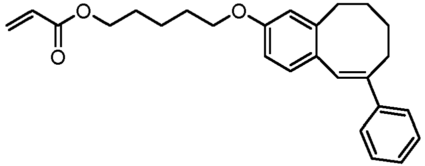

**¹H NMR** (500 MHz, Chloroform-*d*) δ 7.54 (dd, *J* = 7.6, 1.6 Hz, 2H), 7.36 (t, *J* = 7.6 Hz, 2H), 7.31 - 7.27 (m, 1H), 7.10 (d, *J =* 8.4 Hz, 1H), 6.82 (s, 1H), 6.78 (d, *J* = 2.7 Hz, 1H), 6.74 (dd, *J* = 8.4, 2.6 Hz, 1H), 6.41 (dd, *J* = 17.3, 1.5 Hz, 1H), 6.13 (dd, *J* = 17.4, 10.4 Hz, 1H), 5.83 (dd, *J* = 10.5, 1.4 Hz, 1H), 4.20 (t, *J* = 6.7 Hz, 2H), 3.99 (t, *J* = 6.3 Hz, 2H), 2.72 - 2.68 (m, 2H), 2.44 - 2.39 (m, 2H), 1.87 - 1.82 (m, 2H), 1.76 (dt, *J* = 10.9, 5.9 Hz, 4H), 1.62 - 1.56 (m, 4H).

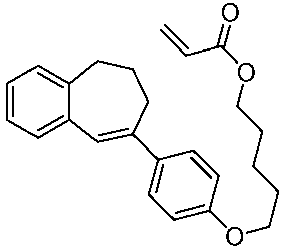

**¹H NMR** (500 MHz, Chloroform-*d*) δ 7.44 (d, *J =* 8.7 Hz, 2H), 7.23 - 7.09 (m, 4H), 6.89 (d, *J* = 8.7 Hz, 2H), 6.75 (s, 1H), 6.41 (dd, *J* = 17.3, 1.5 Hz, 1H), 6.13 (dd, *J* = 17.3, 10.4 Hz, 1H), 5.83 (dd, *J* = 10.4, 1.5 Hz, 1H), 4.20 (t, *J* = 6.6 Hz, 2H), 4.00 (t, *J* = 6.4 Hz, 2H), 2.88 - 2.74 (m, 2H), 2.62 (t, *J =* 6.8 Hz, 2H), 2.30 - 2.08 (m, 2H), 1.90 - 1.81 (m, 2H), 1.77 (m, 2H), 1.67 - 1.48 (m, 3H).

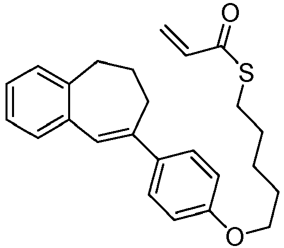

**¹H NMR** (500 MHz, Chloroform-*d*) δ 7.44 (d, *J =* 8.8 Hz, 2H), 7.23 - 7.09 (m, 4H), 6.89 (d, *J* = 8.8 Hz, 2H), 6.75 (s, 1H), 6.47 - 6.21 (m, 2H), 5.68 (dd, *J* = 10.0, 1.4 Hz, 1H), 3.99 (t, *J* = 6.4 Hz, 2H), 3.01 (t, *J* = 7.3 Hz, 2H), 2.88 - 2.76 (m, 2H), 2.62 (t, *J* = 6.8 Hz, 2H), 2.21 (p, *J* = 6.7 Hz, 2H), 1.90 - 1.75 (m, 2H), 1.75 - 1.65 (m, 2H), 1.65 - 1.55 (m, 2H).

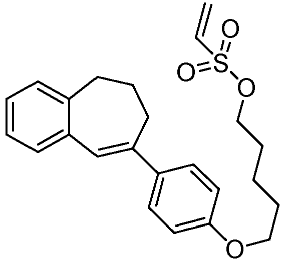

**¹H NMR** (500 MHz, Chloroform-*d*) δ 7.44 (d, *J* = 8.7 Hz, 2H), 7.24 - 7.07 (m, 4H), 6.88 (d, *J* = 8.7 Hz, 2H), 6.75 (s, 1H), 6.54 (dd, *J* = 16.7, 9.9 Hz, 1H), 6.42 (d, *J* = 16.7 Hz, 1H), 6.12 (d, *J* = 9.8 Hz, 1H), 4.17 (t, *J* = 6.4 Hz, 2H), 4.00 (t, *J* = 6.2 Hz, 2H), 2.96 - 2.73 (m, 2H), 2.62 (t, *J* = 6.8 Hz, 2H), 2.21 (p, *J* = 6.7 Hz, 2H), 1.83 (h, *J* = 6.6 Hz, 4H), 1.70 - 1.56 (m, 2H).

### Example 18:

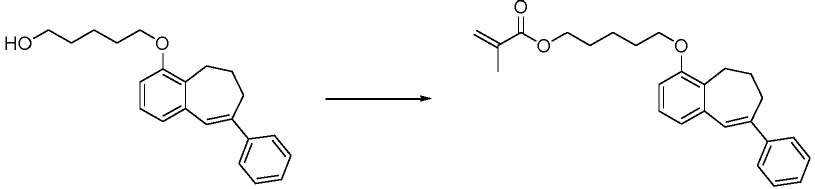

5-({8-phenyl-6,7-dihydro-5H-benzo[7]annulen-4-yl}oxy)pentan-1-ol (1.87 g, 5.80 mmol, 1.00 eq.) is dissolved in DCM (30 mL) and triethylamine (3.22 mL, 23.2 mmol, 4.00 eq.). 4.(Dimethylamino)pyridine (140 mg, 1.16 mmol, 0.20 eq.) and methacrylic anhydride (1.19 mL, 7.54 mmol, 1.30 eq.) are added at 0 °C. After stirring at r.t. for 3 h the suspension is filtered. The solvent of the filtrate is evaporated and the crude product is cleaned by column chromatography (1-10% 2-methyl tetrahydrofuran/cyclohexane). The synthesis yield 2.02 g 5-({8-phenyl-6,7-dihydro-5H-benzo[7]annulen-4-yl}oxy)pentyl 2-methylprop-2-enoate (5.16 mmol, 89% of theory).

**¹H NMR** (500 MHz, Chloroform-d) δ 7.53 - 7.49 (m, 2H), 7.37 (t, J = 7.7 Hz, 2H), 7.30 - 7.26 (m, 1H), 7.13 (t, J = 7.9 Hz, 1H), 6.84 (d, J = 7.6 Hz, 1H), 6.81 (s, 1H), 6.75 (dd, J = 8.1, 1.1 Hz, 1H), 6.13 - 6.06 (m, 1H), 5.58 - 5.51 (m, 1H), 4.20 (t, J = 6.6 Hz, 2H), 4.00 (t, J = 6.2 Hz, 2H), 2.89 - 2.83 (m, 2H), 2.56 (t, J = 6.9 Hz, 2H), 2.25 - 2.17 (m, 2H), 1.95 (t, J = 1.3 Hz, 3H), 1.90 - 1.83 (m, 2H), 1.82 - 1.74 (m, 2H), 1.66 - 1.58 (m, 2H).

Analogously, other derivatives are prepared with methacrylic anhydride in the same manner: R1 means reactant and [P] means product.

| | | | |
|---|---|---|---|
| **No.** | | | **Yield [%]** |
| **18a** | R1 | 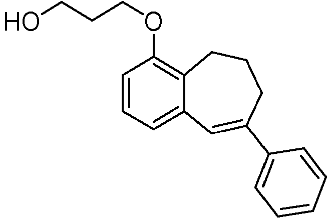 | |
| | [P] | 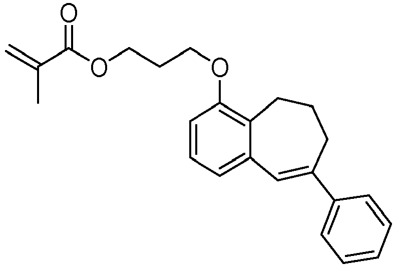 | 79 |
| **18b** | R1 | 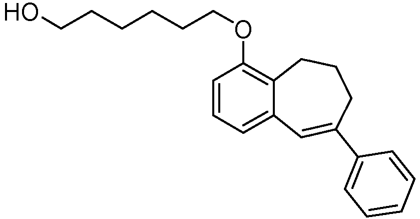 | |
| | [P] | 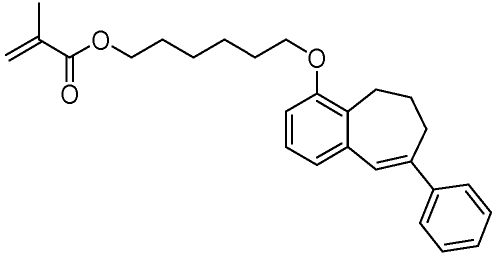 | 85 |
| **18c** | R1 | 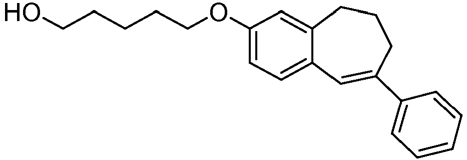 | |
| | [P] | 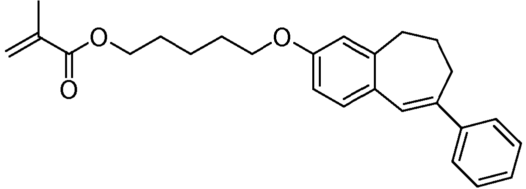 | 80 |
| **18d** | R1 | 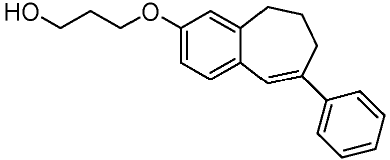 | |
| | [P] | 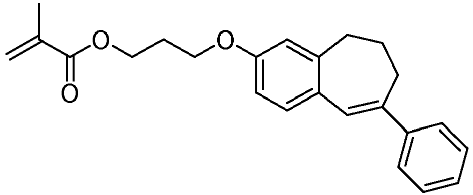 | 83 |
| **18e** | R1 | 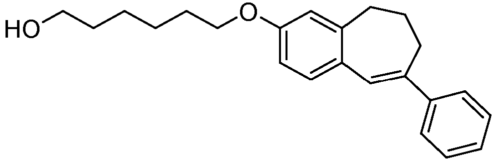 | |
| | [P] | 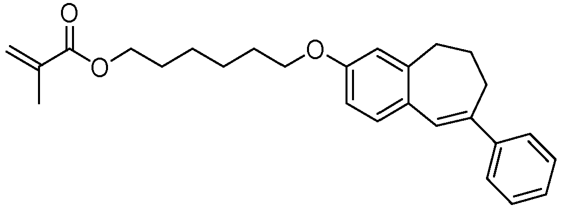 | 88 |
| **18f** | R1 | 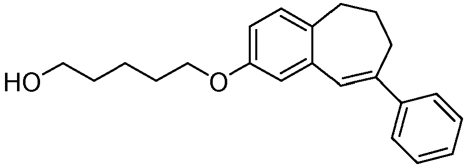 | |
| | [P] | 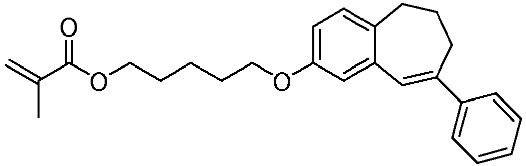 | 86 |
| **18g** | R1 | 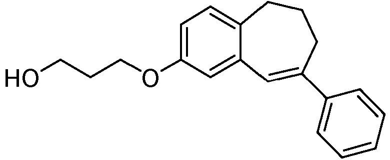 | |
| | [P] | 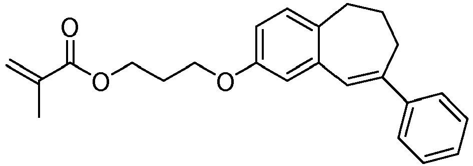 | 80 |
| **18h** | R1 | 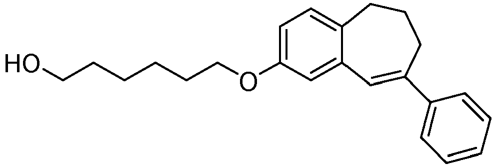 | |
| | [P] | 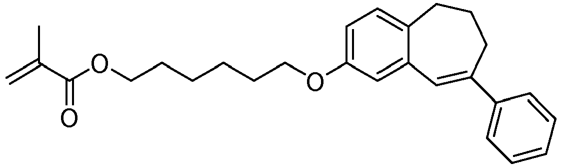 | 81 |
| **18i** | R1 | 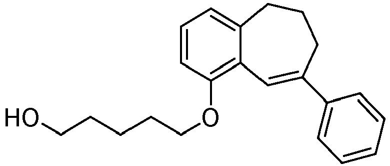 | |
| | | 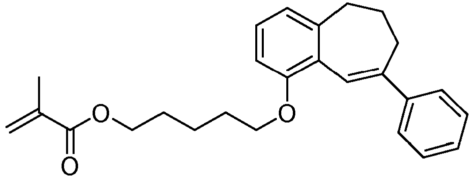 | 77 |
| **18j** | R1 | 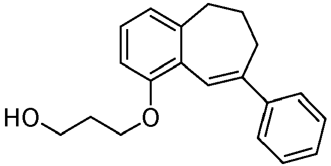 | |
| | | 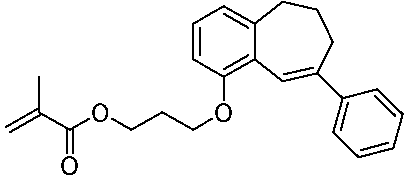 | 76 |
| **18k** | R1 | 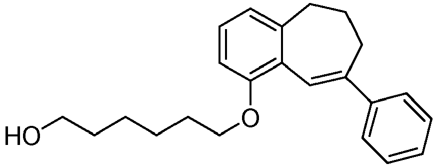 | |
| | [P] | 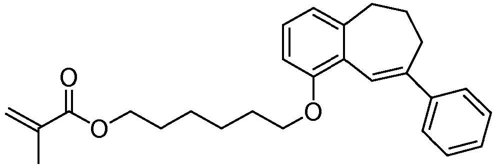 | 74 |
| **18l** | R1 | 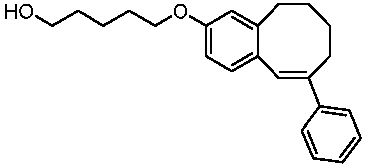 | |
| | [P] | 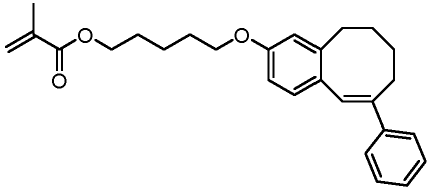 | 88 |
| **18m** | R1 | 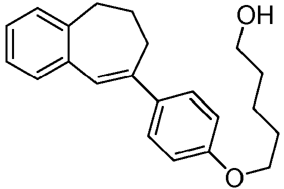 | |
| | [P] | 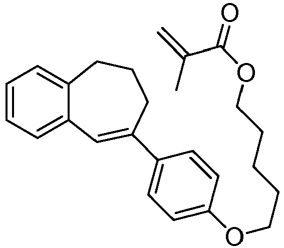 | 83 |
| **18n** | R1 | 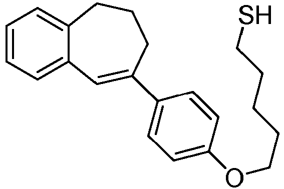 | |
| | [P] | 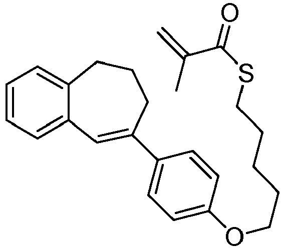 | 52 |
| **180** | R1 | 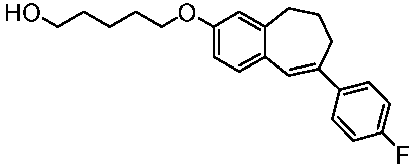 | |
| | [P] | 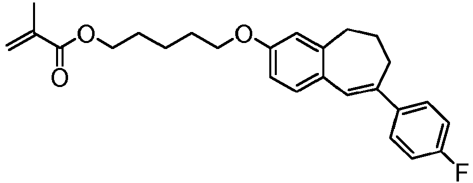 | 96 |
| **18p** | R1 | 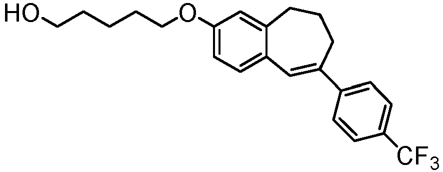 | |
| | [P] | 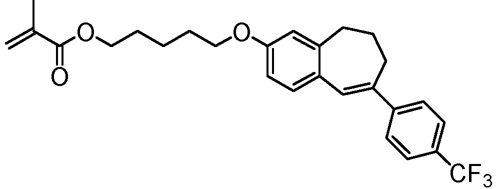 | 70 |
| **18q** | R1 | 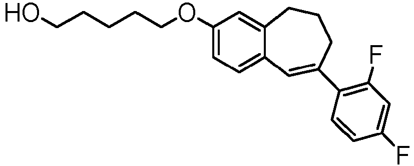 | |
| | [P] | 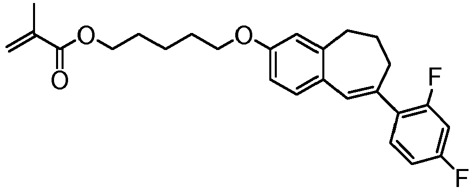 | 83 |
| **18r** | R1 | 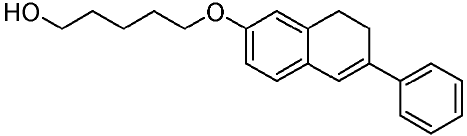 | |
| | [P] | 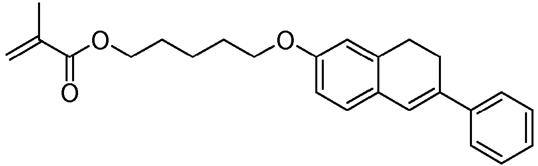 | 28 |
| **18s** | R1 | 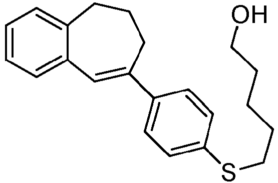 | |
| | [P] | 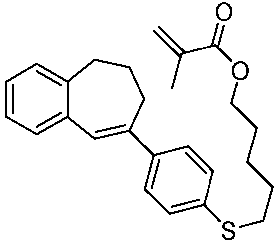 | 95 |
| **18t** | R1 | 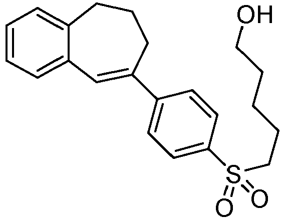 | |
| | [P] | 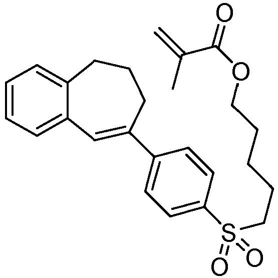 | 81 |

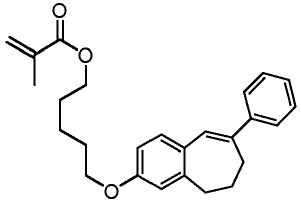

**¹H NMR** (500 MHz, Chloroform-*d*) δ 7.52 - 7.49 (m, 2H), 7.36 (t, *J* = 7.7 Hz, 2H), 7.29-7.26 (m, 1H), 7.16 - 7.13 (m, 1H), 6.75 - 6.73 (m, 3H), 6.12 (t, *J* = 1.3 Hz, 1H), 5.57 (t, *J* = 1.7 Hz, 1H), 4.20 (t, *J* = 6.6 Hz, 2H), 4.00 (t, *J =* 6.4 Hz, 2H), 2.82 - 2.79 (m, 2H), 2.67 (t, *J* = 6.7 Hz, 2H), 2.23 - 2.18 (m, 2H), 1.97 (s, 3H), 1.88 - 1.82 (m, 2H), 1.81 - 1.75 (m, 2H), 1.63 - 1.58 (m, 2H).

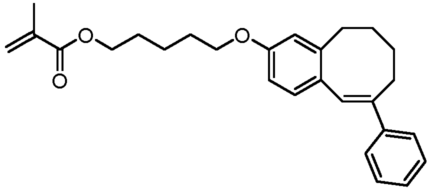

**¹H NMR** (500 MHz, Chloroform-*d*) δ 7.55 - 7.52 (m, 2H), 7.36 (dd, *J* = 8.3, 6.8 Hz, 2H), 7.31 - 7.27 (m, 1H), 7.10 (d, *J* = 8.4 Hz, 1H), 6.82 (s, 1H), 6.78 (d, *J* = 2.6 Hz, 1H), 6.74 (dd, *J* = 8.4, 2.7 Hz, 1H), 6.11 (t, *J* = 1.4 Hz, 1H), 5.56 (p, *J* = 1.6 Hz, 1H), 4.19 (t, *J* = 6.6 Hz, 2H), 4.00 (t, *J* = 6.4 Hz, 2H), 2.72 - 2.69 (m, 2H), 2.44 - 2.39 (m, 2H), 1.96 (t, *J* = 1.3 Hz, 3H), 1.88 - 1.82 (m, 2H), 1.80 - 1.72 (m, 2H), 1.63 - 1.55 (m, 4H).

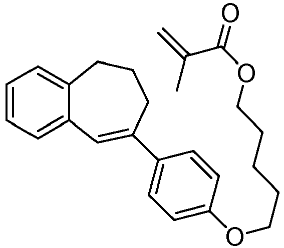

**¹H NMR** (500 MHz, Chloroform-*d*) δ 7.44 (d, *J* = 8.7 Hz, 2H), 7.25 - 7.08 (m, 4H), 6.89 (d, *J* = 8.7 Hz, 2H), 6.75 (s, 1H), 6.11 (s, 1H), 5.56 (t, *J* = 1.7 Hz, 1H), 4.19 (t, *J* = 6.6 Hz, 2H), 4.00 (t, *J* = 6.3 Hz, 2H), 2.84 - 2.75 (m, 2H), 2.61 (t, *J* = 6.8 Hz, 2H), 2.21 (p, *J* = 6.7 Hz, 2H), 1.95 (t, *J* = 1.2 Hz, 3H), 1.91 - 1.81 (m, 2H), 1.77 (m, 2H), 1.65 - 1.55 (m, 2H).

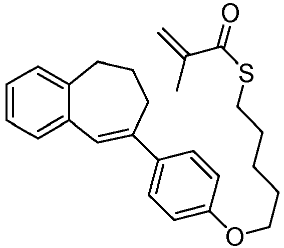

**¹H NMR** (500 MHz, Chloroform-*d*) δ 7.43 (d, *J =* 8.8 Hz, 2H), 7.23 - 7.08 (m, 4H), 6.89 (d, *J =* 8.8 Hz, 2H), 6.74 (s, 1H), 6.08 (d, *J* = 1.3 Hz, 1H), 5.57 (d, *J =* 1.6 Hz, 1H), 3.99 (t, *J* = 6.4 Hz, 2H), 2.96 (t, *J* = 7.3 Hz, 2H), 2.85 - 2.75 (m, 2H), 2.61 (t, *J* = 6.8 Hz, 2H), 2.32 - 2.12 (m, 2H), 1.98 (dd, *J* = 1.6, 1.0 Hz, 3H), 1.83 (p, *J* = 6.6 Hz, 2H), 1.73 - 1.64 (m, 2H), 1.64 - 1.55 (m, 2H).

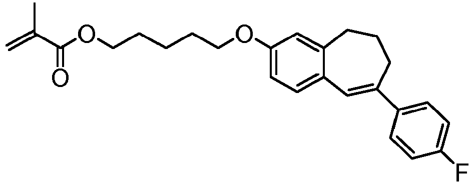

**¹H NMR** (500 MHz, Chloroform-*d*) δ 7.44 (dd, *J* = 8.7, 5.4 Hz, 2H), 7.12 (d, *J* = 8.2 HZ, 1H), 7.03 (t, *J =* 8.7 Hz, 2H), 6.72 (d, *J* = 7.4 Hz, 2H), 6.67 (s, 1H), 6.11 (t, *J =* 1.4 Hz, 1H), 5.56 (p, *J =* 1.6 Hz, 1H), 4.19 (t, *J* = 6.6 Hz, 2H), 3.99 (t, *J* = 6.3 Hz, 2H), 2.81 - 2.77 (m, 2H), 2.65 - 2.61 (m, 2H), 2.18 (p, *J* = 6.5 Hz, 2H), 1.95 (s, 3H), 1.87 - 1.81 (m, 2H), 1.80 - 1.74 (m, 2H), 1.62 - 1.58 (m, 2H).

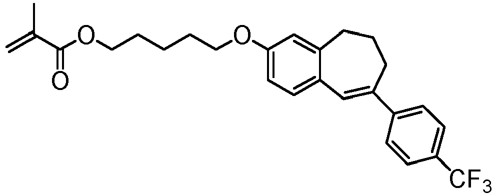

**¹H NMR** (500 MHz, Chloroform-*d*) δ 7.61 - 7.55 (m, 4H), 7.14 (d, *J* = 8.0 Hz, 1H), 6.78 (s, 1H), 6.73 (d, *J* = 7.6 Hz, 2H), 6.11 (s, 1H), 5.56 (t, *J* = 1.8 Hz, 1H), 4.19 (t, *J* = 6.6 Hz, 2H), 3.99 (t, *J* = 6.4 Hz, 2H), 2.84 - 2.78 (m, 2H), 2.66 (t, *J* = 6.7 Hz, 2H), 2.20 (p, *J* = 6.5 Hz, 2H), 1.95 (s, 3H), 1.84 (p, *J = 6.7* Hz, 2H), 1.77 (p, *J* = 6.8 Hz, 2H).

**¹⁹F NMR** (470 MHz, Chloroform-*d*) δ -62.4.

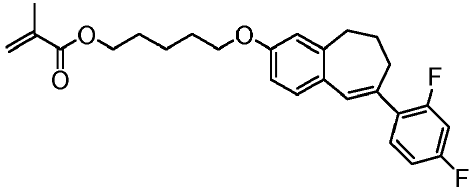

**¹H NMR** (500 MHz, Chloroform-*d*) δ 7.31 (td, *J* = 8.7, 6.6 Hz, 1H), 7.10 - 7.07 (m, 1H), 6.88 - 6.78 (m, 2H), 6.72 (d, *J* = 6.8 Hz, 2H), 6.53 (s, 1H), 6.11 (s, 1H), 5.56 (t, *J* = 1.6 Hz, 1H), 4.19 (t, *J* = 6.6 Hz, 2H), 3.99 (t, *J* = 6.3 Hz, 2H), 2.84 - 2.80 (m, 2H), 2.55 (t, *J* = 6.6 Hz, 2H), 2.17 (p, *J* = 6.5 Hz, 2H), 1.95 (s, 3H), 1.87 - 1.81 (m, 2H), 1.77 (dt, *J* = 14.4, 6.8 Hz, 2H), 1.62 - 1.54 (m, 2H).

**¹⁹F NMR** (470 MHz, Chloroform-*d*) δ -110.8 (q, *J* = 8.9 Hz), -112.7.

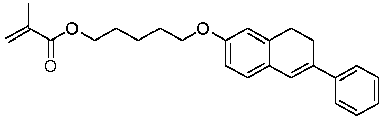

**¹H NMR** (500 MHz, Chloroform-*d*) δ 7.56 - 7.54 (m, 2H), 7.38 (dd, *J* = 8.5, 7.1 Hz, 1H), 7.29-7.27 (m, 1H), 7.07 (d, *J* = 8.2 Hz, 1H), 6.83 (d, *J* = 1.3 Hz, 1H), 6.75 - 6.71 (m, 2H), 6.13 (s, 1H), 5.58 (t, *J* = 1.7 Hz, 1H), 4.21 (t, *J =* 6.5 Hz, 2H), 4.01 (t, *J* = 6.4 Hz, 2H), 2.94 (dd, J = 9.1, 7.1 Hz, 2H), 2.76 - 2.73 (m, 2H), 1.98 (dt, *J* = 4.7, 1.3 Hz, 5H), 1.88 - 1.84 (m, 2H), 1.81 - 1.77 (m, 2H), 1.63-1.59 (m, 2H).

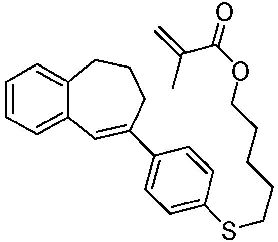

**¹H NMR** (500 MHz, Chloroform-*d*) δ 7.43 (d, *J* = 8.4 Hz, 2H), 7.31 (d, *J =* 8.4 Hz, 2H), 7.24 - 7.10 (m, 4H), 6.80 (s, 1H), 6.10 (s, 1H), 5.55 (t, *J* = 1.6 Hz, 1H), 4.15 (t, *J* = 6.6 Hz, 2H), 2.95 (t, *J* = 7.3 Hz, 2H), 2.87 - 2.75 (m, 2H), 2.62 (t, *J* = 6.7 Hz, 2H), 2.21 (dq, *J* = 10.1, 6.7 Hz, 2H), 1.95 (t, *J =* 1.3 Hz, 3H), 1.75 - 1.68 (m, 4H), 1.57 - 1.51 (m, 2H).

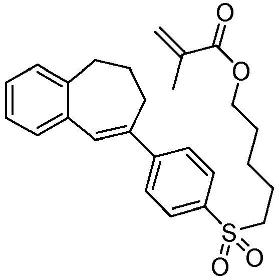

**¹H NMR** (500 MHz, Chloroform-*d*) δ 7.88 (d, *J* = 8.5 Hz, 2H), 7.67 (d, *J =* 8.5 Hz, 2H), 7.25 - 7.15 (m, 4H), 6.89 (s, 1H), 6.07 (s, 1H), 5.55 (t, *J* = 1.6 Hz, 1H), 4.12 (dd, *J =* 7.1, 5.6 Hz, 2H), 3.18 - 3.01 (m, 2H), 2.88 - 2.78 (m, 2H), 2.66 (t, *J* = 6.7 Hz, 2H), 2.24 (p, *J* = 6.6 Hz, 2H), 1.93 (t, *J* = 1.2 Hz, 3H), 1.86 - 1.74 (m, 2H), 1.68 (dt, *J* = 14.8, 6.7 Hz, 2H), 1.50 (td, *J* = 8.1, 5.7 Hz, 2H).

### Example 19:

Characterization of inventive compounds through melting points: Melting points are measured with a TA Instruments Q2000 differential scanning calorimeter during heating in the first heating run with 20 K/min from -100 °C to 200 °C in a hermetic aluminium pans.

| **Compound** | **m.p. [°C]** |
|---|---|
| 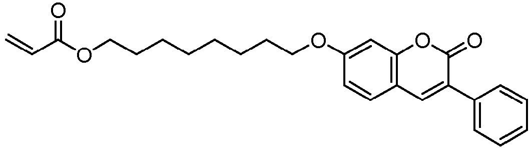 reference | **100** |
| | |
| 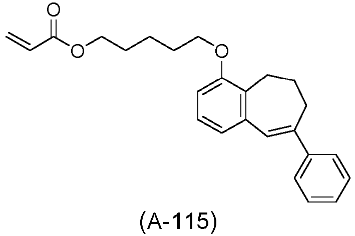 | **46** |
| 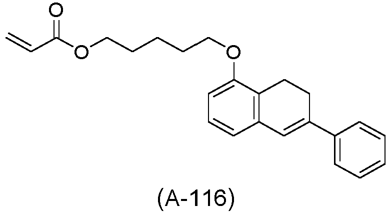 | **Not observed** |
| 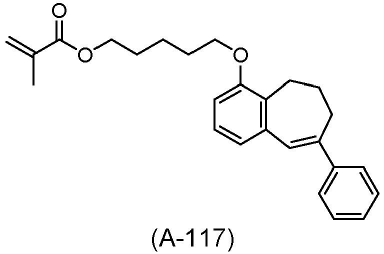 | **Not observed** |
| 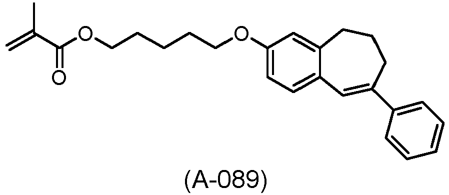 | **Not observed** |
| 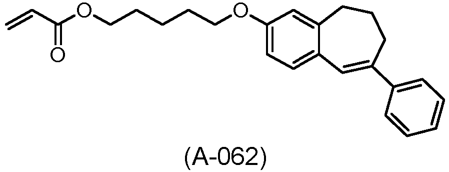 | **Not observed** |
| 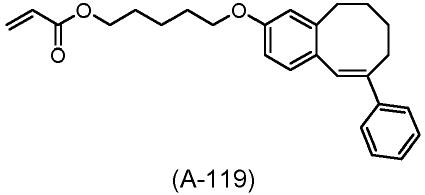 | **62** |
| 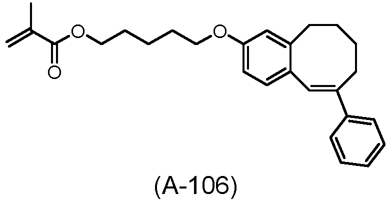 | **59** |
| 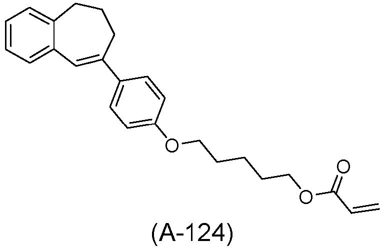 | **49** |
| 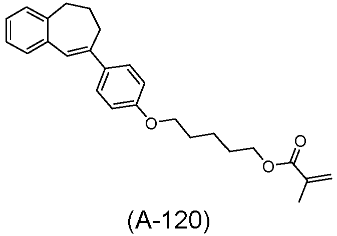 | **58** |
| 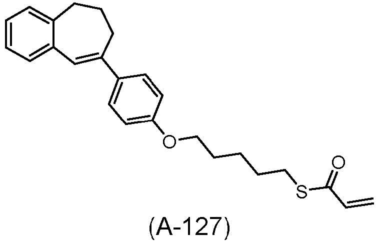 | **43** |
| 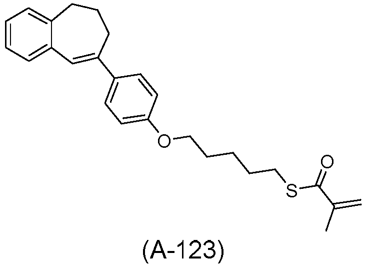 | **69** |
| 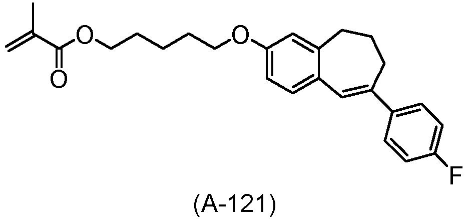 | **Not observed** |
| 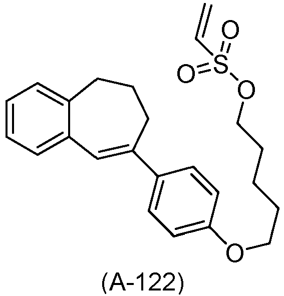 | **Not observed** |
| 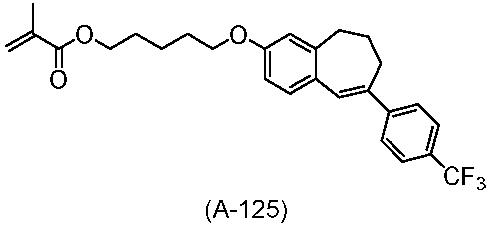 | **64** |
| 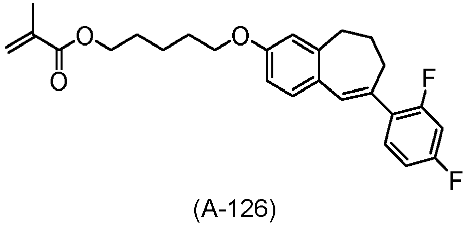 | **Not observed** |
| 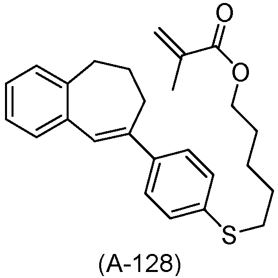 | **33** |
| 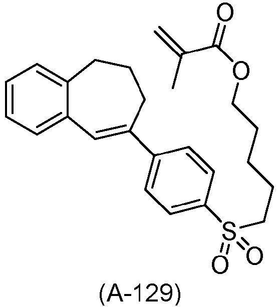 | **Not observed** |

### Examples of application:

### Example 20 - General polymerization procedure to produce bulk copolymer

For production of bulk copolymer blanks, the monomers are melted under vacuum.

These monomers in a composition as indicated in table 3 below are well mixed under stirring using gentle heat and degassed by three freeze-pump-thaw cycles. Appropriate amounts (0.02 - 0.12 equiv.) of a radical initiator are added (e. g. 1,1'-(3,3,5-trimethylcyclohexylidene)bis[2-(1,1-dimethylethyl)peroxide [Luperox^{®} 231] or 2-[(E)-2-(1-cyano-1-methylethyl)diazen-1-yl]-2-methylpropanenitrile).

Two glass plates are coated with a polyethylene sheet and a 0.5 mm thick cell is created between the polyethylene sheets using a silicone rubber gasket. The coated faces of the glass sheets are clipped together using spring clips with a syringe needle being placed between the gasket and the polyethylene sheets. The cavity is then filled with the above formulation through the needle using a gastight syringe. Once the cavity is filled the syringe needle is removed, a final clip is used to seal the mould and the assembly is placed in an oven. The polymerization temperature is between 60 °C and 180 °C and the individual polymerization conditions are choosen for the respective initiators, which can be extracted for the skilled person from the formulation mixture. The moulds are allowed to cool to room temperature before the film is removed from the mould.

**Table 3: Compositions - Amount of compounds is given in mol-%:**

| **Example** | **Monomer** | **mol% monomer** | **mol% *n*-BuAc** | **mol% EGDMA** | **mol% HEMA** | **mol% UV-Abs.** | **mol% EtMAc** |
|---|---|---|---|---|---|---|---|
| 1 | A-021 | 23.1 | 74.6 | 2.3 | 0 | 0 | 0 |
| 2 | A-115 | 89.2 | 0 | 8.9 | 0 | 0 | 0 |
| 3 | A-116 | 89.2 | 0 | 8.9 | 0 | 0 | 0 |
| 4 | A-116 | 24.6 | 69.5 | 4.9 | 0 | 0 | 0 |
| 5 | A-115 | 23.9 | 70.3 | 4.8 | 0 | 0 | 0 |
| 6 | A-115 | 48.7 | 0 | 2.4 | 18.0 | 0 | 25.9 |
| 7 | A-115 | 33.3 | 9.8 | 1.7 | 15.4 | 0.8 | 35.1 |
| 8 | A-117 | 33.9 | 43.3 | 1.7 | 16.3 | 0.8 | 0 |
| 9 | A-117 | 34.3 | 43.9 | 3.4 | 16.5 | 0.8 | 0 |
| 10 | A-117 | 27.2 | 51.0 | 0.9 | 14.9 | 0.8 | 0 |
| 11 | A-038 | 29.0 | 68.8 | 5.3 | 0 | 1.2 | 0 |
| 12 | A-062 | 39.0 | 37.4*^{a}* | 3.8*^{b}* | 18.1 | 0.9 | 0 |
| 13 | A-062 | 38.0 | 38.4*^{c}* | 3.8*^{b}* | 17.8 | 0.9 | 0 |
| 14 | A-089 | 38.9 | 37.4*^{a}* | 3.8*^{b}* | 18.1 | 0.9 | 0 |
| 15 | A-062 | 9.0 | 67.6*^{a}* | 3.8*^{b}* | 17.9 | 0.9 | 0 |
| 16 | A-089 | 20.3 | 58.2*^{a}* | 3.5*^{b}* | 16.5 | 0.8 | 0 |
| 17 | A-062 | 20.4 | 58.1*^{a}* | 3.5*^{b}* | 16.5 | 0.8 | 0 |
| 18 | A-119 | 92.0 | 0 | 6.6 | 0 | 0 | 0 |
| 19 | A-106 | 91.0 | 0 | 8.7*^{d}* | 0 | 0 | 0 |
| 20 | A-120 | 91.1 | 0 | 7.9*^{d}* | 0 | 0 | 0 |
| 21 | A-120 | 35.8 | 59.2*^{e}* | 4.0*^{f}* | 0 | 0 | 0 |
| 22 | A-121 | 35.0 | 60.0*^{e}* | 4.1*^{f}* | 0 | 0 | 0 |
| 23 | A-122 | 34.6 | 60.4*^{e}* | 4.1*^{f}* | 0 | 0 | 0 |
| 24 | A-123 | 34.9 | 60.0*^{e}* | 4.1*^{f}* | 0 | 0 | 0 |
| 25 | A-126 | 33.7 | 61.2*^{e}* | 5.9*^{f}* | 0 | 0 | 0 |
| 26 | A-128 | 89.8 | 0 | 9.0 | 0 | 0 | 0 |
| 27 | A-129 | 89.1 | 0 | 9.7 | 0 | 0 | 0 |

| | | | | | | | |
|---|---|---|---|---|---|---|---|
| Table 3: *n*-BuAc = *n*-butylacrylate; EGDMA = Ethylene glycol dimethacrylate; HEMA = Hydroxyethylmethacrylate; EtMAc = Ethylmethacrylate. *^{a}* 2,2,3,4,4,4-hexafluorobutyl acrylate is used instead of *n*-butylacrylate. *^{b}* Polyethyleneglycol dimethacrylate (Mₙ 750) is used instead of EGDMA. *^{c}* 8-methylnonyl methacrylate is used instead of EGDMA. *^{d}* oxybis(ethane-2,1-diyl) bis(2-methylacrylate) is used instead of EGDMA. *^{e}* iso-decyl methacrylate is used instead of *n*-butylacrylate. *^{f}* Polyethyleneglycol diacrylate (Mₙ 250) is used instead of EGDMA. | | | | | | | |

The compositions shown in table 3 are formulated in the same way as described in Example 20 mixing all compounds together while stirring. A heating bath for the stirring is used, where necessary.

Refractive index change is induced by irradiation at 340 - 365 nm. The refractive indices (n) of the polymer films and blanks at 590 nm are measured on Schmidt+Haensch AR12 before and after irradiation. The following table shows the refractive indice after irradiation as well as the change in refractive index (max. Δn).

The phase transition temperatures are determined with a TA Instruments Q2000 differential scanning calorimeter during heating in the second heating run with 20 K/min from -100 °C to 200 °C in a hermetic aluminium pans.

**Table 4: Results after irradiation and refractive index change**

| **Example** | **T*_{g}* [°C]** | **n_{D, 35 °C}** | **Δn** | **Absorption maximum [nm]** |
|---|---|---|---|---|
| 1 | - | 1.554 | 0.009 | - |
| 2 | 7.85 | 1.601 | 0.024 | - |
| 3 | -3.34 | 1.603 | 0.016 | - |
| 4 | -15.55 | 1.547 | 0.004 | - |
| 5 | -9.24 | 1.540 | 0.001 | - |
| 6 | - | 1.576 | 0.026 | - |
| 7 | - | 1.564 | 0.020 | - |
| 8 | 15.03 | 1.562 | 0.026 | 287 |
| 9 | - | 1.564 | 0.029 | - |
| 10 | | 1.552 | 0.018 | - |
| 11 | 30.14 | 1.587 | 0.042 | - |
| 12 | 3.37 | 1.543 | 0.026 | - |
| 13 | -4.32 | 1.549 | 0.015 | - |
| 14 | 17.39 | 1.520 | 0.021 | - |
| 15 | 3.23 | 1.477 | 0.011 | - |
| 16 | 10.40 | 1.484 | 0.019 | - |
| 17 | 3.82 | 1.502 | 0.014 | - |
| 18 | 33.13 | 1.601 | 0.007 | 281 |
| 19 | 39.58 | 1.591 | 0.018 | 282 |
| 20 | 43.12 | 1.613 | 0.006 | 285 |
| 21 | 7.88 | 1.549 | 0.015 | 308 |
| 22 | 5.59 | 1.543 | 0.013 | - |
| 23 | -30.02 | - | - | - |
| 24 | 1.87 | 1.565 | 0.015 | - |
| 25 | -0.05 | 1.528 | 0.007 | 319 |
| 26 | 30.12 | 1.636 | 0.021 | - |
| 27 | 46.33 | 1.608 | 0.008 | - |

The results of the copolymer examples 2, 3, 6-17, 19, 21, 22, 24, 26 show a significant refractive index change after irradiation. Also the glass transition temperature of most of the respective polymers show values below room temperature documenting the advantages of the compounds.

## Claims

1. An ophthalmic device or a precursor article for an ophthalmic device comprising at least one polymerized compound of formula (I) 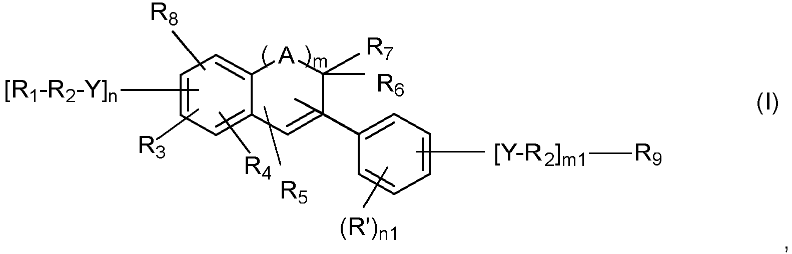 wherein
A is the same or different at each instance and is CH₂, CHR₀ or C(R₀)₂;
m is 0, 1, 2 or 3;
Y is independently of each other O, S, SO₂, or a bond;
n is 0 or 1;
m1 is 0 or 1;
n+m1 is 1;
n1 is 0, 1, 2, 3 or 4;
R₀ is at each occurrence independently selected from the group consisting of F, a linear or branched alkyl group having 1 to 4 C atoms and a linear or branched partially or fully fluorinated alkyl group having 1 to 4 C atoms;
R' is at each occurrence independently selected from the group consisting of F, a linear or branched, non-halogenated, partially or completely halogenated alkyl group having 1 to 20 C atoms, a non-halogenated, partially or completely halogenated cycloalkyl group having 3 to 6 C atoms, a linear or branched, non-halogenated, partially or completely halogenated alkoxy group having 1 to 20 C atoms and a linear or branched, non-halogenated, partially or completely halogenated thioalkyl group having 1 to 20 C atoms;
R₁ is a trialkoxysilyl group or a dialkoxyalkylsilyl group where the alkyl and/or alkoxy groups are each independently linear or branched having 1 to 6 C atoms, or a silyl group of formula (1), (2) or (3) or a polymerizable group of formula (4), 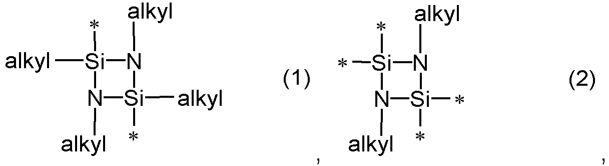 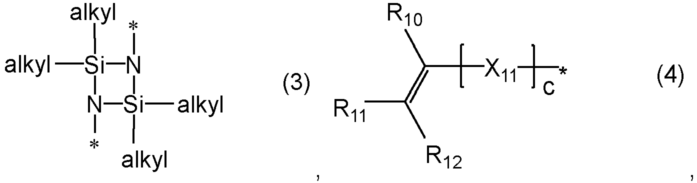
where alkyl means at each occurrence independently of each other a linear or branched alkyl group having 1 to 6 C atoms and the asterisk "*" denotes at each occurrence independently of each other a linkage to the linker [-R₂-Y]ₙ or [Y-R₂-]ₘ₁;
and wherein
X₁₁ is selected from the group consisting of O, S, O-SO₂, SO₂-O, C(=O), OC(=O), C(=O)O, S(C=O) and (C=O)S, R₁₀, R₁₁, R₁₂ are at each occurrence independently of each other selected from the group consisting of H, F, a linear or branched, non-fluorinated, partially or completely fluorinated alkyl group having 1 to 20 C atoms and aryl with 6 to 14 C atoms and
c is 0 or 1;
-R₂- is -(C(R)₂)ₒ-, or =(C(R)₂)ₚ-X₈-(C(R)₂)_{q}-(X₉)ₛ-(C(R)₂)ᵣ-(X₁₀)ₜ-(C(R)₂)ᵤ-;
R is at each occurrence independently selected from the group consisting of H, F, a linear or branched alkyl group having 1 to 4 C atoms or a linear or branched partially or fully fluorinated alkyl group having 1 to 4 C atoms;
o is selected from the group consisting of 0 to 20,
X₈, X₉, X₁₀ are at each occurrence independently O, S, SO₂, or NR₀,
s, t is 0 or 1,
p, q are at each occurrence independently selected from the group consisting of 1 to 10,
r, u are at each occurrence independently selected from the group consisting of 0 to 10, wherein the overall number of atoms for -(C(R)₂)ₚ-X₈-(C(R)₂)_{q}-(X₉)ₛ-(C(R)₂)ᵣ-(X₁₀)ₜ-(C(R)₂)ᵤ- is up to 20 atoms,
R₃, R₄, R₅, R₆, R₇, R₈ are at each occurrence independently selected from the group consisting of H, F, Cl, Br, CN, SO₂CF₃, a linear or branched, non-halogenated, partially or completely halogenated alkyl group having 1 to 20 C atoms, a non-halogenated, partially or completely halogenated cycloalkyl group having 3 to 6 C atoms, a linear or branched, non-halogenated, partially or completely halogenated alkoxy group having 1 to 20 C atoms and a linear or branched, non-halogenated and partially or completely halogenated thioalkyl group having 1 to 20 C atoms;
R₉ is H or R' in case m1 is 0 and
R₉ is R₁ in case m1 is 1.

2. The ophthalmic device or the precursor article for an ophthalmic device according to claim 1 wherein in polymerized compounds of formula (I) n is 1 and m1 is 0 said compounds being of formula (I') 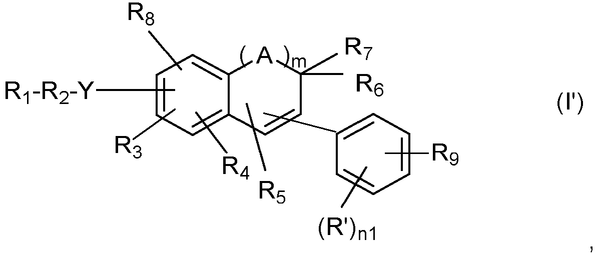 wherein R₁, -R₂-, Y, R₃, R₄, R₅, R₆, R₇, R₈, R₉, A, m, R', and n1 have a meaning as indicated in claim 1.

3. The ophthalmic device or the precursor article for an ophthalmic device according to claim 1 wherein in polymerized compounds of formula (I) n is 0 and m1 is 1 said compounds being of formula (I") 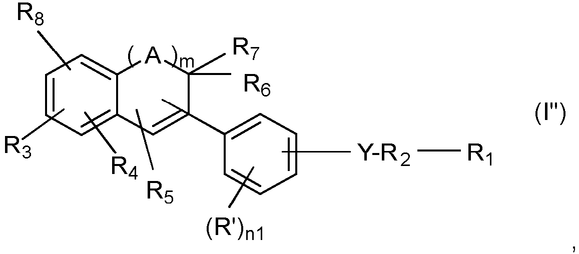 wherein R₁, -R₂-, Y, R₃, R₄, R₅, R₆, R₇, R₈, A, m, R' and n1 have a meaning as indicated in claim 1.

4. The ophthalmic device or the precursor article for an ophthalmic device according to one or more of claims 1 to 3 comprising an oligomer, polymer or copolymer comprising a constitutional unit M⁰ based on formulae (I), (I') or (I") where R₁ on each occurrence is polymerized, R₁ thus forms the regioregular, alternated, regiorandom, statistical, block or random oligomer or polymer backbone or is part of the copolymer backbone.

5. The ophthalmic device or the precursor article for an ophthalmic device according to one or more of claims 1 to 4 where said polymerized group R₁ is of formulae (1-p), (2-p), (3-p) or (4-p) 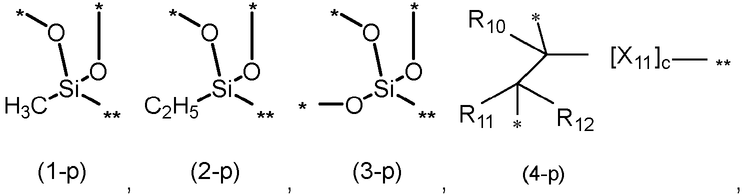 where the asterisk "*" within formulae (1-p) to (4-p) denotes a linkage to the adjacent repeating unit in the polymer chain or oligomer chain or to a terminal end group, the asterisk "**" within formulae (1-p) to (4-p) denotes the linkage to the remainder of formula (I), (I') or (I") and R₁₀, R₁₁, R₁₂, X₁₁ and c has a meaning according to claim 1.

6. The ophthalmic device or the precursor article for an ophthalmic device according to one or more of claims 1 to 5 wherein the constitutional unit M⁰ is of formulae (M⁰-I'-a), (M⁰-I'-b), (M⁰-I'-c), (M⁰-I'-d) or (M⁰-I"), 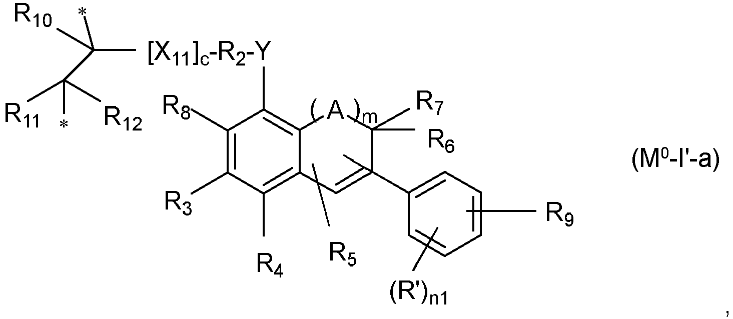 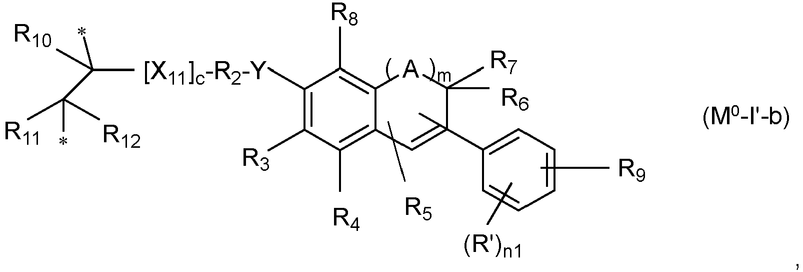 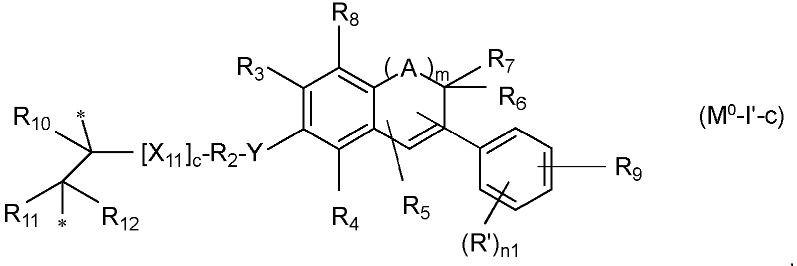 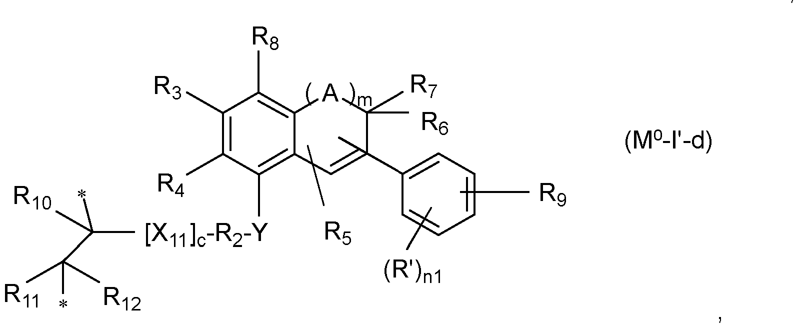 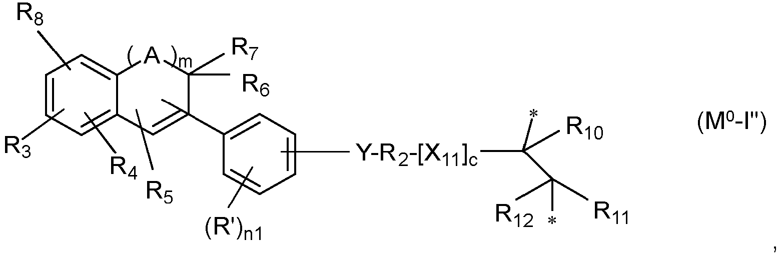 where -R₂-, Y, R₃, R₄, R₅, R₆, R₇, R₈, A, m, R', n1, X₁₁, R₁₀, R₁₁, R₁₂ and c have a meaning according to claim 1 and R⁹ is H or R' and where the asterisk "*" denotes at each occurrence a linkage to the adjacent repeating unit in the polymer chain or oligomer chain or to a terminal end group.

7. The ophthalmic device or the precursor article for an ophthalmic device according to one or more of claims 1 to 6 comprising beside of the at least one polymerized compound of formulae (I), (I') or (I") or the constitutional unit M⁰ of formulae (M⁰-I'-a), (M⁰-I'-b), (M⁰-I'-c), (M⁰-I'-d) or (M⁰-I") at least one further polymerized monomer selected from the group consisting of styrene, ethoxyethyl methacrylate (EOEMA), methyl methacrylate (MMA), methyl acrylate, n-alkyl acrylates (the n-alkyl group comprising 2-20 C-atoms), n-alkyl methacrylates (the n-alkyl group comprising 2-20 C-atoms), ethoxyethoxy ethylacrylate (EEEA), 2-hydroxyethyl methacrylate (HEMA), tetrahydrofuryl methacrylate (THFMA), glycidylmethacrylate (GMA), 16-hydroxyhexadecyl acrylate, 16-hydroxyhexadecyl methacrylate, 18-hydroxyoctadecyl acrylate, 18-hydroxyoctadecyl methacrylate, 2-phenoxyethyl acrylate (EGPEA), Bisphenol A diacrylate-1 EO/Phenol (BPADA), 2-[3'-2'H-benzotriazol- 2'-yl)-4'-hydroxyphenyl]ethyl methacrylate (BTPEM) or ehtyleneglycoldimethacrylate.

8. The ophthalmic device or the precursor article for an ophthalmic device according to claim 7 wherein the at least one further polymerized monomer is selected from methyl methacrylate, 2-hydroxyethyl methacrylate, 2-phenoxyethyl acrylate, ethoxyethoxy ethylacrylate, 8-methylnonyl methacrylate, n-butyl methacrylate, 2-ethyl hexylmethacrylate or a mixture thereof.

9. The ophthalmic device or the precursor article for an ophthalmic device according to one or more of claims 1 to 8 wherein -R₂- is at each occurrence independently -(C(R)₂)ₒ- and R and o have a meaning as indicated in claim 1.

10. The ophthalmic device or the precursor article for an ophthalmic device according to one or more of claims 1 to 9 wherein A is independently at each occurrence CH₂.

11. The ophthalmic device or the precursor article for an ophthalmic device according to one or more of claims 1 to 10 wherein polymerized R₁ is at each occurrence independently derived from an acryl or methacryl radical.

12. The precursor article for an ophthalmic device according to one or more of claims 1 to 11 wherein said precursor article is a blank which may be transformed into an eye implant, preferably an intraocular lens.

13. Process of forming an ophthalmic device or a precursor article for an ophthalmic device according to one or more of claims 1 to 12, said process comprising the steps of
- providing a composition comprising at least one compound of formulae (I), (I') or (I") as described in one or more of claims 1 to 3, 9 and 10 and/or an oligomer or polymer as described in one or more of claims 4 to 8 and 11 but having at least one reactive group left for polymerization and optionally further monomers different from compounds of formulae (I), (I') or (I") and/or crosslinking agents and/or UV absorbers and/or radical initiators;
- subsequently forming the ophthalmic device or precursor article of said composition.

14. Process of changing the optical properties of an ophthalmic device or a precursor article for an ophthalmic device according to one or more of claims 1 to 12, said process comprising the steps of
- providing an ophthalmic device or a precursor article according to claim 13, and
- subsequently exposing said ophthalmic device or precursor article to irradiation having a wavelength of at least 200 nm and at most 1500 nm.

15. Oligomer, polymer or copolymer comprising at least one polymerized compound of formula (I) as described in claim 1 where oligomers, polymers and copolymers of 2-(4-vinylphenyl)indene and 2-[4-(2-propen-1-yl)phenyl-1H-indene are excluded.

16. Polymer according to claim 15 comprising beside of the polymerized compounds of formula (I) at least one further polymerized monomer selected from the group consisting of styrene, ethoxyethyl methacrylate (EOEMA), methyl methacrylate (MMA), methyl acrylate, n-alkyl acrylates (the n-alkyl group comprising 2-20 C-atoms), n-alkyl methacrylates (the n-alkyl group comprising 2-20 C-atoms), ethoxyethoxy ethylacrylate (EEEA), 2-hydroxyethyl methacrylate (HEMA), tetrahydrofuryl methacrylate (THFMA), glycidylmethacrylate (GMA), 16-hydroxyhexadecyl acrylate, 16-hydroxyhexadecyl methacrylate, 18-hydroxyoctadecyl acrylate, 18-hydroxyoctadecyl methacrylate, 2-phenoxyethyl acrylate (EGPEA), Bisphenol A diacrylate-1 EO/Phenol (BPADA), 2-[3'-2'H-benzotriazol- 2'-yl)-4'-hydroxyphenyl]ethyl methacrylate (BTPEM) or ehtyleneglycoldimethacrylate.

17. Composition for polymerization comprising at least one compound of formulae (I), (I') or (I") as described in one or more of claims 1 to 3, 9 and 10 and/or an oligomer or polymer as described in one or more of claims 4 to 8 and 11 but having at least one reactive group left for polymerization excluding 2-(vinylphenyl)indene, 2-[4-(2-propen-1-yl)phenyl-1H-indene and any oligomer, polymer or copolymer derived from 2-(vinylphenyl)indene and 2-[4-(2-propen-1-yl)phenyl-1H-indene and/or a crosslinking agent and/or a UV absorber and/or a radical initiator and optionally further monomers different from compounds of formulae (I), (I') or (I").

18. Compounds of formula (I) 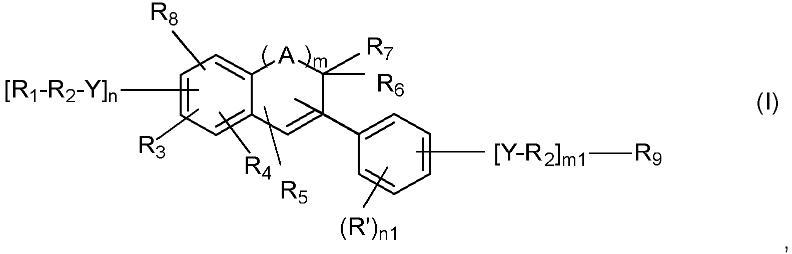 wherein
A is the same or different at each instance and is CH₂, CHR₀ or C(R₀)₂;
m is 0, 1, 2 or 3;
Y is independently of each other O, S, SO₂, or a bond;
n is 0 or 1;
m1 is 0 or 1;
n+m1 is 1;
n1 is 0, 1, 2, 3 or 4;
R₀ is at each occurrence independently selected from the group consisting of F, a linear or branched alkyl group having 1 to 4 C atoms and a linear or branched partially or fully fluorinated alkyl group having 1 to 4 C atoms;
R' is at each occurrence independently selected from the group consisting of F, a linear or branched, non-halogenated, partially or completely halogenated alkyl group having 1 to 20 C atoms, a non-halogenated, partially or completely halogenated cycloalkyl group having 3 to 6 C atoms, a linear or branched, non-halogenated, partially or completely halogenated alkoxy group having 1 to 20 C atoms and a linear or branched, non-halogenated, partially or completely halogenated thioalkyl group having 1 to 20 C atoms;
R₁ is a trialkoxysilyl group or a dialkoxyalkylsilyl group where the alkyl and/or alkoxy groups are each independently linear or branched having 1 to 6 C atoms, or a silyl group of formula (1), (2) or (3) or a polymerizable group of formula (4), 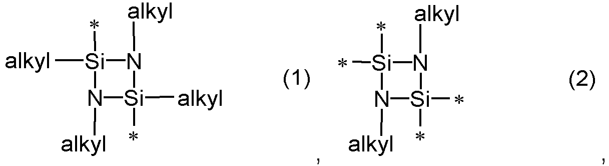 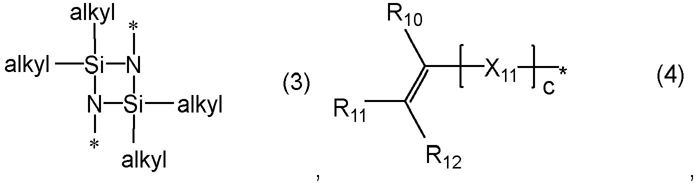
where alkyl means at each occurrence independently of each other a linear or branched alkyl group having 1 to 6 C atoms and the asterisk "*" denotes at each occurrence independently of each other a linkage to the linker [-R₂-Y]ₙ or [Y-R₂-]ₘ₁;
and wherein
X₁₁ is selected from the group consisting of O, S, O-SO₂, SO₂-O, C(=O), OC(=O), C(=O)O, S(C=O) and (C=O)S , R₁₀, R₁₁, R₁₂ are at each occurrence independently of each other selected from the group consisting of H, F, a linear or branched, non-fluorinated, partially or completely fluorinated alkyl group having 1 to 20 C atoms and aryl with 6 to 14 C atoms and
c is 0 or 1;
-R₂- is -(C(R)₂)ₒ-, or -(C(R)₂)ₚ-X₈-(C(R)₂)_{q}-(X₉)ₛ-(C(R)₂)ᵣ-(X₁₀)ₜ-(C(R)₂)ᵤ-;
R is at each occurrence independently selected from the group consisting of H, F, a linear or branched alkyl group having 1 to 4 C atoms or a linear or branched partially or fully fluorinated alkyl group having 1 to 4 C atoms;
o is selected from the group consisting of 0 to 20;
X₈, X₉, X₁₀ are at each occurrence independently O, S, SO₂, or NR₀;
s, t is 0 or 1;
p, q are at each occurrence independently selected from the group consisting of 1 to 10;
r, u are at each occurrence independently selected from the group consisting of 0 to 10, wherein the overall number of atoms for - (C(R)₂)ₚ-X₈-(C(R)₂)_{q}-(X₉)ₛ-(C(R)₂)ᵣ-(X₁₀)ₜ-(C(R)₂)ᵤ- is up to 20 atoms;
R₃, R₄, R₅, R₆, R₇, R₈ are at each occurrence independently selected from the group consisting of H, F, Cl, Br, CN, SO₂CF₃, a linear or branched, non-halogenated, partially or completely halogenated alkyl group having 1 to 20 C atoms, a non-halogenated, partially or completely halogenated cycloalkyl group having 3 to 6 C atoms, a linear or branched, non-halogenated, partially or completely halogenated alkoxy group having 1 to 20 C atoms and a linear or branched, non-halogenated and partially or completely halogenated thioalkyl group having 1 to 20 C atoms;
R₉ is H or R' in case m1 is 0 and
R₉ is R₁ in case m1 is 1,
provided that in case of m is 0, m1 is 1 and Y is a bond, o is selected from the group consisting of 3 to 20, and
provided that in case of m is 0 and m1 is 1, two substituents from the group of R₃, R₄ and R₈ are not simultaneously F, and
provided that in case of m is 0, m1 is 1 and Y is a bond or O, R' is on each occurrence not F; and
provided that in case of m is 0, R₆ and R₇ are on each occurrence not a non-halogenated, partially or completely halogenated cycloalkyl group having 3 to 6 C atoms; and
provided that in case of m is 1, n is 1 and Y is a bond, o is selected from 1 to 20; and
provided that in case of m is 2, m1 is 1 and Y is a bond, o is selected from the group consisting of 3 to 20; and
provided that in case of m is 2, m1 is 1 and Y is O, o is selected from the group consisting of 1 to 20; and
provided that in case of m is 2, n is 1 and Y is a bond, o is selected from the group consisting of 3 to 20; and
provided that in case of m is 2, n is 1 and Y is O, o is selected from the group consisting of 1 to 20.

## Patentansprüche

1. Ophthalmische Vorrichtung oder Vorläuferartikel für eine ophthalmische Vorrichtung, umfassend mindestens eine polymerisierte Verbindung der Formel (I) 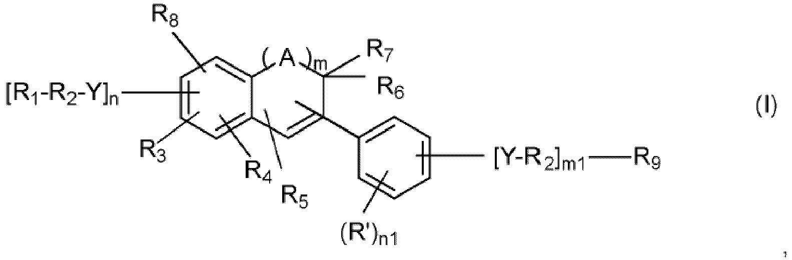 wobei
A bei jedem Auftreten gleich oder verschieden ist und CH₂, CHR₀ oder C(R₀)₂ ist;
m 0, 1, 2 oder 3 ist;
Y unabhängig voneinander O, S, SO₂ oder eine Bindung ist;
n 0 oder 1 ist;
m1 0 oder 1 ist;
n+m1 1 ist;
n1 0, 1, 2, 3 oder 4 ist;
R₀ bei jedem Auftreten unabhängig ausgewählt ist aus der Gruppe bestehend aus F, einer linearen oder verzweigten Alkylgruppe mit 1 bis 4 C-Atomen und einer linearen oder verzweigten teilweise oder vollständig fluorierten Alkylgruppe mit 1 bis 4 C-Atomen;
R' bei jedem Auftreten unabhängig ausgewählt ist aus der Gruppe bestehend aus F, einer linearen oder verzweigten, nichthalogenierten, teilweise oder vollständig halogenierten Alkylgruppe mit 1 bis 20 C-Atomen, einer nichthalogenierten, teilweise oder vollständig halogenierten Cycloalkylgruppe mit 3 bis 6 C-Atomen, einer linearen oder verzweigten, nichthalogenierten, teilweise oder vollständig halogenierten Alkoxygruppe mit 1 bis 20 C-Atomen und einer linearen oder verzweigten, nichthalogenierten, teilweise oder vollständig halogenierten Thioalkylgruppe mit 1 bis 20 C-Atomen;
R₁ eine Trialkoxysilylgruppe oder eine Dialkoxyalkylsilylgruppe, wobei die Alkyl- und/oder Alkoxygruppen jeweils unabhängig voneinander linear oder verzweigt sind und 1 bis 6 C-Atome aufweisen, oder eine Silylgruppe der Formel (1), (2) oder (3) oder eine polymerisierbare Gruppe der Formel (4) ist, 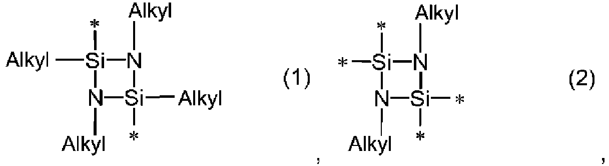 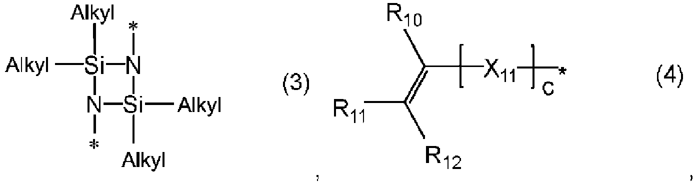
wobei Alkyl bei jedem Auftreten unabhängig voneinander eine lineare oder verzweigte Alkylgruppe mit 1 bis 6 C-Atomen bedeutet und der Stern "*" bei jedem Auftreten unabhängig voneinander eine Verknüpfung mit dem Linker [-R₂-Y]ₙ oder [Y-R₂-]ₘ₁ bezeichnet;
und wobei
X₁₁ ausgewählt ist aus der Gruppe bestehend aus O, S, O-SO₂, SO₂-O, C(=O), OC(=O), C(=O)O, S(C=O) und (C=O)S,
R₁₀, R₁₁, R₁₂ bei jedem Auftreten unabhängig voneinander ausgewählt sind aus der Gruppe bestehend aus H, F, einer linearen oder verzweigten, nichtfluorierten, teilweise oder vollständig fluorierten Alkylgruppe mit 1 bis 20 C-Atomen und Aryl mit 6 bis 14 C-Atomen
und
c 0 oder 1 ist;
-R₂- -(C(R)₂)ₒ- oder -(C(R)₂)ₚ-X₈-(C(R)₂)_{q}-(X₉)ₛ-(C(R)₂)ᵣ-(X₁₀)ₜ-(C(R)₂)ᵤ-ist;
R bei jedem Auftreten unabhängig ausgewählt ist aus der Gruppe bestehend aus H, F, einer linearen oder verzweigten Alkylgruppe mit 1 bis 4 C-Atomen oder einer linearen oder verzweigten teilweise oder vollständig fluorierten Alkylgruppe mit 1 bis 4 C-Atomen;
o ausgewählt ist aus der Gruppe bestehend aus 0 bis 20,
X₈, X₉, X₁₀ bei jedem Auftreten unabhängig voneinander O, S, SO₂ oder NR₀ sind,
s, t 0 oder 1 ist,
p, q bei jedem Auftreten unabhängig voneinander ausgewählt sind aus der Gruppe bestehend aus 1 bis 10,
r, u bei jedem Auftreten unabhängig voneinander ausgewählt sind aus der Gruppe bestehend aus 0 bis 10, wobei die Gesamtzahl der Atome für -(C(R)₂)ₚ-X₈-(C(R)₂)_{q}-(X₉)ₛ-(C(R)₂)ᵣ-(X₁₀)ₜ-(C(R)₂)ᵤ- bis zu 20 Atome beträgt,
R₃, R₄, R₅, R₆, R₇, R₈ bei jedem Auftreten unabhängig ausgewählt sind aus der Gruppe bestehend aus H, F, Cl, Br, CN, SO₂CF₃, einer linearen oder verzweigten, nichthalogenierten, teilweise oder vollständig halogenierten Alkylgruppe mit 1 bis 20 C-Atomen, einer nichthalogenierten, teilweise oder vollständig halogenierten Cycloalkylgruppe mit 3 bis 6 C-Atomen, einer linearen oder verzweigten, nichthalogenierten, teilweise oder vollständig halogenierten Alkoxygruppe mit 1 bis 20 C-Atomen und einer linearen oder verzweigten, nichthalogenierten und teilweise oder vollständig halogenierten Thioalkylgruppe mit 1 bis 20 C-Atomen;
R₉ H oder R' ist, wenn m1 0 ist, und
R₉ R₁ ist, wenn m1 1 ist.

2. Ophthalmische Vorrichtung oder Vorläuferartikel für eine ophthalmische Vorrichtung nach Anspruch 1, wobei in polymerisierten Verbindungen der Formel (I) n 1 ist und m1 0 ist und die Verbindungen die Formel (I') aufweisen 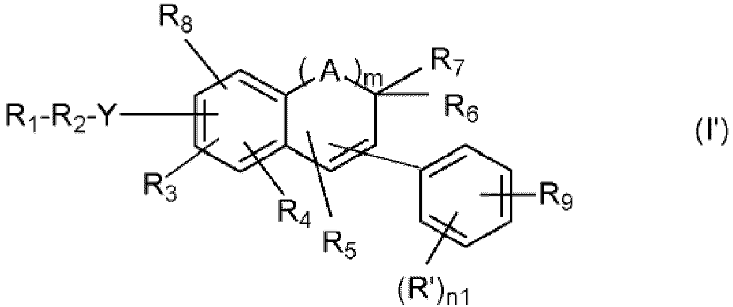 wobei R₁, -R₂-, Y, R₃, R₄, R₅, R₆, R₇, R₈, R₉, A, m, R' und n1 eine Bedeutung wie in Anspruch 1 angegeben haben.

3. Ophthalmische Vorrichtung oder Vorläuferartikel für eine ophthalmische Vorrichtung nach Anspruch 1, wobei in polymerisierten Verbindungen der Formel (I) n 0 ist und m1 1 ist, wobei die Verbindungen die Formel (I) aufweisen 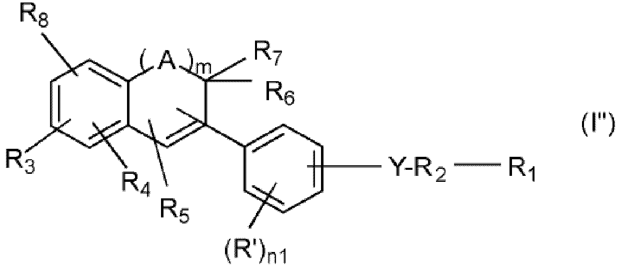 wobei R₁, -R₂-, Y, R₃, R₄, R₅, R₆, R₇, R₈, A, m, R' und n1 eine Bedeutung wie in Anspruch 1 angegeben haben.

4. Ophthalmische Vorrichtung oder Vorläuferartikel für eine ophthalmische Vorrichtung nach einem oder mehreren der Ansprüche 1 bis 3, umfassend ein Oligomer, Polymer oder Copolymer, das eine konstitutionelle Einheit M⁰ basierend auf den Formeln (I), (I') oder (I") umfasst, wobei R₁ bei jedem Auftreten polymerisiert ist, R₁ somit die regioreguläre, alternierte, regiozufällige, statistische, Block- oder zufällige Oligomer- oder Polymerhauptkette bildet oder Teil der Copolymerhauptkette ist.

5. Ophthalmische Vorrichtung oder Vorläuferartikel für eine ophthalmische Vorrichtung nach einem oder mehreren der Ansprüche 1 bis 4, wobei die polymerisierte Gruppe R₁ die Formeln (1-p), (2-p), (3-p) oder (4-p) aufweist, 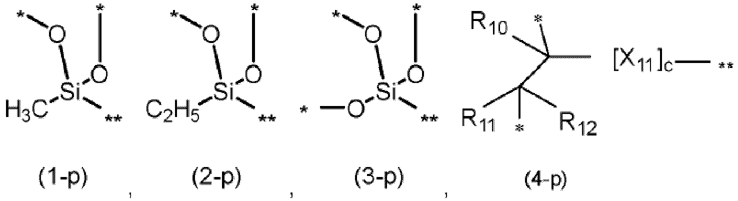 wobei der Stern "*" in den Formeln (1-p) bis (4-p) eine Verknüpfung mit der benachbarten Wiederholungseinheit in der Polymerkette oder Oligomerkette oder mit einer terminalen Endgruppe bezeichnet, der Stern "**" in den Formeln (1-p) bis (4-p) die Verknüpfung mit dem Rest der Formel (I), (I') oder (I") bezeichnet und R₁₀, R₁₁, R₁₂, X₁₁ und c eine Bedeutung gemäß Anspruch 1 haben.

6. Ophthalmische Vorrichtung oder Vorläuferartikel für eine ophthalmische Vorrichtung nach einem oder mehreren der Ansprüche 1 bis 5, wobei die konstitutionelle Einheit M⁰ die Formeln (M⁰-I'-a), (M⁰-I'-b), (M⁰-I'-c), (M⁰-I'-d) oder (M⁰-I") aufweist, 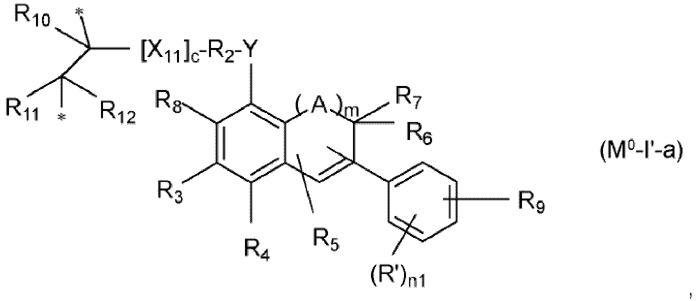 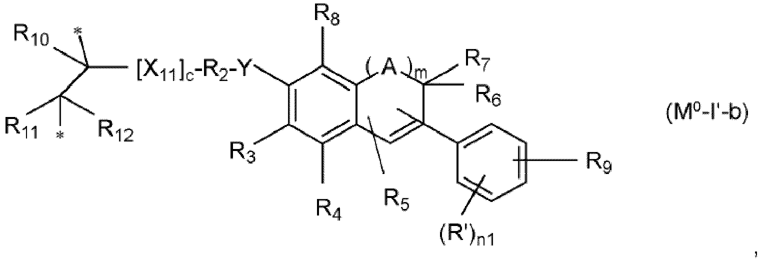 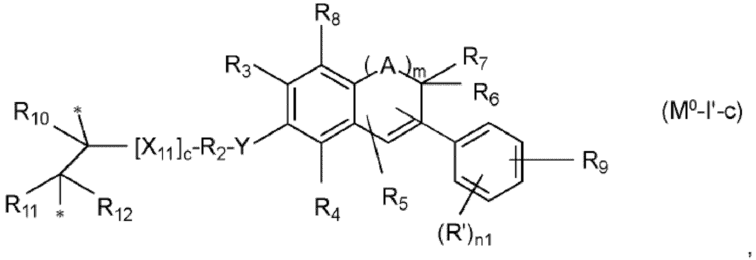 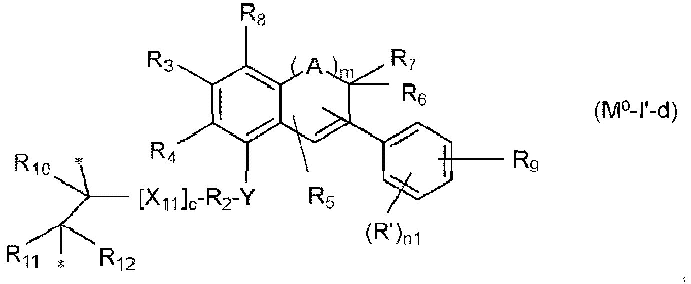 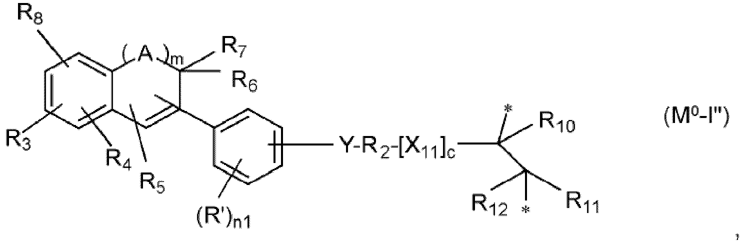 wobei -R₂-, Y, R₃, R₄, R₅, R₆, R₇, R₈, A, m, R', n1, X₁₁, R₁₀, R₁₁, R₁₂ und c eine Bedeutung gemäß Anspruch 1 haben und R⁹ H oder R' ist und wobei der Stern "*" bei jedem Auftreten eine Verknüpfung mit der benachbarten Wiederholungseinheit in der Polymerkette oder Oligomerkette oder mit einer terminalen Endgruppe bezeichnet.

7. Ophthalmische Vorrichtung oder Vorläuferartikel für eine ophthalmische Vorrichtung nach einem oder mehreren der Ansprüche 1 bis 6, umfassend neben der mindestens einen polymerisierten Verbindung der Formeln (I), (I') oder (I") oder der konstitutionellen Einheit M⁰ der Formeln (M⁰-I'-a), (M⁰-I'-b), (M⁰-I'-c), (M⁰-I'-d) oder (M⁰-I") mindestens ein weiteres polymerisiertes Monomer, ausgewählt aus der Gruppe bestehend aus Styrol, Ethoxyethylmethacrylat (EOEMA), Methylmethacrylat (MMA), Methylacrylat, n-Alkylacrylaten (wobei die n-Alkylgruppe 2-20 C-Atome umfasst), n-Alkylmethacrylaten (wobei die n-Alkylgruppe 2-20 C-Atome umfasst), Ethoxyethoxyethylacrylat (EEEA), 2-Hydroxyethylmethacrylat (HEMA), Tetrahydrofurylmethacrylat (THFMA), Glycidylmethacrylat (GMA), 16-Hydroxyhexadecylacrylat, 16-Hydroxyhexadecylmethacrylat, 18-Hydroxyoctadecylacrylat, 18-Hydroxyoctadecylmethacrylat, 2-Phenoxyethylacrylat (EGPEA), Bisphenol-A-diacrylat-1-EO/Phenol (BPADA), 2-[3'-2'H-Benzotriazol-2'-yl)-4'-hydroxyphenyl]ethylmethacrylat (BTPEM) oder Ethylenglycoldimethacrylat.

8. Ophthalmische Vorrichtung oder Vorläuferartikel für eine ophthalmische Vorrichtung nach Anspruch 7, wobei das mindestens eine weitere polymerisierte Monomer ausgewählt ist aus Methylmethacrylat, 2-Hydroxyethylmethacrylat, 2-Phenoxyethylacrylat, Ethoxyethoxyethylacrylat, 8-Methylnonylmethacrylat, n-Butylmethacrylat, 2-Ethylhexylmethacrylat oder einer Mischung davon.

9. Ophthalmische Vorrichtung oder Vorläuferartikel für eine ophthalmische Vorrichtung nach einem oder mehreren der Ansprüche 1 bis 8, wobei -R₂- bei jedem Auftreten unabhängig -(C(R)₂)_{O}- ist und R und o eine wie in Anspruch 1 angegebene Bedeutung haben.

10. Ophthalmische Vorrichtung oder Vorläuferartikel für eine ophthalmische Vorrichtung nach einem oder mehreren der Ansprüche 1 bis 9, wobei A unabhängig bei jedem Auftreten CH₂ ist.

11. Ophthalmische Vorrichtung oder Vorläuferartikel für eine ophthalmische Vorrichtung nach einem oder mehreren der Ansprüche 1 bis 10, wobei polymerisiertes R₁ bei jedem Auftreten unabhängig von einem Acryl- oder Methacrylsäureester-Radikal abgeleitet ist.

12. Vorläuferartikel für eine ophthalmische Vorrichtung nach einem oder mehreren der Ansprüche 1 bis 11, wobei der Vorläuferartikel ein Rohling ist, der in ein Augenimplantat, vorzugsweise eine Intraokularlinse, umgewandelt werden kann.

13. Verfahren zum Herstellen einer ophthalmischen Vorrichtung oder eines Vorläuferartikels für eine ophthalmische Vorrichtung gemäß einem oder mehreren der Ansprüche 1 bis 12, das Verfahren umfassend die Schritte
- Bereitstellen einer Zusammensetzung, umfassend mindestens eine Verbindung der Formeln (I), (I') oder (I") wie in einem oder mehreren der Ansprüche 1 bis 3, 9 und 10 beschrieben und/oder ein Oligomer oder Polymer wie in einem oder mehreren der Ansprüche 4 bis 8 und 11 beschrieben, jedoch mit mindestens einer für die Polymerisation verbleibenden reaktiven Gruppe, und optional weitere von den Verbindungen der Formeln (I), (I') oder (I") verschiedene Monomere und/oder Vernetzungsmittel und/oder UV-Absorber und/oder Radikalstarter;
- anschließendes Bilden der ophthalmischen Vorrichtung oder des Vorläuferartikels aus dieser Zusammensetzung.

14. Verfahren zum Verändern der optischen Eigenschaften einer ophthalmischen Vorrichtung oder eines Vorläuferprodukts für eine ophthalmische Vorrichtung nach einem oder mehreren der Ansprüche 1 bis 12, das Verfahren umfassend die Schritte
- Bereitstellen einer ophthalmischen Vorrichtung oder eines Vorläuferartikels nach Anspruch 13 und
- anschließendes Aussetzen der ophthalmologischen Vorrichtung oder des Vorläuferartikels einer Strahlung mit einer Wellenlänge von mindestens 200 nm und höchstens 1500 nm.

15. Oligomer, Polymer oder Copolymer, umfassend mindestens eine polymerisierte Verbindung der Formel (I), wie in Anspruch 1 beschrieben, wobei Oligomere, Polymere und Copolymere von 2-(4-Vinylphenyl)inden und 2-[4-(2-Propen-1-yl)phenyl-1H-inden] ausgeschlossen sind.

16. Polymer nach Anspruch 15, umfassend neben den polymerisierten Verbindungen der Formel (I) mindestens ein weiteres polymerisiertes Monomer ausgewählt aus der Gruppe bestehend aus Styrol, Ethoxyethylmethacrylat (EOEMA), Methylmethacrylat (MMA), Methylacrylat, n-Alkylacrylaten (wobei die n-Alkylgruppe 2-20 C-Atome umfasst), n-Alkylmethacrylaten (wobei die n-Alkylgruppe 2-20 C-Atome umfasst), Ethoxyethoxyethylacrylat (EEEA), 2-Hydroxyethylmethacrylat (HEMA), Tetrahydrofurylmethacrylat (THFMA), Glycidylmethacrylat (GMA), 16-Hydroxyhexadecylacrylat, 16-Hydroxyhexadecylmethacrylat, 18-Hydroxyoctadecylacrylat, 18-Hydroxyoctadecylmethacrylat, 2-Phenoxyethylacrylat (EGPEA), Bisphenol-A-diacrylat-1-EO/Phenol (BPADA), 2-[3'-2'H-Benzotriazol-2'-yl)-4'-hydroxyphenyl]ethylmethacrylat (BTPEM) oder Ethylenglycoldimethacrylat.

17. Zusammensetzung zur Polymerisation, umfassend mindestens eine Verbindung der Formeln (I), (I') oder (I") wie in einem oder mehreren der Ansprüche 1 bis 3, 9 und 10 beschrieben und/oder ein Oligomer oder Polymer wie in einem oder mehreren der Ansprüche 4 bis 8 und 11 beschrieben, jedoch mindestens aufweisend eine für die Polymerisation übrige reaktive Gruppe ausgenommen 2-(Vinylphenyl)inden, 2-[4-(2-Propen-1-yl)phenyl-1H-inden und jedes von 2-(Vinylphenyl)inden und 2-[4-(2-Propen-1-yl)phenyl-1H-inden abgeleitete Oligomer, Polymer oder Copolymer und/oder ein Vernetzungsmittel und/oder einen UV-Absorber und/oder einen Radikalstarter und optional weitere Monomere, die sich von den Verbindungen der Formeln (I), (I') oder (I") unterscheiden.

18. Verbindung der Formel (I) 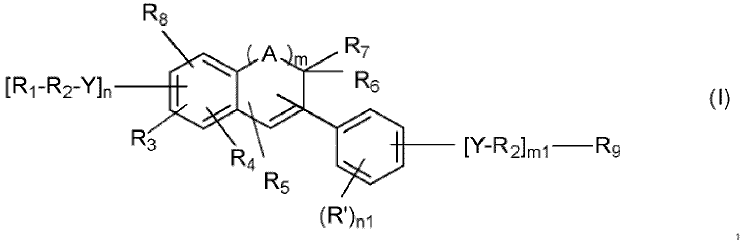 wobei
A bei jedem Auftreten gleich oder verschieden ist und CH₂, CHR₀ oder C(R₀)₂ ist;
m 0, 1, 2 oder 3 ist;
Y unabhängig voneinander O, S, SO₂ oder eine Bindung ist;
n 0 oder 1 ist;
m1 0 oder 1 ist;
n+m1 1 ist;
n1 0, 1, 2, 3 oder 4 ist;
R₀ bei jedem Auftreten unabhängig ausgewählt ist aus der Gruppe bestehend aus F, einer linearen oder verzweigten Alkylgruppe mit 1 bis 4 C-Atomen und einer linearen oder verzweigten teilweise oder vollständig fluorierten Alkylgruppe mit 1 bis 4 C-Atomen;
R' bei jedem Auftreten unabhängig ausgewählt ist aus der Gruppe bestehend aus F, einer linearen oder verzweigten, nichthalogenierten, teilweise oder vollständig halogenierten Alkylgruppe mit 1 bis 20 C-Atomen, einer nichthalogenierten, teilweise oder vollständig halogenierten Cycloalkylgruppe mit 3 bis 6 C-Atomen, einer linearen oder verzweigten, nichthalogenierten, teilweise oder vollständig halogenierten Alkoxygruppe mit 1 bis 20 C-Atomen und einer linearen oder verzweigten, nichthalogenierten, teilweise oder vollständig halogenierten Thioalkylgruppe mit 1 bis 20 C-Atomen;
R₁ eine Trialkoxysilylgruppe oder eine Dialkoxyalkylsilylgruppe, wobei die Alkyl- und/oder Alkoxygruppen jeweils unabhängig voneinander linear oder verzweigt sind und 1 bis 6 C-Atome aufweisen, oder eine Silylgruppe der Formel (1), (2) oder (3) oder eine polymerisierbare Gruppe der Formel (4) ist, 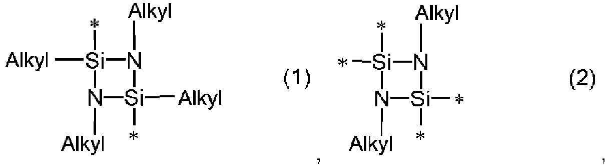 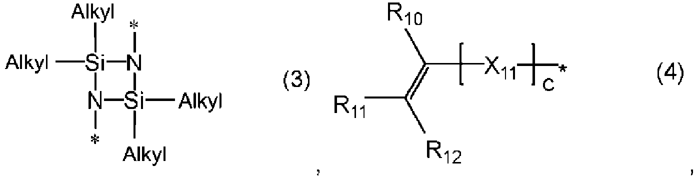
wobei Alkyl bei jedem Auftreten unabhängig voneinander eine lineare oder verzweigte Alkylgruppe mit 1 bis 6 C-Atomen bedeutet und der Stern "*" bei jedem Auftreten unabhängig voneinander eine Verknüpfung mit dem Linker [-R₂-Y]ₙ oder [Y-R₂-]ₘ₁ bezeichnet;
und wobei
X₁₁ ausgewählt ist aus der Gruppe bestehend aus O, S, O-SO₂, SO₂-O, C(=O), OC(=O), C(=O)O, S(C=O) und (C=O)S,
R₁₀, R₁₁, R₁₂ bei jedem Auftreten unabhängig voneinander ausgewählt sind aus der Gruppe bestehend aus H, F, einer linearen oder verzweigten, nichtfluorierten, teilweise oder vollständig fluorierten Alkylgruppe mit 1 bis 20 C-Atomen und Aryl mit 6 bis 14 C-Atomen
c 0 oder 1 ist;
-R₂- -(C(R)₂)ₒ- oder -(C(R)₂)ₚ-X₈-(C(R)₂)_{q}-(X₉)ₛ-(C(R)₂)ᵣ-(X₁₀)ₜ-(C(R)₂)ᵤ-ist;
R bei jedem Auftreten unabhängig ausgewählt ist aus der Gruppe bestehend aus H, F, einer linearen oder verzweigten Alkylgruppe mit 1 bis 4 C-Atomen oder einer linearen oder verzweigten teilweise oder vollständig fluorierten Alkylgruppe mit 1 bis 4 C-Atomen;
o ausgewählt ist aus der Gruppe bestehend aus 0 bis 20;
X₈, X₉, X₁₀ bei jedem Auftreten unabhängig voneinander O, S, SO₂ oder NR₀ sind;
s, t 0 oder 1 ist;
p, q bei jedem Auftreten unabhängig voneinander ausgewählt sind aus der Gruppe bestehend aus 1 bis 10;
r, u bei jedem Auftreten unabhängig voneinander ausgewählt sind aus der Gruppe bestehend aus 0 bis 10, wobei die Gesamtzahl der Atome für -(C(R)₂)ₚ-X₈-(C(R)₂)_{q}-(X₉)ₛ-(C(R)₂)ᵣ-(X₁₀)ₜ-(C(R)₂)ᵤ- bis zu 20 Atome beträgt,
R₃, R₄, R₅, R₆, R₇, R₈ bei jedem Auftreten unabhängig ausgewählt sind aus der Gruppe bestehend aus H, F, Cl, Br, CN, SO₂CF₃, einer linearen oder verzweigten, nichthalogenierten, teilweise oder vollständig halogenierten Alkylgruppe mit 1 bis 20 C-Atomen, einer nichthalogenierten, teilweise oder vollständig halogenierten Cycloalkylgruppe mit 3 bis 6 C-Atomen, einer linearen oder verzweigten, nichthalogenierten, teilweise oder vollständig halogenierten Alkoxygruppe mit 1 bis 20 C-Atomen und einer linearen oder verzweigten, nichthalogenierten und teilweise oder vollständig halogenierten Thioalkylgruppe mit 1 bis 20 C-Atomen;
R₉ H oder R' ist, wenn m1 0 ist, und
R₉ R₁ ist, wenn m1 1 ist,
vorausgesetzt, dass, wenn m 0 ist, m1 1 ist und Y eine Bindung ist, o aus der Gruppe ausgewählt ist bestehend aus 3 bis 20, und
vorausgesetzt, dass, wenn m 0 ist und m1 1 ist, zwei Substituenten aus der Gruppe von R₃, R₄ und R₈ nicht gleichzeitig F sind, und
vorausgesetzt, dass, wenn m 0 ist, m1 gleich 1 ist und Y eine Bindung oder O ist, R' bei jedem Auftreten nicht F ist; und
vorausgesetzt, dass, wenn m 0 ist, R₆ und R₇ bei jedem Auftreten keine nichthalogenierte, teilweise oder vollständig halogenierte Cycloalkylgruppe mit 3 bis 6 C-Atomen sind; und
vorausgesetzt, dass, wenn m 1 ist, n 1 ist und Y eine Bindung ist, o aus 1 bis 20 ausgewählt ist; und
vorausgesetzt, dass, wenn m 2 ist, m1 1 ist und Y eine Bindung ist, o aus der Gruppe ausgewählt ist bestehend aus 3 bis 20; und
vorausgesetzt, dass, wenn m 2 ist, m1 1 ist und Y O ist, o aus der Gruppe ausgewählt ist bestehend aus 1 bis 20; und
vorausgesetzt, dass, wenn m 2 ist, n 1 ist und Y eine Bindung ist, o aus der Gruppe ausgewählt ist bestehend aus 3 bis 20; und
vorausgesetzt, dass, wenn m 2 ist, n 1 ist und Y O ist, o aus der Gruppe ausgewählt ist bestehend aus 1 bis 20.

## Revendications

1. Dispositif ophtalmique ou article précurseur pour un dispositif ophtalmique comprenant au moins un composé polymérisé de formule (I) 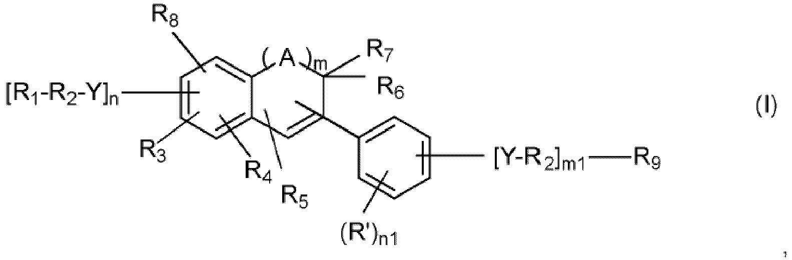 dans lequel
A est identique ou différent à chaque fois et représente CH₂, CHR₀ ou C(R₀)₂ ;
m vaut 0, 1, 2 ou 3 ;
Y représente à chaque fois indépendamment des autres O, S, SO₂, ou une liaison ;
n vaut 0 ou 1 ;
m1 vaut 0 ou 1 ;
n + m1 vaut 1 ;
n1 vaut 0, 1, 2, 3 ou 4 ;
R₀ est à chaque occurrence indépendamment choisi dans le groupe constitué de F, groupe alkyle linéaire ou ramifié ayant 1 à 4 atomes de C et groupe alkyle linéaire ou ramifié partiellement ou totalement fluoré ayant 1 à 4 atomes de C ;
R' est à chaque occurrence indépendamment choisi dans le groupe constitué de F, groupe alkyle linéaire ou ramifié, non halogéné, partiellement ou complètement halogéné ayant 1 à 20 atomes de C, groupe cycloalkyle non halogéné, partiellement ou complètement halogéné ayant 3 à 6 atomes de C, groupe alcoxy linéaire ou ramifié, non halogéné, partiellement ou complètement halogéné ayant 1 à 20 atomes de C et groupe thioalkyle linéaire ou ramifié, non halogéné, partiellement ou complètement halogéné ayant 1 à 20 atomes de C ;
R₁ représente un groupe trialcoxysilyle ou un groupe dialcoxyalkylsilyle où les groupes alkyle et/ou alcoxy sont chacun indépendamment linéaires ou ramifiés ayant 1 à 6 atomes de C, ou un groupe silyle de formule (1), (2) ou (3) ou un groupe polymérisable de formule (4), 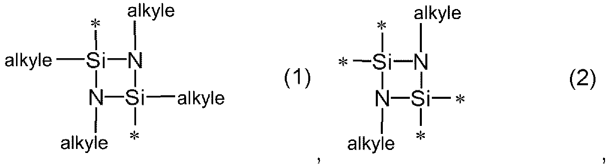 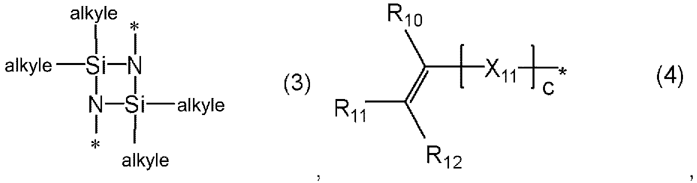
où alkyle signifie à chaque occurrence indépendamment des autres un groupe alkyle linéaire ou ramifié ayant 1 à 6 atomes de C et l'astérisque « * » désigne à chaque occurrence indépendamment des autres une liaison au lieur [-R₂-Y]ₙ ou [Y-R₂-]ₘ₁ ;
et dans lequel
X₁₁ est choisi dans le groupe constitué de O, S, O-SO₂, SO₂-O, C(=O), OC(=O), C(=O)O, S(C=O) et (C=O)S,
R₁₀, R₁₁, R₁₂ sont à chaque occurrence indépendamment les uns des autres choisis dans le groupe constitué de H, F, groupe alkyle linéaire ou ramifié, non fluoré, partiellement ou complètement fluoré ayant 1 à 20 atomes de C et aryle de 6 à 14 atomes de C
et
c vaut 0 ou 1 ;
-R₂- représente -(C(R)₂)ₒ-, ou -(C(R)₂)ₚ-X₈-(C(R)₂)_{q}-(X₉)ₛ-(C(R)₂)ᵣ-(X₁₀)ₜ-(C(R)₂)ᵤ- ;
R est à chaque occurrence indépendamment choisi dans le groupe constitué de H, F, groupe alkyle linéaire ou ramifié ayant 1 à 4 atomes de C ou groupe alkyle linéaire ou ramifié partiellement ou totalement fluoré ayant 1 à 4 atomes de C ;
o est choisi dans le groupe constitué de 0 à 20,
X₈, X₉, X₁₀ représentent à chaque occurrence indépendamment O, S, SO₂, ou NR₀,
s, t valent 0 ou 1,
p, q sont à chaque occurrence indépendamment choisis dans le groupe constitué de 1 à 10,
r, u sont à chaque occurrence indépendamment choisis dans le groupe constitué de 0 à 10, le nombre total d'atomes pour -(C(R)₂)ₚ-X₈-(C(R)₂)_{q}-(X₉)ₛ-(C(R)₂)ᵣ-(X₁₀)ₜ-(C(R)₂)ᵤ- allant jusqu'à 20 atomes,
R₃, R₄, R₅, R₆, R₇, R₈ sont à chaque occurrence indépendamment choisis dans le groupe constitué de H, F, Cl, Br, CN, SO₂CF₃, groupe alkyle linéaire ou ramifié, non halogéné, partiellement ou complètement halogéné ayant 1 à 20 atomes de C, groupe cycloalkyle non halogéné, partiellement ou complètement halogéné ayant 3 à 6 atomes de C, groupe alcoxy linéaire ou ramifié, non halogéné, partiellement ou complètement halogéné ayant 1 à 20 atomes de C et groupe thioalkyle linéaire ou ramifié, non halogéné et partiellement ou complètement halogéné ayant 1 à 20 atomes de C ;
R₉ représente H ou R' dans le cas où m1 vaut 0 et
R₉ représente R₁ dans le cas où m1 vaut 1.

2. Dispositif ophtalmique ou article précurseur pour un dispositif ophtalmique selon la revendication 1 dans lequel dans les composés polymérisés de formule (I) n vaut 1 et m1 vaut 0, lesdits composés étant de formule (I') 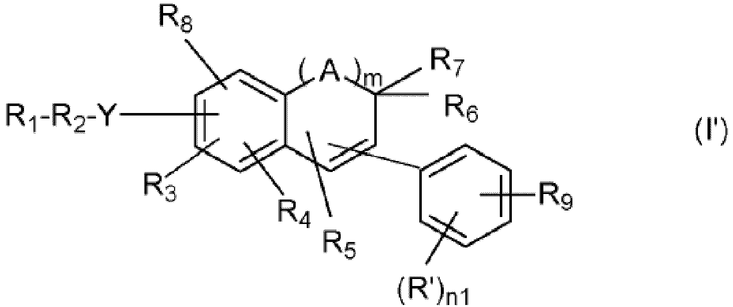 où R₁, -R₂-, Y, R₃, R₄, R₅, R₆, R₇, R₈, R₉, A, m, R', et n1 ont une signification telle qu'indiquée dans la revendication 1.

3. Dispositif ophtalmique ou article précurseur pour un dispositif ophtalmique selon la revendication 1 dans lequel dans les composés polymérisés de formule (I) n vaut 0 et m1 vaut 1 lesdits composés étant de formule (I") 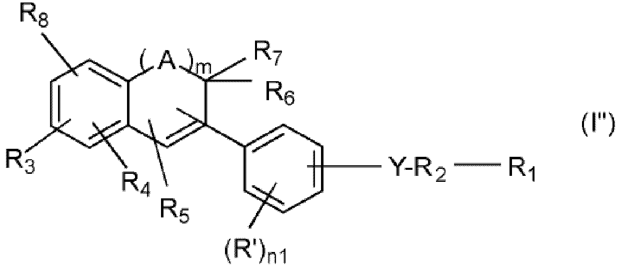 dans laquelle R₁, -R₂-, Y, R₃, R₄, R₅, R₆, R₇, R₈, A, m, R' et n1 ont une signification telle qu'indiquée dans la revendication 1.

4. Dispositif ophtalmique ou article précurseur pour un dispositif ophtalmique selon une ou plusieurs des revendications 1 à 3 comprenant un oligomère, polymère ou copolymère comprenant un motif constitutif M⁰ basé sur les formules (I), (I') ou (I") où R₁ à chaque occurrence est polymérisé, R₁ formant ainsi le squelette de l'oligomère ou du polymère régiorégulier, alterné, régioaléatoire, statistique, à blocs ou aléatoire ou faisant partie du squelette du copolymère.

5. Dispositif ophtalmique ou article précurseur pour un dispositif ophtalmique selon une ou plusieurs des revendications 1 à 4 où ledit groupe polymérisé R₁ est de formule (1-p), (2-p), (3-p) ou (4-p) 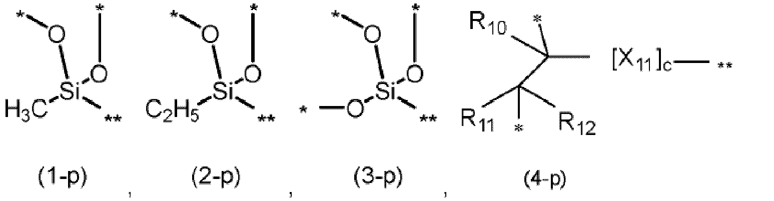 où l'astérisque « * » au sein des formules (1-p) à (4-p) désigne une liaison au motif répété adjacent dans la chaîne du polymère ou la chaîne de l'oligomère ou à un groupe terminal, l'astérisque « ** » au sein des formules (1-p) à (4-p) désigne la liaison au reste de la formule (I), (I') ou (I") et R₁₀, R₁₁, R₁₂, X₁₁ et c ont une signification selon la revendication 1.

6. Dispositif ophtalmique ou article précurseur pour un dispositif ophtalmique selon une ou plusieurs des revendications 1 à 5 dans lequel le motif constitutif M⁰ est de formule (M⁰-I'-a), (M⁰-I'-b), (M⁰-I'-c), (M⁰-I'-d) ou (M⁰-I"), 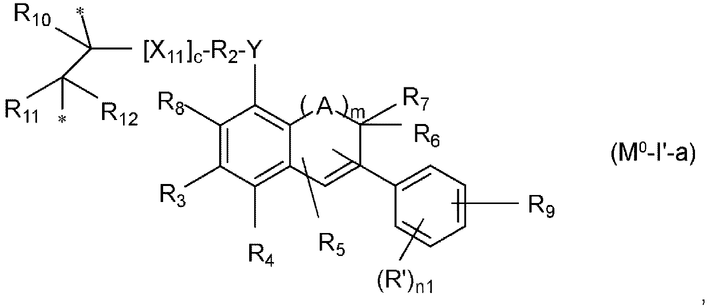 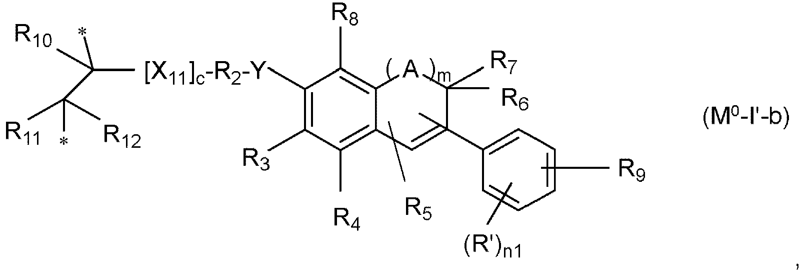 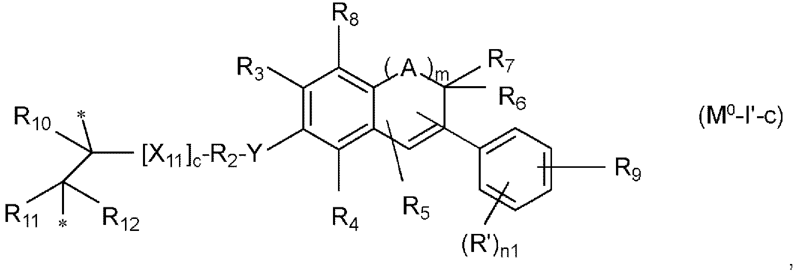 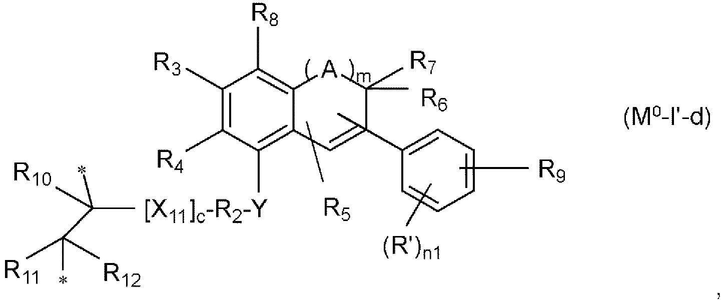 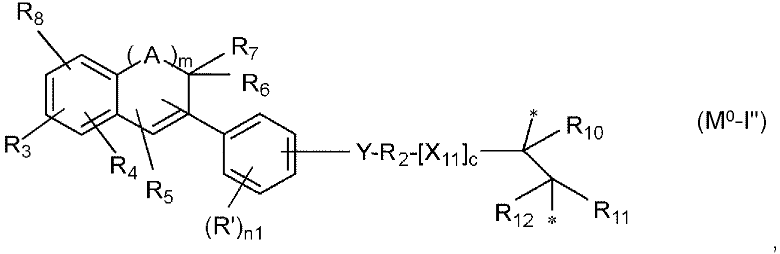 où -R₂-, Y, R₃, R₄, R₅, R₆, R₇, R₈, A, m, R', n1, X₁₁, R₁₀, R₁₁, R₁₂ et c ont une signification selon la revendication 1 et R⁹ représente H ou R' et où l'astérisque « * » désigne à chaque occurrence une liaison au motif répété adjacent dans la chaîne du polymère ou la chaîne de l'oligomère ou à un groupe terminal.

7. Dispositif ophtalmique ou article précurseur pour un dispositif ophtalmique selon une ou plusieurs des revendications 1 à 6 comprenant outre l'au moins un composé polymérisé de formule (I), (I') ou (I") ou le motif constitutif M⁰ de formule (M⁰-I'-a), (M⁰-I'-b), (M⁰-I'-c), (M⁰-I'-d) ou (M⁰-I") au moins un autre monomère polymérisé choisi dans le groupe constitué de styrène, méthacrylate d'éthoxyéthyle (EOEMA), méthacrylate de méthyle (MMA), acrylate de méthyle, acrylates de n-alkyle (le groupe n-alkyle comprenant 2 à 20 atomes de C), méthacrylates de n-alkyle (le groupe n-alkyle comprenant 2 à 20 atomes de C), acrylate d'éthoxyéthoxyéthyle (EEEA), méthacrylate de 2-hydroxyéthyle (HEMA), méthacrylate de tétrahydrofuryle (THFMA), méthacrylate de glycidyle (GMA), acrylate de 16-hydroxyhexadécyle, méthacrylate de 16-hydroxyhexadécyle, acrylate de 18-hydroxyoctadécyle, méthacrylate de 18-hydroxyoctadécyle, acrylate de 2-phénoxyéthyle (EGPEA), diacrylate de bisphénol A-1 OE/phénol (BPADA), méthacrylate de 2-[3'-2'H-benzotriazol-2'-yl)-4'-hydroxyphényl]éthyle (BTPEM) ou diméthacrylate d'éthylèneglycol.

8. Dispositif ophtalmique ou article précurseur pour un dispositif ophtalmique selon la revendication 7 dans lequel l'au moins un autre monomère polymérisé est choisi parmi méthacrylate de méthyle, méthacrylate de 2-hydroxyéthyle, acrylate de 2-phénoxyéthyle, acrylate d'éthoxyéthoxyéthyle, méthacrylate de 8-méthylnonyle, n-butyl-méthacrylate, méthacrylate de 2-éthylhexyle ou mélange de ceux-ci.

9. Dispositif ophtalmique ou article précurseur pour un dispositif ophtalmique selon une ou plusieurs des revendications 1 à 8 dans lequel -R₂- représente à chaque occurrence indépendamment -(C(R)₂)ₒ- et R et o ont une signification telle qu'indiquée dans la revendication 1.

10. Dispositif ophtalmique ou article précurseur pour un dispositif ophtalmique selon une ou plusieurs des revendications 1 à 9 dans lequel A représente indépendamment à chaque occurrence CH₂.

11. Dispositif ophtalmique ou article précurseur pour un dispositif ophtalmique selon une ou plusieurs des revendications 1 à 10 dans lequel R₁ polymérisé est à chaque occurrence indépendamment dérivé d'un radical acryle ou méthacryle .

12. Article précurseur pour un dispositif ophtalmique selon une ou plusieurs des revendications 1 à 11 dans lequel ledit article précurseur est une ébauche qui peut être transformée en un implant oculaire, de préférence une lentille intraoculaire.

13. Procédé de formation d'un dispositif ophtalmique ou d'un article précurseur pour un dispositif ophtalmique selon une ou plusieurs des revendications 1 à 12, ledit procédé comprenant les étapes consistant à
- fournir une composition comprenant au moins un composé de formule (I), (I') ou (I") tel que décrit dans une ou plusieurs des
revendications 1 à 3, 9 et 10 et/ou un oligomère ou polymère tel que décrit dans une ou plusieurs des revendications 4 à 8 et 11 mais ayant au moins un groupe réactif laissé pour la polymérisation et éventuellement d'autres monomères différents des composés de formule (I), (I') ou (I") et/ou des agents de réticulation et/ou des absorbeurs UV et/ou des inducteurs de radicaux ;
- former ultérieurement le dispositif ophtalmique ou l'article précurseur de ladite composition.

14. Procédé de modification des propriétés optiques d'un dispositif ophtalmique ou d'un article précurseur pour un dispositif ophtalmique selon une ou plusieurs des revendications 1 à 12, ledit procédé comprenant les étapes consistant à
- fournir un dispositif ophtalmique ou un article précurseur selon la revendication 13, et
- ultérieurement exposer ledit dispositif ophtalmique ou article précurseur à une irradiation ayant une longueur d'onde d'au moins 200 nm et d'au plus 1 500 nm.

15. Oligomère, polymère ou copolymère comprenant au moins un composé polymérisé de formule (I) tel que décrit dans la revendication 1, les oligomères, polymères et copolymères de 2-(4-vinylphényl)indène et de 2-[4-(2-propén-1-yl)phényl-1H-indène étant exclus.

16. Polymère selon la revendication 15 comprenant outre les composés polymérisés de formule (I) au moins un autre monomère polymérisé choisi dans le groupe constitué de styrène, méthacrylate d'éthoxyéthyle (EOEMA), méthacrylate de méthyle (MMA), acrylate de méthyle, acrylates de n-alkyle (le groupe n-alkyle comprenant 2 à 20 atomes de C), méthacrylates de n-alkyle (le groupe n-alkyle comprenant 2 à 20 atomes de C), acrylate d'éthoxyéthoxyéthyle (EEEA), méthacrylate de 2-hydroxyéthyle (HEMA), méthacrylate de tétrahydrofuryle (THFMA), méthacrylate de glycidyle (GMA), acrylate de 16-hydroxyhexadécyle, méthacrylate de 16-hydroxyhexadécyle, acrylate de 18-hydroxyoctadécyle, méthacrylate de 18-hydroxyoctadécyle, acrylate de 2-phénoxyéthyle (EGPEA), diacrylate de bisphénol A-1 OE/phénol (BPADA), méthacrylate de 2-[3'-2'H-benzotriazol-2'-yl)-4'-hydroxyphényl]éthyle (BTPEM) ou diméthacrylate d'éthylèneglycol.

17. Composition pour polymérisation comprenant au moins un composé de formule (I), (I') ou (I") tel que décrit dans une ou plusieurs des
revendications 1 à 3, 9 et 10 et/ou un oligomère ou polymère tel que décrit dans une ou plusieurs des revendications 4 à 8 et 11 mais ayant au moins un groupe réactif laissé pour la polymérisation à l'exclusion du 2-(vinylphényl)indène, du 2-[4-(2-propén-1-yl)phényl-1H-indène et d'un quelconque oligomère, polymère ou copolymère dérivé de 2-(vinylphényl)indène et de 2-[4-(2-propén-1-yl)phényl-1H-indène et/ou un agent de réticulation et/ou un absorbeur UV et/ou un inducteur de radicaux et éventuellement d'autres monomères différents des composés de formule (I), (I') ou (I").

18. Composé de formule (I) 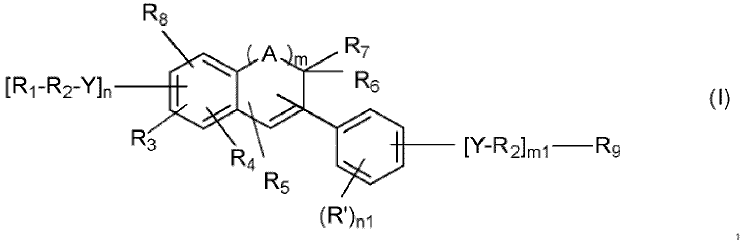 dans lequel
A est identique ou différent à chaque fois et représente CH₂, CHR₀ ou C(R₀)₂ ;
m vaut 0, 1, 2 ou 3 ;
Y représente à chaque fois indépendamment des autres O, S, SO₂, ou une liaison ;
n vaut 0 ou 1 ;
m1 vaut 0 ou 1 ;
n + m1 vaut 1 ;
n1 vaut 0, 1, 2, 3 ou 4 ;
R₀ est à chaque occurrence indépendamment choisi dans le groupe constitué de F, groupe alkyle linéaire ou ramifié ayant 1 à 4 atomes de C et groupe alkyle linéaire ou ramifié partiellement ou totalement fluoré ayant 1 à 4 atomes de C ;
R' est à chaque occurrence indépendamment choisi dans le groupe constitué de F, groupe alkyle linéaire ou ramifié, non halogéné, partiellement ou complètement halogéné ayant 1 à 20 atomes de C, groupe cycloalkyle non halogéné, partiellement ou complètement halogéné ayant 3 à 6 atomes de C, groupe alcoxy linéaire ou ramifié, non halogéné, partiellement ou complètement halogéné ayant 1 à 20 atomes de C et groupe thioalkyle linéaire ou ramifié, non halogéné, partiellement ou complètement halogéné ayant 1 à 20 atomes de C ;
R₁ représente un groupe trialcoxysilyle ou un groupe dialcoxyalkylsilyle où les groupes alkyle et/ou alcoxy sont chacun indépendamment linéaires ou ramifiés ayant 1 à 6 atomes de C, ou un groupe silyle de formule (1), (2) ou (3) ou un groupe polymérisable de formule (4), 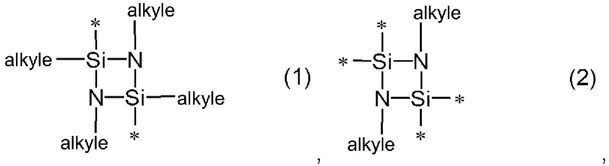 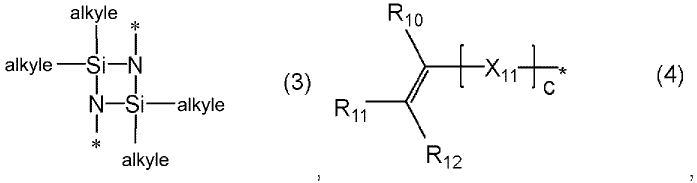
où alkyle signifie à chaque occurrence indépendamment des autres un groupe alkyle linéaire ou ramifié ayant 1 à 6 atomes de C et l'astérisque « * » désigne à chaque occurrence indépendamment des autres une liaison au lieur [-R₂-Y]ₙ ou [Y-R₂-]ₘ₁ ;
et dans lequel
X₁₁ est choisi dans le groupe constitué de O, S, O-SO₂, SO₂-O, C(=O), OC(=O), C(=O)O, S(C=O) et (C=O)S,
R₁₀, R₁₁, R₁₂ sont à chaque occurrence indépendamment les uns des autres choisis dans le groupe constitué de H, F, groupe alkyle linéaire ou ramifié, non fluoré, partiellement ou complètement fluoré ayant 1 à 20 atomes de C et aryle de 6 à 14 atomes de C et
c vaut 0 ou 1 ;
-R₂- représente -(C(R)₂)ₒ- ou -(C(R)₂)ₚ-X₈-(C(R)₂)_{q}-(X₉)ₛ-(C(R)₂)ᵣ(X₁₀)ₜ-(C(R)₂)ᵤ- ;
R est à chaque occurrence indépendamment choisi dans le groupe constitué de H, F, groupe alkyle linéaire ou ramifié ayant 1 à 4 atomes de C ou groupe alkyle linéaire ou ramifié partiellement ou totalement fluoré ayant 1 à 4 atomes de C ;
o est choisi dans le groupe constitué de 0 à 20 ;
X₈, X₉, X₁₀ représentent à chaque occurrence indépendamment 0, S, SO₂, ou NR₀ ;
s, t valent 0 ou 1 ;
p, q sont à chaque occurrence indépendamment choisis dans le groupe constitué de 1 à 10 ;
r, u sont à chaque occurrence indépendamment choisis dans le groupe constitué de 0 à 10, le nombre total d'atomes pour -(C(R)₂)ₚ-X₈-(C(R)₂)_{q}-(X₉)ₛ-(C(R)₂)ᵣ(X₁₀)ₜ-(C(R)₂)ᵤ- allant jusqu'à 20 atomes ;
R₃, R₄, R₅, R₆, R₇, R₈ sont à chaque occurrence indépendamment choisis dans le groupe constitué de H, F, Cl, Br, CN, SO₂CF₃, groupe alkyle linéaire ou ramifié, non halogéné, partiellement ou complètement halogéné ayant 1 à 20 atomes de C, groupe cycloalkyle non halogéné, partiellement ou complètement halogéné ayant 3 à 6 atomes de C, groupe alcoxy linéaire ou ramifié, non halogéné, partiellement ou complètement halogéné ayant 1 à 20 atomes de C et groupe thioalkyle linéaire ou ramifié, non halogéné et partiellement ou complètement halogéné ayant 1 à 20 atomes de C ;
R₉ représente H ou R' dans le cas où m1 vaut 0 et
R₉ représente R₁ dans le cas où m1 vaut 1,
à condition que dans le cas où m vaut 0, m1 vaut 1 et Y représente une liaison, o soit choisi dans le groupe constitué de 3 à 20, et
à condition que dans le cas où m vaut 0 et m1 vaut 1, deux substituants du groupe de R₃, R₄ et R₈ ne soient pas simultanément F, et
à condition que dans le cas où m vaut 0, m1 vaut 1 et Y représente une liaison ou O, R' à chaque occurrence ne soit pas F ; et
à condition que dans le cas où m vaut 0, R₆ et R₇ à chaque occurrence ne soient pas un groupe cycloalkyle non halogéné, partiellement ou complètement halogéné ayant 3 à 6 atomes de C ; et
à condition que dans le cas où m vaut 1, n vaut 1 et Y représente une liaison, o soit choisi parmi 1 à 20 ; et
à condition que dans le cas où m vaut 2, m1 vaut 1 et Y représente une liaison, o soit choisi dans le groupe constitué de 3 à 20 ; et
à condition que dans le cas où m vaut 2, m1 vaut 1 et Y représente O, o soit choisi dans le groupe constitué de 1 à 20 ; et
à condition que dans le cas où m vaut 2, n vaut 1 et Y représente une liaison, o soit choisi dans le groupe constitué de 3 à 20 ; et
à condition que dans le cas où m vaut 2, n vaut 1 et Y représente O, o soit choisi dans le groupe constitué de 1 à 20.
